# EUROPEAN PATENT APPLICATION

(11) **EP 2 420 493 A1**
(43) Date of publication of application: **22.02.2012**
(21) Application number: 10764490.8
(22) Date of filing: 14.04.2010
(51) Int. Cl.: C07D 265/08, A01N 47/04, A01N 47/24, A01P 13/02, C07D 265/10, C07D 265/32, C07D 267/04, C07D 267/10, C07D 273/01, C07D 273/04, C07D 279/06, C07D 279/12

(54) **HALOALKYLSULFONANILIDE DERIVATIVE**

(30) Priority: 14.04.2009 JP 2009098429; 03.03.2010 JP 2010046451; 02.04.2010 JP 2010086422
(71) Applicant: Nissan Chemical Industries, Ltd., Chiyoda-ku Tokyo 101-0054 (JP)
(72) Inventor: KAI, Masanori, Funabashi-shi Chiba 274-8507 (JP); FURUHASHI, Takamasa, Minamisaitama-gun Saitama 349-0294 (JP); MASUZAWA, Yoshihide, Funabashi-shi Chiba 274-8507 (JP); YANO, Tetsuhiko, Minamisaitama-gun Saitama 349-0294 (JP); SAITO, Fumiyo, Funabashi-shi Chiba 274-8507 (JP); NAKAYA, Yoshihiko, Funabashi-shi Chiba 274-8507 (JP)
(74) Representative: Blodig, Wolfgang
(86) International application number: PCT/JP2010/056711
(87) International publication number: WO 2010/119906

(57) **Abstract**

Novel herbicides are provided.

A haloalkylsulfonanilide derivative represented by the formula (1) or an agrochemically acceptable salt thereof:
the formula (1): 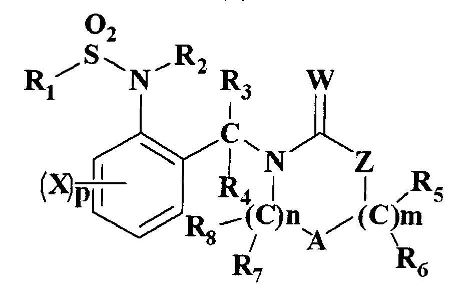 wherein Z is -C(R₉)(R₁₀)- or -N(R₁₁)-, A is an oxygen atom, a sulfur atom or -N(R₁₂)-, W is an oxygen atom or a sulfur atom, m is an integer of from 0 to 3, n is an integer of from 0 to 3, m+n is from 1 to 3, R₁ is halo C₁-C₆ alkyl, R₂ is a hydrogen atom, C₁-C₆ alkyl or the like, each of R₃ and R₄ is independently a hydrogen atom, C₁-C₆ alkyl or the like, each of R₅, R₆, R₇,R₈, R₉ and R₁₀ is independently a hydrogen atom, a halogen, C₁-C₆ alkyl or the like, each of R₁₁ and R₁₂ is a hydrogen atom, C₁-C₆ alkyl, halo C₁-C₆ alkyl or the like, and X is independently a hydrogen atom, a halogen, C₁-C₆ alkyl, or the like.

## Description

### TECHNICAL FIELD

The present invention relates to haloalkylsulfonanilide derivatives and agrochemicals, especially herbicides, containing them as active ingredients.

### BACKGROUND ART

Some haloalkylsulfonanilide derivatives are known to have herbicidal activities (Patent Documents 1 to 6), but nothing has been disclosed about the heterocyclic-N-alkyl substituted haloalkylsulfonanilide structure of the present invention.

### PRIOR ART DOCUMENTS

### PATENT DOCUMENTS

| | |
|---|---|
| Patent Document 1: | WO 04/011429 |
| Patent Document 2: | JP-A-2005-213168 |
| Patent Document 3: | WO 06/090792 |
| Patent Document 4: | JP-A-2008-074840 |
| Patent Document 5: | JP-A-2008-074841 |
| Patent Document 6: | WO 08/059948 |

### DISCLOSURE OF INVENTION

### TECHNICAL PROBLEM

The object of the present invention is to provide highly safe chemicals useful as active ingredients of herbicides, which have secure effects against various weeds at lower doses, and of which problems such as soil pollution and influences over aftercrop plants have been reduced.

### SOLUTION TO PROBLEM

The present inventors conducted extensive research to attain the above-mentioned object and, as a result, found novel haloalkylsulfonanilide derivatives and their high herbicidal activities and crop specificities. The present invention was accomplished on the basis of the discovery.

That is, the present invention provides the compound according to the following [1] to [7] (hereinafter referred to also as the compound of the present invention), the method for producing the compound as defined in [1] according to the following [8], the agrochemical according to the following [9], and the herbicide according to the following [10].

In the present invention, Cₛ-Cₜ (wherein s and t are integers) means the number of carbon atoms of s to t. For example, C₁-C₆ alkyl means an alkyl having 1 to 6 carbon atoms.
[1] A haloalkylsulfonanilide derivative represented by the formula (1) or an agrochemically acceptable salt thereof:
   the formula (1): 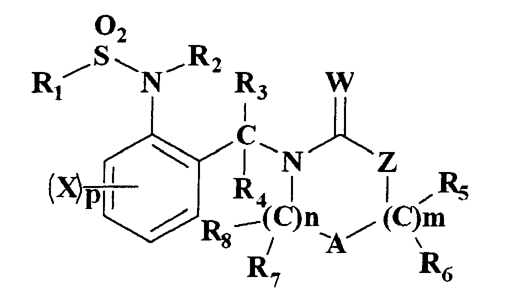
      wherein Z is an oxygen atom, a sulfur atom, -C(R₉)(R₁₀)- or -N(R₁₁)-,
      A is an oxygen atom, a sulfur atom or -N(R₁₂)-,
      W is an oxygen atom or a sulfur atom,
      m is an integer of from 0 to 3,
      n is an integer of from 0 to 3,
      m+n is from 1 to 3,
      p is an integer of from 0 to 4,
      R₁ is halo C₁-C₆ alkyl,
      R₂ is a hydrogen atom, C₁-C₆ alkyl, halo C₁-C₆ alkyl, C₂-C₆ alkenyl, halo C₂-C₆ alkenyl, C₂-C₆ alkynyl, halo C₂-C₆ alkynyl, C₁-C₆ alkoxy C₁-C₆ alkyl, halo C₁-C₆ alkoxy C₁-C₆ alkyl, C₁-C₆ alkoxy C₁-C₆ alkoxy C₁-C₆ alkyl, tri C₁-C₆ alkylsilyl C₁-C₆ alkoxy C₁-C₆ alkyl, phenyl C₁-C₆ alkyl, phenyl C₁-C₆ alkyl substituted with at least one Y, C₁-C₁₂ alkylcarbonyl, halo C₁-C₁₂ alkylcarbonyl, cyclo C₃-C₆ alkylcarbonyl, cyclo C₃-C₆ alkyl C₁-C₆ alkylcarbonyl, C₂-C₆ alkenylcarbonyl, phenylcarbonyl, phenylcarbonyl substituted with at least one Y, heterocyclic carbonyl, heterocyclic carbonyl substituted with at least one Y, C₁-C₁₂ alkoxycarbonyl, halo C₁-C₁₂ alkoxycarbonyl, C₁-C₆ alkoxy C₁-C₆ alkoxycarbonyl, C₁-C₆ alkylthio C₁-C₆ alkoxycarbonyl, C₁-C₆ alkylsulfinyl C₁-C₆ alkoxycarbonyl, C₁-C₆ alkylsulfonyl C₁-C₆ alkoxycarbonyl, C₂-C₆ alkenyloxycarbonyl, halo C₂-C₆ alkenyloxycarbonyl, C₂-C₆ alkynyloxycarbonyl, phenoxycarbonyl, phenoxycarbonyl substituted with at least one Y, phenyl C₁-C₆ alkoxycarbonyl, phenyl C₁-C₆ alkoxycarbonyl substituted with at least one Y, phenoxy C₁-C₆ alkylcarbonyl, phenoxy C₁-C₆ alkylcarbonyl substituted with at least one Y, mono(C₁-C₆ alkyl)aminocarbonyl, mono(halo C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, C₁-C₆ alkylthiocarbonyl, halo C₁-C₆ alkylthiocarbonyl, C₁-C₆ alkylsulfonyl, halo C₁-C₆ alkylsulfonyl, phenylsulfonyl, phenylsulfonyl substituted with at least one Y, C₁-C₆ alkylthio C₁-C₆ alkyl, halo C₁-C₆ alkylthio C₁-C₆ alkyl, phenyl C₁-C₆ alkoxy C₁-C₆ alkyl, phenyl C₁-C₆ alkoxy C₁-C₆ alkyl substituted with at least one Y, phenylthio C₁-C₆ alkyl, phenylthio C₁-C₆ alkyl substituted with at least one Y, phenyl C₁-C₆ alkylthio C₁-C₆ alkyl, phenyl C₁-C₆ alkylthio C₁-C₆ alkyl substituted with at least one Y, phenylsulfonyl C₁-C₆ alkyl, phenylsulfonyl C₁-C₆ alkyl substituted with at least one Y, phenyl C₁-C₆ alkylsulfonyl C₁-C₆ alkyl, phenyl C₁-C₆ alkylsulfonyl C₁-C₆ alkyl substituted with at least one Y, C₁-C₆ alkylcarbonyloxy C₁-C₆ alkyl, phenylcarbonyloxy C₁-C₆ alkyl, phenylcarbonyloxy C₁-C₆ alkyl substituted with at least one Y, phenylcarbonyl C₁-C₆ alkyl, phenylcarbonyl C₁-C₆ alkyl substituted with at least one Y, C₁-C₆ alkoxycarbonyloxy C₁-C₆ alkyl, phenylcarbonyloxy C₁-C₆ alkoxy C₁-C₆ alkyl, phenylcarbonyloxy C₁-C₆ alkoxy C₁-C₆ alkyl substituted with at least one Y, mono C₁-C₆ alkylaminocarbonyloxy C₁-C₆ alkyl, symmetric or asymmetric di(C₁-C₆ alkyl)aminocarbonyloxy C₁-C₆ alkyl, phenylaminocarbonyloxy C₁-C₆ alkyl, phenylaminocarbonyloxy C₁-C₆ alkyl substituted with at least one Y, C₁-C₆ alkyl(phenyl)aminocarbonyloxy C₁-C₆ alkyl, C₁-C₆ alkyl(phenyl)aminocarbonyloxy C₁-C₆ alkyl substituted with at least one Y, C₁-C₆ alkylcarbonyl C₁-C₆ alkyl, C₁-C₆ alkoxycarbonyl C₁-C₆ alkyl, halo C₁-C₆ alkylthio, symmetric or asymmetric di(C₁-C₆ alkyl)aminothio or cyano,
      each of R₃ and R₄ is independently a hydrogen atom, C₁-C₆ alkyl, halo C₁-C₆ alkyl, cyclo C₃-C₆ alkyl, halocyclo C₃-C₆ alkyl, C₁-C₆ alkoxy, halo C₁-C₆ alkoxy, halogen or cyano, or R₃ and R₄ may form a 3- to 7-membered ring together with each other,
      each of R₅, R₆, R₇, R₈, R₉ and R₁₀ is independently a hydrogen atom, a halogen, C₁-C₆ alkyl, halo C₁-C₆ alkyl, C₂-C₆ alkenyl, C₂-C₆ alkynyl, cyclo C₃-C₆ alkyl, halocyclo C₃-C₆ alkyl, C₁₋C₆ alkoxy C₁-C₆ alkyl, halo C₁-C₆ alkoxy C₁-C₆ alkyl, hydroxy C₁-C₆ alkyl, C₁-C₆ alkylthio C₁-C₆ alkyl, halo C₁-C₆ alkylthio C₁-C₆ alkyl, mono(C₁-C₆ alkyl)amino C₁-C₆ alkyl, symmetric or asymmetric di(C₁-C₆ alkyl)amino C₁-C₆ alkyl, phenyl, phenyl substituted with at least one Y, heterocyclyl, heterocyclyl substituted with at least one Y, phenyl C₁-C₆ alkyl, phenyl C₁-C₆ alkyl substituted with at least one Y, phenoxy C₁-C₆ alkyl, phenoxy C₁-C₆ alkyl substituted with at least one Y, C₁-C₆ alkylcarbonyl, halo C₁-C₆ alkylcarbonyl, phenylcarbonyl, phenylcarbonyl substituted with at least one Y, C₁-C₆ alkoxycarbonyl, halo C₁-C₆ alkoxycarbonyl, carboxyl, mono(C₁-C₆ alkyl)aminocarbonyl, symmetric or asymmetric di(C₁-C₆ alkyl)aminocarbonyl, phenylaminocarbonyl, phenylaminocarbonyl substituted with at least one Y, phenyl C₁-C₆ alkylaminocarbonyl, phenyl C₁-C₆ alkylaminocarbonyl substituted with at least one Y, C₁-C₆ alkoxy, halo C₁-C₆ alkoxy, phenoxy, phenoxy substituted with at least one Y, C₁-C₆ alkylthio, halo C₁-C₆ alkylthio, phenylthio, phenylthio substituted with at least one Y, C₁-C₆ alkylsulfonyl, halo C₁-C₆ alkylsulfonyl, mono(C₁-C₆ alkyl)amino, di(C₁-C₆ alkyl)amino, C₁-C₆ alkylcarbonyloxy, hydroxy, amino, cyano or nitro,
      each of R₁₁ and R₁₂ is independently a hydrogen atom, C₁-C₆ alkyl, halo C₁-C₆ alkyl, cyclo C₃-C₆ alkyl, halocyclo C₃-C₆ alkyl, C₂-C₆ alkenyl, halo C₂-C₆ alkenyl, C₂-C₆ alkynyl, C₁-C₆ alkoxy C₁-C₆ alkyl, halo C₁-C₆ alkoxy C₁-C₆ alkyl, hydroxy C₁-C₆ alkyl, cyclo C₃-C₆ alkyl C₁-C₆ alkyl, phenyl C₁-C₆ alkyl, phenyl C₁-C₆ alkyl substituted with at least one Y, phenyl, phenyl substituted with at least one Y, phenoxy C₁-C₆ alkyl, phenoxy C₁-C₆ alkyl substituted with at least one Y, C₁-C₆ alkylcarbonyl C₁-C₆ alkyl, halo C₁-C₆ alkylcarbonyl C₁-C₆ alkyl, C₁-C₆ alkoxycarbonyl C₁-C₆ alkyl, halo C₁-C₆ alkoxycarbonyl C₁-C₆ alkyl, C₁-C₆ alkylcarbonyl, halo C₁-C₆ alkylcarbonyl, cyclo C₃-C₆ alkylcarbonyl, cyclo C₃-C₆ alkyl C₁-C₆ alkylcarbonyl, C₂-C₆ alkenylcarbonyl, phenylcarbonyl, phenylcarbonyl substituted with at least one Y, heterocyclic carbonyl, heterocyclic carbonyl substituted with at least one Y, C₁-C₆ alkoxycarbonyl, halo C₁-C₆ alkoxycarbonyl, phenoxycarbonyl, phenoxycarbonyl substituted with at least one Y, aminocarbonyl, mono(C₁-C₆ alkyl)aminocarbonyl, monohalo(C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, C₁-C₆ alkylthiocarbonyl, halo C₁-C₆ alkylthiocarbonyl, C₁-C₆ alkylsulfonyl, halo C₁-C₆ alkylsulfonyl, phenylsulfonyl, phenylsulfonyl substituted with at least one Y, C₁-C₆ alkylthio C₁-C₆ alkyl, halo C₁-C₆ alkylthio C₁-C₆ alkyl, phenylthio C₁-C₆ alkyl, phenylthio C₁-C₆ alkyl substituted with at least one Y, C₁-C₆ alkylsulfinyl C₁-C₆ alkyl, halo C₁-C₆alkylsulfinyl C₁-C₆ alkyl, phenylsulfinyl C₁-C₆ alkyl, phenylsulfinyl C₁-C₆ alkyl substituted with at least one Y, C₁-C₆ alkylsulfonyl C₁-C₆ alkyl, halo C₁-C₆ alkylsulfonyl C₁-C₆ alkyl, phenylsulfonyl C₁-C₆ alkyl, phenylsulfonyl C₁-C₆ alkyl substituted with at least one Y, cyano, amino or hydroxy, or
      R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), an olefin or carbonyl, or
      R₅, R₆, R₇, R₈, R₉, R₁₀, R₁₁ or R₁₂ may form, together with R₅, R₆, R₇, R₈, R₉, R₁₀, R₁₁ or R₁₂ on an adjacent carbon or nitrogen, an optionally substituted 3- to 8-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), or
      R₅, R₆, R₇, or R₈ may form a bond together with R₅, R₆, R₇, or R₈ on an adjacent carbon,
      when m is 3 or 4, R₅ or R₆ may be the same as or different from adjacent R₅ or R₆, when n is 3 or 4, R₇ or R₈ may be the same as or different from adjacent R₇ or R₈,
      X is independently a halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl, phenyl substituted with at least one Y, heterocyclyl, heterocyclyl substituted with at least one Y, phenoxy, phenoxy substituted with at least one Y, phenylthio, phenylthio substituted with at least one Y, phenylsulfinyl, phenylsulfinyl substituted with at least one Y, phenylsulfonyl, phenylsulfonyl substituted with at least one Y, C₁-C₆ alkylcarbonyl, halo C₁-C₆ alkylcarbonyl, phenylcarbonyl, phenylcarbonyl substituted with at least one Y, C₁-C₆ alkoxycarbonyl, halo C₁-C₆ alkoxycarbonyl, carboxyl, mono(C₁-C₆ alkyl)aminocarbonyl, monohalo(C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, phenylaminocarbonyl, phenylaminocarbonyl substituted with at least one Y, phenyl C₁-C₆ alkylaminocarbonyl, phenyl C₁-C₆ alkylaminocarbonyl substituted with at least one Y, hydroxy, amino, cyano or nitro,
      p is an integer of from 0 to 4,
      Y is a halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl, phenyl having at least one substituent selected from the group consisting of halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, heterocyclyl, heterocyclyl having at least one substituent selected from the group consisting of halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, phenoxy, phenoxy having at least one substituent selected from the group consisting of halogen, C₁-C₆ alkyl, C₂-C₆ alkenyl, C₂-G₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, phenylthio, phenylthio having at least one substituent selected from the group consisting of halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, phenylsulfinyl, phenylsulfinyl having at least one substituent selected from the group consisting of halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, phenylsulfonyl, phenylsulfonyl having at least one substituent selected from the group consisting of halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, C₁-C₆ alkylcarbonyl, halo C₁-C₆ alkylcarbonyl, phenylcarbonyl, phenylcarbonyl having at least one substituent selected from the group consisting of halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, C₁-C₆ alkoxycarbonyl, carboxyl, mono(C₁-C₆ alkyl)aminocarbonyl, symmetric or asymmetric di(C₁-C₆ alkyl)aminocarbonyl, phenylaminocarbonyl, phenylaminocarbonyl having at least one substituent selected from the group consisting of halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, phenyl C₁-C₆ alkylaminocarbonyl, phenyl C₁-C₆ alkylaminocarbonyl having at least one substituent selected from the group consisting of halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, hydroxy, amino, cyano or nitro, or
      Y may be C₁-C₄ alkylene, halo C₁-C₄ alkylene, C₂-C₄ alkenylene or halo C₂-C₄ alkenylene which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl) and form a 5- or 6-membered ring together with an adjacent carbon or nitrogen atom on a benzene ring or a heterocyclyl, and
      the heterocyclyl is thienyl, furyl, pyrrolyl, oxazolyl, isoxazolyl, isoxazolinyl, thiazolyl, isothiazolyl, pyrazolyl, imidazolyl, 1,3,4-oxadiazolyl, 1,2,4-oxadiazolyl, 1,3,4-thiadiazolyl, 1,2,4-thiadiazolyl, 1,2,4-triazolyl, 1,2,3-thiadiazolyl, 1,2,3-triazolyl, 1,2,3,4-tetrazolyl, pyridyl, pyrimidinyl, pyrazinyl, pyridazinyl, 1,3,5-triazinyl, 1,2,4-triazinyl, benzothienyl, benzofuryl, indolyl, benzothiazolyl, benzoimidazolyl, benzoisoxazolyl, benzoisothiazolyl, indazolyl, benzoxazolyl, quinolyl, isoquinolyl, quinoxalinyl, phthalazinyl, cinnolinyl or quinazolinyl,
      provided that in a group having two or more substituents, the substituents may be the same or different.
[2] The haloalkylsulfonanilide derivative according to the above [1] or an agrochemically acceptable salt thereof, wherein Z is an oxygen atom or -C(R₉)(R₁₀)-,
   R₁ is halo C₁-C₂ alkyl,
   R₂ is a hydrogen atom, C₁-C₁₂ alkylcarbonyl, halo C₁-C₁₂ alkylcarbonyl, cyclo C₃-C₆ alkylcarbonyl, cyclo C₃-C₆ alkyl C₁-C₆ alkylcarbonyl, C₂-C₆ alkenylcarbonyl, phenylcarbonyl, phenylcarbonyl substituted with at least one Y, heterocyclic carbonyl, heterocyclic carbonyl substituted with at least one Y, C₁-C₁₂ alkoxycarbonyl, halo C₁-C₁₂ alkoxycarbonyl, C₁-C₆ alkoxy C₁-C₆ alkoxycarbonyl, C₂-C₆ alkenyloxycarbonyl, halo C₂-C₆ alkenyloxycarbonyl, C₂-C₆ alkynyloxycarbonyl, phenoxycarbonyl, phenoxycarbonyl substituted with at least one Y, phenyl C₁-C₆ alkoxycarbonyl, phenyl C₁-C₆ alkoxycarbonyl substituted with at least one Y, phenoxy C₁-C₆ alkylcarbonyl, phenoxy C₁-C₆ alkylcarbonyl substituted with at least one Y, mono(C₁-C₆ alkyl)aminocarbonyl, mono(halo C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, C₁-C₆ alkylsulfonyl or halo C₁-C₆ alkylsulfonyl,
   R₃ and R₄ are hydrogen atoms,
   each of R₅, R₆, R₇, R₈, R₉ and R₁₀ is independently a hydrogen atom, a halogen, C₁-C₆ alkyl, halo C₁-C₆ alkyl, cyclo C₃-C₆ alkyl, halocyclo C₃-C₆ alkyl, C₁-C₆ alkoxy C₁-C₆ alkyl, halo C₁-C₆ alkoxy C₁-C₆ alkyl, hydroxy C₁-C₆ alkyl, C₁-C₆ alkylthio C₁-C₆ alkyl, halo C₁-C₆ alkylthio C₁-C₆ alkyl, phenyl, phenyl substituted with at least one Y, heterocyclyl, heterocyclyl substituted with at least one Y, phenyl C₁-C₆ alkyl, phenyl C₁-C₆ alkyl substituted with at least one Y, C₁-C₆ alkylcarbonyl, halo C₁-C₆ alkylcabonyl, phenylcarbonyl, phenylcarbonyl substituted with at least one Y, C₁-C₆ alkoxycarbonyl, halo C₁-C₆ alkoxycarbonyl, carboxyl, mono(C₁-C₆ alkyl)aminocarbonyl, symmetric or asymmetric di(C₁-C₆ alkyl)aminocarbonyl, C₁-C₆ alkoxy, halo C₁-C₆ alkoxy, phenoxy, phenoxy substituted with at least one Y, C₁-C₆ alkylthio, halo C₁-C₆ alkylthio, phenylthio, phenylthio substituted with at least one Y, C₁-C₆ alkylsulfonyl, halo C₁-C₆ alkylsulfonyl or hydroxy, or
   R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), an olefin or carbonyl, or
   R₅, R₆, R₇, R₈, R₉, R₁₀, R₁₁ or R₁₂ may form, together with R₅, R₆, R₇, R₈, R₉, R₁₀, R₁₁ or R₁₂ on an adjacent carbon or nitrogen, an optionally substituted 3- to 8-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), or
   R₅, R₆, R₇ or R₈ may form a bond together with R₅, R₆, R₇ or R₈ on an adjacent carbon,
   X is a halogen, and
   pis 0 or 1.
[3] The haloalkylsulfonanilide derivative according to the above [1] or an agrochemically acceptable salt thereof, wherein Z is an oxygen atom or -C(R₉)(R₁₀)-,
   A is an oxygen atom or a sulfur atom,
   R₁ is trifluoromethyl,
   R₂ is a hydrogen atom, C₁-C₅ alkylcarbonyl, halo C₁-C₅ alkylcarbonyl, cyclo C₃-C₆ alkylcarbonyl, C₁-C₅ alkoxycarbonyl, halo C₁-C₅ alkoxycarbonyl or phenylcarbonyl,
   R₃ and R₄ are hydrogen atoms,
   each of R₅, R₆, R₇, R₈, R₉ and R₁₀ is independently a hydrogen atom, a halogen, C₁-C₃ alkyl, halo C₁-C₃ alkyl, C₁-C₃ alkoxy C₁-C₃ alkyl, phenyl, phenyl substituted with at least one Y, heterocyclyl, or heterocyclyl substituted with at least one Y, or
   R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), an olefin or carbonyl, or
   R₅, R₆, R₇, or R₈ may form a bond together with R₅, R₆, R₇, or R₈ on an adjacent carbon, and
   pis 0.
[4] The haloalkylsulfonanilide derivative according to the above [3] or an agrochemically acceptable salt thereof, wherein Z is -C(R₉)(R₁₀)-, and m and n are 1.
[5] The haloalkylsulfonanilide derivative according to the above [3] or an agrochemically acceptable salt thereof, wherein Z is -C(R₉)(R₁₀)-, m is 0, and n is 2.
[6] A compound represented by the formula (2):
   the formula (2) 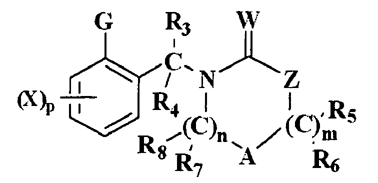
      wherein Z, W, R₃, R₄, R₅, R₆, R₇, R₈, m, n, p and X are the same as defined in the above [1],
      and G is amino or nitro.
[7] The compound according to the above [6], wherein Z is an oxygen atom or - C(R₉)(R₁₀)-,
   R₃ and R₄ are hydrogen atoms,
   each of R₅, R₆, R₇, R₈, R₉ and R₁₀ is independently a hydrogen atom, a halogen, C₁-C₃ alkyl, halo C₁-C₃ alkyl, C₁-C₃ alkoxy C₁-C₃ alkyl, phenyl, phenyl substituted with at least one Y, heterocyclyl or heterocyclyl substituted with at least one Y, or
   R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), an olefin or carbonyl, or
   R₅, R₆, R₇ or R₈ may form a bond together with R₅, R₆, R₇ or R₈ on an adjacent carbon, and
   pis 0.
[8] A method of use of the compound of the formula (2) as defined in the above [6], as an intermediated for production of the haloalkylsulfonanilide derivative as defined in the above [1].
[9] An agrochemical containing the haloalkylsulfonanilide derivative as defined in any one of the above [1] to [5] or an agrochemically acceptable salt thereof, as an active ingredient.
[10] A herbicide containing the haloalkylsulfonanilide derivative as defined in any one of the above [1] to [5] or an agrochemically acceptable salt thereof, as an active ingredient.
The compounds of the present invention and agrochemically acceptable salts thereof show synergic herbicidal effect when used in the form of a mixture with a certain kind of herbicide.

### ADVANTAGEOUS EFFECTS OF INVENTION

The compounds of the present invention have excellent herbicidal effects and crop specificities. Further, they have little adverse effects on the mammals and fishes, have low residual properties and present low environmental burden.

Accordingly, the present invention can provide herbicides which are agriculturally and horticulturally useful in paddy fields, crop plant fields and orchards.

### DESCRIPTION OF EMBODIMENTS

When the compounds of the present invention have at least one asymmetric carbon atom, the present invention covers all the optical isomers, racemates and diastereomers thereof.

Now, the options for Z, A, W, R₁, R₂, R₃, R₄, R₅, R₆, R₇, R₈, R₉, R₁₀, R₁₁, R₁₂, X, m and n will be illustrated.

As options for Z, the following are, for example, mentioned:
ZI: -C(R₉)(R₁₀)-,
ZII: -N(R₁₁)-, and
ZIII: an oxygen atom or a sulfur atom.

As options for A, the following are, for example, mentioned:
AI: an oxygen atom,
AII: a sulfur atom, and
AIII: -N(R₁₂)-.

As options for W, the following are, for example, mentioned:
WI: an oxygen atom, and
WII: a sulfur atom.

As options for R₁, the following are, for example, mentioned:
R₁I: trifluoromethyl, and
R₁II: halo C₁-C₆ alkyl.

As options for R₂, the following are, for example, mentioned:
R₂I: a hydrogen atom, C₁-C₁₂ alkylcarbonyl, halo C₁-C₁₂ alkylcarbonyl, C₂-C₆ alkenylcarbonyl, phenylcarbonyl, phenylcarbonyl substituted with at least one Y, C₁-C₁₂ alkoxycarbonyl, halo C₁-C₁₂ alkoxycarbonyl, C₂-C₆ alkenyloxycarbonyl, phenoxycarbonyl, C₁-C₆ alkylthiocarbonyl or halo C₁-C₆ alkylthiocarbonyl,
R₂II: C₁-C₆ alkyl, C₁-C₆ alkoxy C₁-C₆ alkyl, phenyl C₁-C₆ alkyl, phenyl C₁-C₆ alkyl substituted with at least one Y, C₁-C₆ alkylthio C₁-C₆ alkyl, halo C₁-C₆ alkylthio C₁-C₆ alkyl, phenoxy C₁-C₆ alkyl or C₁-C₆ alkylcarbonyloxy C₁-C₆ alkyl, and R₂III: C₁-C₆ alkylsulfonyl, halo C₁-C₆ alkylsulfonyl, phenylsulfonyl or phenylsulfonyl substituted with at least one Y.

As options for R₃ and R₄, the following are, for example, mentioned:
R₃I or R₄I: a hydrogen atom, and
R₃II or R₄II: C₁-C₆ alkyl, halo C₁-C₆ alkyl, C₃-C₆ cycloalkyl, halo C₃-C₆ cycloalkyl, C₁-C₆ alkoxy, halo C₁-C₆ alkoxy, halogen or cyano.

As options for R₅, R₆, R₇, R₈, R₉ and R₁₀, the following are, for example, mentioned:
R₅I, R₆I, R₇I R₈I, R₉I or R₁₀I: a hydrogen atom, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy C₁-C₆ alkyl, halo C₁-C₆ alkoxy C₁-C₆ alkyl, C₁-C₆ alkylthio C₁-C₆ alkyl or halo C₁-C₆ alkylthio C₁-C₆ alkyl,
R₅II, R₆II, R₇II R₈II, R₉II or R₁₀II: phenyl or phenyl substituted with at least one Y,
R₅III, R₆III, R₇III R₈III, R₉III or R₁₀III: halogen, C₁-C₆ alkoxy, C₁-C₆ alkylthio, hydroxy, amino, cyano or nitro, and
R₅IV, R₆IV, R₇IV R₈IV, R₉IV or R₁₀IV: C₁-C₆ alkylcarbonyl, halo C₁-C₆ alkylcarbonyl, phenylcarbonyl, phenylcarbonyl substituted with at least one Y or C₁-C₆ alkoxycarbonyl.

As options for R₁₁ and R₁₂, the following are, for example, mentioned:
R₁₁I or R₁₂I: a hydrogen atom, C₁-C₆ alkyl, halo C₁-C₆ alkyl, cyclo C₃-C₆ alkyl, halocyclo C₃-C₆ alkyl, C₁-C₆ alkoxy C₁-C₆ alkyl, halo C₁-C₆ alkoxy C₁-C₆ alkyl, phenyl C₁-C₆ alkyl, phenyl C₁-C₆ alkyl substituted with at least one Y, phenyl or phenyl substituted with at least one Y, and
R₁₁II or R₁₂II: C₁-C₆ alkylcarbonyl, halo C₁-C₆ alkylcarbonyl, cyclo C₃-C₆ alkylcarbonyl, cyclo C₃-C₆ alkyl C₁-C₆ alkylcarbonyl, phenylcarbonyl, phenylcarbonyl substituted with at least one Y, C₁-C₆ alkoxycarbonyl, halo C₁-C₆ alkoxycarbonyl, phenoxycarbonyl or phenoxycarbonyl substituted with at least one Y, and
R₁₁III or R₁₂III: C₁-C₆ alkylsulfonyl, halo C₁-C₆ alkylsulfonyl, phenylsulfonyl or phenylsulfonyl substituted with at least one Y.

As options for X, the following are, for example, mentioned:
XI: a halogen, C₁-C₆ alkoxy, halo C₁-C₆ alkoxy, C₁-C₆ alkylthio, halo C₁-C₆ alkylthio, phenoxy, phenoxy substituted with at least one identical or different Y, phenylthio, phenylthio substituted with at least one identical or different Y, hydroxy, amino, cyano or nitro,
XII: C₁-C₆ alkyl, C₂-C₆ alkenyl, C₂-C₆ alkynyl, C₃-C₆ cycloalkyl, halo C₁-C₆ alkyl, halo C₃-C₆ cycloalkyl, C₁-C₆ alkoxy C₁-C₆ alkyl or halo C₁-C₆ alkoxy C₁-C₆ alkyl,
XIII: C₁-C₆ alkylcarbonyl, halo C₁-C₆ alkylcarbonyl, phenylcarbonyl, phenylcarbonyl substituted with at least one identical or different Y or C₁-C₆ alkoxycarbonyl, and
XIV: phenyl, phenyl substituted with at least one identical or different Y, heterocyclyl or heterocyclyl substituted with at least one identical or different Y.

As options for m, the following are, for example, mentioned:
mI: m=0,
mII: m=1 or 2, and
mIII: m=3.

As options for n, the following are, for example, mentioned:
nI: n=0,
nII: n=1 or 2, and
nlll: n=3.

As options for p, the following are, for example, mentioned:
pI: n=0,
pII: n=1 or 2, and
pIII: n=3 or 4.

These options for the respective groups may be combined arbitrarily to define the scope of the compounds of the present invention. Examples of combinations of these options for the respective groups are shown in Table 1. However, the combinations shown in Table 1 are mere examples, and the present invention is by no means restricted thereto.

**TABLE 1**

| R₃=R₃I, R₄=R₄I, R₆=R₆I, R₈=R₈I, R₁₀=R₁₀I, p=pI | | | | | | | | | | | | |
|---|---|---|---|---|---|---|---|---|---|---|---|---|
| W | Z | A | R₁ | R₂ | R₅ | R₇ | R₉ | R₁₁ | R₁₂ | X | m | n |
| WI | ZI | AI | R₁I | R₂I | R₅I | R₇I | R₉I | - | - | XI | mI | nII |
| WI | ZI | AI | R₁I | R₂I | - | R₇I | R₉I | - | - | XI | mI | nIII |
| WI | ZI | AI | R₁I | R₂I | R₅I | - | R₉I | - | - | XI | mII | nI |
| WI | ZI | AI | R₁I | R₂I | R₅I | R₇I | R₉I | - | - | XI | mII | nII |
| WI | ZI | AI | R₁I | R₂I | R₅I | - | R₉I | - | - | XI | mIII | nI |
| WI | ZI | AI | R₁I | R₂I | - | R₇I | R₉I | - | - | XII | mI | nII |
| WI | ZI | AI | R₁I | R₂I | R₅I | - | R₉I | - | - | XII | mII | nI |
| WI | ZI | AI | R₁I | R₂I | R₅I | R₇I | R₉I | - | - | XII | mII | nII |
| WI | ZI | AI | R₁I | R₂I | - | R₇I | R₉I | - | - | XIII | mI | nII |
| WI | ZI | AI | R₁I | R₂I | R₅I | - | R₉I | - | - | XIII | mII | nI |
| WI | ZI | AI | R₁I | R₂I | R₅I | R₇I | R₉I | - | - | XIII | mII | nII |
| WI | ZI | AI | R₁I | R₂I | - | R₇I | R₉I | - | - | XIV | mI | nII |
| WI | ZI | AI | R₁I | R₂I | R₅I | - | R₉I | - | - | XIV | mII | nI |
| WI | ZI | AI | R₁I | R₂I | R₅I | R₇I | R₉I | - | - | XIV | mII | nII |
| WI | ZI | AI | R₁I | R₂I | - | R₇I | R₉II | - | - | XI | mI | nII |
| WI | ZI | AI | R₁I | R₂I | R₅I | - | R₉II | - | - | XI | mII | nI |
| WI | ZI | AI | R₁I | R₂I | R₅I | R₇I | R₉II | - | - | XI | mII | nII |
| WI | ZI | AI | R₁I | R₂I | - | R₇I | R₉III | - | - | XI | mI | nII |
| WI | ZI | AI | R₁I | R₂I | R₅I | - | R₉III | - | - | XI | mII | nI |
| WI | ZI | AI | R₁I | R₂I | R₅I | R₇I | R₉III | - | - | XI | mII | nII |
| WI | ZI | AI | R₁I | R₂I | - | R₇I | R₉IV | - | - | XI | mI | nII |
| WI | ZI | AI | R₃I | R₂I | R₅I | - | R₉IV | - | - | XI | mII | nI |
| WI | ZI | AI | R₁I | R₂I | R₅I | R₇I | R₉IV | - | - | XI | mII | nII |
| WI | ZI | AI | R₁I | R₂I | - | R₇II | R₉I | - | - | XI | mI | nII |
| WI | ZI | AI | R₁I | R₂I | R₅I | - | R₉I | - | - | XI | mII | nI |
| WI | ZI | AI | R₁I | R₂I | R₅I | R₇II | R₉I | - | - | XI | mII | nII |
| WI | ZI | AI | R₁I | R₂I | - | R₇III | R₉I | - | - | XI | mI | nII |
| WI | ZI | AI | R₁I | R₂I | R₅I | - | R₉I | - | - | XI | mII | nI |
| WI | ZI | AI | R₁I | R₂I | R₅I | R₇III | R₉I | - | - | XI | mII | nII |
| WI | ZI | AI | R₁I | R₂I | - | R₇IV | R₉I | - | - | XI | mI | nII |
| WI | ZI | AI | R₁I | R₂I | R₅I | - | R₉I | - | - | XI | mII | nI |
| WI | ZI | AI | R₁I | R₂I | R₅I | R₇IV | R₉I | - | - | XI | mII | nII |
| WI | ZI | AI | R₁I | R₂I | - | R₇I | R₉I | - | - | XI | mI | nII |
| WI | ZI | AI | R₁I | R₂I | R₅II | - | R₉I | - | - | X I | mII | nI |
| WI | ZI | AI | R₁I | R₂I | R₅II | R₇I | R₉I | - | - | XI | mI | nII |
| WI | ZI | AI | R₁I | R₂I | - | R₇I | R₉I | - | - | XI | mI | nII |
| WI | ZI | AI | R₁I | R₂I | R₅III | - | R₉I | - | - | XI | mII | nI |
| WI | ZI | AI | R₁I | R₂I | R₅III | R₇I | R₉I | - | - | XI | mII | nII |
| WI | ZI | AI | R₁I | R₂I | - | R₇I | R₉I | - | - | XI | mI | nII |
| WI | ZI | AI | R₁I | R₂I | R₅IV | - | R₉I | - | - | XI | mII | nI |
| WI | ZI | AI | R₁I | R₂I | R₅IV | R₇I | R₉I | - | - | XI | mII | nII |
| WI | ZI | AI | R₁I | R₂I I | - | R₇I | R₉I | - | - | XI | mII | nII |
| WI | ZI | AI | R₁I | R₂II | R₅I | - | R₉I | - | - | XI | mII | nI |
| WI | ZI | AI | R₃I | R₂II | R₅I | R₇I | R₉I | - | - | XI | mII | nII |
| WI | ZI | AI | R₁I | R₂III | - | RI | R₉I | - | - | XI | mI | nII |
| WI | ZI | AI | R₁I | R₂III | R₅I | - | R₉I | - | - | XI | mII | nII |
| WI | ZI | AI | R₁I | R₂III | R₅I | R₇I | R₉I | - | - | XI | mII | nII |
| WI | ZI | AI | R₁II | R₂I | - | R₇I | R₉I | - | - | XI | mI | nII |
| WI | ZI | AI | R₁II | R₂I | R₅I | - | R₉I | - | - | XI | mII | nI |
| WI | ZI | AI | R₁II | R₂I | R₅I | R₇I | R₉I | - | - | XI | mII | nII |
| WI | ZI | AII | R₁I | R₂I | - | R₇I | - | R₁₁I | - | XI | mI | nII |
| WI | ZI | AII | R₁I | R₂I | R₅I | - | - | R₁₁I | - | XI | mII | nI |
| WI | ZI | AII | R₁I | R₂I | - | R₇I | - R₁₁I | I | - | XI | mII | nII |
| WI | ZI | AII | R₁I | R₂I | R₅I | - | - R₁₁I | I | - | XI | mII | nI |
| WI | ZI | AII | R₁I | R₂I | R₅I | R₇I | - R₁₁I | I | - | XI | mII | nII |
| WI | ZI | AII | R₁I | R₂I | - | R₇I | - | R₁₁III | - | XI | mII | nII |
| WI | ZI | AII | R₁I | R₂I | R₅I | - | - | R₁₁IIII | - | XI | mII | nI |
| WI | ZI | AII | R₁I | R₂I | R₅I | R₇I | - | R₁₁III | - | XI | mII | nII |
| WI | ZII | AI | R₁I | R₂I | - | R₇I | R₉I | - | - | XI | mI | nII |
| WI | ZII | AI | R₁I | R₂I | R₅I | - | R₉I | - | - | XI | mII | nI |
| WI | ZII | AI | R₁I | R₂I | R₅I | R₇I | Rel | - | - | XI | mII | nII |
| WI | ZIII | AI | R₁I | R₂I | - | R₇I | R₉I | - | R₁₂I | XI | mI | nII |
| WI | ZIII | AI | R₁I | R₂I | R₅I | - | R₉I | - | R₁₂I | XI | mII | nII |
| WI | ZIII | AI | RI | R₂I | R₅I | R₇I | R₉I | - | R₁₂I | XI | mII | nII |
| WI | ZIII | AI | R₁I | R₂I | - | R₇I | R₉I | - | R₁₂II | XI | mII | nII |
| WI | ZIII | AI | R₁I | R₂I | R₅I | - | R₉I | - | R₁₂II | XI | mII | nI |
| WI | ZIII | AI | R₁I | R₂I | R₅I | R₇I | R₉I | - | R₂II | XI | mII | nII |
| WI | ZIII | AI | R₁I | R₂I | - | R₇I | R₉I | - | R₁₂III | XI | mII | nII |
| WI | ZIII | AI | R₁I | R₂I | R₅I | - | R₉I | - | R₁₂III | XI | mII | nI |
| WI | ZIII | AI | R₁I | R₂I | R₅I | R₇I | R₉I | - | R₁₂III | XI | mII | nII |
| WII | ZI | AI | R₁I | R₂I | - | R₇I | R₉I | - | - | XI | mI | nII |
| WII | ZI | AI | R₁I | R₂I | R₆I | - | R₉I | - | - | XI | mII | nI |
| WII | ZI | AI | R₁I | R₂I | R₅I | R₇I | R₈I | - | - | XI | mII | nII |

Now, examples of each atom or group in the definitions of R₁, R₂, R₃, R₄, R₅, R₆, R₇, R₈, R₉, R₁₀, R₁₁, R₁₂, X and Y will be given.

The following symbols herein have the following meanings.
i. iso, s: secondary, t: tertiary, c: cyclo, p: para.

Heterocyclyl means the following: namely, thienyl such as thiophen-2-yl or thiophen-3-yl, furyl such as furan-2-yl or furan-3-yl, pyrrolyl such as pyrrol-1-yl, pyrrol-2-yl or pyrrol-3-yl, oxazolyl such as oxazol-2-yl, oxazol-4-yl or oxazol-5-yl, isoxazolyl such as isoxazol-3-yl, isoxazol-4-yl or isoxazol-5-yl, isoxazolinyl such as isoxazolin-3-yl, isoxazolin-4-yl or isoxazolin-5-yl, thiazolyl such as thiazol-2-yl, thiazol-4-yl or thiazol-5-yl, isothiazolyl such as isothiazol-3-yl, isothiazol-4-yl or isothiazol-5-yl, pyrazolyl such as pyrazol-1-yl, pyrazol-3-yl, pyrazol-4-yl or pyrazol-5-yl, imidazolyl such as imidazol-1-yl, imidazol-2-yl or imidazol-4-yl, 1,3,4-oxadiazolyl such as 1,3,4-oxadiazol-2-yl, 1,2,4-oxadiazolyl such as 1,2,4-oxadiazol-3-yl or 1,2,4-oxadiazol-5-yl, 1,3,4-thiadiazolyl such as 1,3,4-thiadiazol-2-yl, 1,2,4-thiadiazolyl such as 1,2,4-thiadiazol-3-yl or 1,2,4-thiadiazol-5-yl, 1,2,4-triazolyl such as1,2,4-triazol-1-yl, 1,2,4-triazol-3-yl or 1,2,4-triazol-5-yl, 1,2,3-thiadiazolyl such as 1,2,3-thiadiazol-4-yl or 1,2,3-thiadiazol-5-yl, 1,2,3-triazolyl such as 1,2,3-triazol-1-yl, 1,2,3-triazol-2-yl or 1,2,3-triazol-4-yl, 1,2,3,4-tetrazolyl such as 1,2,3,4-tetrazol-1-yl, 1,2,3,4-tetrazol-2-yl or 1,2,3,4-tetrazol-5-yl, pyridyl such as pyridin-2-yl, pyridin-3-yl or pyridin-4-yl, pyrimidinyl such as pyrimidin-2-yl, pyrimidin-4-yl or pyrimidin-5-yl, pyrazinyl such as pyrazin-2-yl, pyridazinyl such as pyridazin-3-yl or pyridazin-4-yl, 1,3,5-triazinyl such as1,3,5-triazin-2-yl, 1,2,4-triazinyl such as 1,2,4-triazin-3-yl, 1,2,4-triazin-5-yl or 1,2,4-triazin-6-yl, benzothienyl such as benzothiophen-2-yl, benzothiophen-3-yl, benzothiophen-4-yl, benzothiophen-5-yl, benzothiophen-6-yl or benzothiophen-7-yl, benzofuryl such as benzofuran-2-yl, benzofuran-3-yl, benzofuran-4-yl, benzofuran-5-yl, benzofuran-6-yl or benzofuran-7-yl, indolyl such as indol-1-yl, indol-2-yl, indol-3-yl, indol-4-yl, indol-5-yl, indol-6-yl or indol-7-yl, benzothiazolyl such as benzothiazol-2-yl, benzothiazol-4-yl, benzothiazol-5-yl, benzothiazol-6-yl or benzothiazol-7-yl, benzimidazolyl such as benzimidazol-1-yl, benzimidazol-2-yl, benzimidazol-4-yl, benzimidazol-5-yl, benzimidazol-6-yl or benzimidazol-7-yl, benzoisoxazolyl such as benzisoxazol-3-yl, benzisoxazol-4-yl, benzisoxazol-5-yl, benzisoxazol-6-yl or benzisoxazol-7-yl, benzisothiazolyl such as benzisothiazol-3-yl, benzisothiazol-4-yl, benzisothiazol-5-yl, benzisothiazol-6-yl or benzisothiazol-7-yl, indazolyl such as indazol-1-yl, indazol-3-yl, indazol-4-yl, indazol-5-yl, indazol-6-yl or indazol-7-yl, benzoxazolyl such as benzoxazol-2-yl, benzoxazol-4-yl, benzoxazol-5-yl, benzoxazol-6-yl or benzoxazol-7-yl, quinolyl such as quinolin-2-yl, quinolin-3-yl, quinolin-4-yl, quinolin-5-yl, quinolin-6-yl, quinolin-7-yl or quinolin-8-yl, isoquinolyl such as isoquinolin-1-yl, isoquinolin-3-yl, isoquinolin-4-yl, isoquinolin-5-yl, isoquinolin-6-yl, isoquinolin-7-yl or isoquinolin-8-yl, quinoxalinyl such as quinoxalin-2-yl, quinoxalin-3-yl, quinoxalin-5-yl, quinoxalin-6-yl, quinoxalin-7-yl or quinoxalin-8-yl, phthalazinyl such as phthalazin-1-yl, phthalazin-4-yl, phthalazin-5-yl, phthalazin-6-yl, phthalazin-7-yl or phthalazin-8-yl, cinnolinyl such as cinnolin-3-yl, cinnolin-4-yl, cinnolin-5-yl, cinnolin-6-yl, cinnolin-7-yl or cinnolin-8-yl, and quinazolinyl such as quinazolin-2-yl, quinazolin-4-yl, quinazolin-5-yl, quinazolin-6-yl, quinazolin-7-yl or quinazolin-8-yl. In this specification, "heterocyclylcarbonyl" means the above-mentioned groups attached to carbonyl.

As a halogen, a fluorine atom, a chlorine atom, a bromine atom and an iodine atom may be mentioned. In this specification, "halo" means such halogen.

Alkyl may be methyl, ethyl, propyl, i-propyl, butyl, i-butyl, t-butyl, s-butyl, pentyl, 1-methylbutyl, 2-methylbutyl, 3-methylbutyl, 1-ethylpropyl, 1,1-dimethylpropyl, 1,2-dimethylpropyl, neo-pentyl, hexyl, 1-methylpentyl, 2-methylpentyl, 3-methylpentyl, 4-methylpentyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1,1,2-trimethylpropyl, 1-ethyl-1-methylpropyl, 1-ethyl-2-methylpropyl or the like and is selected within a given range of carbon atoms.

Haloalkyl may be fluoromethyl, chloromethyl, bromomethyl, 2-fluoroethyl, 2-chloroethyl, 2-bromoethyl, 3-fluoropropyl, 3-chloropropyl, difluoromethyl, chlorodifluoromethyl, trifluoromethyl, dichloromethyl, trichloromethyl, 2,2-difluoroethyl, 2,2,2-trifluoroethyl, 2,2,2-trichloroethyl, chlorodifluoromethyl, bromodifluoromethyl, pentafluoroethyl, heptafluoropropyl, heptafluoroisopropyl, 4-chlorobutyl, 4-fluorobutyl or the like and is selected within a given range of carbon atoms.

Cycloalkyl may be cyclopropyl, cyclobutyl, cyclopentyl, cyclohexyl or the like and is selected within a given range of carbon atoms.

Halocycloalkyl may be 2-fluorocyclopropyl, 1-chlorocyclopropyl, 2-bromo-1-methylcyclopropyl, 2,2-difluorocyclopropyl, 1,2-dichlorocyclopropyl or the like and is selected within a given range of carbon atoms.

Cycloalkylalkyl may be cyclopropylmethyl, 2-cyclopropylethyl, cyclopentylmethyl, cyclohexylmethyl or the like and is selected within a given range of carbon atoms.

Alkenyl may be ethenyl, 1-propenyl, 2-propenyl, 1-butenyl, 2-butenyl, 3-butenyl, 1-methyl-2-propenyl, 2-methyl-2-propenyl, 1-pentenyl, 2-pentenyl, 3-pentenyl, 4-pentenyl, 1,1-dimethyl-2-propenyl, 1,2-dimethyl-2-propenyl, 1-hexenyl, 1,1-dimethyl-2-butenyl, 1,2-dimethyl-2-butenyl, 1,3-dimethyl-2-butenyl, 2,3-dimethyl-2-butenyl, 1-ethyl-2-butenyl, 2-ethyl-2-butenyl, 1,1,2-trimethyl-2-propenyl, 1-ethyl-1-methyl-2-propenyl or the like and is selected within a given range of carbon atoms.

Haloalkenyl may be 1-chloroethenyl, 2-chloroethenyl, 2-fluoroethenyl, 2,2-dichloroethenyl, 3-chloro-2-propenyl, 3-fluoro-2-propenyl, 2-chloro-2-propenyl, 4-chloro-3-butenyl or the like and is selected within a given range of carbon atoms.

Alkynyl may be ethynyl, 1-propynyl, 2-propynyl, 1-butynyl, 2-butynyl, 3-butynyl, 1-methyl-2-propynyl, 1-pentynyl, 1-methyl-2-butynyl, 2-methyl-3-butynyl, 1-hexynyl, 1-methyl-3-pentynyl, 2-methyl-3-pentynyl, 3-methyl-4-pentynyl, 4-methyl-2-pentynyl, 1,1-dimethyl-2-butynyl, 1,2-dimethyl-3-butynyl, 2,2-dimethyl-3-butynyl, 1-ethyl-2-butynyl, 2-ethyl-3-butynyl or the like and is selected within a given range of carbon atoms.

Haloalkynyl may be chloroethynyl, fluoroethynyl, bromoethynyl, 3-chloro-2-propynyl, 4-chloro-2-butynyl or the like and is selected within a given range of carbon atoms.

Phenyl substituted with at least one Y may be 2-fluorophenyl, 3-fluorophenyl, 4-fluorophenyl, 2-chlorophenyl, 3-bromophenyl, 4-iodophenyl, 2,3-difluorophenyl, 2,4-difluorophenyl, 2,6-difluorophenyl, 3,4-difluorophenyl, 3,5-difluorophenyl, 2,4-dichlorophenyl, 2-fluoro-4-chlorophenyl, 2,3,4,5,6-pentafluorophenyl, 2-methylphenyl, 2,5-dimethylphenyl, 4-ethenylphenyl, 2-ethynylphenyl, 3-cyclopropylphenyl, 2-cyclopentylphenyl, 4-cyclohexylphenyl, 2-methoxyphenyl, 3,4-dimethoxyphenyl, 3,4,5-trimethoxyphenyl, 4-methoxymethylphenyl, 2-aminophenyl, 4-dimethylaminophenyl, 2-aminocarbonylphenyl, 2-dimethylaminocarbonylphenyl, 4-trifluoromethoxyphenyl, 2-difluoromethoxyphenyl, 2-ethenyloxyphenyl, 3-cyclopropyloxyphenyl, 2-biphenyl, 4-acetylphenyl, 3-methoxycarbonylphenyl, 2-methylthiophenyl, 3-trifluoromethylthiophenyl, 4-difluoromethylthiophenyl, 2-methylsulfinylphenyl, 3-trifluoromethylsulfinylphenyl, 4-difluoromethylsulfinylphenyl, 2-methylsulfonylphenyl, 3-trifluoromethylsulfonylphenyl, 4-difluoromethylsulfonylphenyl, 2-cyanophenyl, 3-nitrophenyl, 4-trifluoromethylphenyl, 2-difluoromethylphenyl, 2,3-methylenedioxyphenyl, 3,4-methylenedioxyphenyl or the like. In a case where the phenyl group has two or more substituents Y's, the respective Y's may be the same or different from one another.

Alkoxy may be methoxy, ethoxy, propyloxy, i-propyloxy, butyloxy, i-butyloxy, s-butyloxy, t-butyloxy, pentyloxy, 1-methylbutyloxy, 2-methylbutyloxy, 3-methylbutyloxy, 1,1-dimethylpropyloxy, 1,2-dimethylpropyloxy, 2,2-dimethylpropyloxy, 1-ethylpropyloxy, hexyloxy, 1-methylpentyloxy, 2-methylpentyloxy, 3-methylpentyloxy, 4-methylpentyloxy, 1,1-dimethylbutyloxy, 1,2-dimethylbutyloxy, 1,3-dimethylbutyloxy, 2,2-dimethylbutyloxy, 2,3-dimethylbutyloxy, 3,3-dimethylbutyloxy, 1-ethylbutyloxy, 2-ethylbutyloxy, 1,1,2-trimethylpropyloxy, 1,2,2-trimethylpropyloxy, 1-ethyl-1-methylpropyloxy, 1-ethyl-2-methylpropyloxy or the like and is selected within a given range of carbon atoms.

Haloalkoxy may be fluoromethoxy, difluoromethoxy, trifluoromethoxy, chlorodifluoromethoxy, bromodifluoromethoxy, dichlorofluoromethoxy, chloromethoxy, dichloromethoxy, trichloromethoxy, bromomethoxy, 1-fluoroethoxy, 2-fluoroethoxy, 2-chloroethoxy, 2-bromoethoxy, 2,2-difluoroethoxy, 2,2,2-trifluoroethoxy, 1,1,2,2-tetrafluoroethoxy, pentafluoroethoxy, 2,2,2-trichloroethoxy, 2,2,2-trifluoroethoxy, 1-fluoropropyloxy, 2-fluoropropyloxy, 3-fluoropropyloxy, 3-chloropropyloxy, 3-bromopropyloxy, 1-fluorobutyloxy, 2-fluorobutyloxy, 3-fluorobutyloxy, 4-fluorobutyloxy, 4-chlorobutyloxy or the like and is selected within a given range of carbon atoms.

Phenylalkyl and phenylalkyl substituted with at least one Y may be benzyl, 2-fluorobenzyl, 3-fluorobenzyl, 4-fluorobenzyl, 2-chlorobenzyl, 3-bromobenzyl, 4-iodobenzyl, 2,4-difluorobenzyl, 2-methylbenzyl, 3-methylbenzyl, 4-methylbenzyl, 2-methoxybenzyl, 4-aminobenzyl, 4-trifluoromethoxybenzyl, 2-methylthiobenzyl, 3-trifluoromethylthiobenzyl, 2-cyanobenzyl, 4-nitrobenzyl, 2-trifluoromethylbenzyl, 2-phenethyl or the like and is selected within a given range of carbon atoms. In a case where the phenyl group has two or more substituents Y's, the respective Y's may be, the same or different from one another.

Phenylcarbonylalkyl and phenylcarbonylalkyl substituted with at least one Y may be phenylcarbonylmethyl, 2-fluorophenylcarbonylmethyl, 2-chlorophenylcarbonylmethyl, 3-bromophenylcarbonylmethyl, 4-iodophenylcarbonylmethyl, 4-methylphenylcarbonylmethyl, 2-methoxyphenylcarbonylmethyl, 4-aminophenylcarbonylmethyl, 4-trifluoromethoxyphenylcarbonylmethyl, 2-methylthiophenylcarbonylmethyl, 3-trifluoromethylthiophenylcarbonylmethyl, 2-cyanophenylcarbonylmethyl, 4-nitrophenylcarbonylmethyl, 2-trifluoromethylphenylcarbonylmethyl, 2-phenylcarbonylethyl or the like and is selected within a given range of carbon atoms. In a case where the phenyl group has two or more substituents Y's, the respective Y's may be the same or different from one another.

Phenoxy substituted with at least one Y may be 2-fluorophenoxy, 2-chlorophenoxy, 4-bromophenoxy, 4-methylphenoxy, 2-methoxyphenoxy, 3-aminophenoxy, 4-trifluoromethoxyphenoxy, 2-methylthiophenoxy, 3-trifluoromethylthiophenoxy, 4-cyanophenoxy, 2-nitrophenoxy, 4-trifluoromethylphenoxy or the like. In a case where the phenyl group has two or more substituents Y's, the respective Y's may be the same or different from one another.

Alkylthio may be methylthio, ethylthio, propylthio, i-propylthio, butylthio, i-butylthio, s-butylthio, t-butylthio or the like and is selected within a given range of carbon atoms.

Haloalkylthio may be fluoromethylthio, chloromethylthio, chlorodifluoromethylthio, bromodifluoromethylthio, trifluoromethylthio, trichloromethylthio, 2,2,2-trifluoroethylthio, 1,1,2,2-tetrafluoroethylthio, 2-fluoroethylthio, pentafluoroethylthio or the like and is selected within a given range of carbon atoms.

Phenylthio substituted with at least one Y may be 2-fluorophenylthio, 2-chlorophenylthio, 4-bromophenylthio, 4-methylphenylthio, 2-methoxyphenylthio, 3-aminophenylthio, 4-trifluoromethoxyphenylthio, 2-methylthiophenylthio, 3-trifluoromethylthiophenylthio, 4-cyanophenylthio, 2-nitrophenylthio, 4-trifluoromethylphenylthio or the like. In a case where the phenyl group has two or more substituents Y's, the respective Y's may be the same or different from one another.

Alkylsulfinyl may be methylsulfinyl, ethylsulfinyl, propylsulfinyl, i-propylsulfinyl, butylsulfinyl, i-butylsulfinyl, s-butylsulfinyl, t-butylsulfinyl or the like and is selected within a given range of carbon atoms.

Haloalkylsulfinyl may be fluoromethylsulfinyl, chloromethylsulfinyl, chlorodifluoromethylsulfinyl, bromodifluoromethylsulfinyl, trifluoromethylsulfinyl, trichloromethylsulfinyl, 2,2,2-trifluoroethylsulfinyl, 1,1,2,2-tetrafluoroethylsulfinyl, 2-fluoroethylsulfinyl, pentafluoroethylsulfinyl or the like and is selected within a given range of carbon atoms.

Phenylsulfinyl substituted with at least one Y may be 2-fluorophenylsulfinyl, 2-chlorophenylsulfinyl, 4-bromophenylsulfinyl, 4-methylphenylsulfinyl, 2-methoxyphenylsulfinyl, 3-aminophenylsulfinyl, 4-trifluoromethoxyphenylsulfinyl, 2-methylthiophenylsulfinyl, 3-trifluoromethylthiophenylsulfinyl, 4-cyanophenylsulfinyl, 2-nitrophenylsulfinyl, 4-trifluoromethylphenylsulfinyl or the like. In a case where the phenyl group has two or more substituents Y's, the respective Y's may be the same or different from one another.

Alkylsulfonyl may be methylsulfonyl, ethylsulfonyl, propylsulfonyl, i-propylsulfonyl, butylsulfonyl, i-butylsulfonyl, s-butylsulfonyl, t-butylsulfonyl or the like and is selected within a given range of carbon atoms.

Haloalkylsulfonyl may be fluoromethylsulfonyl, chloromethylsulfonyl, chlorodifluoromethylsulfonyl, bromodifluoromethylsulfonyl, trifluoromethylsulfonyl, trichloromethylsulfonyl, 2,2,2-trifluoroethylsulfonyl, 1,1,2,2-tetrafluoroethylsulfonyl, 2-fluoroethylsulfonyl, pentafluoroethylsulfonyl or the like and is selected within a given range of carbon atoms.

Phenylsulfonyl substituted with at least Y may be 2-fluorophenylsulfonyl, 2-chlorophenylsulfonyl, 4-bromophenylsulfonyl, 4-methylphenylsulfonyl, 2-methoxyphenylsulfonyl, 3-aminophenylsulfonyl, 4-trifluoromethoxyphenylsulfonyl, 2-methylthiophenylsulfonyl, 3-trifluoromethylthiophenylsulfonyl, 4-cyanophenylsulfonyl, 2-nitrophenylsulfonyl, 4-trifluoromethylphenylsulfonyl or the like. In a case where the phenyl group has two or more substituents Y's, the respective Y's may be the same or different from one another.

Alkylcarbonyl may be acetyl, propionyl, butyryl, i-butyryl, valeroyl, i-valeroyl, 2-methylbutyryl, pivaloyl, hexanoyl, 2-ethylbutyryl, 2-methylvaleroyl, 4-methylvaleroyl, heptanoyl, 2,2-dimethylvaleroyl, 2-ethylhexanoyl, 2-propylvaleroyl, octanoyl, nonanoyl, 3,5,5-trimethylhexanoyl, decanoyl or the like and is selected within a given range of carbon atoms.

Haloalkylcarbonyl may be fluoroacetyl, chloroacetyl, difluoroacetyl, dichloroacetyl, trifluoroacetyl, chlorodifluoroacetyl, bromodifluoroacetyl, trichloroacetyl, pentafluoropropionyl, 4-chlorobutyryl, heptafluorobutyryl, 3-chloro-2,2-dimethylpropionyl or the like and is selected within a given range of carbon atoms.

Cycloalkylcarbony may be cyclopropanecarbonyl, cyclobutanecarbonyl, cyclopentanecarbonyl, cyclohexanecarbonyl or the like and is selected within a given range of carbon atoms.

Cycloalkylalkylcarbonyl may be cyclopropylmethylcarbonyl, 2-cyclopropylethylcarbonyl, cyclopentylmethylcarbonyl, cyclohexylmethylcarbonyl or the like and is selected within a given range of carbon atoms.

Alkenylcarbonyl may be ethenylcarbonyl, 1-propenylcarbonyl, 2-propenylcarbonyl, 1-butenylcarbonyl, 1-methyl-2-propenylcarbonyl, 2-methyl-2-propenylcarbonyl, 1-hexenylcarbonyl or the like and is selected within a given range of carbon atoms.

Phenylcarbonyl substituted with at least one Y may be 2-fluorophenylcarbonyl, 2-chlorophenylcarbonyl, 4-bromophenylcarbonyl, 4-methylphenylcarbonyl, 2-methoxyphenylcarbonyl, 3-aminophenylcarbonyl, 4-trifluoromethoxyphenylcarbonyl, 2-methylthiophenylcarbonyl, 3-trifluoromethylthiophenylcarbonyl, 4-cyanophenylcarbonyl, 2-nitrophenylcarbonyl, 4-trifluoromethylphenylcarbonyl. In a case where the phenyl group has two or more substituents Y's, the respective Y's may be the same or different from one another.

Alkoxycarbonyl may be methoxycarbonyl, ethoxycarbonyl, propyloxycarbonyl, i-propyloxycarbonyl, butyloxycarbonyl, s-butyloxycarbonyl, i-butyloxycarbonyl, t-butyloxycarbonyl, pentyloxycarbonyl, hexyloxycarbonyl or the like and is selected within a given range of carbon atoms.

Haloalkoxycarbonyl may be fluoromethoxycarbonyl, difluoromethoxycarbonyl, trifluoromethoxycarbonyl, 1-fluoroethoxycarbonyl, 2-fluoroethoxycarbonyl, 2-chloroethoxycarbonyl, 2-bromoethoxycarbonyl, 2,2-difluoroethoxycarbonyl, 2,2,2-trifluoroethoxycarbonyl, 1-fluoropropyloxycarbonyl, 2-fluoropropyloxycarbonyl, 3-fluoropropyloxycarbonyl, 3-chloropropyloxycarbonyl, 3-bromopropyloxycarbonyl, 4-fluorobutyloxycarbonyl, 4-chlorobutyloxycarbonyl or the like and is selected within a given range of carbon atoms.

Alkoxyalkoxycarbonyl may be methoxymethoxycarbonyl, ethoxymethoxycarbonyl, propyloxymethoxycarbonyl, i-propyloxymethoxycarbonyl, butyloxymethoxycarbonyl, 2-methoxyethoxycarbonyl, 1-methoxyethoxycarbonyl or the like and is selected within a given range of carbon atoms.

Alkylthioalkoxycarbonyl may be methylthiomethoxycarbonyl, ethylthiomethoxycarbonyl, propylthiomethoxycarbonyl, i-propylthiomethoxycarbonyl, butylthiomethoxycarbonyl, 2-methylthioethoxycarbonyl, 1-methylthioethoxycarbonyl or the like and is selected within a given range of carbon atoms.

Alkylsulfinylalkoxycarbonyl may be methylsulfinylmethoxycarbonyl, ethylsulfinylmethoxycarbonyl, propylsulfinylmethoxycarbonyl, i-propylsulfinylmethoxycarbonyl, butylsulfinylmethoxycarbonyl, 2-methylsulfinylethoxycarbonyl, 1-methylsulfinylethoxycarbonyl or the like and is selected within a given range of carbon atoms.

Alkylsulfonylalkoxycarbonyl may be methylsulfonylmethoxycarbonyl, ethylsulfonylmethoxycarbonyl, propylsulfonylmethoxycarbonyl, i-propylsulfonylmethoxycarbonyl, butylsulfonylmethoxycarbonyl, 2-methylsulfonylethoxycarbonyl, 1-methylsulfonylethoxycarbonyl or the like and is selected within a given range of carbon atoms.

Alkenyloxycarbonyl may be ethenyloxycarbonyl, 1-propenyloxycarbonyl, 2-propenyloxycarbonyl, 1-butenyloxycarbonyl, 1-methyl-2-propenyloxycarbonyl, 2-methyl-2-propenyloxycarbonyl, 1-hexenyloxycarbonyl or the like and is selected within a given range of carbon atoms.

Haloalkenyloxycarbonyl may be 1-chloroethenyloxycarbonyl, 2-chloroethenyloxycarbonyl, 2-fluoroethenyloxycarbonyl, 2,2-dichloroethenyloxycarbonyl, 3-chloro-2-propenyloxycarbonyl, 3-fluoro-2-propenyloxycarbonyl, 2-chloro-2-propenyloxycarbonyl, 4-chloro-3-butenyloxycarbonyl or the like and is selected within a given range of carbon atoms.

Alkynyloxycarbonyl may be ethynyloxycarbonyl, 1-propynyloxycarbonyl, 2-propynyloxycarbonyl, 1-butynyloxycarbonyl, 1-methyl-2-propynyloxycarbonyl, 2-methyl-2-propynyloxycarbonyl, 1-hexynyloxycarbonyl or the like and is selected within a given range of carbon atoms.

Phenoxycarbonyl substituted with at least one Y may be 2-fluorophenoxycarbonyl, 2-chlorophenoxycarbonyl, 4-bromophenoxycarbonyl, 4-methylphenoxycarbonyl, 2-methoxyphenoxycarbonyl, 3-aminophenoxycarbonyl, 4-trifluoromethoxyphenoxycarbonyl, 2-methylthiophenoxycarbonyl, 3-trifluoromethylthiophenoxycarbonyl, 4-cyanophenoxycarbonyl, 2-nitrophenoxycarbonyl, 4-trifluoromethylphenoxycarbonyl or the like. In a case where the phenyl group has two or more substituents Y's, the respective Y's may be the same or different from one another.

Phenylalkoxycarbonyl and phenylalkoxycarbonyl substituted with at least one Y may be benzyloxycarbonyl, 2-fluorobenzyloxycarbonyl, 2-chlorobenzyloxycarbonyl, 3-bromobenzyloxycarbonyl, 4-iodobenzyloxycarbonyl, 4-methylbenzyloxycarbonyl, 2-methoxybenzyloxycarbonyl, 4-aminobenzyloxycarbonyl, 4-trifluoromethoxybenzyloxycarbonyl, 2-methylthiobenzyloxycarbonyl, 3-trifluoromethylthiobenzyloxycarbonyl, 2-cyanobenzyloxycarbonyl, 4-nitrobenzyloxycarbonyl, 2-trifluoromethylbenzyloxycarbony, 2-phenethyloxycarbonyl or the like. In a case where the phenyl group has two or more substituents Y's, the respective Y's may be the same or different from one another.

Phenoxyalkylcarbonyl and phenoxyalkylcarbonyl substituted with at least one Y may be phenoxymethylcarbonyl, 2-phenoxyethylcarbonyl, 2-fluorophenoxymethylcarbonyl, 2-chlorophenoxymethylcarbonyl, 4-bromophenoxymethylcarbonyl, 4-methylphenoxymethylcarbonyl, 2-methoxyphenoxymethylcarbonyl, 3-aminophenoxymethylcarbonyl, 4-trifluoromethoxyphenoxymethylcarbonyl, 2-methylthiophenoxymethylcarbonyl, 3-trifluoromethylthiophenoxymethylcarbonyl, 4-cyanophenoxymethylcarbonyl, 2-nitrophenoxymethylcarbonyl, 4-trifluoromethylphenoxymethylcarbonyl or the like. In a case where the phenyl group has two or more substituents Y's, the respective Y's may be the same or different from one another.

Alkylthiocarbonyl may be methylthiocarbonyl, ethylthiocarbonyl, propylthiocarbonyl, i-propylthiocarbonyl, butylthiocarbonyl, i-butylthiocarbonyl, s-butylthiocarbonyl, t-butylthiocarbonyl or the like and is selected within a given range of carbon atoms.

Haloalkylthiocarbonyl may be fluoromethylthiocarbonyl, chlorodifluoromethylthiocarbonyl, bromodifluoromethylthiocarbonyl, trifluoromethylthiocarbonyl, trichloromethylthiocarbonyl, 2,2,2-trifluoroethylthiocarbonyl, 1,1,2,2-tetrafluoroethylthiocarbonyl, 2-fluoroethylthiocarbonyl, pentafluoroethylthiocarbonyl or the like and is selected within a given range of carbon atoms.

Monoalkylaminocarbonyl may be methylaminocarbonyl, ethylaminocarbonyl, propylaminocarbonyl, i-propylaminocarbonyl, butylaminocarbonyl, s-butylaminocarbonyl, i-butylaminocarbonyl, t-butylaminocarbonyl, pentylaminocarbonyl, hexylaminocarbonyl or the like and is selected within a given range of carbon atoms.

Monohaloalkylaminocarbonyl may be fluoromethylaminocarbonyl, difluoromethylaminocarbonyl, trifluoromethylaminocarbonyl, chloromethylaminocarbonyl, bromomethylaminocarbonyl, iodomethylaminocarbonyl, 2-fluoroethylaminocarbonyl, 2-chloroethylaminocarbonyl, 3-fluoropropylaminocarbonyl, 2-fluoropropylaminocarbonyl, 1-fluoropropylaminocarbonyl or the like and is selected within a given range of carbon atoms.

Dialkylaminocarbonyl may be dimethylaminocarbonyl, diethylaminocarbonyl, dipropylaminocarbonyl, di(i-propyl)aminocarbonyl, dibutylaminocarbonyl, di(s-butyl)aminocarbonyl, di(i-butyl)aminocarbonyl, di(t-butyl)aminocarbonyl, dipentylaminocarbonyl, dihexylaminocarbonyl, ethyl(methyl)aminocarbonyl, methyl(propyl)aminocarbonyl or the like and is selected within a given range of carbon atoms.

Halo di(alkyl)aminocarbonyl may be di(fluoromethyl)aminocarbonyl, di(chloromethyl)aminocarbonyl, di(bromomethyl)aminocarbonyl, fluoromethyl(methyl)aminocarbonyl, chloromethyl(methyl)aminocarbonyl, chloromethyl(ethyl)aminocarbonyl, di(2-fluoroethyl)aminocarbonyl, (2-fluoroethyl)methylaminocarbonyl or the like and is selected within a given range of carbon atoms.

Phenylaminocarbonyl substituted with at least one Y may be 2-fluorophenylaminocarbonyl, 2-chlorophenylaminocarbonyl, 4-bromophenylaminocarbonyl, 4-methylphenylaminocarbonyl, 2-methoxyphenylaminocarbonyl, 3-aminophenylaminocarbonyl, 4-trifluoromethoxyphenylaminocarbonyl, 2-methylthiophenylaminocarbonyl, 3-trifluoromethylthiophenylaminocarbonyl, 4-cyanophenylaminocarbonyl, 2-nitrophenylaminocarbonyl, 4-trifluoromethylphenylaminocarbonyl or the like. In a case where the phenyl group has two or more substituents Y's, the respective Y's may be the same or different from one another.

Phenylalkylaminocarbonyl and phenylalkylaminocarbonyl substituted with at least one Y may be benzylaminocarbonyl, 2-fluorobenzylaminocarbonyl, 2-chlorobenzylaminocarbonyl, 3-bromobenzylaminocarbonyl, 4-iodobenzylaminocarbonyl, 4-methylbenzylaminocarbonyl, 2-methoxybenzylaminocarbonyl, 4-aminobenzylaminocarbonyl, 4-trifluoromethoxybenzylaminocarbonyl, 2-methylthiobenzylaminocarbonyl, 3-trifluoromethylthiobenzylaminocarbonyl, 2-cyanobenzylaminocarbonyl, 4-nitrobenzylaminocarbonyl, 2-trifluoromethylbenzylaminocarbony, 2-phenethylaminocarbonyl or the like. In a case where the phenyl group has two or more substituents Y's, the respective Y's may be the same or different from one another.

Alkoxyalkyl may be methoxymethyl, ethoxymethyl, propyloxymethyl, i-propyloxymethyl, butyloxymethyl, i-butyloxymethyl, s-butyloxymethyl, t-butyloxymethyl, pentyloxymethyl, 1-methoxyethyl, 2-methoxyethyl, 2-ethoxyethyl, 3-methoxypropyl or the like and is selected within a given range of carbon atoms.

Haloalkoxyalkyl may be fluoromethoxymethyl, chloromethoxymethyl, bromomethoxymethyl, 2,2,2-trifluoroethoxymethyl, 2-(chrolomethoxy)ethyl or the like and is selected within a given range of carbon atoms.

Alkoxyalkoxyalkyl may be methoxymethoxymethyl, ethoxymethoxymethyl, propyloxymethoxymethyl, i-propyloxymethoxymethyl, butyloxymethoxymethyl, 1-(methoxymethoxy)ethyl, 2-(methoxymethoxy)ethyl, 2-(ethoxymethoxy)ethyl, 2-(ethoxy)ethoxymethyl or the like and is selected within a given range of carbon atoms.

Trialkylsilylalkoxyalkyl may be trimethylsilylmethoxymethyl, 2-(trimethylsilyl)ethoxymethyl, 2-(trimethylsilylmethoxy)ethyl, 2-[2-(trimethylsilyl)ethoxy]ethyl or the like and is selected within a given range of carbon atoms.

Hydroxyalkyl may be hydroxymethyl, 1-hydroxyethyl, 2-hydroxyethyl, 1-hydroxypropyl, 2-hydroxypropyl, 3-hydroxypropyl, 1-hydroxy-1-methylethyl, 2-hydroxy-1-methylethyl, 2-hydroxy-1,1-dimethylethyl or the like and is selected within a given range of carbon atoms.

Phenoxyalky and phenoxyalky substituted with at least one Y may be phenoxymethyl, 2-fluorophenoxymethyl, 2-chlorophenoxymethyl, 3-bromophenoxymethyl, 4-iodophenoxymethyl, 4-methylphenoxymethyl, 2-methoxyphenoxymethyl, 4-aminophenoxymethyl, 4-trifluoromethoxyphenoxymethyl, 2-methylthiophenoxymethyl, 3-trifluoromethylthiophenoxymethyl, 2-cyanophenoxymethyl, 4-nitrophenoxymethyl, 2-trifluoromethylphenoxymethyl, 2-(phenoxy)ethyl or the like and is selected within a given range of carbon atoms. In a case where the phenyl group has two or more substituents Y's, the respective Y's may be the same or different from one another.

Phenylcarbonyloxyalkoxyalkyl and phenylcarbonyloxyalkoxyalkyl substituted with at least one Y may be phenylcarbonyloxymethoxymethyl, 2-fluorophenylcarbonyloxymethoxymethyl, 2-chlorophenylcarbonyloxymethoxymethyl, 3-bromophenylcarbonyloxymethoxymethyl, 4-iodophenylcarbonyloxymethoxymethyl, 4-methylphenylcarbonyloxymethoxymethyl, 2-methoxyphenylcarbonyloxymethoxymethyl, 4-aminophenylcarbonyloxymethoxymethyl, 4-trifluoromethoxyphenylcarbonyloxymethoxymethyl, 2-methylthiophenylcarbonyloxymethoxymethyl, 3-trifluoromethylthiophenylcarbonyloxymethoxymethyl, 2-cyanophenylcarbonyloxymethoxymethyl, 4-nitrophenylcarbonyloxymethoxymethyl, 2-trifluoromethylphenylcarbonyloxymethoxymethyl, 2-(phenylcarbonyloxy)ethoxymethyl, 2-(phenylcarbonyloxymethoxy)ethyl or the like. In a case where the phenyl group has two or more substituents Y's, the respective Y's may be the same or different from one another.

Alkylthioalkyl may be methylthiomethyl, ethylthiomethyl, propylthiomethyl, i-propylthiomethyl, butylthiomethyl, i-butylthiomethyl, s-butylthiomethyl, t-butylthiomethyl, pentylthiomethyl, 1-methylthioethyl, 2-methylthioethyl, 2-ethylthioethy, 3-methylthiopropyl or the like and is selected within a given range of carbon atoms.

Haloalkylthioalkyl may be fluoromethylthiomethyl, chloromethylthiomethyl, bromomethylthiomethyl, 2,2,2-trifluoroethylthiomethyl, 2-(chloromethylthio)ethyl or the like and is selected within a given range of carbon atoms.

Phenylthioalkyl and phenylthioalkyl substituted with at least one Y may be phenylthiomethyl, 2-fluorophenylthiomethyl, 2-chlorophenylthiomethyl, 3-bromophenylthiomethyl, 4-iodophenylthiomethyl, 4-methylphenylthiomethyl, 2-methoxyphenylthiomethyl, 4-aminophenylthiomethyl, 4-trifluoromethoxyphenylthiomethyl, 2-methylthiophenylthiomethyl, 3-trifluoromethylthiophenylthiomethyl, 2-cyanophenylthiomethyl, 4-nitrophenylthiomethyl, 2-trifluoromethylphenylthiomethyl, 2-(phenylthio)ethyl or the like and is selected within a given range of carbon atoms. In a case where the phenyl group has two or more substituents Y's, the respective Y's may be the same or different from one another.

Phenylalkylthioalkyl and phenylalkylthioalkyl substituted with at least one Y may be benzylthiomethyl, 2-fluorobenzylthiomethyl, 2-chlorobenzylthiomethyl, 3-bromobenzylthiomethyl, 4-iodobenzylthiomethyl, 4-methylbenzylthiomethyl, 2-methoxybenzylthiomethyl, 4-aminobenzylthiomethyl, 4-trifluoromethoxybenzylthiomethyl, 2-methylthiobenzylthiomethyl, 3-trifluoromethylthiobenzylthiomethyl, 2-cyanobenzylthiomethyl, 4-nitrobenzylthiomethyl, 2-trifluoromethylbenzylthiomethyl, phenethylthiomethyl, 2-(benzylthio)ethyl or the like and is selected within a given range of carbon atoms. In a case where the phenyl group has two or more substituents Y's, the respective Y's may be the same or different from one another.

Alkylcarbonylalkyl may be methylcarbonylmethyl, ethylcarbonylmethyl, propylcarbonylmethyl, i-propylcarbonylmethyl, butylcarbonylmethyl, i-butylcarbonylmethyl, s-butylcarbonylmethyl, t-butylcarbonylmethyl, pentylcarbonylmethyl, 1-(methylcarbonyl)ethyl, 2-(methylcarbonyl)ethyl, 2-(ethylcarbonyl)ethyl, 3-(methylcarbonyl)propyl or the like and is selected within a given range of carbon atoms.

Haloalkylcarbonylalkyl may be fluoromethylcarbonylmethyl, chloromethylcarbonylmethyl, bromomethylcarbonylmethyl, 2,2,2-trifluoroethylcarbonylmethyl, 2-(chloromethylcarbonyl)ethyl or the like and is selected within a given range of carbon atoms.

Alkoxycarbonylalkyl may be methoxycarbonylmethyl, ethoxycarbonylmethyl, propyloxycarbonylmethyl, i-propyloxycarbonylmethyl, butyloxycarbonylmethyl, 2-(methoxycarbonyl)ethyl, 2-(ethoxycarbonyl)ethyl, 3-(methoxycarbonyl)propyl or the like and is selected within a given range of carbon atoms.

Haloalkoxycarbonylalkyl may be fluoromethoxycarbonylmethyl, chloromethoxycarbonylmethyl, bromomethoxycarbonylmethyl, 2,2,2-trifluoroethoxycarbonylmethyl, 2-(chloromethoxycarbonyl)ethyl or the like and is selected within a given range of carbon atoms.

Alkoxycarbonyloxyalkyl may be methoxycarbonyloxymethyl, ethoxycarbonyloxymethyl, propyloxycarbonyloxymethyl, i-propyloxycarbonyloxymethyl, butyloxycarbonyloxymethyl, 2-methoxycarbonyloxyethyl, 2-(ethoxycarbonyloxy)ethyl, 3-(methoxycarbonyloxy)propyl or the like and is selected within a given range of carbon atoms.

Monoalkylaminocarbonyloxyalkyl may be methylaminocarbonyloxymethyl, ethylaminocarbonyloxymethyl, propylaminocarbonyloxymethyl, i-propylaminocarbonyloxymethyl, butylaminocarbonyloxymethyl, 2-(methylaminocarbonyloxy)ethyl, 2-(ethylaminocarbonyloxy)ethyl, 3-(methylaminocarbonyloxy)propyl or the like and is selected within a given range of carbon atoms.

Dialkylaminocarbonyloxyalkyl may be dimethylaminocarbonyloxymethyl, diethylaminocarbonyloxymethyl, ethyl(methyl)aminocarbonyloxymethyl, methyl(propyl)aminocarbonyloxymethyl, butyl(methyl)aminocarbonyloxymethyl, 2-(diethylaminocarbonyloxy)ethyl, 2-{ethyl(methyl)aminocarbonyloxy}propyl or the like and is selected within a given range of carbon atoms.

Phenylaminocarbonyloxyalkyl and phenylaminocarbonyloxyalkyl substituted with at least one Y may be phenylaminocarbonyloxymethyl, 2-fluorophenylaminocarbonyloxymethyl, 2-chlorophenylaminocarbonyloxymethyl, 3-bromophenylaminocarbonyloxymethyl, 4-iodophenylaminocarbonyloxymethyl, 4-methylphenylaminocarbonyloxymethyl, 2-methoxyphenylaminocarbonyloxymethyl, 4-aminophenylaminocarbonyloxymethyl, 4-trifluoromethoxyphenylaminocarbonyloxymethyl, 2-methylthiophenylaminocarbonyloxymethyl, 3-trifluoromethylphenylaminocarbonyloxymethyl, 2-cyanophenylaminocarbonyloxymethyl, 4-nitrophenylaminocarbonyloxymethyl, 2-trifluoromethylphenylaminocarbonyloxymethyl, 2-(phenylaminocarbonyloxy)ethyl or the like and is selected within a given range of carbon atoms. In a case where the phenyl group has two or more substituents Y's, the respective Y's may be the same or different from one another.

Alkyl(phenyl)aminocarbonyloxyalkyl and alkyl(phenyl)aminocarbonyloxyalkyl substituted with at least one Y may be methyl(phenyl)aminocarbonyloxymethyl, methyl(2-fluorophenyl)aminocarbonyloxymethyl, methyl(2-chlorophenyl)aminocarbonyloxymethyl, methyl(3-bromophenyl)aminocarbonyloxymethyl, methyl(4-iodophenyl)aminocarbonyloxymethyl, methyl(4-methylphenyl)aminocarbonyloxymethyl, methyl(2-methoxyphenyl)aminocarbonytoxymethyl, methyl(4-aminophenyl)aminocarbonyloxymethyl, methyl(4-trifluoromethoxy)phenylaminocarbonyloxymethyl, methyl(2-methylthiophenyl)aminocarbonyloxymethyl, methyl(3-trifluoromethylphenyl)aminocarbonyloxymethyl, methyl(2-cyanophenyl)aminocarbonyloxymethyl, methyl(4-nitrophenyl)aminocarbonyloxymethyl, ethyl(phenyl)aminocarbonyloxymethyl, 2-{methyl(phenyl)aminocarbonyloxy}ethyl or the like and is selected within a given range of carbon atoms. In a case where the phenyl group has two or more substituents Y's, the respective Y's may be the same or different from one another.

Alkylsulfinylalkyl may be methylsulfinylmethyl, ethylsulfinylmethyl, propylsulfinylmethyl, i-propylsulfinylmethyl, butylsulfinylmethyl, i-butylsulfinylmethyl, s-butylsulfinylmethyl, t-butylsulfinylmethyl, pentylsulfinylmethyl, 1-(methylsulfinyl)ethyl, 2-(methylsulfinyl)ethyl, 2-(ethylsulfinyl)ethyl, 3-(methylsulfinyl)propyl or the like and is selected within a given range of carbon atoms.

Haloalkylsulfinylalkyl may be fluoromethylsulfinylmethyl, chloromethylsulfinylmethyl, bromomethylsulfinylmethyl, 2,2,2-trifluoroethylsulfinylmethyl, 2-(chloromethylsulfinyl)ethyl or the like and is selected within a given range of carbon atoms.

Phenylsulfinylalkyl and phenylsulfinylalkyl substituted with at least one Y may be phenylsulfinylmethyl, 2-fluorophenylsulfinylmethyl, 2-chlorophenylsulfinylmethyl, 3-bromophenylsulfinylmethyl, 4-iodophenylsulfinylmethyl, 4-methylphenylsulfinylmethyl, 2-methoxyphenylsulfinylmethyl, 4-aminophenylsulfinylmethyl, 4-trifluoromethoxyphenylsulfinylmethyl, 2-methylthiophenylsulfinylmethyl, 3-trifluoromethylthiophenylsulfinylmethyl, 2-cyanophenylsulfinylmethyl, 4-nitrophenylsulfinylmethyl, 2-trifluoromethylphenylsulfinylmethyl, 2-(phenylsulfinyl)ethyl or the like and is selected within a given range of carbon atoms. In a case where the phenyl group has two or more substituents Y's, the respective Y's may be the same or different from one another.

Alkylsulfonylalkyl may be methylsulfonylmethyl, ethylsulfonylmethyl, propylsulfonylmethyl, i-propylsulfonylmethyl, butylsulfonylmethyl, i-butylsulfonylmethyl, s-butylsulfonylmethyl, t-butylsulfonylmethyl, pentylsulfonylmethyl, 1-(methylsulfonyl)ethyl, 2-(methylsulfonyl)ethyl, 2-(ethylsulfonyl)ethyl, 3-(methylsulfonyl)propyl or the like and is selected within a given range of carbon atoms.

Haloalkylsulfonylalkyl may be fluoromethylsulfonylmethyl, chloromethylsulfonylmethyl, bromomethylsulfonylmethyl, 2,2,2-trifluoroethylsulfonylmethyl, 2-(chloromethylsulfonyl)ethyl or the like and is selected within a given range of carbon atoms.

Phenylsulfonylalkyl and phenylsulfonylalkyl substituted with at least one Y may be phenylsulfonylmethyl, 2-fluorophenylsulfonylmethyl, 2-chlorophenylsulfonylmethyl, 3-bromophenylsulfonylmethyl, 4-iodophenylsulfonylmethyl, 4-methylphenylsulfonylmethyl, 2-methoxyphenylsulfonylmethyl, 4-aminophenylsulfonylmethyl, 4-trifluoromethoxyphenylsulfonylmethyl, 2-methylthiophenylsulfonylmethyl, 3-trifluoromethylthiophenylsulfonylmethyl, 2-cyanophenylsulfonylmethyl, 4-nitrophenylsulfonylmethyl, 2-trifluoromethylphenylsulfonylmethyl, 2-(phenylsulfonyl)ethyl or the like and is selected within a given range of carbon atoms. In a case where the phenyl group has two or more substituents Y's, the respective Y's may be the same or different from one another.

Phenylalkylsulfonylalkyl and phenylalkylsulfonylalkyl substituted with at least one Y may be benzylsulfonylmethyl, 2-fluorobenzylsulfonylmethyl, 2-chlorobenzylsulfonylmethyl, 3-bromobenzylsulfonylmethyl, 4-iodobenzylsulfonylmethyl, 4-methylbenzylsulfonylmethyl, 2-methoxybenzylsulfonylmethyl, 4-aminobenzylsulfonylmethyl, 4-trifluoromethoxybenzylsulfonylmethyl, 2-methylthiobenzylsulfonylmethyl, 3-trifluoromethylthiobenzylsulfonylmethyl, 2-cyanobenzylsulfonylmethyl, 4-nitrobenzylsulfonylmethyl, 2-trifluoromethylbenzylsulfonylmethyl, phenethylsulfonylmethyl, 2-(benzylsulfonyl)ethyl or the like and is selected within a given range of carbon atoms. In a case where the phenyl group has two or more substituents Y's, the respective Y's may be the same or different from one another.

Phenylalkoxyalkyl and phenylalkoxyalkyl substituted with at least one Y may be benzyloxymethyl, 2-fluorobenzyloxymethyl, 2-chlorobenzyloxymethyl, 3-bromobenzyloxymethyl, 4-iodobenzyloxymethyl, 4-methylbenzyloxymethyl, 2-methoxybenzyloxymethyl, 4-aminobenzyloxymethyl, 4-trifluoromethoxybenzyloxymethyl, 2-methylthiobenzyloxymethyl, 3-trifluoromethylthiobenzyloxymethyl, 2-cyanobenzyloxymethyl, 4-nitrobenzyloxymethyl, 2-trifluoromethylbenzyloxymethyl, 2-phenethyloxymethyl, 1-(benzyloxy)ethyl, 2-(benzyloxy)ethyl or the like and is selected within a given range of carbon atoms. In a case where the phenyl group has two or more substituents Y's, the respective Y's may be the same or different from one another.

Alkylcarbonyloxyalkyl may be acetoxymethyl, propionyloxymethyl, butyryloxymethyl, i-butyryloxymethyl, valeroyloxymethyl, i-valeroyloxymethyl, 2-methylbutyryloxymethyl, pivaloyloxymethyl, heptanoyloxymethyl, 1-(acetoxy)ethyl, 2-(acetoxy)ethyl, 2-(propionyloxy)ethyl, 3-(acetoxy)propyl or the like and is selected within a given range of carbon atoms.

Phenylcarbonyloxyalkyl and phenylcarbonyloxyalkyl substituted with at least one Y may be phenylcarbonyloxymethyl, 2-fluorophenylcarbonyloxymethyl, 2-chlorophenylcarbonyloxymethyl, 3-bromophenylcarbonyloxymethyl, 4-iodophenylcarbonyloxymethyl, 4-methylphenylcarbonyloxymethyl, 2-methoxyphenylcarbonyloxymethyl, 4-aminophenylcarbonyloxymethyl, 4-trifluoromethoxyphenylcarbonyloxymethyl, 2-methylthiophenylcarbonyloxymethyl, 3-trifluoromethylthiophenylcarbonyloxymethyl, 2-cyanophenylcarbonyloxymethyl, 4-nitrophenylcarbonyloxymethyl, 2-trifluoromethylphenylcarbonyloxymethyl, 2-(phenylcarbonyloxy)ethyl or the like and is selected within a given range of carbon atoms. In a case where the phenyl group has two or more substituents Y's, the respective Y's may be the same or different from one another.

Dialkylaminothio may be dimethylaminothio, diethylaminothio, dipropylaminothio, di(i-propyl)aminothio, dibutylaminothio, di(s-butyl)aminothio, di(i-butyl)aminothio, di(t-butyl)aminothio, dipentylaminothio, dihexylaminothio, ethyl(methyl)aminothio, methyl(propyl)aminothio or the like and is selected within a given range of carbon atoms.

Monoalkylaminoalkyl may be methylaminomethyl, ethylaminomethyl, propylaminomethyl, i-propylaminomethyl, butylaminomethyl, 2-(methylamino)ethyl, 2-(ethylamino)ethyl, 3-(methylamino)propyl or the like and is selected within a given range of carbon atoms.

Dialkylaminoalkyl may be dimethylaminomethyl, diethylaminomethyl, ethyl(methyl)aminomethyl, methyl(propyl)aminomethyl, butyl(methyl)aminomethyl, 2-(diethylamino)ethyl, 2-{ethyl(methyl)amino}propyl or the like and is selected within a given range of carbon atoms.

Monoalkylamino may be methylamino, ethylamino, propylamino, i-propylamino, butylamino, s-butylamino, i-butylamino, t-butylamino, pentylamino, hexylamino or the like and is selected within a given range of carbon atoms.

Di(alkyl)amino may be dimethylamino, diethylamino, dipropylamino, di(i-propyl)amino, dibutylamino, di(s-butyl)amino, di(i-butyl)amino, di(t-butyl)amino, dipentylamino, dihexylamino, ethyl(methyl)amino, methyl(propyl)amino or the like and is selected within a given range of carbon atoms.

Alkylcarbonyloxy may be acetoxy, propionyloxy, butyryloxy, i-butyryloxy, valeroyloxy, i-valeroyloxy, 2-methylbutyryloxy, pivaloyloxy, hexanoyloxy, 2-ethylbutyryloxy, 2-methylvaleroyloxy, 4-methylvaleroyloxy, heptanoyloxy, 2,2-dimethylvaleroyloxy, 2-ethylhexanoyloxy, 2-propylvaleroyloxy, octanoyloxy, nonanoyloxy, 3,5,5-trimethylhexanoyloxy, decanoyloxy or the like and is selected within a given range of carbon atoms.

"R₃ and R₄ may form a 3- to 7-membered ring together with each other" means that R₃ and R₄ may form cyclopropyl, cyclobutyl, cyclopentyl, cyclohexyl, cycloheptyl, epoxy, tetrahydrofuran , tetrahydropyran, tetrahydrothiophene, tetrahydrothiopyran, pyrrolidine, piperidine or the like containing the carbon to which R₃ and R₄ are bonded.

"R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (the nitrogen atoms may be substituted with C₁₋C₆ alkyl, C₂₋C₆ alkenyl, C₂₋C₆ alkynyl or cyclo C₃₋C₆ alkyl)" means that R₅, R₆, R₇, R₈, R₉ or R₁₀ on the same carbon may form cyclopropyl, cyclobutyl, cyclopentyl, cyclohexyl, cycloheptyl, epoxy, tetrahydrofuran , tetrahydropyran, tetrahydrothiophene, tetrahydrothiopyran, pyrrolidine, piperidine or the like containing the carbon to which R₅, R₆, R₇, R₈, R₉ or R₁₀ is bonded.

"R₅, R₆, R₇, R₈ R₉, R₁₀, R₁₁ or R₁₂ may form, together with R₅, R₆, R₇, R₈ R₉, R₁₀, R₁₁ or R₁₂ on an adjacent carbon or nitrogen, a 3- to 8-membered ring which may contain one or two hetero atoms selected from oxygen atoms, sulfur atoms and nitrogen atoms (the nitrogen atoms may be substituted with C₁₋C₆ alkyl, C₂₋C₆ alkenyl, C₂₋C₆ alkynyl or cyclo C₃₋C₆ alkyl), or may form a double bond" means that R₅, R₆, R₇, R₈ R₉, R₁₀, R₁₁ or R₁₂ may form, together with R₅, R₆, R₇, R₈ R₉, R₁₀, R₁₁ or R₁₂ on an adjacent carbon or nitrogen, a linkage such as -CH₂-, -CH₂-CH₂-, -O-, -O-CH₂-, -S-, -S-CH₂-, -NR₁₃- (R₁₃ is C₁₋C₆ alkyl, C₂₋C₆ alkenyl, C₂₋C₆ alkynyl or cyclo C₃₋C₆ alkyl) and -NR₁₃-CH₂- (R₁₃ is the same as defined previously), or may form a double bond.

"Y may be C₁₋C₄ alkylene, halo C₁₋C₄ alkylene, C₂₋C₄ alkenylene or halo C₂₋C₄ alkenylene which may contain one or identical or different two hetero atoms selected from oxygen atoms, sulfur atoms and nitrogen atoms (the nitrogen atoms may be substituted with C₁₋C₆ alkyl, C₂₋C₆ alkenyl, C₂₋C₆ alkynyl or C₃₋C₆ cycloalkyl) and form a 5- or 6-membered ring together with an adjacent carbon or nitrogen atom on a benzene ring or a heterocyclyl" means that indene, dihydroindene, naphthalene, dihydronaphthalene, tetrahydronaphthalene, benzofuran, dihydrobenzofuran, chromene, chromane, benzothiophene, dihydrobenzothiophene, thiochromene, thiochromane, indole, indoline, quinoline, dihydroquinoline, tetrahydroquinoline, cyclopentapyridine, pyrrolopyridine, naphthylidine, pyrazolopyridine, triazolopyrimidine or the like containing the benzene ring or the heterocyclyl to which Y is bonded may be formed.

The compounds of the present invention can be prepared, for example, by the processes represented by the following Reaction Schemes 1 to 10, but may be prepared by other processes. 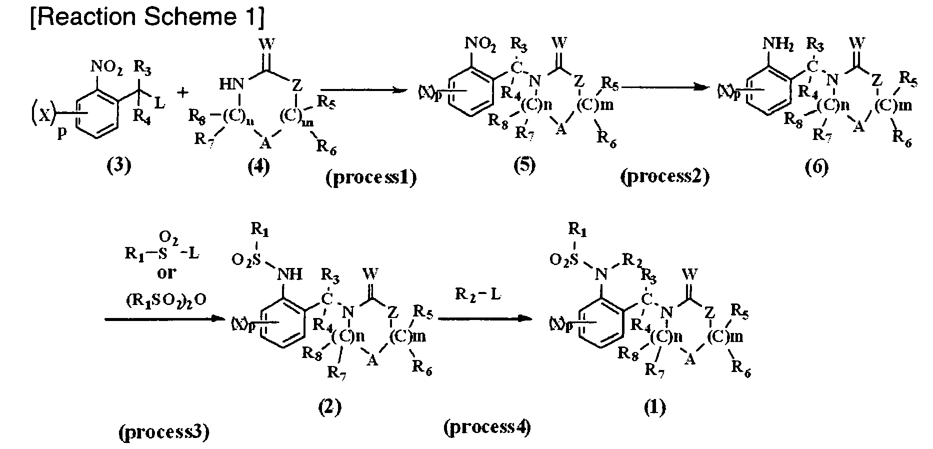 [wherein R₁, R₂, R₃, R₄, R₅, R₆, R₇, R₈, A, W, Z, X, m, n and p are the same as defined previously, and L is a leaving group such as a halogen atom.]

As shown in Reaction Scheme 1, a compound represented by the formula (3) and a compound represented by the formula (4) are reacted to give a preparation intermediate represented by the formula (5). After or without isolation, the preparation intermediate represented by the formula (5) is converted to a preparation intermediate represented by the formula (6) by reduction of the nitro group. After or without isolation, the preparation intermediate represented by the formula (6) is reacted with a known haloalkylsulfonyl derivative represented by the formula R₁SO₂L or (R₁SO₂)₂O to give a compound of the present invention represented by the formula (2). Further, after or without isolation, the compound of the present invention represented by the formula (2) is reacted with a compound represented by the formula R₂-L to give a compound of the present invention represented by the formula (1).

The reaction intermediate (3) may be commercially available or may be synthesized in accordance with Journal of Organic Chemistry 73(15),5989-5992(2008) or Bioorganic & Medicinal Chemistry 15(20),6574-6595(2007).

The reaction intermediate (4) may be synthesized in accordance with WO06/055951, Tetrahedron 57(11),2115-2120(2001), Synlett (8),915-916 (1997). 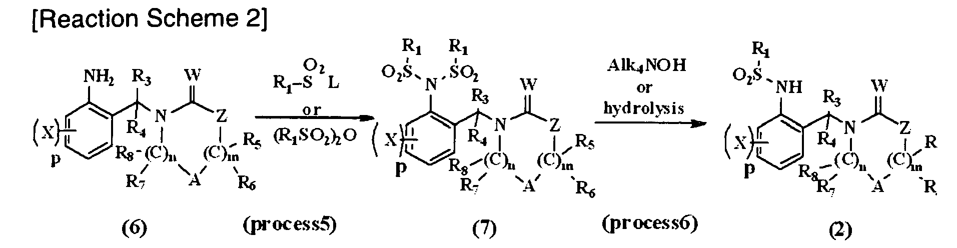 [wherein R₁ R₃, R₄, R₅, R₆, R₇, R₈, A, W, Z, X, m, n and p are the same as defined previously, L is a leaving group such as a halogen atom, and Alk is an alkyl group.]

As shown in Reaction Scheme 2, a compound represented by the formula (6) is reacted with an excess of a known haloalkylsulfonyl derivative represented by the formula R₁SO₂L or (R₁SO₂)₂O to give a compound of the present invention represented by the formula (7). After or without isolation, the compound represented by the formula (7) is reacted with a known compound represented by the formula Alk₄NOH or hydrolyzed with sodium hydroxide, potassium hydroxide or the like to give a compound of the present invention represented by the formula (2).

The alkyl group of Alk₄NOH used in Process 6 in Reaction Scheme 2 is not particularly limited and may, for example, be tetrabutylammonium hydroxide, tetramethylammonium hydroxide or tetraethylammonium hydroxide. Alk₄NOH is used usually in an amount of from 0 to 10 eq., preferably from 0 to 2 eq., in relation to 1 eq. of the compound used. 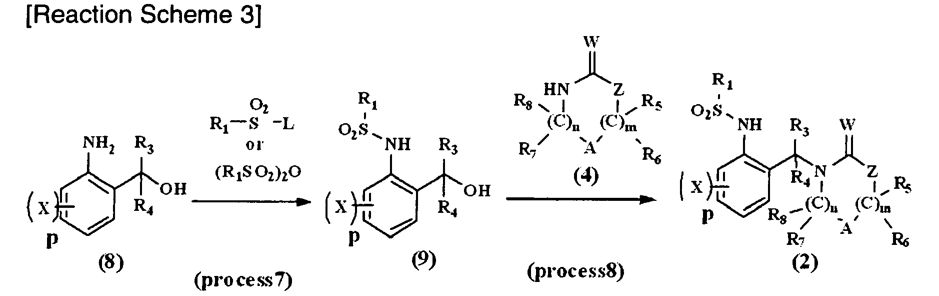 [wherein R₁, R₃, R₄, R₅, R₆, R₇, R₈, A, W, Z, X, m, n and p are the same as defined previously, and L is a leaving group such as a halogen atom.]

As shown in Reaction Scheme 3, a compound represented by the formula (8) is reacted with a known haloalkylsulfonyl derivative represented by the formula R₁SO₂L or (R₁SO₂)₂O to give a preparation intermediate represented by the formula (9). Further, after or without isolation, the preparation intermediate represented by the formula (9) is reacted with a compound represented by the formula (4) to give a compound of the present invention represented by the formula (2).

The reaction intermediate (8) may be commercially available or may be synthesized in accordance with Medicinal Chemistry 4(4),298-308(2008) or W02006/113432. 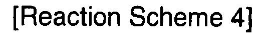 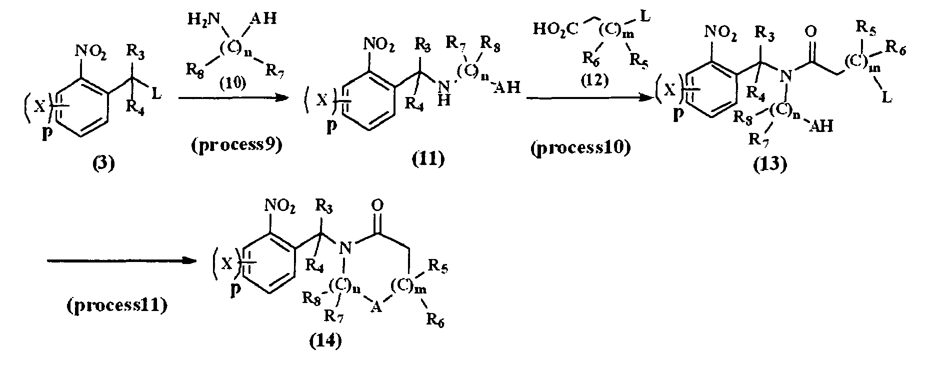 [wherein R₃, R₄, R₅, R₆, R₇, R₈, A, X, m, n and p are the same as defined previously, and L is a leaving group such as a halogen atom.]

As shown in Reaction Scheme 4, a compound represented by the formula (3) and a compound represented by the formula (10) which is known or can be easily synthesized in accordance with a known method are reacted to give a preparation intermediate represented by the formula (11). After or without isolation, the preparation intermediate represented by the formula (11) and a compound represented by the formula (12) which is known or can be easily synthesized in accordance with a known method are reacted with e.g. a condensation agent to give a preparation intermediate represented by the formula (13). Further, after or without isolation, the preparation intermediate represented by the formula (13) is cyclized to give a preparation intermediate represented by the formula (14). 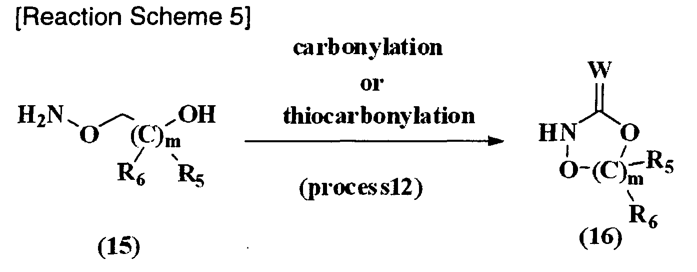 [wherein R₅, R₆, W and m are the same as defined previously.]

As shown in Reaction Scheme 5, a compound represented by the formula (15) and a carbonylating agent or a thiocarbonylating agent are reacted to give a preparation intermediate represented by the formula (16).

The reaction intermediate (15) may be commercially available or may be synthesized in accordance with W02008/024725 or Journal of Organic chemistry 47(3),517-523(1982). 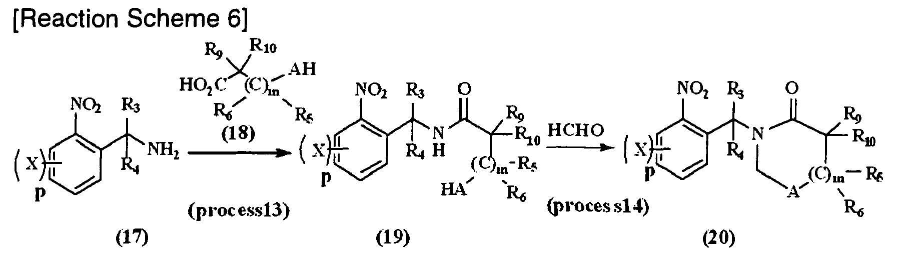 [wherein R₃, R₄, R₅, R₆, A, X, m and p are the same as defined previously.]

As shown in Reaction Scheme 6, a compound represented by the formula (17) and a compound represented by the formula (18) are reacted with e.g. a condensation agent to give a preparation intermediate represented by the formula (19). After or without isolation, the preparation intermediate represented by the formula (19) is cyclized with formaldehyde to give a preparation intermediate represented by the formula (20).

The reaction intermediate (17) may be commercially available or may be synthesized in accordance with W02006/113432, Bioorganic & Medicinal Chemistry Letters 16(13),3463-3468(2006), Chemical Communications (14),1557-1559(2006).

The reaction intermediate (18) may be commercially available or may be synthesized in accordance with W02009/075557, Bioorganic & Medicinal Chemistry Letters 18(21)5781-5784 (2008), Journal of Organic Chemistry 74(2), 917-920 (2009), Journal of Organic Chemistry 67(1), 72-78 (2002) and Tetrahedron 62(35), 8410-8418 (2006). 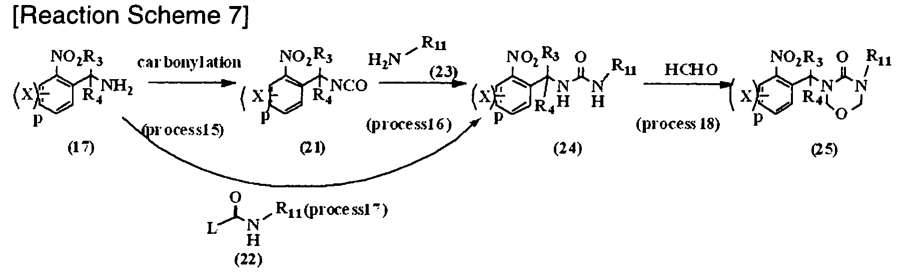 [wherein R₃, R₄, R₁₁, X and p are the same as defined previously, and L is a leaving group such as a halogen atom.]

As shown in Reaction Scheme 7, a compound represented by the formula (17) is isocyanated with e.g. a carbonylating agent to give a compound represented by the formula(21). After or without isolation, the compound represented by the formula (21) is reacted with an amine represented by the formula (23) which is known or can be easily synthesized in accordance with a known method to give a preparation intermediate represented by the formula (24). Further, the preparation intermediate represented by the formula (24) can be produced also by reacting the compound represented by the formula (17) with a known compound represented by the formula (22). Further, after or without isolation, the preparation intermediate represented by the formula (24) is cyclized with formaldehyde to give a preparation intermediate represented by the formula (25). 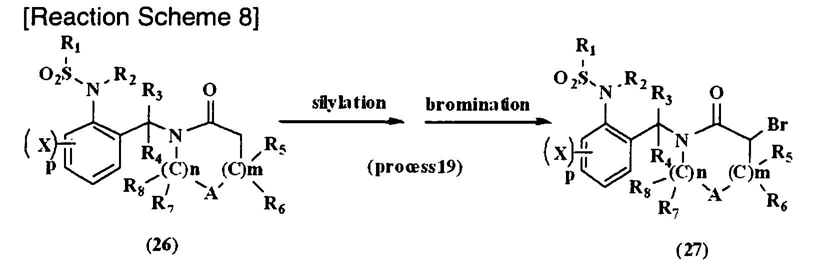 [wherein R₁, R₂, R₃, R₄, R₅, R₆, R₇, R₈, A, X, m, n and p are the same as defined previously.]

As shown in Reaction Scheme 8, a compound represented by the formula (26) is silyl-enol-etherified, and without isolation, brominated to give a compound of the present invention represented by the formula (27). 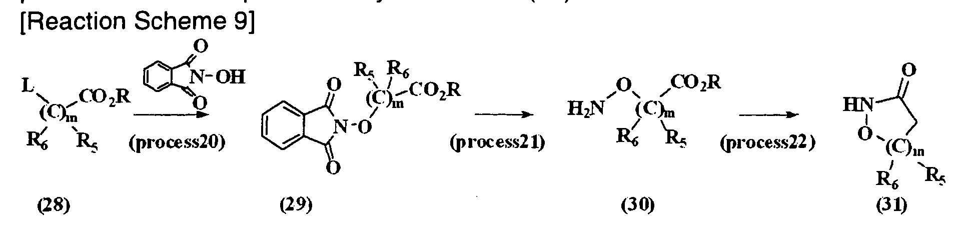 [wherein R₅, R₆, m and L are the same as defined previously, and R is an optional group.]

As shown in Reaction Scheme 9, a known compound represented by the formula (28) and known N-hydroxyphthalimide are reacted to give a preparation intermediate represented by the formula (29), and after or without isolation, the preparation intermediate represented by the formula (29) is hydrolyzed to give a preparation intermediate represented by the formula (30). Further, after or without isolation, the preparation intermediate represented by the formula (30) is cyclized to give a preparation intermediate represented by the formula (31). 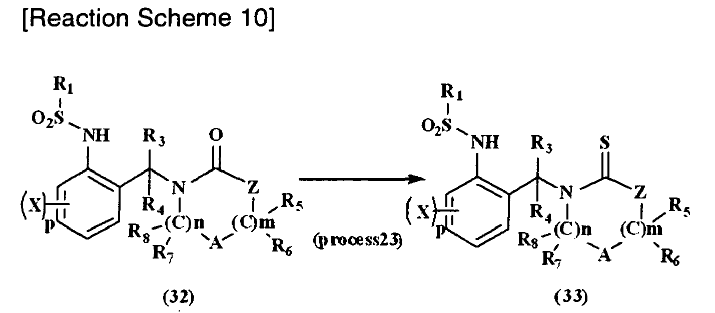 [wherein R₁, R₃, R₄, R₅, R₆, R₇, R₈, A, Z, X, m, n and p are the same as defined previously.]

As shown in Reaction Scheme 10, a compound represented by the formula (32) is thiocarbonylated to give a compound of the present invention represented by the formula (33).

The reactions of Processes 1, 3 to 7, 9 to 13, 15, 17 and 19 in Reaction Schemes 1 to 10 may be carried out in the presence of a base, and as the base, an inorganic base such as sodium hydroxide, potassium hydroxide, sodium carbonate, potassium carbonate, sodium hydrogen carbonate, potassium hydrogen carbonate or sodium hydride, an organic base such as pyridine, 4-dimethylaminopyridine, triethylamine, tributylamine, N,N-dimethylaniline or 1,8-diazabicyclo[5.4.0]-7-undecene, an organic lithium such as butyllithium or s-butyllithium, an organic lithium amide such as lithiumdiisopropylamide or lithiumbis(trimethylsilyl)amide, or a metal alkoxide such as sodium methoxide, sodium ethoxide or potassium t-butoxide may be mentioned. The base is used usually in an amount of from 0 to 10 eq., preferably from 0 to 2 eq., in relation to 1 eq. of the compound as the reactant.

In the reactions in Reaction Schemes 1 to 10, a solvent may be used, if necessary. Any solvents inert to the reactions may be used without any particular restrictions. For example, hydrocarbons such as hexane, cyclohexane, benzene and toluene, halogenated hydrocarbons such as carbon tetrachloride, chloroform and 1,2-dichloroethane, ethers such as diethyl ether, diisopropyl ether, 1,4-dioxane and tetrahydrofuran, ketones such as acetone, methyl ethyl ketone and methyl isobutyl ketone, nitriles such as acetonitrile and propionitrile, carboxylic acid esters such as ethyl acetate and ethyl propionate, nitrogen-containing aprotic polar solvents such as N,N-dimethylformamide, N,N-dimethylacetamide, N-methyl-2-pyrrolidone and 1,3-dimethyl-2-imidazolidinone, sulfur-containing aprotic polar solvents such as dimethyl sulfoxide and sulfolane, pyridines such as pyridine and picoline and the like may be mentioned. These solvents may be used singly or in combination of at least two.

The reactions in Reaction Schemes 1 to 10 may be carried out in a homogeneous system or a two-phase system. In the case of a two-phase system, use of a phase transfer catalyst can be effective. As the phase transfer catalyst to be used, a quaternary ammonium salt such as tetrabutylammonium chloride or tetrabutylammonium bromide or a crown ether such as 18-crown-6 may be mentioned.

The reactions in Process 8 in Reaction Scheme 3, Process 14 in reaction Scheme 6 and Process 18 in Reaction Scheme 7 may be carried out in the presence of an acid catalyst. As the acid catalyst to be used, a mineral acid such as hydrochloric acid, sulfuric acid or nitric acid, an organic acid such as formic acid, acetic acid, trifluroacetic acid, methanesulfonic acid or p-toluenesulfonic acid, a Lewis acid such as zinc chloride, titanium tetrachloride or boron trifluoride-ether complex may be mentioned. The acid catalyst is used usually in an amount of from 0 to 10 eq., preferably from 0 to 2 eq., in relation to 1 eq. of the compound as the reactant.

In the reaction of Process 2 in Reaction Scheme 1, the reduction may be carried out without any particular restrictions, for example, by metal reduction with e.g. iron, zinc chloride or tin chloride, by hydrogenation in the presence of a palladium catalyst, or by reduction with a metal hydride such as sodium borohydride, lithium borohydride or lithium aluminum hydride. The reducing agent is used usually in an amount of from 0 to 10 eq., preferably from 0 to 2 eq., in relation to 1 eq. of the compound as the reactant.

In the reactions of Process 10 in Reaction Scheme 4 and Process 13 in Reaction Scheme 6, any condensation agent may be used without any particular restrictions. For example, 1-ethyl-3-(3-dimethylaminopropyl)carbodiimide hydrochloride (WSC), N,N'-dicyclohexylcarbodiimide, N,N'-diisopropylcarbodiimide, diethyl cyanohydrochloride, carbonyldiimidazole, 4-(4,6-dimethoxy-1,3,5-triazin-2-yl)-4-methylmorpholinium chloride, a chlorocarbonate ester, 2-chloro-1-methylpyridinium iodide, oxalyl chloride or thionyl chloride may be mentioned. The condensation agent is used usually in an amount of from 0 to 10 eq., preferably from 0 to 2 eq., in relation to 1 eq. of the compound as the reactant.

In Process 12 in Reaction Scheme 5 and Process 15 in Reaction Scheme 7, any carbonylating agent may be used without any particular restrictions. For example, phosgene, diphosgene, triphosgene, diethyl carbonate or 1,1'-carbonyldiimidazole may be mentioned. Further, in Process 12 in Reaction Scheme 5, any thiocarbonylating agent may be used without any particular restrictions. For example, thiophosgene or 1,1 '-thiocarbonytdiimidazote may be used. The carbonylating agent or the thiocarbonylating agent is used usually in an amount of from 0 to 10 eq., preferably from 0 to 2 eq., in relation to 1 eq. of the compound as the reactant.

In Process 19 in Reaction Scheme 8, any silyl-enol-etherifying agent may be used without any particular restrictions. For example, trimethylsilyl trifluoromethanesulfonate, triethylsilyl trifluoromethanesulfonate or triisopropylsilyl trifluoromethanesulfonate may be mentioned.

In Process 19 in Reaction Scheme 8, any brominating agent may be used without any particular restrictions. For example, phenyltrimethylammonium tribromide, tetramethylammonium tribromide, tetrabutylammonium tribromide or benzyltrimethylammonium tribromide may be mentioned.

In Process 21 in Reaction Scheme 9, any reagent may be used without any particular restrictions. For example, a mineral acid such as hydrazine monohydrate, hydrochloric acid, sulfuric acid or nitric acid, a primary amine such as ammonia water or methylamine, or an inorganic base such as sodium hydroxide, potassium hydroxide, sodium carbonate, potassium carbonate, sodium hydrogen carbonate or potassium hydrogen carbonate may be mentioned.

In the cyclization in Process 22 of Reaction Scheme 9, any reagent may be used without any particular restrictions. For example, a Lewis acid such as trimethylalminum, zinc chloride, titanium tetrachloride or a boron trifluoride-ether complex, 1-ethyl-3-(3-dimethylaminopropyl)carbodiimide hydrochloride (WSC), N,N'-dicyclohexylcarbodiimide, N,N'-diisoproplylcarbodiimide, diethyl cyanohydrochloride, carbonyldiimidazole, 4-(4,6-dimethoxy-1,3,5-triazin-2-yl)-4-methylmorpholinium chloride, a chlorocarbonate ester or 2-chloro-1-methylpyridinium iodide may be mentioned.

The thiocarbonylating agent used in Reaction Scheme 10 is not particularly restricted and may be Lawesson's reagent, Yokoyama's reagent, PSCI₃, P₂S₅ or the like. The thiocarbonylating agent is used usually in an amount of from 0 to 10 eq., preferably from 0 to 2 eq., in relation to 1 eq. of the compound as the reactant.

In Reaction Schemes 1 to 10, the reaction temperatures are usually from -90 to 200°C, preferably 0 to 120°C.

In Reaction Schemes 1 to 10, the reaction times are usually from 0.05 to 100 hours, preferably from 0.5 to 10 hours.

The desired product obtained by the above-mentioned reactions may be isolated and purified by filtration, extraction, washing, column chromatography, recrystallization, distillation and other operations.

Next, examples of the compounds covered by the present invention are shown in Tables 2 and 3, but the present invention should not be restricted thereto.

The symbols in the tables have the following meanings, and for example, Ph-2-CI means 2-chlorophenyl.

Me: methyl group, Et: ethyl group, Pr: propyl group, Bu: butyl group, Pen: pentyl group, Hex: hexyl group, Ph: phenyl group.

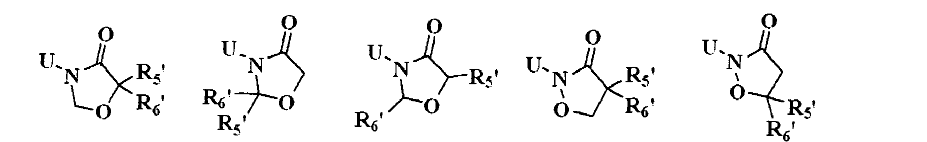

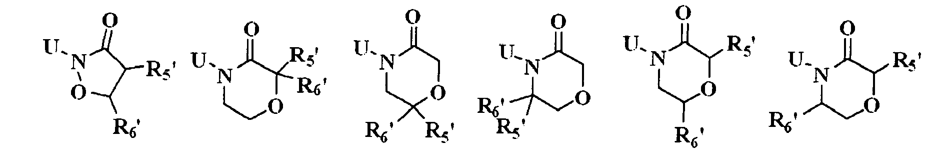

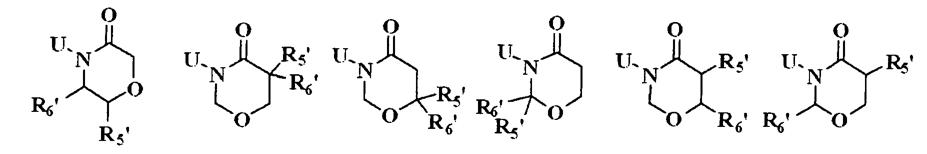

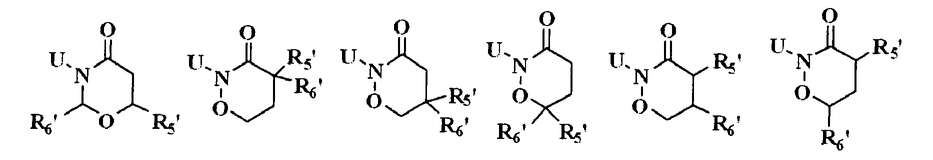

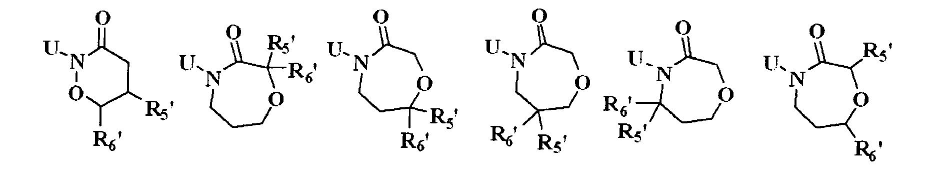

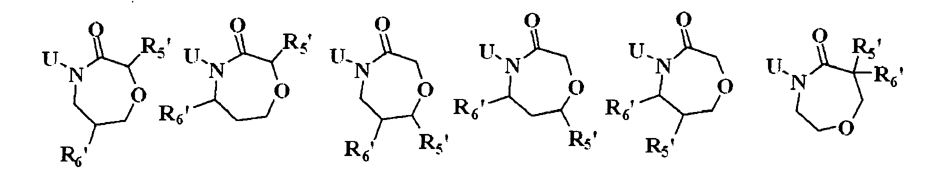

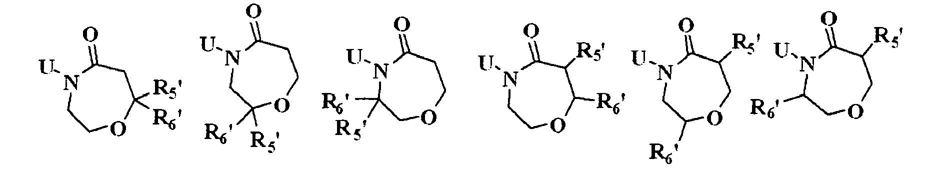

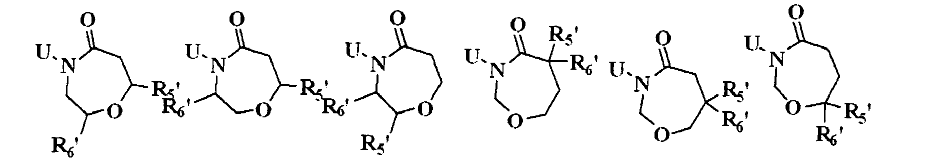

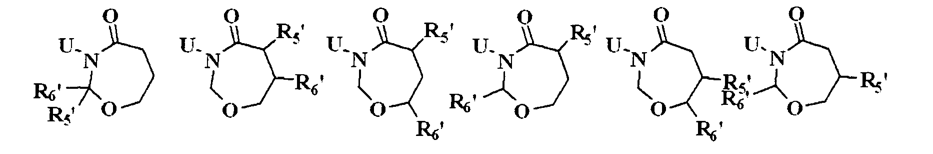

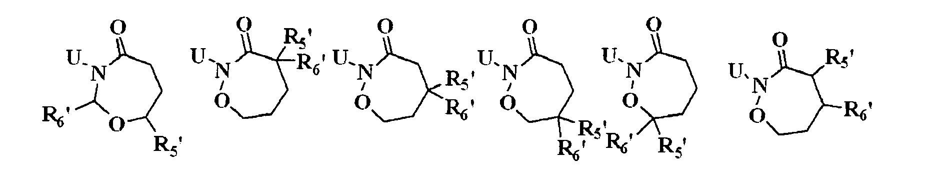

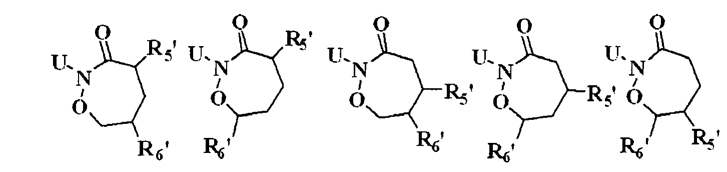

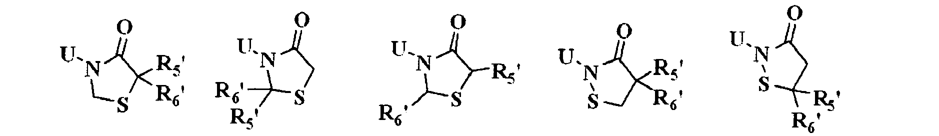

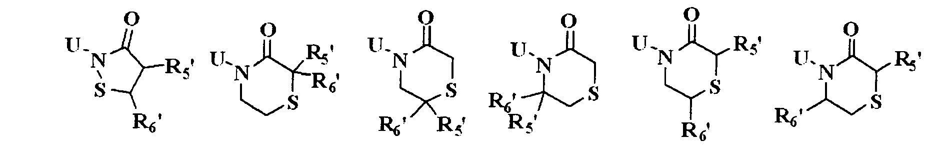

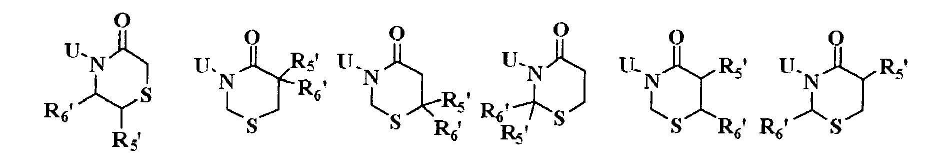

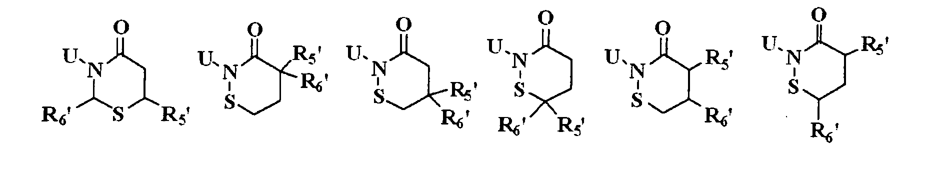

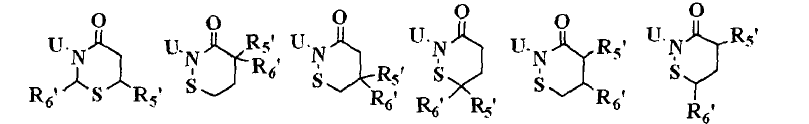

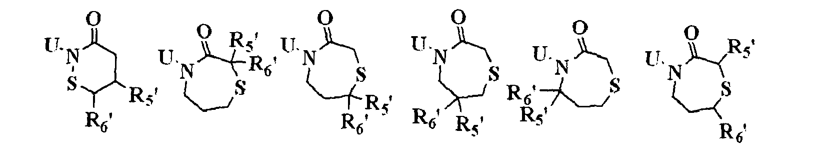

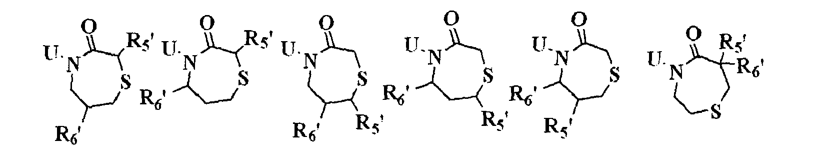

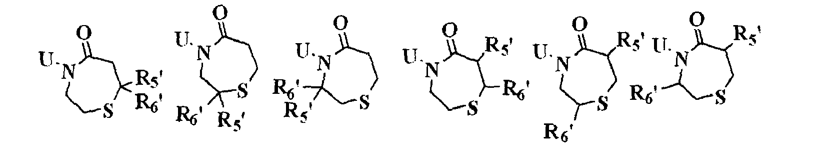

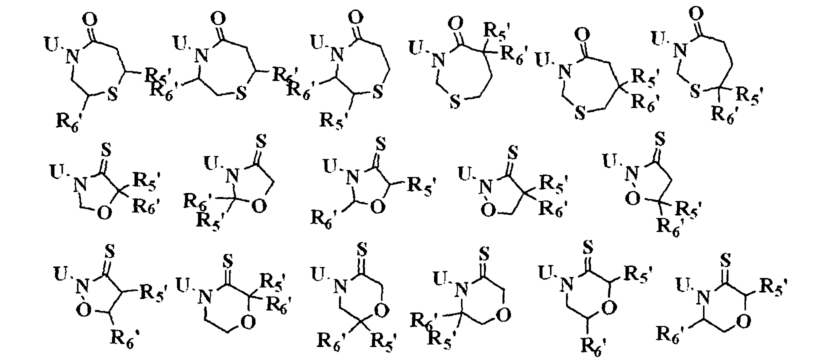

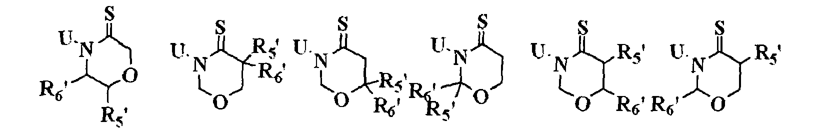

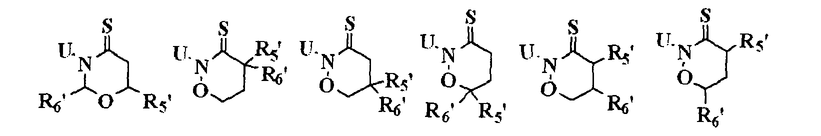

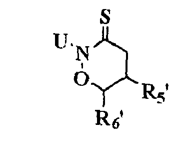

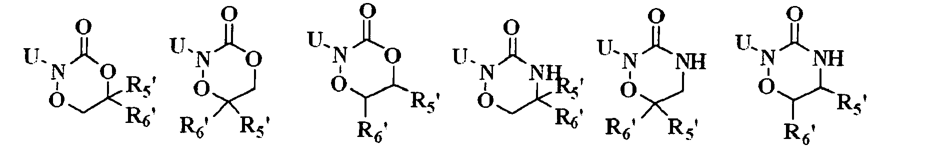

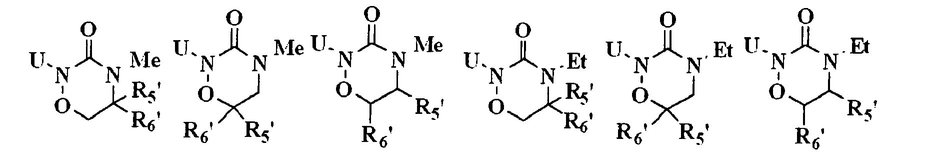

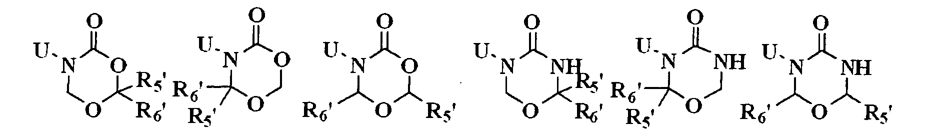

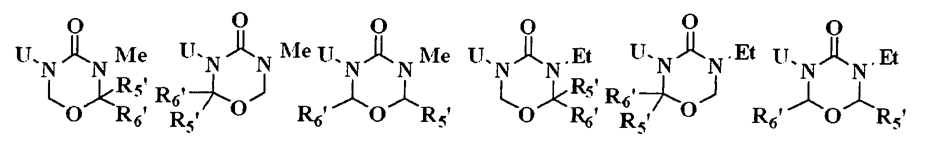

U is represented by the following formula (34).

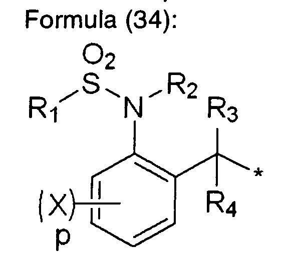

**TABLE 2 R₃=R₄=H,p=0**

| R₁ | R₂ | R₅' | R₆' | X |
|---|---|---|---|---|
| CF₃ | H | H | H | H |
| CF₃ | H | Me | H | H |
| CF₃ | H | Me | Me | H |
| CF₃ | H | Et | H | H |
| CF₃ | H | Et | Me | H |
| CF₃ | H | Pr-n | H | H |
| CF₃ | H | Pr-n | Me | H |
| CF₃ | H | Pr-i | H | H |
| CF₃ | H | Pr-i | Me | H |
| CF₃ | H | Pr-c | H | H |
| CF₃ | H | Pr-c | Me | H |
| CF₃ | H | Bu-n | H | H |
| CF₃ | H | Bu-n | Me | H |
| CF₃ | H | Bu-i | H | H |
| CF₃ | H | Bu-i | Me | H |
| CF₃ | H | Bu-s | H | H |
| CF₃ | H | Bu-s | Me | H |
| CF₃ | H | Bu-t | H | H |
| CF₃ | H | Bu-t | Me | H |
| CF₃ | H | Bu-c | H | H |
| CF₃ | H | Bu-c | Me | H |
| CF₃ | H | Pen-n | H | H |
| CF₃ | H | Pen-n | Me | H |
| CF₃ | H | Pen-c | H | H |
| CF₃ | H | Pen-c | Me | H |
| CF₃ | H | Hex-n | H | H |
| CF₃ | H | Hex-n | Me | H |
| CF₃ | H | Hex-c | H | H |
| CF₃ | H | Hex-c | Me | H |
| CF₃ | H | Cl | H | H |
| CF₃ | H | Cl | Me | H |
| CF₃ | H | Cl | Cl | H |
| CF₃ | H | Br | H | H |
| CF₃ | H | Br | Me | H |
| CF₃ | H | I | H | H |
| CF₃ | H | I | Me | H |
| CF₃ | H | F | H | H |
| CF₃ | H | F | Me | H |
| CF₃ | H | F | F | H |
| CF₃ | H | CH=CH₂ | H | H |
| CF₃ | H | CH=CH₂ | Me | H |
| CF₃ | H | C≡CH | H | H |
| CF₃ | H | C≡CH | Me | H |
| CF₃ | H | CH₂CH=CH₂ | H | H |
| CF₃ | H | CH₂CH=CH₂ | Me | H |
| CF₃ | H | CH₂C≡CH | H | H |
| CF₃ | H | CH₂C≡CH | Me | H |
| CF₃ | H | CH₂Cl | H | H |
| CF₃ | H | CH₂Cl | Me | H |
| CF₃ | H | CHCl₂ | H | H |
| CF₃ | H | CCl₃ | H | H |
| CF₃ | H | CH₂F | H | H |
| CF₃ | H | CHF₂ | H | H |
| CF₃ | H | CF₃ | H | H |
| CF₃ | H | CF₃ | Me | H |
| CF₃ | H | CF₃ | Et | H |
| CF₃ | H | CH₂CH₂Cl | H | H |
| CF₃ | H | CH₂OH | H | H |
| CF₃ | H | CH₂OMe | H | H |
| CF₃ | H | CH₂OMe | Me | H |
| CF₃ | H | CH₂SMe | H | H |
| CF₃ | H | CH₂SCH₂Cl | H | H |
| CF₃ | H | Ph | H | H |
| CF₃ | H | Ph | Me | H |
| CF₃ | H | Ph-2-Cl | H | H |
| CF₃ | H | Ph-2-Cl | Me | H |
| CF₃ | H | Ph-3-Cl | H | H |
| CF₃ | H | Ph-3-Cl | Me | H |
| CF₃ | H | Ph-4-Cl | H | H |
| CF₃ | H | Ph-4-Cl | Me | H |
| CF₃ | H | Ph-3-I | H | H |
| CF₃ | H | Ph-4-F | H | H |
| CF₃ | H | Ph-2-Me | H | H |
| CF₃ | H | Ph-3-OMe | H | H |
| CF₃ | H | Ph-4-SMe | H | H |
| CF₃ | H | Ph-3-CN | H | H |
| CF₃ | H | Ph-4-NO₂ | H | H |
| CF₃ | H | Ph-2-CO₂Me | H | H |
| CF₃ | H | Ph-3-NH₂ | H | H |
| CF₃ | H | Ph-4-Ph | H | H |
| CF₃ | H | Ph-2-OPh | H | H |
| CF₃ | H | Ph-3-SPh | H | H |
| CF₃ | H | Ph-4-CONMe₂ | H | H |
| CF₃ | H | Ph-2-OH | H | H |
| CF₃ | H | COMe | H | H |
| CF₃ | H | COEt | H | H |
| CF₃ | H | COCH₂Cl | H | H |
| CF₃ | H | CO₂H | H | H |
| CF₃ | H | CO₂Me | H | H |
| CF₃ | H | CO₂Me | Me | H |
| CF₃ | H | CO₂Et | H | H |
| CF₃ | H | CO₂Et | Me | H |
| CF₃ | H | CONH₂ | H | H |
| CF₃ | H | CONHMe | H | H |
| CF₃ | H | CONMe₂ | H | H |
| CF₃ | H | CH₂Ph | H | H |
| CF₃ | H | CH₂Ph-2-Cl | H | H |
| CF₃ | H | CH₂Ph-3-Cl | H | H |
| CF₃ | H | CH₂Ph-4-Cl | H | H |
| CF₃ | H | COPh | H | H |
| CF₃ | H | COPh-2-Cl | H | H |
| CF₃ | H | COPh-3-Cl | H | H |
| CF₃ | H | COPh-4-Cl | H | H |
| CF₃ | H | OH | H | H |
| CF₃ | H | OH | Me | H |
| CF₃ | H | OMe | H | H |
| CF₃ | H | OMe | Me | H |
| CF₃ | H | OEt | H | H |
| CF₃ | H | OEt | Me | H |
| CF₃ | H | OPh | H | H |
| CF₃ | H | OPh | Me | H |
| CF₃ | H | OCOMe | H | H |
| CF₃ | H | OCOMe | Me | H |
| CF₃ | H | OCO₂Me | H | H |
| CF₃ | H | OCO₂Me | Me | H |
| CF₃ | H | OCO₂Et | H | H |
| CF₃ | H | OCO₂Et | Me | H |
| CF₃ | H | OCH₂OMe | H | H |
| CF₃ | H | OCH₂OMe | Me | H |
| CF₃ | H | SMe | H | H |
| CF₃ | H | SMe | Me | H |
| CF₃ | H | SPh | H | H |
| CF₃ | H | CN | H | H |
| CF₃ | H | NH₂ | H | H |
| CF₃ | H | NHMe | H | H |
| CF₃ | H | NHMe | Me | H |
| CF₃ | H | NMe₂ | H | H |
| CF₃ | H | NMe₂ | Me | H |
| CF₃ | H | E-5 | H | H |
| CF₃ | H | E-5 | Me | H |
| CF₃ | H | E-6 | H | H |
| CF₃ | H | E-6 | Me | H |
| CF₃ | H | E-7 | H | H |
| CF₃ | H | E-7 | Me | H |
| CF₃ | H | E-8 | H | H |
| CF₃ | H | E-8 | Me | H |
| CF₃ | H | -CH₂-O-CH₂- | | H |
| CF₃ | H | -(CH2)₂-O-(CH₂)₂- | | H |
| CF₃ | H | -(CH₂)₂- | | H |
| CF₃ | H | -(CH₂)₃- | | H |
| CF₃ | H | -(CH₂)₄- | | H |
| CF₃ | H | -(CH₂)₅- | | H |
| CF₃ | H | H | H | 3-F |
| CF₃ | H | H | H | 4-F |
| CF₃ | H | H | H | 5-F |
| CF₃ | H | H | H | 6-F |
| CF₃ | H | H | H | 3-Cl |
| CF₃ | H | H | H | 4-Cl |
| CF₃ | H | H | H | 5-Cl |
| CF₃ | H | H | H | 6-Cl |
| CF₃ | H | H | H | 3-Br |
| CF₃ | H | H | H | 4-Br |
| CF₃ | H | H | H | 5-Br |
| CF₃ | H | H | H | 6-Br |
| CF₃ | H | H | H | 3-I |
| CF₃ | H | H | H | 4-I |
| CF₃ | H | H | H | 5-I |
| CF₃ | H | H | H | 6-I |
| CF₃ | H | H | H | 3-Me |
| CF₃ | H | H | H | 4-Me |
| CF₃ | H | H | H | 5-Me |
| CF₃ | H | H | H | 6-Me |
| CF₃ | H | H | H | 3-OMe |
| CF₃ | H | H | H | 4-OMe |
| CF₃ | H | H | H | 5-OMe |
| CF₃ | H | H | H | 6-OMe |
| CF₃ | H | H | H | 3-SMe |
| CF₃ | H | H | H | 4-SMe |
| CF₃ | H | H | H | 5-SMe |
| CF₃ | H | H | H | 6-SMe |
| CF₃ | H | Me | H | 3-F |
| CF₃ | H | Me | H | 4-F |
| CF₃ | H | Me | H | 5-F |
| CF₃ | H | Me | H | 6-F |
| CF₃ | H | Me | H | 3-Cl |
| CF₃ | H | Me | H | 4-Cl |
| CF₃ | H | Me | H | 5-Cl |
| CF₃ | H | Me | H | 6-Cl |
| CF₃ | H | Me | H | 3-Br |
| CF₃ | H | Me | H | 4-Br |
| CF₃ | H | Me | H | 5-Br |
| CF₃ | H | Me | H | 6-Br |
| CF₃ | H | Me | H | 3-I |
| CF₃ | H | Me | H | 4-I |
| CF₃ | H | Me | H | 5-I |
| CF₃ | H | Me | H | 6-I |
| CF₃ | H | Me | H | 3-Me |
| CF₃ | H | Me | H | 4-Me |
| CF₃ | H | Me | H | 5-Me |
| CF₃ | H | Me | H | 6-Me |
| CF₃ | H | Me | H | 3-OMe |
| CF₃ | H | Me | H | 4-OMe |
| CF₃ | H | Me | H | 5-OMe |
| CF₃ | H | Me | H | 6-OMe |
| CF₃ | H | Me | H | 3-SMe |
| CF₃ | H | Me | H | 4-SMe |
| CF₃ | H | Me | H | 5-SMe |
| CF₃ | H | Me | H | 6-SMe |
| CF₃ | H | Me | Me | 3-F |
| CF₃ | H | Me | Me | 4-F |
| CF₃ | H | Me | Me | 5-F |
| CF₃ | H | Me | Me | 6-F |
| CF₃ | H | Me | Me | 3-Cl |
| CF₃ | H | Me | Me | 4-Cl |
| CF₃ | H | Me | Me | 5-Cl |
| CF₃ | H | Me | Me | 6-Cl |
| CF₃ | H | Me | Me | 3-Br |
| CF₃ | H | Me | Me | 4-Br |
| CF₃ | H | Me | Me | 5-Br |
| CF₃ | H | Me | Me | 6-Br |
| CF₃ | H | Me | Me | 3-I |
| CF₃ | H | Me | Me | 4-I |
| CF₃ | H | Me | Me | 5-I |
| CF₃ | H | Me | Me | 6-I |
| CF₃ | H | Me | Me | 3-Me |
| CF₃ | H | Me | Me | 4-Me |
| CF₃ | H | Me | Me | 5-Me |
| CF₃ | H | Me | Me | 6-Me |
| CF₃ | H | Me | Me | 3 -OMe |
| CF₃ | H | Me | Me | 4 -OMe |
| CF₃ | H | Me | Me | 5 -OMe |
| CF₃ | H | Me | Me | 6 -OMe |
| CF₃ | H | Me | Me | 3 -SMe |
| CF₃ | H | Me | Me | 4 -SMe |
| CF₃ | H | Me | Me | 5 -SMe |
| CF₃ | H | Me | Me | 6 -SMe |
| CF₃ | Me | H | H | H |
| CF₃ | Me | Me | H | H |
| CF₃ | Me | Me | Me | H |
| CF₃ | Et | H | H | H |
| CF₃ | Et | Me | H | H |
| CF₃ | Et | Me | Me | H |
| CF₃ | COMe | H | H | H |
| CF₃ | COMe | Me | H | H |
| CF₃ | COMe | Me | Me | H |
| CF₃ | COMe | Et | H | H |
| CF₃ | COMe | Et | Me | H |
| CF₃ | COMe | Pr-n | H | H |
| CF₃ | COMe | Pr-n | Me | H |
| CF₃ | COMe | Cl | H | H |
| CF₃ | COMe | Cl | Me | H |
| CF₃ | COMe | Br | H | H |
| CF₃ | COMe | Br | Me | H |
| CF₃ | COMe | F | H | H |
| CF₃ | COMe | F | Me | H |
| CF₃ | COMe | CH₂Cl | H | H |
| CF₃ | COMe | CH₂Cl | Me | H |
| CF₃ | COMe | CH₂OMe | H | H |
| CF₃ | COMe | CH₂OMe | Me | H |
| CF₃ | COMe | CF₃ | H | H |
| CF₃ | COMe | CF₃ | Me | H |
| CF₃ | COMe | Ph | H | H |
| CF₃ | COMe | Ph | Me | H |
| CF₃ | COMe | Ph-2-Cl | H | H |
| CF₃ | COMe | Ph-3-Cl | H | H |
| CF₃ | COMe | Ph-4-Cl | H | H |
| CF₃ | COMe | COMe | H | H |
| CF₃ | COMe | CO₂H | H | H |
| CF₃ | COMe | CO₂Me | H | H |
| CF₃ | COMe | CONH₂ | H | H |
| CF₃ | COMe | CONHMe | H | H |
| CF₃ | COMe | CONMe₂ | H | H |
| CF₃ | COMe | OH | H | H |
| CF₃ | COMe | OH | Me | H |
| CF₃ | COMe | OMe | H | H |
| CF₃ | COMe | OMe | Me | H |
| CF₃ | COMe | OPh | H | H |
| CF₃ | COMe | OPh | Me | H |
| CF₃ | COMe | SMe | H | H |
| CF₃ | COMe | SMe | Me | H |
| CF₃ | COMe | NHMe | H | H |
| CF₃ | COMe | NHMe | Me | H |
| CF₃ | COMe | CH₂Ph | H | H |
| CF₃ | COMe | CH₂Ph-2-Cl | H | H |
| CF₃ | COMe | CH₂Ph-3-Cl | H | H |
| CF₃ | COMe | CH₂Ph-4-Cl | H | H |
| CF₃ | COMe | COPh | H | H |
| CF₃ | COMe | COPh-2-Cl | H | H |
| CF₃ | COMe | COPh-3-Cl | H | H |
| CF₃ | COMe | COPh-4-Cl | H | H |
| CF₃ | COEt | H | H | H |
| CF₃ | COEt | Me | H | H |
| CF₃ | COEt | Me | Me | H |
| CF₃ | COEt | Et | H | H |
| CF₃ | COEt | Et | Me | H |
| CF₃ | COEt | Pr-n | H | H |
| CF₃ | COEt | Pr-n | Me | H |
| CF₃ | COEt | Pr-i | H | H |
| CF₃ | COEt | Pr-i | Me | H |
| CF₃ | COEt | Pr-c | H | H |
| CF₃ | COEt | Pr-c | Me | H |
| CF₃ | COEt | Bu-n | H | H |
| CF₃ | COEt | Bu-n | Me | H |
| CF₃ | COEt | Bu-i | H | H |
| CF₃ | COEt | Bu-i | Me | H |
| CF₃ | COEt | Bu-s | H | H |
| CF₃ | COEt | Bu-s | Me | H |
| CF₃ | COEt | Bu-t | H | H |
| CF₃ | COEt | Bu-t | Me | H |
| CF₃ | COEt | Bu-c | H | H |
| CF₃ | COEt | Bu-c | Me | H |
| CF₃ | COEt | Pen-n | H | H |
| CF₃ | COEt | Pen-n | Me | H |
| CF₃ | COEt | Pen-c | H | H |
| CF₃ | COEt | Pen-c | Me | H |
| CF₃ | COEt | Hex-n | H | H |
| CF₃ | COEt | Hex-n | Me | H |
| CF₃ | COEt | Hex-c | H | H |
| CF₃ | COEt | Hex-c | Me | H |
| CF₃ | COEt | Cl | H | H |
| CF₃ | COEt | Cl | Me | H |
| CF₃ | COEt | Cl | Cl | H |
| CF₃ | COEt | Br | H | H |
| CF₃ | COEt | Br | Me | H |
| CF₃ | COEt | I | H | H |
| CF₃ | COEt | I | Me | H |
| CF₃ | COEt | F | H | H |
| CF₃ | COEt | F | Me | H |
| CF₃ | COEt | F | F | H |
| CF₃ | COEt | CH=CH₂ | H | H |
| CF₃ | COEt | CH=CH₂ | Me | H |
| CF₃ | COEt | C≡CH | H | H |
| CF₃ | COEt | C≡CH | Me | H |
| CF₃ | COEt | CH₂CH₌CH₂ | H | H |
| CF₃ | COEt | CH₂CH=CH₂ | Me | H |
| CF₃ | COEt | CH₂C≡CH | H | H |
| CF₃ | COEt | CH₂C≡CH | Me | H |
| CF₃ | COEt | CH₂Cl | H | H |
| CF₃ | COEt | CH₂Cl | Me | H |
| CF₃ | COEt | CHCl₂ | H | H |
| CF₃ | COEt | CCl₃ | H | H |
| CF₃ | COEt | CH₂F | H | H |
| CF₃ | COEt | CHF₂ | H | H |
| CF₃ | COEt | CF₃ | H | H |
| CF₃ | COEt | CF₃ | Me | H |
| CF₃ | COEt | CF₃ | Et | H |
| CF₃ | COEt | CH₂CH₂Cl | H | H |
| CF₃ | COEt | CH₂OH | H | H |
| CF₃ | COEt | CH₂OMe | H | H |
| CF₃ | COEt | CH₂OMe | Me | H |
| CF₃ | COEt | CH₂SMe | H | H |
| CF₃ | COEt | CH₂SCH₂Cl | H | H |
| CF₃ | COEt | Ph | H | H |
| CF₃ | COEt | Ph | Me | H |
| CF₃ | COEt | Ph-2-Cl | H | H |
| CF₃ | COEt | Ph-2-Cl | Me | H |
| CF₃ | COEt | Ph-3-Cl | H | H |
| CF₃ | COEt | Ph-3-Cl | Me | H |
| CF₃ | COEt | Ph-4-Cl | H | H |
| CF₃ | COEt | Ph-4-Cl | Me | H |
| CF₃ | COEt | Ph-2-Br | H | H |
| CF₃ | COEt | Ph-3-I | H | H |
| CF₃ | COEt | Ph-4-F | H | H |
| CF₃ | COEt | Ph-2-Me | H | H |
| CF₃ | COEt | Ph-3-OMe | H | H |
| CF₃ | COEt | Ph-4-SMe | H | H |
| CF₃ | COEt | Ph-2-SO₂Me | H | H |
| CF₃ | COEt | Ph-3-CN | H | H |
| CF₃ | COEt | Ph-4-NO₂ | H | H |
| CF₃ | COEt | Ph-2-CO₂Me | H | H |
| CF₃ | COEt | Ph-3-NH₂ | H | H |
| CF₃ | COEt | Ph-4-Ph | H | H |
| CF₃ | COEt | Ph-2-OPh | H | H |
| CF₃ | COEt | Ph-3-SPh | H | H |
| CF₃ | COEt | Ph-4-CONMe₂ | H | H |
| CF₃ | COEt | Ph-2-OH | H | H |
| CF₃ | COEt | COMe | H | H |
| CF₃ | COEt | COEt | H | H |
| CF₃ | COEt | COCH₂Cl | H | H |
| CF₃ | COEt | CO₂H | H | H |
| CF₃ | COEt | CO₂Me | H | H |
| CF₃ | COEt | CO₂Me | Me | H |
| CF₃ | COEt | CO₂Et | H | H |
| CF₃ | COEt | CO₂Et | Me | H |
| CF₃ | COEt | CONH₂ | H | H |
| CF₃ | COEt | CONHMe | H | H |
| CF₃ | COEt | CONMe₂ | H | H |
| CF₃ | COEt | CH₂Ph | H | H |
| CF₃ | COEt | CH₂Ph-2-Cl | H | H |
| CF₃ | COEt | CH₂Ph-3-Cl | H | H |
| CF₃ | COEt | CH₂Ph-4-Cl | H | H |
| CF₃ | COEt | COPh | H | H |
| CF₃ | COEt | COPh-2-Cl | H | H |
| CF₃ | COEt | COPh-3-Cl | H | H |
| CF₃ | COEt | COPh-4-Cl | H | H |
| CF₃ | COEt | OH | H | H |
| CF₃ | COEt | OH | Me | H |
| CF₃ | COEt | OMe | H | H |
| CF₃ | COEt | OMe | Me | H |
| CF₃ | COEt | OEt | H | H |
| CF₃ | COEt | OEt | Me | H |
| CF₃ | COEt | OPh | H | H |
| CF₃ | COEt | OPh | Me | H |
| CF₃ | COEt | OCOMe | H | H |
| CF₃ | COEt | OCOMe | Me | H |
| CF₃ | COEt | OCO₂Me | H | H |
| CF₃ | COEt | OCO₂Me | Me | H |
| CF₃ | COEt | OCO₂Et | H | H |
| CF₃ | COEt | OCO₂Et | Me | H |
| CF₃ | COEt | OCH₂OMe | H | H |
| CF₃ | COEt | OCH₂OMe | Me | H |
| CF₃ | COEt | SMe | H | H |
| CF₃ | COEt | SMe | Me | H |
| CF₃ | COEt | SPh | H | H |
| CF₃ | COEt | CN | H | H |
| CF₃ | COEt | NH₂ | H | H |
| CF₃ | COEt | NHMe | H | H |
| CF₃ | COEt | NHMe | Me | H |
| CF₃ | COEt | NMe₂ | H | H |
| CF₃ | COEt | NMe₂ | Me | H |
| CF₃ | COEt | E-5 | H | H |
| CF₃ | COEt | E-5 | Me | H |
| CF₃ | COEt | E-6 | H | H |
| CF₃ | COEt | E-6 | Me | H |
| CF₃ | COEt | E-7 | H | H |
| CF₃ | COEt | E-7 | Me | H |
| CF₃ | COEt | E-8 | H | H |
| CF₃ | COEt | E-8 | Me | H |
| CF₃ | COEt | -CH₂-O-CH₂- | | H |
| CF₃ | COEt | - (CH₂)₂-O- (CH₂)₂- | | H |
| CF₃ | COEt | - (CH₂)₂- | | H |
| CF₃ | COEt | - (CH₂)₃- | | H |
| CF₃ | COEt | -(CH₂)₄- | | H |
| CF₃ | COEt | -(CH₂)₅- | | H |
| CF₃ | COPr-n | H | H | H |
| CF₃ | COPr-n | Me | H | H |
| CF₃ | COPr-n | Me | Me | H |
| CF₃ | COPr-n | Et | H | H |
| CF₃ | COPr-n | Et | Me | H |
| CF₃ | COPr-n | Pr-n | H | H |
| CF₃ | COPr-n | Pr-n | Me | H |
| CF₃ | COPr-n | Cl | H | H |
| CF₃ | COPr-n | Cl | Me | H |
| CF₃ | COPr-n | Br | H | H |
| CF₃ | COPr-n | Br | Me | H |
| CF₃ | COPr-n | F | H | H |
| CF₃ | COPr-n | F | Me | H |
| CF₃ | COPr-n | CH₂Cl | H | H |
| CF₃ | COPr-n | CH₂Cl | Me | H |
| CF₃ | COPr-n | CH₂OMe | H | H |
| CF₃ | COPr-n | CH₂OMe | Me | H |
| CF₃ | COPr-n | CF₃ | H | H |
| CF₃ | COPr-n | CF₃ | Me | H |
| CF₃ | COPr-n | Ph | H | H |
| CF₃ | COPr-n | Ph | Me | H |
| CF₃ | COPr-n | Ph-2-Cl | H | H |
| CF₃ | COPr-n | Ph-3-Cl | H | H |
| CF₃ | COPr-n | Ph-4-Cl | H | H |
| C_{F}3 | COPr-n | COMe | H | H |
| CF₃ | COPr-n | CO₂H | H | H |
| CF₃ | COPr-n | CO₂Me | H | H |
| CF₃ | COPr-n | CONH₂ | H | H |
| CF₃ | COPr-n | CONHMe | H | H |
| CF₃ | COPr-n | CONMe₂ | H | H |
| CF₃ | COPr-n | OH | H | H |
| CF₃ | COPr-n | OH | Me | H |
| CF₃ | COPr-n | OMe | H | H |
| CF₃ | COPr-n | OMe | Me | H |
| CF₃ | COPr-n | OPh | H | H |
| CF₃ | COPr-n | OPh | Me | H |
| CF₃ | COPr-n | SMe | H | H |
| CF₃ | COPr-n | SMe | Me | H |
| CF₃ | COPr-n | NHMe | H | H |
| CF₃ | COPr-n | NHMe | Me | H |
| CF₃ | COPr-n | CH₂Ph | H | H |
| CF₃ | COPr-n | CH₂Ph-2-Cl | H | H |
| CF₃ | COPr-n | CH₂Ph-3-Cl | H | H |
| CF₃ | COPr-n | CH₂Ph-4-Cl | H | H |
| CF₃ | COPr-n | COPh | H | H |
| CF₃ | COPr-n | COPh-2-Cl | H | H |
| CF₃ | COPr-n | COPh-3-Cl | H | H |
| CF₃ | COPr-n | COPh-4-Cl | H | H |
| CF₃ | COPr-i | H | H | H |
| CF₃ | COPr-i | Me | H | H |
| CF₃ | COPr-i | Me | Me | H |
| CF₃ | COPr-i | Et | H | H |
| CF₃ | COPr-i | Et | Me | H |
| CF₃ | COPr-i | Pr-n | H | H |
| CF₃ | COPr-i | Pr-n | Me | H |
| CF₃ | COPr-i | Cl | H | H |
| CF₃ | COPr-i | Cl | Me | H |
| CF₃ | COPr-i | Br | H | H |
| CF₃ | COPr-i | Br | Me | H |
| CF₃ | COPr-i | F | H | H |
| CF₃ | COPr-i | F | Me | H |
| CF₃ | COPr-i | CH₂Cl | H | H |
| CF₃ | COPr-i | CH₂Cl | Me | H |
| CF₃ | COPr-i | CH₂OMe | H | H |
| CF₃ | COPr-i | CH₂OMe | Me | H |
| CF₃ | COPr-i | CF₃ | H | H |
| CF₃ | COPr-i | CF₃ | Me | H |
| CF₃ | COPr-i | Ph | H | H |
| CF₃ | COPr-i | Ph | Me | H |
| CF₃ | COPr-i | Ph-2-Cl | H | H |
| CF₃ | COPr-i | Ph-3-Cl | H | H |
| CF₃ | COPr-i | Ph-4-Cl | H | H |
| CF₃ | COPr-i | COMe | H | H |
| CF₃ | COPr-i | CO₂H | H | H |
| CF₃ | COPr-i | CO₂Me | H | H |
| CF₃ | COPr-i | CONH₂ | H | H |
| CF₃ | COPr-i | CONHMe | H | H |
| CF₃ | COPr-i | CONMe₂ | H | H |
| CF₃ | COPr-i | OH | H | H |
| CF₃ | COPr-i | OH | Me | H |
| CF₃ | COPr-i | OMe | H | H |
| CF₃ | COPr-i | OMe | Me | H |
| CF₃ | COPr-i | OPh | H | H |
| CF₃ | COPr-i | OPh | Me | H |
| CF₃ | COPr-i | SMe | H | H |
| CF₃ | COPr-i | SMe | Me | H |
| CF₃ | COPr-i | NHMe | H | H |
| CF₃ | COPr-i | NHMe | Me | H |
| CF₃ | COPr-i | CH₂Ph | H | H |
| CF₃ | COPr-i | CH₂Ph-2-Cl | H | H |
| CF₃ | COPr-i | CH₂Ph-3-Cl | H | H |
| CF₃ | COPr-i | CH₂Ph-4-Cl | H | H |
| CF₃ | COPr-i | COPh | H | H |
| CF₃ | COPr-i | COPh-2-Cl | H | H |
| CF₃ | COPr-i | COPh-3-Cl | H | H |
| CF₃ | COPr-i | COPh-4-Cl | H | H |
| CF₃ | COPr-c | H | H | H |
| CF₃ | COPr-c | Me | H | H |
| CF₃ | COPr-c | Me | Me | H |
| CF₃ | COPr-c | Et | H | H |
| CF₃ | COPr-c | Et | Me | H |
| CF₃ | COPr-c | Pr-n | H | H |
| CF₃ | COPr-c | Pr-n | Me | H |
| CF₃ | COPr-c | Cl | H | H |
| CF₃ | COPr-c | Cl | Me | H |
| CF₃ | COPr-c | Br | H | H |
| CF₃ | COPr-c | Br | Me | H |
| CF₃ | COPr-c | F | H | H |
| CF₃ | COPr-c | F | Me | H |
| CF₃ | COPr-c | CH₂Cl | H | H |
| CF₃ | COPr-c | CH₂Cl | Me | H |
| CF₃ | COPr-c | CH₂OMe | H | H |
| CF₃ | COPr-c | CH₂OMe | Me | H |
| CF₃ | COPr-c | CF₃ | H | H |
| CF₃ | COPr-c | CF₃ | Me | H |
| CF₃ | COPr-c | Ph | H | H |
| CF₃ | COPr-c | Ph | Me | H |
| CF₃ | COPr-c | Ph-2-Cl | H | H |
| CF₃ | COPr-c | Ph-3-Cl | H | H |
| CF₃ | COPr-c | Ph-4-Cl | H | H |
| CF₃ | COPr-c | COMe | H | H |
| CF₃ | COPr-c | CO₂H | H | H |
| CF₃ | COPr-c | CO₂Me | H | H |
| CF₃ | COPr-c | CONH₂ | H | H |
| CF₃ | COPr-c | CONHMe | H | H |
| CF₃ | COPr-c | CONMe₂ | H | H |
| CF₃ | COPr-c | OH | H | H |
| CF₃ | COPr-c | OH | Me | H |
| CF₃ | COPr-c | OMe | H | H |
| CF₃ | COPr-c | OMe | Me | H |
| CF₃ | COPr-c | OPh | H | H |
| CF₃ | COPr-c | OPh | Me | H |
| CF₃ | COPr-c | SMe | H | H |
| CF₃ | COPr-c | SMe | Me | H |
| CF₃ | COPr-c | NHMe | H | H |
| CF₃ | COPr-c | NHMe | Me | H |
| CF₃ | COPr-c | CH₂Ph | H | H |
| CF₃ | COPr-c | CH₂Ph-2-Cl | H | H |
| CF₃ | COPr-c | CH₂Ph-3-Cl | H | H |
| CF₃ | COPr-c | CH₂Ph-4-Cl | H | H |
| CF₃ | COPr-c | COPh | H | H |
| CF₃ | COPr-c | COPh-2-Cl | H | H |
| CF₃ | COPr-c | COPh-3-Cl | H | H |
| CF₃ | COPr-c | COPh-4-Cl | H | H |
| CF₃ | COBu-t | H | H | H |
| CF₃ | COBu-t | Me | H | H |
| CF₃ | COBu-t | Me | Me | H |
| CF₃ | COBu-t | Et | H | H |
| CF₃ | COBu-t | Et | Me | H |
| CF₃ | COBu-t | Pr-n | H | H |
| CF₃ | COBu-t | Pr-n | Me | H |
| CF₃ | COBu-t | Cl | H | H |
| CF₃ | COBu-t | Cl | Me | H |
| CF₃ | COBu-t | Br | H | H |
| CF₃ | COBu-t | Br | Me | H |
| CF₃ | COBu-t | F | H | H |
| CF₃ | COBu-t | F | Me | H |
| CF₃ | COBu-t | CH₂Cl | H | H |
| CF₃ | COBu-t | CH₂Cl | Me | H |
| CF₃ | COBu-t | CH₂OMe | H | H |
| CF₃ | COBu-t | CH₂OMe | Me | H |
| CF₃ | COBu-t | CF₃ | H | H |
| CF₃ | COBu-t | CF₃ | Me | H |
| CF₃ | COBu-t | Ph | H | H |
| CF₃ | COBu-t | Ph | Me | H |
| CF₃ | COBu-t | Ph-2-Cl | H | H |
| CF₃ | COBu-t | Ph-3-Cl | H | H |
| CF₃ | COBu-t | Ph-4-Cl | H | H |
| CF₃ | COBu-t | COMe | H | H |
| CF₃ | COBu-t | CO₂H | H | H |
| CF₃ | COBu-t | CO₂Me | H | H |
| CF₃ | COBu-t | CONH₂ | H | H |
| CF₃ | COBu-t | CONHMe | H | H |
| CF₃ | COBu-t | CONMe₂ | H | H |
| CF₃ | COBu-t | OH | H | H |
| CF₃ | COBu-t | OH | Me | H |
| CF₃ | COBu-t | OMe | H | H |
| CF₃ | COBu-t | OMe | Me | H |
| CF₃ | COBu-t | OPh | H | H |
| CF₃ | COBu-t | OPh | Me | H |
| CF₃ | COBu-t | SMe | H | H |
| CF₃ | COBu-t | SMe | Me | H |
| CF₃ | COBu-t | NHMe | H | H |
| CF₃ | COBu-t | NHMe | Me | H |
| CF₃ | COBu-t | CH₂Ph | H | H |
| CF₃ | COBu-t | CH₂Ph-2-Cl | H | H |
| CF₃ | COBu-t | CH₂Ph-3-Cl | H | H |
| CF₃ | COBu-t | CH2Ph-4-Cl | H | H |
| CF₃ | COBu-t | COPh | H | H |
| CF₃ | COBu-t | COPh-2-Cl | H | H |
| CF₃ | COBu-t | COPh-3-Cl | H | H |
| CF₃ | COBu-t | COPh-4-Cl | H | H |
| CF₃ | COPh | H | H | H |
| CF₃ | COPh | Me | H | H |
| CF₃ | COPh | Me | Me | H |
| CF₃ | COPh | E t | H | H |
| CF₃ | COPh | E t | Me | H |
| CF₃ | COPh | Pr-n | H | H |
| CF₃ | COPh | Pr-n | Me | H |
| CF₃ | COPh | Cl | H | H |
| CF₃ | COPh | Cl | Me | H |
| CF₃ | COPh | Br | H | H |
| CF₃ | COPh | Br | Me | H |
| CF₃ | COPh | F | H | H |
| CF₃ | COPh | F | Me | H |
| CF₃ | COPh | CH₂Cl | H | H |
| CF₃ | COPh | CH₂Cl | Me | H |
| CF₃ | COPh | CH₂OMe | H | H |
| CF₃ | COPh | CH₂OMe | Me | H |
| CF₃ | COPh | CF₃ | H | H |
| CF₃ | COPh | CF₃ | Me | H |
| CF₃ | COPh | Ph | H | H |
| CF₃ | COPh | Ph | Me | H |
| CF₃ | COPh | Ph-2-Cl | H | H |
| CF₃ | COPh | Ph-3-Cl | H | H |
| CF₃ | COPh | Ph-4-Cl | H | H |
| CF₃ | COPh | COMe | H | H |
| CF₃ | COPh | CO₂H | H | H |
| CF₃ | COPh | CO₂Me | H | H |
| CF₃ | COPh | CONH₂ | H | H |
| CF₃ | COPh | CONHMe | H | H |
| CF₃ | COPh | CONMe₂ | H | H |
| CF₃ | COPh | OH | H | H |
| CF₃ | COPh | OH | Me | H |
| CF₃ | COPh | OMe | H | H |
| CF₃ | COPh | OMe | Me | H |
| CF₃ | COPh | OPh | H | H |
| CF₃ | COPh | OPh | Me | H |
| CF₃ | COPh | SMe | H | H |
| CF₃ | COPh | SMe | Me | H |
| CF₃ | COPh | NHMe | H | H |
| CF₃ | COPh | NHMe | Me | H |
| CF₃ | COPh | CH₂Ph | H | H |
| CF₃ | COPh | CH₂Ph-2-Cl | H | H |
| CF₃ | COPh | CH₂Ph-3-Cl | H | H |
| CF₃ | COPh | CH₂Ph-4-Cl | H | H |
| CF₃ | COPh | COPh | H | H |
| CF₃ | COPh | COPh-2-Cl | H | H |
| CF₃ | COPh | COPh-3-Cl | H | H |
| CF₃ | COPh | COPh-4-Cl | H | H |
| CF₃ | CO₂Me | H | H | H |
| CF₃ | CO₂Me | Me | H | H |
| CF₃ | CO₂Me | Me | Me | H |
| CF₃ | CO₂Me | Et | H | H |
| CF₃ | CO₂Me | Et | Me | H |
| CF₃ | CO₂Me | Pr-n | H | H |
| CF₃ | CO₂Me | Pr-n | Me | H |
| CF₃ | CO₂Me | Cl | H | H |
| CF₃ | CO₂Me | Cl | Me | H |
| CF₃ | CO₂Me | Br | H | H |
| CF₃ | CO₂Me | Br | Me | H |
| CF₃ | CO₂Me | F | H | H |
| CF₃ | CO₂Me | F | Me | H |
| CF₃ | CO₂Me | CH₂Cl | H | H |
| CF₃ | CO₂Me | CH₂Cl | Me | H |
| CF₃ | CO₂Me | CH₂OMe | H | H |
| CF₃ | CO₂Me | CH₂OMe | Me | H |
| CF₃ | CO₂Me | CF₃ | H | H |
| CF₃ | CO₂Me | CF₃ | Me | H |
| CF₃ | CO₂Me | Ph | H | H |
| CF₃ | CO₂Me | Ph | Me | H |
| CF₃ | CO₂Me | Ph-2-Cl | H | H |
| CF₃ | CO₂Me | Ph-3-Cl | H | H |
| CF₃ | CO₂Me | Ph-4-Cl | H | H |
| CF₃ | CO₂Me | COMe | H | H |
| CF₃ | CO₂Me | CO₂H | H | H |
| CF₃ | CO₂Me | CO₂Me | H | H |
| CF₃ | CO₂Me | CONH₂ | H | H |
| CF₃ | CO₂Me | CONHMe | H | H |
| CF₃ | CO₂Me | CONMe₂ | H | H |
| CF₃ | CO₂Me | OH | H | H |
| CF₃ | CO₂Me | OH | Me | H |
| CF₃ | CO₂Me | OMe | H | H |
| CF₃ | CO₂Me | OMe | Me | H |
| CF₃ | CO₂Me | OPh | H | H |
| CF₃ | CO₂Me | OPh | Me | H |
| CF₃ | CO₂Me | SMe | H | H |
| CF₃ | CO₂Me | SMe | Me | H |
| CF₃ | CO₂Me | NHMe | H | H |
| CF₃ | CO₂Me | NHMe | Me | H |
| CF₃ | CO₂Me | CH₂Ph | H | H |
| CF₃ | CO₂Me | CH₂Ph-2-Cl | H | H |
| CF₃ | CO₂Me | CH₂Ph-3-Cl | H | H |
| CF₃ | CO₂Me | CH₂Ph-4-Cl | H | H |
| CF₃ | CO₂Me | COPh | H | H |
| CF₃ | CO₂Me | COPh-2-Cl | H | H |
| CF₃ | CO₂Me | COPh-3-Cl | H | H |
| CF₃ | CO₂Me | COPh-4-Cl | H | H |
| CF₃ | CO₂Et | H | H | H |
| CF₃ | CO₂Et | Me | H | H |
| CF₃ | CO₂Et | Me | Me | H |
| CF₃ | CO₂Et | Et | H | H |
| CF₃ | CO₂Et | Et | Me | H |
| CF₃ | CO₂Et | Pr-n | H | H |
| CF₃ | CO₂Et | Pr-n | Me | H |
| CF₃ | CO₂Et | Pr-i | H | H |
| CF₃ | CO₂Et | Pr-i | Me | H |
| CF₃ | CO₂Et | Pr-c | H | H |
| CF₃ | CO₂Et | Pr-c | Me | H |
| CF₃ | CO₂Et | Bu-n | H | H |
| CF₃ | CO₂Et | Bu-n | Me | H |
| CF₃ | CO₂Et | Bu-i | H | H |
| CF₃ | CO₂Et | Bu-i | Me | H |
| CF₃ | CO₂Et | Bu-s | H | H |
| CF₃ | CO₂Et | Bu-s | Me | H |
| CF₃ | CO₂Et | Bu-t | H | H |
| CF₃ | CO₂Et | Bu-t | Me | H |
| CF₃ | CO₂Et | Bu-c | H | H |
| CF₃ | CO₂Et | Bu-c | Me | H |
| CF₃ | CO₂Et | Pen-n | H | H |
| CF₃ | CO₂Et | Pen-n | Me | H |
| CF₃ | CO₂Et | Pen-c | H | H |
| CF₃ | CO₂Et | Pen-c | Me | H |
| CF₃ | CO₂Et | Hex-n | H | H |
| CF₃ | CO₂Et | Hex-n | Me | H |
| CF₃ | CO₂Et | Hex-c | H | H |
| CF₃ | CO₂Et | Hex-c | Me | H |
| CF₃ | CO₂Et | Cl | H | H |
| CF₃ | CO₂Et | Cl | Me | H |
| CF₃ | CO₂Et | Cl | Cl | H |
| CF₃ | CO₂Et | Br | H | H |
| CF₃ | CO₂Et | Br | Me | H |
| CF₃ | CO₂Et | I | H | H |
| CF₃ | CO₂Et | I | Me | H |
| CF₃ | CO₂Et | F | H | H |
| CF₃ | CO₂Et | F | Me | H |
| CF₃ | CO₂Et | F | F | H |
| CF₃ | CO₂Et | CH=CH₂ | H | H |
| CF₃ | CO₂Et | CH=CH₂ | Me | H |
| CF₃ | CO₂Et | C≡CH | H | H |
| CF₃ | CO₂Et | C≡CH | Me | H |
| CF₃ | CO₂Et | CH₂CH=CH₂ | H | H |
| CF₃ | CO₂Et | CH₂CH=CH₂ | Me | H |
| CF₃ | CO₂Et | CH₂C=CH | H | H |
| CF₃ | CO₂Et | CH₂C≡CH | Me | H |
| CF₃ | CO₂Et | CH₂Cl | H | H |
| CF₃ | CO₂Et | CH₂Cl | Me | H |
| CF₃ | CO₂Et | CHCl₂ | H | H |
| CF₃ | CO₂Et | CCl₃ | H | H |
| CF₃ | CO₂Et | CH₂F | H | H |
| CF₃ | CO₂Et | CHF₂ | H | H |
| CF₃ | CO₂Et | CF₃ | H | H |
| CF₃ | CO₂Et | CF₃ | Me | H |
| CF₃ | CO₂Et | CF₃ | Et | H |
| CF₃ | CO₂Et | CH₂CH₂Cl | H | H |
| CF₃ | CO₂Et | CH₂OH | H | H |
| CF₃ | CO₂Et | CH₂OMe | H | H |
| CF₃ | CO₂Et | CH₂OMe | Me | H |
| CF₃ | CO₂Et | CH₂SMe | H | H |
| CF₃ | CO₂Et | CH₂SCH₂Cl | H | H |
| CF₃ | CO₂Et | Ph | H | H |
| CF₃ | CO₂Et | Ph | Me | H |
| CF₃ | CO₂Et | Ph-2-Cl | H | H |
| CF₃ | CO₂Et | Ph-3-Cl | H | H |
| CF₃ | CO₂Et | Ph-4-Cl | H | H |
| CF₃ | CO₂Et | Ph-2-Br | H | H |
| CF₃ | CO₂Et | Ph-3-I | H | H |
| CF₃ | CO₂Et | Ph-4-F | H | H |
| CF₃ | CO₂Et | Ph-2-Me | H | H |
| CF₃ | CO₂Et | Ph-3-OMe | H | H |
| CF₃ | CO₂Et | Ph-4-SMe | H | H |
| CF₃ | CO₂Et | Ph-2-SO₂Me | H | H |
| CF₃ | CO₂Et | Ph-3-CN | H | H |
| CF₃ | CO₂Et | Ph-4-NO₂ | H | H |
| CF₃ | CO₂Et | Ph-2-CO₂Me | H | H |
| CF₃ | CO₂Et | Ph-3-NH₂ | H | H |
| CF₃ | CO₂Et | Ph-4-Ph | H | H |
| CF₃ | CO₂Et | Ph-2-OPh | H | H |
| CF₃ | CO₂Et | Ph-3-SPh | H | H |
| CF₃ | CO₂Et | Ph-4-CONMe₂ | H | H |
| CF₃ | CO₂Et | Ph-2-OH | H | H |
| CF₃ | CO₂Et | COMe | H | H |
| CF₃ | CO₂Et | COEt | H | H |
| CF₃ | CO₂Et | COCH₂Cl | H | H |
| CF₃ | CO₂Et | CO₂H | H | H |
| CF₃ | CO₂Et | CO₂Me | H | H |
| CF₃ | CO₂Et | CO₂Me | Me | H |
| CF₃ | CO₂Et | CO₂Et | H | H |
| CF₃ | CO₂Et | CO₂Bu-i | H | H |
| CF₃ | CO₂Et | CONH₂ | H | H |
| CF₃ | CO₂Et | CONHMe | H | H |
| CF₃ | CO₂Et | CONMe₂ | H | H |
| CF₃ | CO₂Et | CH₂Ph | H | H |
| CF₃ | CO₂Et | CH₂Ph-2-Cl | H | H |
| CF₃ | CO₂Et | CH₂Ph-3-Cl | H | H |
| CF₃ | CO₂Et | CH₂Ph-4-Cl | H | H |
| CF₃ | CO₂Et | COPh | H | H |
| CF₃ | CO₂Et | COPh-2-Cl | H | H |
| CF₃ | CO₂Et | COPh-3-Cl | H | H |
| CF₃ | CO₂Et | COPh-4-Cl | H | H |
| CF₃ | CO₂Et | OH | H | H |
| CF₃ | CO₂Et | OH | Me | H |
| CF₃ | CO₂Et | OMe | H | H |
| CF₃ | CO₂Et | OMe | Me | H |
| CF₃ | CO₂Et | OEt | H | H |
| CF₃ | CO₂Et | OEt | Me | H |
| CF₃ | CO₂Et | OPh | H | H |
| CF₃ | CO₂Et | OPh | Me | H |
| CF₃ | CO₂Et | OCOMe | H | H |
| CF₃ | CO₂Et | OCOMe | Me | H |
| CF₃ | CO₂Et | OCO₂Me | H | H |
| CF₃ | CO₂Et | OCO₂Me | Me | H |
| CF₃ | CO₂Et | OCO₂Et | H | H |
| CF₃ | CO₂Et | OCO₂Et | Me | H |
| CF₃ | CO₂Et | OCH₂OMe | H | H |
| CF₃ | CO₂Et | OCH₂OMe | Me | H |
| CF₃ | CO₂Et | SMe | H | H |
| CF₃ | CO₂Et | SMe | Me | H |
| CF₃ | CO₂Et | SPh | H | H |
| CF₃ | CO₂Et | CN | H | H |
| CF₃ | CO₂Et | NH₂ | H | H |
| CF₃ | CO₂Et | NHMe | H | H |
| CF₃ | CO₂Et | NHMe | Me | H |
| CF₃ | CO₂Et | NMe₂ | H | H |
| CF₃ | CO₂Et | NMe₂ | Me | H |
| CF₃ | CO₂Et | E-5 | H | H |
| CF₃ | CO₂Et | E-5 | Me | H |
| CF₃ | CO₂Et | E-6 | H | H |
| CF₃ | CO₂Et | E-6 | Me | H |
| CF₃ | CO₂Et | E-7 | H | H |
| CF₃ | CO₂Et | E-7 | Me | H |
| CF₃ | CO₂Et | E-8 | H | H |
| CF₃ | CO₂Et | E-8 | Me | H |
| CF₃ | CO₂Et | -CH₂-O-CH₂- | | H |
| CF₃ | CO₂Et | -(CH₂)₂-O-(CH₂)₂- | | H |
| CF₃ | CO₂Et | -(CH₂)₂- | | H |
| CF₃ | CO₂Et | -(CH₂)₃- | | H |
| CF₃ | CO₂Et | -(CH₂)₄- | | H |
| CF₃ | CO₂Et | -(CH₂)₅₋ | | H |
| CF₃ | CO₂Et | H | H | 3-F |
| CF₃ | CO₂Et | H | H | 4-F |
| CF₃ | CO₂Et | H | H | 5-F |
| CF₃ | CO₂Et | H | H | 6-F |
| CF₃ | CO₂Et | H | H | 3-Cl |
| CF₃ | CO₂Et | H | H | 4-Cl |
| CF₃ | CO₂Et | H | H | 5-Cl |
| CF₃ | CO₂Et | H | H | 6-Cl |
| CF₃ | CO₂Et | H | H | 3-Br |
| CF₃ | CO₂Et | H | H | 4-Br |
| CF₃ | CO₂Et | H | H | 5-Br |
| CF₃ | CO₂Et | H | H | 6-Br |
| CF₃ | CO₂Et | H | H | 3-I |
| CF₃ | CO₂Et | H | H | 4-I |
| CF₃ | CO₂Et | H | H | 5-I |
| CF₃ | CO₂Et | H | H | 6-I |
| CF₃ | CO₂Et | H | H | 3-Me |
| CF₃ | CO₂Et | H | H | 4-Me |
| CF₃ | CO₂Et | H | H | 5-Me |
| CF₃ | CO₂Et | H | H | 6-Me |
| CF₃ | CO₂Et | H | H | 3-OMe |
| CF₃ | CO₂Et | H | H | 4-OMe |
| CF₃ | CO₂Et | H | H | 5-OMe |
| CF₃ | CO₂Et | H | H | 6-OMe |
| CF₃ | CO₂Et | H | H | 3-SMe |
| CF₃ | CO₂Et | H | H | 4-SMe |
| CF₃ | CO₂Et | H | H | 5-SMe |
| CF₃ | CO₂Et | H | H | 6-SMe |
| CF₃ | CO₂Et | Me | H | 3-F |
| CF₃ | CO₂Et | Me | H | 4-F |
| CF₃ | CO₂Et | Me | H | 5-F |
| CF₃ | CO₂Et | Me | H | 6-F |
| CF₃ | CO₂Et | Me | H | 3-Cl |
| CF₃ | CO₂Et | Me | H | 4-Cl |
| CF₃ | CO₂Et | Me | H | 5-Cl |
| CF₃ | CO₂Et | Me | H | 6-Cl |
| CF₃ | CO₂Et | Me | H | 3-Br |
| CF₃ | CO₂Et | Me | H | 4-Br |
| CF₃ | CO₂Et | Me | H | 5-Br |
| CF₃ | CO₂Et | Me | H | 6-Br |
| CF₃ | CO₂Et | Me | H | 3-I |
| CF₃ | CO₂Et | Me | H | 4-I |
| CF₃ | CO₂Et | Me | H | 5-I |
| CF₃ | CO₂Et | Me | H | 6-I |
| CF₃ | CO₂Et | Me | H | 3-Me |
| CF₃ | CO₂Et | Me | H | 4-Me |
| CF₃ | CO₂Et | Me | H | 5-Me |
| CF₃ | CO₂Et | Me | H | 6-Me |
| CF₃ | CO₂Et | Me | H | 3-OMe |
| CF₃ | CO₂Et | Me | H | 4-OMe |
| CF₃ | CO₂Et | Me | H | 5-OMe |
| CF₃ | CO₂Et | Me | H | 6-OMe |
| CF₃ | CO₂Et | Me | H | 3-SMe |
| CF₃ | CO₂Et | Me | H | 4-SMe |
| CF₃ | CO₂Et | Me | H | 5-SMe |
| CF₃ | CO₂Et | Me | H | 6-SMe |
| CF₃ | CO₂Et | Me | Me | 3-F |
| CF₃ | CO₂Et | Me | Me | 4-F |
| CF₃ | CO₂Et | Me | Me | 5-F |
| CF₃ | CO₂Et | Me | Me | 6-F |
| CF₃ | CO₂Et | Me | Me | 3-Cl |
| CF₃ | CO₂Et | Me | Me | 4-Cl |
| CF₃ | CO₂Et | Me | Me | 5-Cl |
| CF₃ | CO₂Et | Me | Me | 6-Cl |
| CF₃ | CO₂Et | Me | Me | 3-Br |
| CF₃ | CO₂Et | Me | Me | 4-Br |
| CF₃ | CO₂Et | Me | Me | 5-Br |
| CF₃ | CO₂Et | Me | Me | 6-Br |
| CF₃ | CO₂Et | Me | Me | 3-I |
| CF₃ | CO₂Et | Me | Me | 4-I |
| CF₃ | CO₂Et | Me | Me | 5-I |
| CF₃ | CO₂Et | Me | Me | 6-I |
| CF₃ | CO₂Et | Me | Me | 3-Me |
| CF₃ | CO₂Et | Me | Me | 4-Me |
| CF₃ | CO₂Et | Me | Me | 5-Me |
| CF₃ | CO₂Et | Me | Me | 6-Me |
| CF₃ | CO₂Et | Me | Me | 3-OMe |
| CF₃ | CO₂Et | Me | Me | 4-OMe |
| CF₃ | CO₂Et | Me | Me | 5-OMe |
| CF₃ | CO₂Et | Me | Me | 6-OMe |
| CF₃ | CO₂Et | Me | Me | 3-SMe |
| CF₃ | CO₂Et | Me | Me | 4-SMe |
| CF₃ | CO₂Et | Me | Me | 5-SMe |
| CF₃ | CO₂Et | Me | Me | 6-SMe |
| CF₃ | CO₂Pr-n | H | H | H |
| CF₃ | CO₂Pr-n | Me | H | H |
| CF₃ | CO₂Pr-n | Me | Me | H |
| CF₃ | CO₂Pr-n | Et | H | H |
| CF₃ | CO₂Pr-n | Et | Me | H |
| CF₃ | CO₂Pr-n | Pr-n | H | H |
| CF₃ | CO₂Pr-n | Pr-n | Me | H |
| CF₃ | CO₂Pr-n | Cl | H | H |
| CF₃ | CO₂Pr-n | Cl | Me | H |
| CF₃ | CO₂Pr-n | Br | H | H |
| CF₃ | CO₂Pr-n | Br | Me | H |
| CF₃ | CO₂Pr-n | F | H | H |
| CF₃ | CO₂Pr-n | F | Me | H |
| CF₃ | CO₂Pr-n | CH₂Cl | H | H |
| CF₃ | CO₂Pr-n | CH₂Cl | Me | H |
| CF₃ | CO₂Pr-n | CH₂OMe | H | H |
| CF₃ | CO₂Pr-n | CH₂OMe | Me | H |
| CF₃ | CO₂Pr-n | CF₃ | H | H |
| CF₃ | CO₂Pr-n | CF₃ | Me | H |
| CF₃ | CO₂Pr-n | Ph | H | H |
| CF₃ | CO₂Pr-n | Ph | Me | H |
| CF₃ | CO₂Pr-n | Ph-2-Cl | H | H |
| CF₃ | CO₂Pr-n | Ph-3-Cl | H | H |
| CF₃ | CO₂Pr-n | Ph-4-Cl | H | H |
| CF₃ | CO₂Pr-n | COMe | H | H |
| CF₃ | CO₂Pr-n | CO₂H | H | H |
| CF₃ | CO₂Pr-n | CO₂Me | H | H |
| CF₃ | CO₂Pr-n | CONH₂ | H | H |
| CF₃ | CO₂Pr-n | CONHMe | H | H |
| CF₃ | CO₂Pr-n | CONMe₂ | H | H |
| CF₃ | CO₂Pr-n | OH | H | H |
| CF₃ | CO₂Pr-n | OH | Me | H |
| CF₃ | CO₂Pr-n | OMe | H | H |
| CF₃ | CO₂Pr-n | OMe | Me | H |
| CF₃ | CO₂Pr-n | OPh | H | H |
| CF₃ | CO₂Pr-n | OPh | Me | H |
| CF₃ | CO₂Pr-n | SMe | H | H |
| CF₃ | CO₂Pr-n | SMe | Me | H |
| CF₃ | CO₂Pr-n | NHMe | H | H |
| CF₃ | CO₂Pr-n | NHMe | Me | H |
| CF₃ | CO₂Pr-n | CH₂Ph | H | H |
| CF₃ | CO₂Pr-n | CH₂Ph-2-Cl | H | H |
| CF₃ | CO₂Pr-n | CH₂Ph-3-Cl | H | H |
| CF₃ | CO₂Pr-n | CH₂Ph-4-Cl | H | H |
| CF₃ | CO₂Pr-n | COPh | H | H |
| CF₃ | CO₂Pr-n | COPh-2-Cl | H | H |
| CF₃ | CO₂Pr-n | COPh-3-Cl | H | H |
| CF₃ | CO₂Pr-n | COPh-4-Cl | H | H |
| CF₃ | CO₂Pr-i | H | H | H |
| CF₃ | CO₂Pr-i | Me | H | H |
| CF₃ | CO₂Pr-i | Me | Me | H |
| CF₃ | CO₂Pr-i | Et | H | H |
| CF₃ | CO₂Pr-i | Et | Me | H |
| CF₃ | CO₂Pr-i | Pr-n | H | H |
| CF₃ | CO₂Pr-i | Pr-n | Me | H |
| CF₃ | CO₂Pr-i | Cl | H | H |
| CF₃ | CO₂Pr-i | Cl | Me | H |
| CF₃ | CO₂Pr-i | Br | H | H |
| CF₃ | CO₂Pr-i | Br | Me | H |
| CF₃ | CO₂Pr-i | F | H | H |
| CF₃ | CO₂Pr-i | F | Me | H |
| CF₃ | CO₂Pr-i | CH₂Cl | H | H |
| CF₃ | CO₂Pr-i | CH₂Cl | Me | H |
| CF₃ | CO₂Pr-i | CH₂OMe | H | H |
| CF₃ | CO₂Pr-i | CH₂OMe | Me | H |
| CF₃ | CO₂Pr-i | CF₃ | H | H |
| CF₃ | CO₂Pr-i | CF₃ | Me | H |
| CF₃ | CO₂Pr-i | Ph | H | H |
| CF₃ | CO₂Pr-i | Ph | Me | H |
| CF₃ | CO₂Pr-i | Ph-2-Cl | H | H |
| CF₃ | CO₂Pr-i | Ph-3-Cl | H | H |
| CF₃ | CO₂Pr-i | Ph-4-Cl | H | H |
| CF₃ | CO₂Pr-i | COMe | H | H |
| CF₃ | CO₂Pr-i | CO₂H | H | H |
| CF₃ | CO₂Pr-i | CO₂Me | H | H |
| CF₃ | CO₂Pr-i | CONH₂ | H | H |
| CF₃ | CO₂Pr-i | CONHM | H | H |
| CF₃ | CO₂Pr-i | CONMe₂ | H | H |
| CF₃ | CO₂Pr-i | OH | H | H |
| CF₃ | CO₂Pr-i | OH | Me | H |
| CF₃ | CO₂Pr-i | OMe | H | H |
| CF₃ | CO₂Pr-i | OMe | Me | H |
| CF₃ | CO₂Pr-i | OPh | H | H |
| CF₃ | CO₂Pr-i | OPh | Me | H |
| CF₃ | CO₂Pr-i | SMe | H | H |
| CF₃ | CO₂Pr-i | SMe | Me | H |
| CF₃ | CO₂Pr-i | NHMe | H | H |
| CF₃ | CO₂Pr-i | NHMe | Me | H |
| CF₃ | CO₂Pr-i | CH₂Ph | H | H |
| CF₃ | CO₂Pr-i | CH₂Ph-2-Cl | H | H |
| CF₃ | CO₂Pr-i | CH₂Ph-3-Cl | H | H |
| CF₃ | CO₂Pr-i | CH₂Ph-4-Cl | H | H |
| CF₃ | CO₂Pr-i | COPh | H | H |
| CF₃ | CO₂Pr-i | COPh-2-Cl | H | H |
| CF₃ | CO₂Pr-i | COPh-3-Cl | H | H |
| CF₃ | CO₂Pr-i | COPh-4-Cl | H | H |
| CF₃ | CO₂Bu-n | H | H | H |
| CF₃ | CO₂Bu-n | Me | H | H |
| CF₃ | CO₂Bu-n | Me | Me | H |
| CF₃ | CO₂Bu-n | Et | H | H |
| CF₃ | CO₂Bu-n | Et | Me | H |
| CF₃ | CO₂Bu-n | Pr-n | H | H |
| CF₃ | CO₂Bu-n | Pr-n | Me | H |
| CF₃ | CO₂Bu-n | Cl | H | H |
| CF₃ | CO₂Bu-n | Cl | Me | H |
| CF₃ | CO₂Bu-n | Br | H | H |
| CF₃ | CO₂Bu-n | Br | Me | H |
| CF₃ | CO₂Bu-n | F | H | H |
| CF₃ | CO₂Bu-n | F | Me | H |
| CF₃ | CO₂Bu-n | CH₂Cl | H | H |
| CF₃ | CO₂Bu-n | CH₂Cl | Me | H |
| CF₃ | CO₂Bu-n | CH₂OMe | H | H |
| CF₃ | CO₂Bu-n | CH₂OMe | Me | H |
| CF₃ | CO₂Bu-n | CF₃ | H | H |
| CF₃ | CO₂Bu-n | CF₃ | Me | H |
| CF₃ | CO₂Bu-n | Ph | H | H |
| CF₃ | CO₂Bu-n | Ph | Me | H |
| CF₃ | CO₂Bu-n | Ph-2-Cl | H | H |
| CF₃ | CO₂Bu-n | Ph-3-Cl | H | H |
| CF₃ | CO₂Bu-n | Ph-4-Cl | H | H |
| CF₃ | CO₂Bu-n | COMe | H | H |
| CF₃ | CO₂Bu-n | CO₂H | H | H |
| CF₃ | CO₂Bu-n | CO₂Me | H | H |
| CF₃ | CO₂Bu-n | CONH₂ | H | H |
| CF₃ | CO₂Bu-n | CONHMe | H | H |
| CF₃ | CO₂Bu-n | CONMe₂ | H | H |
| CF₃ | CO₂Bu-n | OH | H | H |
| CF₃ | CO₂Bu-n | OH | Me | H |
| CF₃ | CO₂Bu-n | OMe | H | H |
| CF₃ | CO₂Bu-n | OMe | Me | H |
| CF₃ | CO₂Bu-n | OPh | H | H |
| CF₃ | CO₂Bu-n | OPh | Me | H |
| CF₃ | CO₂Bu-n | SMe | H | H |
| CF₃ | CO₂Bu-n | SMe | Me | H |
| CF₃ | CO₂Bu-n | NHMe | H | H |
| CF₃ | CO₂Bu-n | NHMe | Me | H |
| CF₃ | CO₂Bu-n | CH₂Ph | H | H |
| CF₃ | CO₂Bu-n | CH₂Ph-2-Cl | H | H |
| CF₃ | CO₂Bu-n | CH₂Ph-3-Cl | H | H |
| CF₃ | CO₂Bu-n | CH₂Ph-4-Cl | H | H |
| CF₃ | CO₂Bu-n | COPh | H | H |
| CF₃ | CO₂Bu-n | COPh-2-Cl | H | H |
| CF₃ | CO₂Bu-n | COPh-3-Cl | H | H |
| CF₃ | CO₂Bu-n | COPh-4-Cl | H | H |
| CF₃ | CO₂Bu-i | H | H | H |
| CF₃ | CO₂Bu-i | Me | H | H |
| CF₃ | CO₂Bu-i | Me | Me | H |
| CF₃ | CO₂Bu-i | Et | H | H |
| CF₃ | CO₂Bu-i | Et | Me | H |
| CF₃ | CO₂Bu-i | Pr-n | H | H |
| CF₃ | CO₂Bu-i | Pr-n | Me | H |
| CF₃ | CO₂Bu-i | Pr-i | H | H |
| CF₃ | CO₂Bu-i | Pr-i | Me | H |
| CF₃ | CO₂Bu-i | Pr-c | H | H |
| CF₃ | CO₂Bu-i | Pr-c | Me | H |
| CF₃ | CO₂Bu-i | Bu-n | H | H |
| CF₃ | CO₂Bu-i | Bu-n | Me | H |
| CF₃ | CO₂Bu-i | Bu-i | H | H |
| CF₃ | CO₂Bu-i | Bu-i | Me | H |
| CF₃ | CO₂Bu-i | Bu-s | H | H |
| CF₃ | CO₂Bu-i | Bu-s | Me | H |
| CF₃ | CO₂Bu-i | Bu-t | H | H |
| CF₃ | CO₂Bu-i | Bu-t | Me | H |
| CF₃ | CO₂Bu-i | Bu-c | H | H |
| CF₃ | CO₂Bu-i | Bu-c | Me | H |
| CF₃ | CO₂Bu-i | Pen-n | H | H |
| CF₃ | CO₂Bu-i | Pen-n | Me | H |
| CF₃ | CO₂Bu-i | Pen-c | H | H |
| CF₃ | CO₂Bu-i | Pen-c | Me | H |
| CF₃ | CO₂Bu-i | Hex-n | H | H |
| CF₃ | CO₂Bu-i | Hex-n | Me | H |
| CF₃ | CO₂Bu-i | Hex-c | H | H |
| CF₃ | CO₂Bu-i | Hex-c | Me | H |
| CF₃ | CO₂Bu-i | Cl | H | H |
| CF₃ | CO₂Bu-i | Cl | Me | H |
| CF₃ | CO₂Bu-i | Cl | Cl | H |
| CF₃ | CO₂Bu-i | Br | H | H |
| CF₃ | CO₂Bu-i | Br | Me | H |
| CF₃ | CO₂Bu-i | I | H | H |
| CF₃ | CO₂Bu-i | I | Me | H |
| CF₃ | CO₂Bu-i | F | H | H |
| CF₃ | CO₂Bu-i | F | Me | H |
| CF₃ | CO₂Bu-i | F | F | H |
| CF₃ | CO₂Bu-i | CH=CH₂ | H | H |
| CF₃ | CO₂Bu-i | CH=CH₂ | Me | H |
| CF₃ | CO₂Bu-i | C≡CH | H | H |
| CF₃ | CO₂Bu-i | C≡CH | Me | H |
| CF₃ | CO₂Bu-i | CH₂CH=CH₂ | H | H |
| CF₃ | CO₂Bu-i | CH₂CH=CH₂ | Me | H |
| CF₃ | CO₂Bu-i | CH₂C≡CH | H | H |
| CF₃ | CO₂Bu-i | CH₂C≡CH | Me | H |
| CF₃ | CO₂Bu-i | CH₂Cl | H | H |
| CF₃ | CO₂Bu-i | CH₂Cl | Me | H |
| CF₃ | CO₂Bu-i | CHCl₂ | H | H |
| CF₃ | CO₂Bu-i | CCl₃ | H | H |
| CF₃ | CO₂Bu-i | CH₂F | H | H |
| CF₃ | CO₂Bu-i | CHF₂ | H | H |
| CF₃ | CO₂Bu-i | CF₃ | H | H |
| CF₃ | CO₂Bu-i | CF₃ | Me | H |
| CF₃ | CO₂Bu-i | CF₃ | Et t | H |
| CF₃ | CO₂Bu-i | CH₂CH₂Cl | H | H |
| CF₃ | CO₂Bu-i | CH₂OH | H | H |
| CF₃ | CO₂Bu-i | CH₂OMe | H | H |
| CF₃ | CO₂Bu-i | CH₂OMe | Me | H |
| CF₃ | CO₂Bu-i | CH₂SMe | H | H |
| CF₃ | CO₂Bu-i | CH₂SCH₂Cl | H | H |
| CF₃ | CO₂Bu-i | Ph | H | H |
| CF₃ | CO₂Bu-i | Ph | Me | H |
| CF₃ | CO₂Bu-i | Ph-2-Cl | H | H |
| CF₃ | CO₂Bu-i | Ph-3-Cl | H | H |
| CF₃ | CO₂Bu-i | Ph-4-Cl | H | H |
| CF₃ | CO₂Bu-i | Ph-2-Br | H | H |
| CF₃ | CO₂Bu-i | Ph-3-I | H | H |
| CF₃ | CO₂Bu-i | Ph-4-F | H | H |
| CF₃ | CO₂Bu-i | Ph-2-Me | H | H |
| CF₃ | CO₂Bu-i | Ph-3-OMe | H | H |
| CF₃ | CO₂Bu-i | Ph-4-SMe | H | H |
| CF₃ | CO₂Bu-i | Ph-2-SO₂Me | H | H |
| CF₃ | CO₂Bu-i | Ph-3-CN | H | H |
| CF₃ | CO₂Bu-i | Ph-4-NO₂ | H | H |
| CF₃ | CO₂Bu-i | Ph-2-CO₂Me | H | H |
| CF₃ | CO₂Bu-i | Ph-3-NH₂ | H | H |
| CF₃ | CO₂Bu-i | Ph-4-Ph | H | H |
| CF₃ | CO₂Bu-i | Ph-2-OPh | H | H |
| CF₃ | CO₂Bu-i | Ph-3-SPh | H | H |
| CF₃ | CO₂Bu-i | Ph-4-CONMe | H | H |
| CF₃ | CO₂Bu-i | Ph-2-OH | H | H |
| CF₃ | CO₂Bu-i | COMe | H | H |
| CF₃ | CO₂Bu-i | COEt | H | H |
| CF₃ | CO₂Bu-i | COCH₂Cl | H | H |
| CF₃ | CO₂Bu-i | CO₂H | H | H |
| CF₃ | CO₂Bu-i | CO₂Me | H | H |
| CF₃ | CO₂Bu-i | CO₂Me | Me | H |
| CF₃ | CO₂Bu-i | CO₂Et | H | H |
| CF₃ | CO₂Bu-i | CO₂Bu-i | H | H |
| CF₃ | CO₂Bu-i | CONH₂ | H | H |
| CF₃ | CO₂Bu-i | CONHMe | H | H |
| CF₃ | CO₂Bu-i | CONMe₂ | H | H |
| CF₃ | CO₂Bu-i | CH₂Ph | H | H |
| CF₃ | CO₂Bu-i | CH₂Ph-2-Cl | H | H |
| CF₃ | CO₂Bu-i | CH₂Ph-3-Cl | H | H |
| CF₃ | CO₂Bu-i | CH₂Ph-4-Cl | H | H |
| CF₃ | CO₂Bu-i | COPh | H | H |
| CF₃ | CO₂Bu-i | COPh-2-Cl | H | H |
| CF₃ | CO₂Bu-i | COPh-3-Cl | H | H |
| CF₃ | CO₂Bu-i | COPh-4-Cl | H | H |
| CF₃ | CO₂Bu-i | OH | H | H |
| CF₃ | CO₂Bu-i | OH | Me | H |
| CF₃ | CO₂Bu-i | OMe | H | H |
| CF₃ | CO₂Bu-i | OMe | Me | H |
| CF₃ | CO₂Bu-i | OEt | H | H |
| CF₃ | CO₂Bu-i | OEt | Me | H |
| CF₃ | CO₂Bu-i | OPh | H | H |
| CF₃ | CO₂Bu-i | OPh | Me | H |
| CF₃ | CO₂Bu-i | OCOMe | H | H |
| CF₃ | CO₂Bu-i | OCOMe | Me | H |
| CF₃ | CO₂Bu-i | OCO₂Me | H | H |
| CF₃ | CO₂Bu-i | OCO₂Me | Me | H |
| CF₃ | CO₂Bu-i | OCO₂Et | H | H |
| CF₃ | CO₂Bu-i | OCO₂Et | Me | H |
| CF₃ | CO₂Bu-i | OCH₂OMe | H | H |
| CF₃ | CO₂Bu-i | OCH₂OMe | Me | H |
| CF₃ | CO₂Bu-i | SMe | H | H |
| CF₃ | CO₂Bu-i | SMe | Me | H |
| CF₃ | CO₂Bu-i | SPh | H | H |
| CF₃ | CO₂Bu-i | CN | H | H |
| CF₃ | CO₂Bu-i | NH₂ | I H | H |
| CF₃ | CO₂Bu-i | NHMe | H | H |
| CF₃ | CO₂Bu-i | NHMe | Me | H |
| CF₃ | CO₂Bu-i | NMe₂ | | H |
| CF₃ | CO₂Bu-i | NMe₂ | Me | H |
| CF₃ | CO₂Bu-i | E-5 | H | H |
| CF₃ | CO₂Bu-i | E-5 | Me | H |
| CF₃ | CO₂Bu-i | E-6 | H | H |
| CF₃ | CO₂Bu-i | E-6 | Me | H |
| CF₃ | CO₂Bu-i | E-7 | H | H |
| CF₃ | CO₂Bu-i | E-7 | Me | H |
| CF₃ | CO₂Bu-i | E-8 | H | H |
| CF₃ | CO₂Bu-i | E-8 | Me | H |
| CF₃ | CO₂Bu-i | -CH₂-O-CH₂- | | H |
| CF₃ | CO₂Bu-i | -(CH₂)₂-O-(CH₂)₂- | | H |
| CF₃ | CO₂Bu-i | -(CH₂)₂- | | H |
| CF₃ | CO₂Bu-i | -(CH₂)₃- | | H |
| CF₃ | CO₂Bu-i | -(CH₂)₄- | | H |
| CF₃ | CO₂Bu-i | -(CH₂)₅- | | H |
| CF₃ | E-1 | H | H | H |
| CF₃ | E-1 | Me | H | H |
| CF₃ | E-1 | Me | Me | H |
| CF₃ | E-1 | Et | H | H |
| CF₃ | E-1 | Et | Me | H |
| CF₃ | E-1 | Pr-n | H | H |
| CF₃ | E-1 | Pr-n | Me | H |
| CF₃ | E-1 | Cl | H | H |
| CF₃ | E-1 | Cl | Me | H |
| CF₃ | E-1 | Br | H | H |
| CF₃ | E-1 | Br | Me | H |
| CF₃ | E-1 | F | H | H |
| CF₃ | E-1 | F | Me | H |
| CF₃ | E-1 | CH₂Cl | H | H |
| CF₃ | E-1 | CH₂Cl | Me | H |
| CF₃ | E-1 | CH₂OMe | H | H |
| CF₃ | E-1 | CH₂OMe | Me | H |
| CF₃ | E-1 | CF₃ | H | H |
| CF₃ | E-1 | CF₃ | Me | H |
| CF₃ | E-1 | Ph | H | H |
| CF₃ | E-1 | Ph | Me | H |
| CF₃ | E-1 | Ph-2-Cl | H | H |
| CF₃ | E-1 | Ph-3-Cl | H | H |
| CF₃ | E-1 | Ph-4-Cl | H | H |
| CF₃ | E-1 | COMe | H | H |
| CF₃ | E-1 | CO₂H | H | H |
| CF₃ | E-1 | CO₂Me | H | H |
| CF₃ | E-1 | CONH₂ | H | H |
| CF₃ | E-1 | CONHMe | H | H |
| CF₃ | E-1 | CONMe₂ | H | H |
| CF₃ | E-1 | OH | H | H |
| CF₃ | E-1 | OH | Me | H |
| CF₃ | E-1 | OMe | H | H |
| CF₃ | E-1 | OMe | Me | H |
| CF₃ | E-1 | OPh | H | H |
| CF₃ | E-1 | OPh | Me | H |
| CF₃ | E-1 | SMe | H | H |
| CF₃ | E-1 | SMe | Me | H |
| CF₃ | E-1 | NHMe | H | H |
| CF₃ | E-1 | NHMe | Me | H |
| CF₃ | E-1 | CH₂Ph | H | H |
| CF₃ | E-1 | CH₂Ph-2-Cl | H | H |
| CF₃ | E-1 | CH₂Ph-3-Cl | H | H |
| CF₃ | E-1 | CH₂Ph-4-Cl | H | H |
| CF₃ | E-1 | COPh | H | H |
| CF₃ | E-1 | COPh-2-Cl | H | H |
| CF₃ | E-1 | COPh-3-Cl | H | H |
| CF₃ | E-1 | COPh-4-Cl | H | H |
| CF₃ | CO₂Bu-t | H | H | H |
| CF₃ | CO₂Bu-t | Me | H | H |
| CF₃ | CO₂Bu-t | Me | Me | H |
| CF₃ | CO₂Bu-t | Et | H | H |
| CF₃ | CO₂Bu-t | Et | Me | H |
| CF₃ | CO₂Bu-t | Pr-n | H | H |
| CF₃ | CO₂Bu-t | Pr-n | Me | H |
| CF₃ | CO₂Bu-t | Cl | H | H |
| CF₃ | CO₂Bu-t | Cl | Me | H |
| CF₃ | CO₂Bu-t | Br | H | H |
| CF₃ | CO₂Bu-t | Br | Me | H |
| CF₃ | CO₂Bu-t | F | H | H |
| CF₃ | CO₂Bu-t | F | Me | H |
| CF₃ | CO₂Bu-t | CH₂Cl | H | H |
| CF₃ | CO₂Bu-t | CH₂Cl | Me | H |
| CF₃ | CO₂Bu-t | CH₂OMe | H | H |
| CF₃ | CO₂Bu-t | CH₂OMe | Me | H |
| CF₃ | CO₂Bu-t | CF₃ | H | H |
| CF₃ | CO₂Bu-t | CF₃ | Me | H |
| CF₃ | CO₂Bu-t | Ph | H | H |
| CF₃ | CO₂Bu-t | Ph | Me | H |
| CF₃ | CO₂Bu-t | Ph-2-Cl | H | H |
| CF₃ | CO₂Bu-t | Ph-3-Cl | H | H |
| CF₃ | CO₂Bu-t | Ph-4-Cl | H | H |
| CF₃ | CO₂Bu-t | COMe | H | H |
| CF₃ | CO₂Bu-t | CO₂H | H | H |
| CF₃ | CO₂Bu-t | CO₂Me | H | H |
| CF₃ | CO₂Bu-t | CONH₂ | H | H |
| CF₃ | CO₂Bu-t | CONHMe | H | H |
| CF₃ | CO₂Bu-t | CONMe₂ | H | H |
| CF₃ | CO₂Bu-t | OH | H | H |
| CF₃ | CO₂Bu-t | OH | Me | H |
| CF₃ | CO₂Bu-t | OMe | H | H |
| CF₃ | CO₂Bu-t | OMe | Me | H |
| CF₃ | CO₂Bu-t | OPh | H | H |
| CF₃ | CO₂Bu-t | OPh | Me | H |
| CF₃ | CO₂Bu-t | SMe | H | H |
| CF₃ | CO₂Bu-t | SMe | Me | H |
| CF₃ | CO₂Bu-t | NHMe | H | H |
| CF₃ | CO₂Bu-t | NHMe | Me | H |
| CF₃ | CO₂Bu-t | CH₂Ph | H | H |
| CF₃ | CO₂Bu-t | CH₂Ph-2-Cl | H | H |
| CF₃ | CO₂Bu-t | CH₂Ph-3-Cl | H | H |
| CF₃ | CO₂Bu-t | CH₂Ph-4-Cl | H | H |
| CF₃ | CO₂Bu-t | COPh | H | H |
| CF₃ | CO₂Bu-t | COPh-2-Cl | H | H |
| CF₃ | CO₂Bu-t | COPh-3-Cl | H | H |
| CF₃ | CO₂Bu-t | COPh-4-Cl | H | H |
| CF₃ | CO₂Ph | H | H | H |
| CF₃ | CO₂Ph | Me | H | H |
| CF₃ | CO₂Ph | Me | Me | H |
| CF₃ | CO₂Ph | Et | H | H |
| CF₃ | CO₂Ph | Et | Me | H |
| CF₃ | CO₂Ph | Pr-n | H | H |
| CF₃ | CO₂Ph | Pr-n | Me | H |
| CF₃ | CO₂Ph | Cl | H | H |
| CF₃ | CO₂Ph | Cl | Me | H |
| CF₃ | CO₂Ph | Br | H | H |
| CF₃ | CO₂Ph | Br | Me | H |
| CF₃ | CO₂Ph | F | H | H |
| CF₃ | CO₂Ph | F | Me | H |
| CF₃ | CO₂Ph | CH₂Cl | H | H |
| CF₃ | CO₂Ph | CH₂Cl | Me | H |
| CF₃ | CO₂Ph | CH₂OMe | H | H |
| CF₃ | CO₂Ph | CH₂OMe | Me | H |
| CF₃ | CO₂Ph | CF₃ | H | H |
| CF₃ | CO₂Ph | CF₃ | Me | H |
| CF₃ | CO₂Ph | Ph I | | H |
| CF₃ | CO₂Ph | Ph | Me | H |
| CF₃ | CO₂Ph | Ph-2-Cl | H | H |
| CF₃ | CO₂Ph | Ph-3-Cl | H | H |
| CF₃ | CO₂Ph | Ph-4-Cl | H | H |
| CF₃ | CO₂Ph | COMe | H | H |
| CF₃ | CO₂Ph | CO₂H | H | H |
| CF₃ | CO₂Ph | CO₂Me | H | H |
| CF₃ | CO₂Ph | CONH₂ | H | H |
| CF₃ | CO₂Ph | CONHMe | H | H |
| CF₃ | CO₂Ph | CONMe₂ | H | H |
| CF₃ | CO₂Ph | OH | H | H |
| CF₃ | CO₂Ph | OH | Me | H |
| CF₃ | CO₂Ph | OMe | H | H |
| CF₃ | CO₂Ph | OMe | Me | H |
| CF₃ | CO₂Ph | OPh | H | H |
| CF₃ | CO₂Ph | OPh | Me | H |
| CF₃ | CO₂Ph | SMe | H | H |
| CF₃ | CO₂Ph | SMe | Me | H |
| CF₃ | CO₂Ph | NHMe | H | H |
| CF₃ | CO₂Ph | NHMe | Me | H |
| CF₃ | CO₂Ph | CH₂Ph | H | H |
| CF₃ | CO₂Ph | CH₂Ph-2-Cl | H | H |
| CF₃ | CO₂Ph | CH₂Ph-3-Cl | H | H |
| CF₃ | CO₂Ph | CH₂Ph-4-Cl | H | H |
| CF₃ | CO₂Ph | COPh | H | H |
| CF₃ | CO₂Ph | COPh-2-Cl | H | H |
| CF₃ | CO₂Ph | COPh-3-Cl | H | H |
| CF₃ | CO₂Ph | COPh-4-Cl | H | H |
| CF₃ | CO₂CH₂CH₂Cl | H | H | H |
| CF₃ | CO₂CH₂CH₂Cl | Me | H | H |
| CF₃ | CO₂CH₂CH₂Cl | Me | Me | H |
| CF₃ | CO₂CH₂CH₂Cl | Et | H | H |
| CF₃ | CO₂CH₂CH₂Cl | Et | Me | H |
| CF₃ | CO₂CH₂CH₂Cl I | Pr-n | H | H |
| CF₃ | CO₂CH₂CH₂Cl | Pr-n | Me | H |
| CF₃ | CO₂CH₂CH₂Cl | Cl | H | H |
| CF₃ | CO₂CH₂CH₂Cl | Cl | Me | H |
| CF₃ | CO₂CH₂CH₂Cl | Br | H | H |
| CF₃ | CO₂CH₂CH₂Cl | Br | Me | H |
| CF₃ | CO₂CH₂CH₂Cl | F | H | H |
| CF₃ | CO₂CH₂CH₂Cl | F | Me | H |
| CF₃ | CO₂CH₂CH₂Cl | CH₂Cl | H | H |
| CF₃ | CO₂CH₂CH₂Cl | CH₂Cl | Me | H |
| CF₃ | CO₂CH₂CH₂Cl | CH₂OMe | H | H |
| CF₃ | CO₂CH₂CH₂Cl | CH₂OMe | Me | H |
| CF₃ | CO₂CH₂CH₂Cl | CF₃ | H | H |
| CF₃ | CO₂CH₂CH₂Cl | CF₃ | Me | H |
| CF₃ | CO₂CH₂CH₂Cl | Ph | H | H |
| CF₃ | CO₂CH₂CH₂Cl | Ph | Me | H |
| CF₃ | CO₂CH₂CH₂Cl | Ph-2-Cl | H | H |
| CF₃ | CO₂CH₂CH₂Cl | Ph-3-Cl | H | H |
| CF₃ | CO₂CH₂CH₂Cl | Ph-4-Cl | H | H |
| CF₃ | CO₂CH₂CH₂Cl | COMe | H | H |
| CF₃ | CO₂CH₂CH₂Cl | CO₂H | H | H |
| CF₃ | CO₂CH₂CH₂Cl | CO₂Me | H | H |
| CF₃ | CO₂CH₂CH₂Cl | CONH₂ | H | H |
| CF₃ | CO₂CH₂CH₂Cl | CONHMe | H | H |
| CF₃ | CO₂CH₂CH₂Cl | CONMe₂ | H | H |
| CF₃ | CO₂CH₂CH₂Cl | OH | H | H |
| CF₃ | CO₂CH₂CH₂Cl | OH | Me | H |
| CF₃ | CO₂CH₂CH₂Cl | OMe | H | H |
| CF₃ | CO₂CH₂CH₂Cl | OMe | Me | H |
| CF₃ | CO₂CH₂CH₂Cl | OPh | H | H |
| CF₃ | CO₂CH₂CH₂Cl | OPh | Me | H |
| CF₃ | CO₂CH₂CH₂Cl | SMe | H | H |
| CF₃ | CO₂CH₂CH₂Cl | SMe | Me | H |
| CF₃ | CO₂CH₂CH₂Cl | NHMe | H | H |
| CF₃ | CO₂CH₂CH₂Cl | NHMe | Me | H |
| CF₃ | CO₂CH₂CH₂Cl | CH₂Ph | H | H |
| CF₃ | CO₂CH₂CH₂Cl | CH₂Ph-2-Cl | H | H |
| CF₃ | CO₂CH₂CH₂Cl | CH₂Ph-3-Cl | H | H |
| CF₃ | CO₂CH₂CH₂Cl | CH₂Ph-4-Cl | H | H |
| CF₃ | CO₂CH₂CH₂Cl | COPh | H | H |
| CF₃ | CO₂CH₂CH₂Cl | COPh-2-Cl | H | H |
| CF₃ | CO₂CH₂CH₂Cl | COPh-3-Cl | H | H |
| CF₃ | CO₂CH₂CH₂Cl | COPh-4-Cl | H | H |
| CF₃ | E-2 | H | H | H |
| CF₃ | E-2 | Me | H | H |
| CF₃ | E-2 | Me | Me | H |
| CF₃ | E-2 | Et | H | H |
| CF₃ | E-2 | Et | Me | H |
| CF₃ | E-2 | Pr-n | H | H |
| CF₃ | E-2 | Pr-n | Me | H |
| CF₃ | E-2 | Cl | H | H |
| CF₃ | E-2 | Cl | Me | H |
| CF₃ | E-2 | Br | H | H |
| CF₃ | E-2 | Br | Me | H |
| CF₃ | E-2 | F | H | H |
| CF₃ | E-2 | F | Me | H |
| CF₃ | E-2 | CH₂Cl | H | H |
| CF₃ | E-2 | CH₂Cl | Me | H |
| CF₃ | E-2 | CH₂OMe | H | H |
| CF₃ | E-2 | CH₂OMe | Me | H |
| CF₃ | E-2 | CF₃ | H | H |
| CF₃ | E-2 | CF₃ | Me | H |
| CF₃ | E-2 | Ph | H | H |
| CF₃ | E-2 | Ph | Me | H |
| CF₃ | E-2 | Ph-2-Cl | H | H |
| CF₃ | E-2 | Ph-3-Cl | H | H |
| CF₃ | E-2 | Ph-4-Cl | H | H |
| CF₃ | E-2 | COMe | H | H |
| CF₃ | E-2 | CO₂H | H | H |
| CF₃ | E-2 | CO₂Me | H | H |
| CF₃ | E- 2 | CONH₂ | H | H |
| CF₃ | E-2 | CONHMe | H | H |
| CF₃ | E-2 | CONMe₂ | H | H |
| CF₃ | E-2 | OH | H | H |
| CF₃ | E-2 | OH | Me | H |
| CF₃ | E-2 | OMe | H | H |
| CF₃ | E-2 | OMe | Me | H |
| CF₃ | E-2 | OPh | H | H |
| CF₃ | E-2 | OPh | Me | H |
| CF₃ | E-2 | SMe | H | H |
| CF₃ | E-2 | SMe | Me | H |
| CF₃ | E-2 | NHMe | H | H |
| CF₃ | E-2 | NHMe | Me | H |
| CF₃ | E-2 | CH₂Ph | H | H |
| CF₃ | E-2 | CH₂Ph-2-Cl | H | H |
| CF₃ | E-2 | CH₂Ph-3-Cl | H | H |
| CF₃ | E-2 | CH₂Ph-4-Cl | H | H |
| CF₃ | E-2 | COPh | H | H |
| CF₃ | E-2 | COPh-2-Cl | H | H |
| CF₃ | E-2 | COPh-3-Cl | H | H |
| CF₃ | E-2 | COPh-4-Cl | H | H |
| CF₃ | E-3 | H | H | H |
| CF₃ | E-3 | Me | H | H |
| CF₃ | E-3 | Me | Me | H |
| CF₃ | E-3 | Et | H | H |
| CF₃ | E-3 | Et | Me | H |
| CF₃ | E-3 | Pr-n | H | H |
| CF₃ | E-3 | Pr-n | Me | H |
| CF₃ | E-3 | Cl | H | H |
| CF₃ | E-3 | Cl | Me | H |
| CF₃ | E-3 | Br | H | H |
| CF₃ | E-3 | Br | Me | H |
| CF₃ | E-3 | F | H | H |
| CF₃ | E-3 | F | Me | H |
| CF₃ | E-3 | CH₂Cl | H | H |
| CF₃ | E-3 | CH₂Cl | Me | H |
| CF₃ | E-3 | CH₂OMe | H | H |
| CF₃ | E-3 | CH₂OMe | Me | H |
| CF₃ | E-3 | CF₃ | H | H |
| CF₃ | E-3 | CF₃ | Me | H |
| CF₃ | E-3 | Ph | H | H |
| CF₃ | E-3 | Ph | Me | H |
| CF₃ | E-3 | Ph-2-Cl | H | H |
| CF₃ | E-3 | Ph-3-Cl | H | H |
| CF₃ | E-3 | Ph-4-Cl | H | H |
| CF₃ | E-3 | COMe | H | H |
| CF₃ | E-3 | CO₂H | H | H |
| CF₃ | E-3 | CO₂Me | H | H |
| CF₃ | E-3 | CONH₂ | H | H |
| CF₃ | E-3 | CONHMe | H | H |
| CF₃ | E-3 | CONMe₂ | H | H |
| CF₃ | E-3 | OH | H | H |
| CF₃ | E-3 | OH | Me | H |
| CF₃ | E-3 | OMe | H | H |
| CF₃ | E-3 | OMe | Me | H |
| CF₃ | E-3 | OPh | H | H |
| CF₃ | E-3 | OPh | Me | H |
| CF₃ | E-3 | SMe | H | H |
| CF₃ | E-3 | SMe | Me | H |
| CF₃ | E-3 | NHMe | H | H |
| CF₃ | E-3 | NHMe | Me | H |
| CF₃ | E-3 | CH₂Ph | H | H |
| CF₃ | E-3 | CH₂Ph-2-Cl | H | H |
| CF₃ | E-3 | CH₂Ph-3-Cl | H | H |
| CF₃ | E-3 | CH₂Ph-4-Cl | H | H |
| CF₃ | E-3 | COPh | H | H |
| CF₃ | E-3 | COPh-2-Cl | H | H |
| CF₃ | E-3 | COPh-3-Cl | H | H |
| CF₃ | E- 3 | COPh-4-Cl | H | H |
| CF₃ | CH₂OMe | H | H | H |
| CF₃ | CH₂OMe | Me | H | H |
| CF₃ | CH₂OMe | Me | Me | H |
| CF₃ | CH₂OMe | Et t | H | H |
| CF₃ | CH₂OMe | E t | Me | H |
| CF₃ | CH₂OMe | Pr-n | H | H |
| CF₃ | CH₂OMe | Pr-n | Me | H |
| CF₃ | CH₂OMe | Cl | H | H |
| CF₃ | CH₂OMe | Cl | Me | H |
| CF₃ | CH₂OMe | Br | H | H |
| CF₃ | CH₂OMe | Br | Me | H |
| CF₃ | CH₂OMe | F | H | H |
| CF₃ | CH₂OMe | F | Me | H |
| CF₃ | CH₂OMe | CH₂Cl | H | H |
| CF₃ | CH₂OMe | CH₂Cl | Me | H |
| CF₃ | CH₂OMe | CH₂OMe | H | H |
| CF₃ | CH₂OMe | CH₂OMe | Me | H |
| CF₃ | CH₂OMe | CF₃ | H | H |
| CF₃ | CH₂OMe | CF₃ | Me | H |
| CF₃ | CH₂OMe | Ph | H | H |
| CF₃ | CH₂OMe | Ph | Me | H |
| CF₃ | CH₂OMe | Ph-2-Cl | H | H |
| CF₃ | CH₂OMe | Ph-3-Cl | H | H |
| CF₃ | CH₂OMe | Ph-4-Cl | H | H |
| CF₃ | CH₂OMe | COMe | H | H |
| CF₃ | CH₂OMe | CO₂H | H | H |
| CF₃ | CH₂OMe | CO₂Me | H | H |
| CF₃ | CH₂OMe | CONH₂ | H | H |
| CF₃ | CH₂OMe | CONHMe | H | H |
| CF₃ | CH₂OMe | CONMe₂ | H | H |
| CF₃ | CH₂OMe | OH | H | H |
| CF₃ | CH₂OMe | OH | Me | H |
| CF₃ | CH₂OMe | OMe | H | H |
| CF₃ | CH₂OMe | OMe | Me | H |
| CF₃ | CH₂OMe | OPh | H | H |
| CF₃ | CH₂OMe | OPh | Me | H |
| CF₃ | CH₂OMe | SMe | H | H |
| CF₃ | CH₂OMe | SMe | Me | H |
| CF₃ | CH₂OMe | NHMe | H | H |
| CF₃ | CH₂OMe | NHMe | Me | H |
| CF₃ | CH₂OMe | CH₂Ph | H | H |
| CF₃ | CH₂OMe | CH₂Ph-2-Cl | H | H |
| CF₃ | CH₂OMe | CH₂Ph-3-Cl | H | H |
| CF₃ | CH₂OMe | CH₂Ph-4-Cl | H | H |
| CF₃ | CH₂OMe | COPh | H | H |
| CF₃ | CH₂OMe | COPh-2-Cl | H | H |
| CF₃ | CH₂OMe | COPh-3-Cl | H | H |
| CF₃ | CH₂OMe | COPh-4-Cl | H | H |
| CF₃ | COCH₂Cl | H | H | H |
| CF₃ | COCH₂Cl | Me | H | H |
| CF₃ | COCH₂Cl | Me | Me | H |
| CF₃ | E-4 | H | H | H |
| CF₃ | E-4 | Me | H | H |
| CF₃ | E-4 | Me | Me | H |
| CF₃ | CH₂SMe | H | H | H |
| CF₃ | CH₂SMe | Me | H | H |
| CF₃ | CH₂SMe | Me | Me | H |
| CF₃ | CH₂OPh | H | H | H |
| CF₃ | CH₂OPh | Me | H | H |
| CF₃ | CH₂OPh | Me | Me | H |
| CF₃ | CH₂OCOE t | H | H | H |
| CF₃ | CH₂OCOE t | Me | H | H |
| CF₃ | CH₂OCOE t | Me | Me | H |
| CF₃ | COSEt | H | H | H |
| CF₃ | COSEt | Me | H | H |
| CF₃ | COSEt | Me | Me | H |
| CF₃ | CH₂SO₂Me | H | H | H |
| CF₃ | CH₂SO₂Me | Me | H | H |
| CF₃ | CH₂SO₂Me | Me | Me | H |
| CF₃ | CH₂SOMe | H | H | H |
| CF₃ | CH₂SOMe | Me | H | H |
| CF₃ | CH₂SOMe | Me | Me | H |
| CF₃ | CH₂Ph | H | H | H |
| CF₃ | CH₂Ph | Me | H | H |
| CF₃ | CH₂Ph | Me | Me | H |
| CF₃ | SO₂CF₃ | H | H | H |
| CF₃ | SO₂CF₃ | Me | H | H |
| CF₃ | SO₂CF₃ | Me | Me | H |
| CHF₂ | H | H | H | H |
| CHF₂ | H | Me | H | H |
| CHF₂ | H | Me | Me | H |
| CHF₂ | COEt | H | H | H |
| CHF₂ | COEt | Me | H | H |
| CHF₂ | COEt | Me | Me | H |
| CHF₂ | CO₂Et | H | H | H |
| CHF₂ | CO₂Et | Me | H | H |
| CHF₂ | CO₂Et | Me | Me | H |
| CHF₂ | CO₂Bu-i | H | H | H |
| CHF₂ | CO₂Bu-i | Me | H | H |
| CHF₂ | CO₂Bu-i | Me | Me | H |
| CClF₂ | H | H | H | H |
| CClF₂ | H | Me | H | H |
| CClF₂ | H | Me | Me | H |
| CClF₂ | COEt | H | H | H |
| CClF₂ | COEt | Me | H | H |
| CClF₂ | COEt | Me | Me | H |
| CClF₂ | CO₂Et | H | H | H |
| CClF₂ | CO₂Et | Me | H | H |
| CClF₂ | CO₂Et | Me | Me | H |
| CClF₂ | CO₂Bu-i | H | H | H |
| CClF₂ | CO₂Bu-i | Me | H | H |
| CClF₂ | CO₂Bu-i | Me | Me | H |
| CBrF₂ | H | H | H | H |
| CBrF₂ | H | Me | H | H |
| CBrF₂ | H | Me | Me | H |
| CBrF₂ | COEt | H | H | H |
| CBrF₂ | COEt | Me | H | H |
| CBrF₂ | COEt | Me | Me | H |
| CBrF₂ | CO₂Et | H | H | H |
| CBrF₂ CBrF₂ | CO₂Et | Me | H | H |
| CBrF₂ | CO₂Et | Me | Me | H |
| CBrF₂ | CO₂Bu-i | H | H | H |
| CBrF₂ | CO₂Bu-i | Me | H | H |
| CBrF₂ | CO₂Bu-i | Me | Me | H |
| CCl₂F | H | H | H | H |
| CCl₂F | H | Me | H | H |
| CCl₂F | H | Me | Me | H |
| CCl₂F | COEt | H | H | H |
| CCl₂F | COEt | Me | H | H |
| CCl₂F | COEt | Me | Me | H |
| CCl₂F | CO₂Et | H | H | H |
| CCl₂F | CO₂Et | Me | H | H |
| CCl₂F | CO₂Et | Me | Me | H |
| CCl₂F | CO₂Bu-i | H | H | H |
| CCl₂F | CO₂Bu-i | Me | H | H |
| CCl₂F | CO₂Bu-i | Me | Me | H |
| | | | | |

In Table, E-1 to E-8 represent the following structures, respectively.

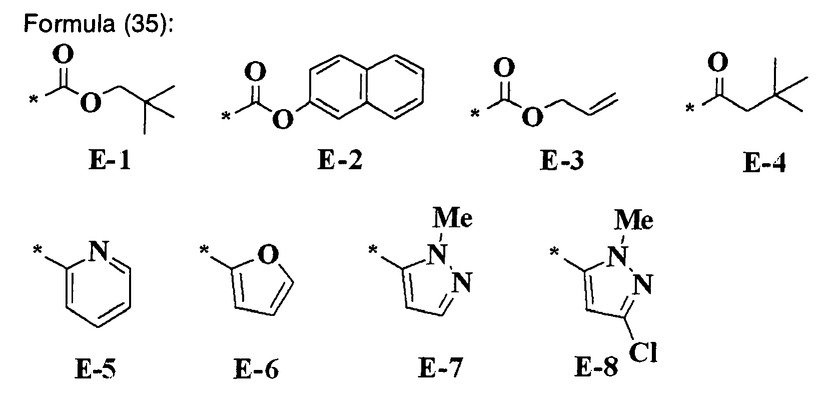

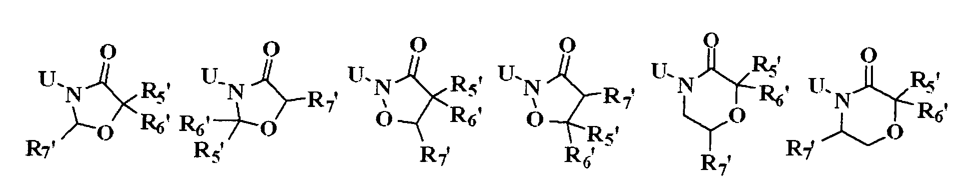

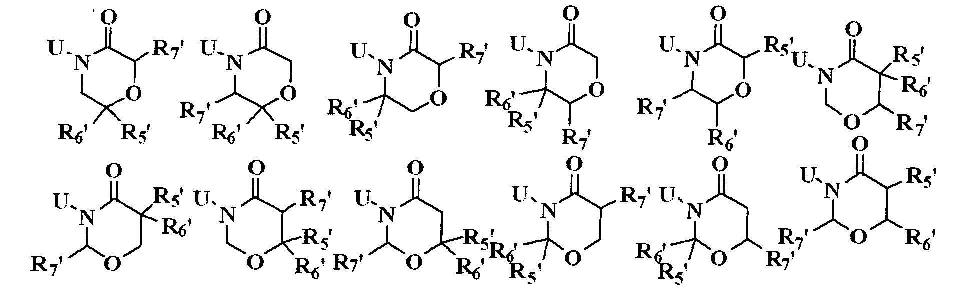

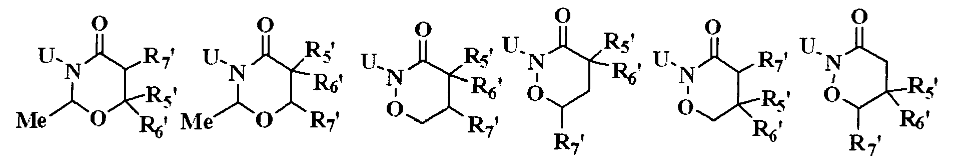

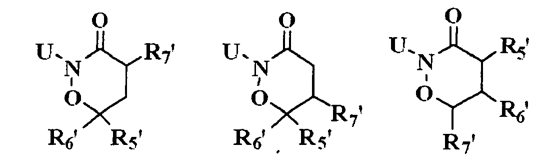

U is the same as defined above.

**TABLE 3 R₃=R₄=H**

| R₁ | R₂ | R₅ | R₆ | R₇ | X |
|---|---|---|---|---|---|
| CF₃ | H | Me | Me | Me | H |
| CF₃ | H | Me | Me | Et | H |
| CF₃ | H | Me | Me | F | H |
| CF₃ | H | Me | Me | Cl | H |
| CF₃ | H | Me | Me | Br | H |
| CF₃ | H | Me | Me | I | H |
| CF₃ | H | Me | Me | OH | H |
| CF₃ | H | Me | Me | OMe | H |
| CF₃ | H | Me | Me | OEt | H |
| CF₃ | H | Me | Me | OPh | H |
| CF₃ | H | Me | Me | OCOMe | H |
| CF₃ | H | Me | Me | OCO₂Me | H |
| CF₃ | H | Me | Me | OCO₂Et | H |
| CF₃ | H | Me | Me | OCH₂OMe | H |
| CF₃ | H | Me | Me | SMe | H |
| CF₃ | H | Me | Me | SPh | H |
| CF₃ | H | Me | Me | NHMe | H |
| CF₃ | H | Me | Me | NMe₂ | H |
| CF₃ | H | Me | Et | Me | H |
| CF₃ | H | Me | Et | F | H |
| CF₃ | H | Me | Et | Cl | H |
| CF₃ | H | Me | Et | Br | H |
| CF₃ | H | Me | Et | OMe | H |
| CF₃ | H | Me | CF₃ | Me | H |
| CF₃ | H | Me | CF₃ | F | H |
| CF₃ | H | Me | CF₃ | Cl | H |
| CF₃ | H | Me | CF₃ | Br | H |
| CF₃ | H | Me | CF₃ | OMe | H |
| CF₃ | H | Me | Pr-c | Me | H |
| CF₃ | H | Me | Pr-c | F | H |
| CF₃ | H | Me | Pr-c | Cl | H |
| CF₃ | H | Me | Pr-c | Br | H |
| CF₃ | H | Me | Pr-c | OMe | H |
| CF₃ | H | Me | CH₂OMe | Me | H |
| CF₃ | H | Me | CH₂OMe | F | H |
| CF₃ | H | Me | CH₂OMe | Cl | H |
| CF₃ | H | Me | CH₂OMe | Br | H |
| CF₃ | H | Me | CH₂OMe | OMe | H |
| CF₃ | H | Me | CH₂Cl | Me | H |
| CF₃ | H | Me | CH₂Cl | F | H |
| CF₃ | H | Me | CH₂Cl | Cl | H |
| CF₃ | H | Me | CH₂Cl | Br | H |
| CF₃ | H | Me | CH₂Cl | OMe | H |
| CF₃ | H | | -(CH₂)₃- | Me | H |
| CF₃ | H | | -(CH₂)₃- | F | H |
| CF₃ | H | | -(CH₂)₃- | Cl | H |
| CF₃ | H | | -(CH₂)₃- | Br | H |
| CF₃ | H | | -(CH₂)₃- | OMe | H |
| CF₃ | H | | -(CH₂)₄- | Me | H |
| CF₃ | H | | -(CH₂)₄- | F | H |
| CF₃ | H | | -(CH₂)₄- | Cl | H |
| CF₃ | H | | -(CH₂)₄- | Br | H |
| CF₃ | H | | -(CH₂)₄- | OMe | H |
| CF₃ | H | | -(CH₂)₅- | Me | H |
| CF₃ | H | | -(CH₂)₅- | F | H |
| CF₃ | H | | -(CH₂)₅- | Cl | H |
| CF₃ | H | | -(CH₂)₅- | Br | H |
| CF₃ | H | | -(CH₂)₅- | OMe | H |
| CF₃ | COMe | Me | Me | Me | H |
| CF₃ | COMe | Me | Me | F | H |
| CF₃ | COMe | Me | Me | Cl | H |
| CF₃ | COMe | Me | Me | Br | H |
| CF₃ | COMe | Me | Me | OH | H |
| CF₃ | COMe | Me | Me | OMe | H |
| CF₃ | COMe | Me | Me | SMe | H |
| CF₃ | COEt | Me | Me | Me | H |
| CF₃ | COEt | Me | Me | Et | H |
| CF₃ | COEt | Me | Me | F | H |
| CF₃ | COEt | Me | Me | Cl | H |
| CF₃ | COEt | Me | Me | Br | H |
| CF₃ | COEt | Me | Me | I | H |
| CF₃ | COEt | Me | Me | OH | H |
| CF₃ | COEt | Me | Me | OMe | H |
| CF₃ | COEt | Me | Me | OEt | H |
| CF₃ | COEt | Me | Me | OPh | H |
| CF₃ | COEt | Me | Me | OCOMe | H |
| CF₃ | COEt | Me | Me | OCO₂Me | H |
| CF₃ | COEt | Me | Me | OCO₂Et | H |
| CF₃ | COEt | Me | Me | OCH₂OMe | H |
| CF₃ | COEt | Me | Me | SMe | H |
| CF₃ | COEt | Me | Me | SPh | H |
| CF₃ | COEt | Me | Me | NHMe | H |
| CF₃ | COEt | Me | Me | NMe₂ | H |
| CF₃ | COEt | Me | Et | Me | H |
| CF₃ | COEt | Me | Et | F | H |
| CF₃ | COEt | Me | Et | Cl | H |
| CF₃ | COEt | Me | Et | Br | H |
| CF₃ | COEt | Me | Et | OMe | H |
| CF₃ | COEt | Me | CF₃ | Me | H |
| CF₃ | COEt | Me | CF₃ | F | H |
| CF₃ | COEt | Me | CF₃ | Cl | H |
| CF₃ | COEt | Me | CF₃ | Br | H |
| CF₃ | COEt | Me | CF₃ | OMe | H |
| CF₃ | COEt | Me | Pr-c | Me | H |
| CF₃ | COEt | Me | Pr-c | F | H |
| CF₃ | COEt | Me | Pr-c | Cl | H |
| CF₃ | COEt | Me | Pr-c | Br | H |
| CF₃ | COEt | Me | Pr-c | OMe | H |
| CF₃ | COEt | Me | CH₂OMe | Me | H |
| CF₃ | COEt | Me | CH₂OMe | F | H |
| CF₃ | COEt | Me | CH₂OMe | Cl | H |
| CF₃ | COEt | Me | CH₂OMe | Br | H |
| CF₃ | COEt | Me | CH₂OMe | OMe | H |
| CF₃ | COEt | Me | CH₂Cl | Me | H |
| CF₃ | COEt | Me | CH₂Cl | F | H |
| CF₃ | COEt | Me | CH₂Cl | Cl | H |
| CF₃ | COEt | Me | CH₂Cl | Br | H |
| CF₃ | COEt | Me | CH₂Cl | OMe | H |
| CF₃ | COEt | | -(CH₂₎₃- | Me | H |
| CF₃ | COEt | | -(CH₂)₃- | F | H |
| CF₃ | COEt | | -(CH₂)₃- | Cl | H |
| CF₃ | COEt | | -(CH₂)₃- | Br | H |
| CF₃ | COEt | | -(CH₂)₃- | OMe | H |
| CF₃ | COEt | | -(CH₂)₄- | Me | H |
| CF₃ | COEt | | -(CH₂)₄- | F | H |
| CF₃ | COEt | | -(CH₂)₄- | Cl | H |
| CF₃ | COEt | | -CH₂)₄- | Br | H |
| CF₃ | COEt | | - (CH₂)₄- | OMe | H |
| CF₃ | COEt | | - (CH₂)₅- | Me | H |
| CF₃ | COEt | | - (CH₂)₅- | F | H |
| CF₃ | COEt | | -(CH₂)₅- | Cl | H |
| CF₃ | COEt | | - (CH₂) ₅- | Br | H |
| CF₃ | COEt | | -(CH₂)₅- | OMe | H |
| CF₃ | COPr-n | Me | Me | Me | H |
| CF₃ | COPr-n | Me | Me | F | H |
| CF₃ | COPr-n | Me | Me | Cl | H |
| CF₃ | COPr-n | Me | Me | Br | H |
| CF₃ | COPr-n | Me | Me | OH | H |
| CF₃ | COPr-n | Me | Me | OMe | H |
| CF₃ | COPr-n | Me | Me | SMe | H |
| CF₃ | COPr-i | Me | Me | Me | H |
| CF₃ | COPr-i | Me | Me | F | H |
| CF₃ | COPr-i | Me | Me | Cl | H |
| CF₃ | COPr-i | Me | Me | Br | H |
| CF₃ | COPr-i | Me | Me | OH | H |
| CF₃ | COPr-i | Me | Me | OMe | H |
| CF₃ | COPr-i | Me | Me | SMe | H |
| CF₃ | COPr-c | Me | Me | Me | H |
| CF₃ | COPr-c | Me | Me | F | H |
| CF₃ | COPr-c | Me | Me | Cl | H |
| CF₃ | COPr-c | Me | Me | Br | H |
| CF₃ | COPr-c | Me | Me | OH | H |
| CF₃ | COPr-c | Me | Me | OMe | H |
| CF₃ | COPr-c | Me | Me | SMe | H |
| CF₃ | COBu-t | Me | Me | Me | H |
| CF₃ | COBu-t | Me | Me | F | H |
| CF₃ | COBu-t | Me | Me | Cl | H |
| CF₃ | COBu-t | Me | Me | Br | H |
| CF₃ | COBu-t | Me | Me | OH | H |
| CF₃ | COBu-t | Me | Me | OMe | H |
| CF₃ | COBu-t | Me | Me | SMe | H |
| CF₃ | COPh | Me | Me | Me | H |
| CF₃ | COPh | Me | Me | F | H |
| CF₃ | COPh | Me | Me | Cl | H |
| CF₃ | COPh | Me | Me | Br | H |
| CF₃ | COPh | Me | Me | OH | H |
| CF₃ | COPh | Me | Me | OMe | H |
| CF₃ | COPh | Me | Me | SMe | H |
| CF₃ | CO₂Me | Me | Me | Me | H |
| CF₃ | CO₂Me | Me | Me | F | H |
| CF₃ | CO₂Me | Me | Me | Cl | H |
| CF₃ | CO₂Me | Me | Me | Br | H |
| CF₃ | CO₂Me | Me | Me | OH | H |
| CF₃ | CO₂Me | Me | Me | OMe | H |
| CF₃ | CO₂Me | Me | Me | SMe | H |
| CF₃ | CO₂Et | Me | Me | Me | H |
| CF₃ | CO₂Et | Me | Me | Et | H |
| CF₃ | CO₂Et | Me | Me | F | H |
| CF₃ | CO₂Et | Me | Me | Cl | H |
| CF₃ | CO₂Et | Me | Me | Br | H |
| CF₃ | CO₂Et | Me | Me | I | H |
| CF₃ | CO₂Et | Me | Me | OH | H |
| CF₃ | CO₂Et | Me | Me | OMe | H |
| CF₃ | CO₂Et | Me | Me | OEt | H |
| CF₃ | CO₂Et | Me | Me | OPh | H |
| CF₃ | CO₂Et | Me | Me | OCOMe | H |
| CF₃ | CO₂Et | Me | Me | OCO₂Me | H |
| CF₃ | CO₂Et | Me | Me | OCO₂Et | H |
| CF₃ | CO₂Et | Me | Me | OCH₂OMe | H |
| CF₃ | CO₂Et | Me | Me | SMe | H |
| CF₃ | CO₂Et | Me | Me | SPh | H |
| CF₃ | CO₂Et | Me | Me | NHMe | H |
| CF₃ | CO₂Et | Me | Me | NMe₂ | H |
| CF₃ | CO₂Et | Me | Et | Me | H |
| CF₃ | CO₂Et | Me | Et | F | H |
| CF₃ | CO₂Et | Me | Et | Cl | H |
| CF₃ | CO₂Et | Me | Et | Br | H |
| CF₃ | CO₂Et | Me | Et | OMe | H |
| CF₃ | CO₂Et | Me | CF₃ | Me | H |
| CF₃ | CO₂Et | Me | CF₃ | F | H |
| CF₃ | CO₂Et | Me | CF₃ | Cl | H |
| CF₃ | CO₂Et | Me | CF₃ | Br | H |
| CF₃ | CO₂Et | Me | CF₃ | OMe | H |
| CF₃ | CO₂Et | Me | Pr-c | Me | H |
| CF₃ | CO₂Et | Me | Pr-c | F | H |
| CF₃ | CO₂Et | Me | Pr-c | Cl | H |
| CF₃ | CO₂Et | Me | Pr-c | Br | H |
| CF₃ | CO₂Et | Me | Pr-c | OMe | H |
| CF₃ | CO₂Et | Me | CH₂OMe | Me | H |
| CF₃ | CO₂Et | Me | CH₂OMe | F | H |
| CF₃ | CO₂Et | Me | CH₂OMe | Cl | H |
| CF₃ | CO₂Et | Me | CH₂OMe | Br | H |
| CF₃ | CO₂Et | Me | CH₂OMe | OMe | H |
| CF₃ | CO₂Et | Me | CH₂Cl | Me | H |
| CF₃ | CO₂Et | Me | CH₂Cl | F | H |
| CF₃ | CO₂Et | Me | CH₂Cl | Cl | H |
| CF₃ | CO₂Et | Me | CH₂Cl | Br | H |
| CF₃ | CO₂Et | Me | CH₂Cl | OMe | H |
| CF₃ | CO₂Et | | -(CH₂)₃- | Me | H |
| CF₃ | CO₂Et | | -(CH2)₃- | F | H |
| CF₃ | CO₂Et | | -(CH₂)₃- | Cl | H |
| CF₃ | CO₂Et | | -(CH₂)₃- | Br | H |
| CF₃ | CO₂Et | | -(CH₂)₃- | OMe | H |
| CF₃ | CO₂Et | | -(CH₂)₄- | Me | H |
| CF₃ | CO₂Et | | -(CH₂)₄- | F | H |
| CF₃ | CO₂Et | | -(CH₂)₄- | Cl | H |
| CF₃ | CO₂Et | | -(CH₂)₄- | Br | H |
| CF₃ | CO₂Et | | -(CH₂)₄- | OMe | H |
| CF₃ | CO₂Et | | -(CH₂)₅- | Me | H |
| CF₃ | CO₂Et | | -(CH₂)₅- | F | H |
| CF₃ | CO₂Et | | -(CH₂)₅- | Cl | H |
| CF₃ | CO₂Et | | -(CH₂)₅- | Br | H |
| CF₃ | CO₂Et | | -(CH₂)₅- | OMe | H |
| CF₃ | CO₂Pr-n | Me | Me | Me | H |
| CF₃ | CO₂Pr-n | Me | Me | F | H |
| CF₃ | CO₂Pr-n | Me | Me | Cl | H |
| CF₃ | CO₂Pr-n | Me | Me | Br | H |
| CF₃ | CO₂Pr-n | Me | Me | OH | H |
| CF₃ | CO₂Pr-n | Me | Me | OMe | H |
| CF₃ | CO₂Pr-n | Me | Me | SMe | H |
| CF₃ | CO₂Pr-i | Me | Me | Me | H |
| CF₃ | CO₂Pr-i | Me | Me | F | H |
| CF₃ | CO₂Pr-i | Me | Me | Cl | H |
| CF₃ | CO₂Pr-i | Me | Me | Br | H |
| CF₃ | CO₂Pr-i | Me | Me | OH | H |
| CF₃ | CO₂Pr-i | Me | Me | OMe | H |
| CF₃ | CO₂Pr-i | Me | Me | SMe | H |
| CF₃ | CO₂Bu-n | Me | Me | Me | H |
| CF₃ | CO₂Bu-n | Me | Me | F | H |
| CF₃ | CO₂Bu-n | Me | Me | Cl | H |
| CF₃ | CO₂Bu-n | Me | Me | Br | H |
| CF₃ | CO₂Bu-n | Me | Me | OH | H |
| CF₃ | CO₂Bu-n | Me | Me | OMe | H |
| CF₃ | CO₂Bu-n | Me | Me | SMe | H |
| CF₃ | CO₂Bu-i | Me | Me | Me | H |
| CF₃ | CO₂Bu-i | Me | Me | Et | H |
| CF₃ | CO₂Bu-i | Me | Me | F | H |
| CF₃ | CO₂Bu-i | Me | Me | Cl | H |
| CF₃ | CO₂Bu-i | Me | Me | Br | H |
| CF₃ | CO₂Bu-i | Me | Me | I | H |
| CF₃ | CO₂Bu-i | Me | Me | OH | H |
| CF₃ | CO₂Bu-i | Me | Me | OMe | H |
| CF₃ | CO₂Bu-i | Me | Me | OEt | H |
| CF₃ | CO₂Bu-i | Me | Me | OPh | H |
| CF₃ | CO₂Bu-i | Me | Me | OCOMe | H |
| CF₃ | CO₂Bu-i | Me | Me | OCO₂Me | H |
| CF₃ | CO₂Bu-i | Me | Me | OCO₂Et | H |
| CF₃ | CO₂Bu-i | Me | Me | OCH₂OMe | H |
| CF₃ | CO₂Bu-i | Me | Me | SMe | H |
| CF₃ | CO₂Bu-i | Me | Me | SPh | H |
| CF₃ | CO₂Bu-i | Me | Me | NHMe | H |
| CF₃ | CO₂Bu-i | Me | Me | NMe₂ | H |
| CF₃ | CO₂Bu-i | Me | Et | Me | H |
| CF₃ | CO₂Bu-i | Me | Et | F | H |
| CF₃ | CO₂Bu-i | Me | Et | Cl | H |
| CF₃ | CO₂Bu-i | Me | Et | Br | H |
| CF₃ | CO₂Bu-i | Me | Et | OMe | H |
| CF₃ | CO₂Bu-i | Me | CF₃ | Me | H |
| CF₃ | CO₂Bu-i | Me | CF₃ | F | H |
| CF₃ | CO₂Bu-i | Me | CF₃ | Cl | H |
| CF₃ | CO₂Bu-i | Me | CF₃ | Br | H |
| CF₃ | CO₂Bu-i | Me | CF₃ | OMe | H |
| CF₃ | CO₂Bu-i | Me | Pr-c | Me | H |
| CF₃ | CO₂Bu-i | Me | Pr-c | F | H |
| CF₃ | CO₂Bu-i | Me | Pr-c | Cl | H |
| CF₃ | CO₂Bu-i | Me | Pr-c | Br | H |
| CF₃ | CO₂Bu-i | Me | Pr-c | OMe | H |
| CF₃ | CO₂Bu-i | Me | CH₂OMe | Me | H |
| CF₃ | CO₂Bu-i | Me | CH₂OMe | F | H |
| CF₃ | CO₂Bu-i | Me | CH₂OMe | Cl | H |
| CF₃ | CO₂Bu-i | Me | CH₂OMe | Br | H |
| CF₃ | CO₂Bu-i | Me | CH₂OMe | OMe | H |
| CF₃ | CO₂Bu-i | Me | CH₂Cl | Me | H |
| CF₃ | CO₂Bu-i | Me | CH₂Cl | F | H |
| CF₃ | CO₂Bu-i | Me | CH₂Cl | Cl | H |
| CF₃ | CO₂Bu-i | Me | CH₂Cl | Br | H |
| CF₃ | CO₂Bu-i | Me | CH₂Cl | OMe | H |
| CF₃ | CO₂Bu-i | -(CH₂)₃- | | Me | H |
| CF₃ | CO₂Bu-i | -(CH₂)₃- | | F | H |
| CF₃ | CO₂Bu-i | -(CH₂)₃- | | Cl | H |
| CF₃ | CO₂Bu-i | -(CH₂)₃- | | Br | H |
| CF₃ | CO₂Bu-i | -(CH₂)₃- | | OMe | H |
| CF₃ | CO₂Bu-i | -(CH₂)₄- | | Me | H |
| CF₃ | CO₂Bu-i | -(CH₂)₄- | | F | H |
| CF₃ | CO₂Bu-i | -(CH₂)₄- | | Cl | H |
| CF₃ | CO₂Bu-i | -(CH₂)₄- | | Br | H |
| CF₃ | CO₂Bu-i | -(CH₂)₄- | | OMe | H |
| CF₃ | CO₂Bu-i | -(CH₂)₅- | | Me | H |
| CF₃ | CO₂Bu-i | -(CH₂)₅- | | F | H |
| CF₃ | CO₂Bu-i | -(CH₂)₅- | | Cl | H |
| CF₃ | CO₂Bu-i | -(CH₂)₅- | | Br | H |
| CF₃ | CO₂Bu-i | -(CH₂)₅- | | OMe | H |
| CF₃ | E-1 | Me | Me | Me | H |
| CF₃ | E-1 | Me | Me | F | H |
| CF₃ | E-1 | Me | Me | Cl | H |
| CF₃ | E-1 | Me | Me | Br | H |
| CF₃ | E-1 | Me | Me | OH | H |
| CF₃ | E-1 | Me | Me | OMe | H |
| CF₃ | E-1 | Me | Me | SMe | H |
| CF₃ | CO₂Bu-t | Me | Me | Me | H |
| CF₃ | CO₂Bu-t | Me | Me | F | H |
| CF₃ | CO₂Bu-t | Me | Me | Cl | H |
| CF₃ | CO₂Bu-t | Me | Me | Br | H |
| CF₃ | CO₂Bu-t | Me | Me | OH | H |
| CF₃ | CO₂Bu-t | Me | Me | OMe | H |
| CF₃ | CO₂Bu-t | Me | Me | SMe | H |
| CF₃ | CO₂Ph | Me | Me | Me | H |
| CF₃ | CO₂Ph | Me | Me | F | H |
| CF₃ | CO₂Ph | Me | Me | Cl | H |
| CF₃ | CO₂Ph | Me | Me | Br | H |
| CF₃ | CO₂Ph | Me | Me | OH | H |
| CF₃ | CO₂Ph | Me | Me | OMe | H |
| CF₃ | CO₂Ph | Me | Me | SMe | H |
| CF₃ | CO₂CH₂CH₂Cl | Me | Me | Me | H |
| CF₃ | CO₂CH₂CH₂Cl | Me | Me | F | H |
| CF₃ | CO₂CH₂CH₂Cl | Me | Me | Cl | H |
| CF₃ | CO₂CH₂CH₂Cl | Me | Me | Br | H |
| CF₃ | CO₂CH₂CH₂Cl | Me | Me | OH | H |
| CF₃ | CO₂CH₂CH₂Cl | Me | Me | OMe | H |
| CF₃ | CO₂CH₂CH₂Cl | Me | Me | SMe | H |
| CF₃ | E-2 | Me | Me | Me | H |
| CF₃ | E-2 | Me | Me | F | H |
| CF₃ | E-2 | Me | Me | Cl | H |
| CF₃ | E-2 | Me | Me | Br | H |
| CF₃ | E-2 | Me | Me | OH | H |
| CF₃ | E-2 | Me | Me | OMe | H |
| CF₃ | E-2 | Me | Me | SMe | H |
| CF₃ | E-3 | Me | Me | Me | H |
| CF₃ | E-3 | Me | Me | F | H |
| CF₃ | E-3 | Me | Me | Cl | H |
| CF₃ | E-3 | Me | Me | Br | H |
| CF₃ | E-3 | Me | Me | OH | H |
| CF₃ | E-3 | Me | Me | OMe | H |
| CF₃ | E-3 | Me | Me | SMe | H |
| CF₃ | CH₂OMe | Me | Me | Me | H |
| CF₃ | CH₂OMe | Me | Me | F | H |
| CF₃ | CH₂OMe | Me | Me | Cl | H |
| CF₃ | CH₂OMe | Me | Me | Br | H |
| CF₃ | CH₂OMe | Me | Me | OH | H |
| CF₃ | CH₂OMe | Me | Me | OMe | H |
| CF₃ | CH₂OMe | Me | Me | SMe | H |
| CF₃ | E-4 | Me | Me | Me | H |
| CF₃ | E-4 | Me | Me | F | H |
| CF₃ | E-4 | Me | Me | Cl | H |
| CF₃ | E-4 | Me | Me | Br | H |
| CF₃ | E-4 | Me | Me | OH | H |
| CF₃ | E-4 | Me | Me | OMe | H |
| CF₃ | E-4 | Me | Me | SMe | H |
| CF₃ | SO₂CF₃ | Me | Me | Me | H |
| CF₃ | SO₂CF₃ | Me | Me | F | H |
| CF₃ | SO₂CF₃ | Me | Me | Cl | H |
| CF₃ | SO₂CF₃ | Me | Me | Br | H |
| CF₃ | SO₂CF₃ | Me | Me | OH | H |
| CF₃ | SO₂CF₃ | Me | Me | OMe | H |
| CF₃ | SO₂CF₃ | Me | Me | SMe | H |
| | | | | | |

In Table, E-1 to E-4 are the same as defined above.

The compounds of the present invention may be used as a herbicide for paddy fields in submerged soil treatment or foliage treatment.

Paddy fields weeds include, for example, Potamogetonaceae weeds such as roundleaf pondweed (Potamogeton distinctus), Alismataceae weeds such as narrowleaf waterplantain (Alisma canaliculatum), japanese ribbon wapato (Sagittaria pygmaea) and arrowhead (Sagittaria trifolia), Gramineae weeds such as sprangletop (Leptochloa chinensis),barnyardgrass (Echinochloa crus-galli), barnyardgrass (Echinochloa oryzicola), leersia japonica Makino (Homalocenchrus japonocus) and Knotgrass (Paspalum distichum), Cyperaceae weeds such as water chestnut (Eleocharis kuroguwai), japanese bulrush (Scirpus juncoides), Shizui (Scirpus nipponicus), water nutgrass (Cyperus serotinus), umbrellaplant (Cyperus difformis) and Hinagayatsuri (Cyperus hakonensis), Lemnaceae weeds such as giant duckweed (Spirodela polyrhiza) and duckmeat (Lemna paucicostata), Commelinaceae weeds such as march dayflower (Murdannia keisak), Pontederiaceae weeds such as monochoria species (Monochoria korsakowii) and monochoria (Monochoria vaginalis), Elatinaceae weeds such as waterwort (Elatine triandra), Lythraceae weeds such as red stem (Ammannia multiflora) and toothcup (Rotala indica), Oenotheraceae weeds such as fireweed ludwigia (Lidwigia epilobioides), Scrophulariaceae weeds such as dopatorium (Dopatrium junceum), Ooabunome (Gratiola japonica), limnophila (Limnophila sessilifolia), false pimpernel (Lindernia pyxidaria) and low falsepimpemel (Lindernia dubia), Leguminosae weeds such as Indian jointvetch (Aeschynomene indica) and Compositae weeds such as devils beggarticks (Bidens frondosa) and bur beggarticks (Bidens tripartite).

The compounds of the present invention may be used as a herbicide for upland fields and orchards in soil treatment, soil incorporation treatment and foliage treatment.

Cropland weeds include, for example, broad-leaved weeds such as Solanaceous weeds (Solanaceae) represented by black nightshade (Solanum nigrum) and jimsonweed (Datura stramonium), Malvaceous weeds (Malvaceae) represented by velvetleaf (Abutilon theophrasti) and prickly sida (Sida spinosa), Convolvulaceous weeds (Convolvulaceae) represented by morningglories (Ipomoea spps.) including common morningglory (Ipomoea purpurea) and bindweeds (Calystegia spps.), Amaranthaceous weeds (Amaranthaceae) represented by livid amaranth (Amaranthus lividus) and redroot pigweed (Amaranthus retroflexus), Composite weeds (Compositae) represented by common cocklebur (xanthium pensylvanicum), common ragweed (Ambrosia artemisiaefolia), sunflower (Helianthus annuus), hairy galinsoga (Galinsoga ciliate), Creeping thistle (Cirsium arvense), common groundsel (Senecio vulgaris) and annual fleabane (Erigeron annus), Cruciferous weeds (Cruciferae) represented by India field cress (Rorippa indica), kedlock (Sinapis arvensis) and shepherd's purse (Capsella Bursapastoris), Polygonaceous weeds (Polygonaceae) represented by posumbu knotweed (Polygonum Blumei) and wild buckwheat (Polygonum convolvulus), Portulacaceous weeds (Portulacaceae) represented by common purslane (Portulaca oleracea), Chenopodiaceous weeds (Chenopodiaceae) represented by common lambsquater (Chenopodium album), figleaved goosefoot (Chenopodium ficifolium) and kochia (Kochia scoparia), Caryophyllaceous weeds (Caryophyllaceae) represented by common chickweed (Stellaria media), Scrophulariaceous weeds (Scrophulariaceae) represented by persian speedwell (Veronica persica), Commelinaceous weeds (Commelinaceae) represented by asiatic dayflower (Commelina communis), Labiate weeds (Labiatae) represented by deadnettle (Lamium amplexicaule) and red deadnettle (Lamium purpureum), Euphorbiaceous weeds (Euphorbiaceae) represented by prostrate spurge (Euphorbia supine) and spotted spurge (Euphorbia maculate), Rubiaceous weeds (Rubiaceae) represented by beadstraw (Galium spurium) and Indian madder (Rubia akane), Violaceous weeds (Violaceae) represented by violet (Viola mandshurica), Leguminous weeds (Leguminosae) represented by hempsesbania (Sesbania exaltata) and sicklepod (Cassia obtusifolia), Oxsaldaseous weeds (Oxsaldaseae) represented by creeping woodsorrel (Oxsalis courniculata), and Graminaceous weeds represented by shatternace (Sorgham bicolor), fall panicum (Panicum dichotomiflorum), johnsongrass (Sorghum halepense), barnyardgrass (Echinochloa crus-galli var. crus-galli), barnyardgrass (Echinochloa crus-galli var. praticola), barnyardgrass (crop) (Echinochloa utilis), large crabgrass (Digitaria ciliaris), wild oat (Avena fatua), blackgrass (Alopecurus myosuroides), goosegrass (Eleusine indica), green foxtail (Setaria viridis), giant foxtail (Setaria faberi) and water foxtail (Alopecurus aegualis), and Cyperaceous weeds represented by purple nutsedge (Cyperus rotundus,Cyperus esculentus).

The compounds of the present invention may be used not only agriculturally and horticulturally for paddy fields, upland fields and orchards but also for non-agricultural fields such as lawns, playgrounds, vacant lots, road shoulders and railroad shoulders in soil treatment, soil incorporation treatment and foliage treatment. The target weeds include, in addition to the cropland and orchard weeds mentioned above, annual bluegrass (Poa annua), dandelion (Taraxacum officinale), tall fleabane (Conyza sumatrensis), flexuous bittercress (Cardamine flexuosa), white clover (Trifolium repens), lawn pennywort (Hydrocotyle sibthorpioides), Asiatic plantain (Plantago asiatica), himekugu (Cyperus brevifolius, Kyllinga brevifolia), field horsetail (Equisetum arvense) and the like.

The compounds of the present invention may be combined with other herbicides, various insecticides, fungicides, plant growth regulators or synergists at the time of the preparation of the formulations or at the time of the application, as the case requires.

Particularly, combined use of the compound of the present invention with another herbicide can be expected to result in lower cost attributable to reduction in the dose, a broader spectrum and a higher herbicidal effect attributable to synergistic action of the combined chemical. In such a case, the compound of the present invention can be combined with plural known herbicides simultaneously.

Herbicides preferably combined with the compounds of the present invention include, for example, alachlor (general name), ametryn (general name), aminocyclopirachlor (general name), aminopyralid (general name), amiprophosmethyl (general name), anilofos (general name), asulam (general name), atrazine (general name), azimsulfuron (general name), bencarbazone (general name), benefin (general name), benfuresate (general name), bensulfuron-methyl (general name), bensulide (general name), bentazone (general name), a salt of bentazone, benthiocarb (general name), benzobicyclon (general name), benzofenap (general name), bialaphos, bicyclopyrone (general name), bifenox (general name), bispyribac (general name), bromacil (general name), bromobutide (general name), butachlor (general name), butamifos (general name), butenachlor (general name), cafenstrole (general name), carfentrazone-ethyl, chlomethoxynil (general name), chloridazon (general name), chlorphtalim (general name), chlorpropham (general name), TCTP (chlorthal-dimethyl, tetorachlorothiophene (general name), cinmethylin (general name), cinosulfuron (general name), clodinafop (general name), clomazone (general name), clomeprop, clopyralid (general name), CNP (general name), cumyluron (general name), cyanazin (general name), cyclosulfamuron (general name), cyhalofop-butyl (general name), dicamba (general name), dichlobenil (general name), diflufenican (general name), dimepiperate (general name), dimethametryn (general name), dimethenamid (general name), dimethenamid-p (general name), dithiopyl (general name), diuron (general name), dymron (general name), esprocarb (general name), ethofumesate (general name), etobenzanid (general name), ethoxysulfuron (general name), flazasulfuron (general name), fenoxaprop-ethyl (general name), fenoxasulfone (general name), fentrazamide (general name), florasulam (general name), fluazifop-butyl (general name), flucarbazone-sodium (general name), flucetosulfuron (general name), flupoxam (general name), glufosinate-ammonium (general name), glyphosate-ammonium (general name), glyphosate-iso-propylammonium (general name), glyphosate-potassium (general name), glyphosate-sodium (general name), glyphosate-trimesium (general name), halosulfuron-methyl (general name), hexazinone (general name), imazamox (general name), imazapyr (general name), imazethapyr (general name), imazaquin (general name), imazosulfuron (general name), indanofan (general name), indaziflam (general name), iodosulfuron-methyl-sodium (general name), ioxynil octanoate (general name), ipufencarbazone (general name), isoproturon (general name), isouron (general name), isoxaben (general name), isoxaflutole (general name), karbutilate (general name), lenacil (general name), linuron (general name), MCC (general name), MCPA (general name), MCPB (general name), MCPPA (general name), mefenacet (general name), mesosulfuron-methyl (general name), mesotrione (general name), metamifop (general name), metamitron (general name), metazosulfuron (general name), methiozolin (general name), methyl dymron (general name), metolachlor (general name), metribuzin (general name), metsulfuron-methyl (general name), molinate (general name), monosulfuron (general name), monosulfuron-methyl (general name), naproanilide (general name), napropamide (general name), nicosulfuron (general name), norflurazon (general name), OK-701 (trial name), orbencarb (general name), orthosulfamuron (general name), oxadiargyl (general name), oxadiazon (general name), oxaziclomefone (general name), pendimethalin (general name), penoxsulam (general name), pentoxazone (general name), phenmedipham (general name), picolinafen (general name), pinoxaden (general name), pretilachlor (general name), prodiamine (general name), prometryn (general name), propanil (general name), propisochlor (general name), propoxycarbazone-sodium (general name), propyrisulfuron (general name), propyzamide (general name), pyraclonil (general name), pyrasulfotole (general name), pyrazolynate (general name), pyrazosulfuron-ethyl (general name), pyrazoxyfen (general name), pyributicarb (general name), pyriftalid (general name), pyriminobac-methyl (general name), pyrimisulfan (general name), pyroxasulfone (general name), pyroxsulam (general name), quinclorac (general name), quinoclamine (general name), quizalofop-ethyl (general name), saflufenacil (general name), sethoxydim (general name), siduron (general name), simazine (general name), simetryn (general name), sulfosulfuron (general name), tebuthiuron (general name), tefuryltrione (general name), tembotrione (general name), tepraloxydim (general name), tetrapion/flupropanate (general name), thenylchlor (general name), thiazafluron (general name), thiencarbazone-methyl (general name), thifensulfuron-methyl (general name), topramezon (general name), triaziflam (general name), trifloxysulfuron (general name), trifluralin (general name), 2,4-PA (general name), an ester of 2,4-PA, and a salt of 2,4-PA. These agrochemically active ingredients may be used singly or as a mixture of at least two, and in the case of a mixture, the ratio may be optionally selected.

When the compounds of the present invention are used as a herbicide, they are usually mixed with a suitable solid carrier or a liquid carrier. Further, if desired, a surfactant, a penetrating agent, a spreader, a thickner, an antifreezing agent, a binder, an anticaking agent, a stabilizing agent or the like is added to prepare an optional formulation such as a liquid formulation, an emulsifiable concentrate, a wettable powder, a dry flowable, a flowable, a dust or a granule.

Further, such formulations as mentioned above may be encapsulated in a watersoluble material to save labor and improve safety.

As solid carriers, for example, natural minerals such as kaolinite, pyrophyllite, sericite, talc, bentonite, acid clay, attapulgite, zeolite and diatomaceous earth, inorganic salts such as calcium carbonate, ammonium sulfate, sodium sulfate and potassium chloride, synthetic silica and synthetic silicates may be mentioned.

As liquid carriers, for example, water, alcohols (such as ethylene glycol, propylene glycol, isopropanol), aromatic hydrocarbons (such as xylene, alkylbenzene, alkylnaphthalene), ethers (such as butyl cellosolve), ketones (scuh as cyclohexanone), esters (such as y-butyrolactone), acid amides (such as N-methylpyrrolidone, N-octylpyrrolidone) and vegetable oils (scuh as soybean oil, rapeseed oil, cottonseed oil and castor oil) may be mentioned.

These solid and liquid carriers may be used singly or in combination of at least two.

As surfactants, for example, nonionic surfactants such as polyoxyethylene alkyl aryl ether, polyoxyethylene styryl phenyl ether, polyoxyethylene polyoxypropylene block copolymers, polyoxyethylene fatty acid esters, sorbitan fatty acid esters and polyoxyethylene sorbitan fatty acid esters, and ionic surfactants such as alkylbenzenesulfonate salts, lignin sulfonate salts, alkylsulfosuccinates, naphthalenesulfonate salts, alkylnaphthalenesulfonate salts, naphthalenesulfonic acid formalin condensate salts, alkylnaphthalenesulfonic acid formalin condensate salts, polyoxyethlene alkyl aryl ether sulfate or phosphate, polyoxyethylene styryl phenyl ether sulfate or phosphate and alkylamine salts may be mentioned.

The amounts of these surfactants are not particularly limited, but usually, they are preferably from 0.05 to 20 parts by weight in relation to 100 parts by weight of the formulation of the present invention. These surfactants may be used singly or in combination of at least two.

Now, examples of formulations of the compounds of the present invention will be given. However, it should be understood that the present invention is by no means restricted to such specific examples. In the following Formulation Examples, "parts" means parts by weight.

**Wettable powder**

| | |
|---|---|
| Compound of the present invention | 0.1-80 parts |
| Solid carrier | 5-98.9 parts |
| Surfactant | 1-10 parts |
| Others | 0-5 parts |

As the others, for example, an anticaking agent and a stabilizing agent may be mentioned.

**Emulsifiable concentrate**

| | |
|---|---|
| Compound of the present invention | 0.1-30 parts |
| Liquid carrier | 55-95 parts |
| Surfactant | 4.9-15 parts |

**Flowable**

| | |
|---|---|
| Compound of the present invention | 0.1-70 parts |
| Liquid carrier | 15-98.89 parts |
| Surfactant | 1-12 parts |
| Others | 0.01-30 parts |

As the others, for example, an antifreezing agent and a thickener may be mentioned.

**Dry flowable**

| | |
|---|---|
| Compound of the present invention | 0.1-90 parts |
| Solid carrier | 0-98.9 parts |
| Surfactant | 1-20 parts |
| Others | 0-10 parts |

As the others, for example, a binder and a stabilizing agent may be mentioned.

**Liquid formulation**

| | |
|---|---|
| Compound of the present invention | 0.01-30 parts |
| Liquid carrier | 0.1-50 parts |
| Water | 50-99.89 parts |
| Others | 0-10 parts |

As the others, for example, an antifreezing agent and a spreader may be mentioned.

**Granule**

| | |
|---|---|
| Compound of the present invention | 0.01-10 parts |
| Solid carrier | 90-99.99 parts |
| Others | 0-10 parts |

As the others, for example, a binder and a stabilizing agent may be mentioned. The above-mentioned formulations are applied directly or after diluted with water by a factor of 1 to 10000.

### PREPARATION EXAMPLES

Now, specific examples of agrochemical preparations containing compounds of the present invention as active ingredients will be given. However, it should be understood that the present invention is by no means restricted to such specific examples. In the following Formulation Examples, "parts" means parts by weight.

**[Formulation Example 1] Wettable powder**

| | |
|---|---|
| Compound I-1 of the present invention | 20 parts |
| Pyrophyllite | 76 parts |
| Sorpol 5039 | 2 parts |
| (trade name for a mixture of a nonionic surfactant and an anionic surfactant manufactured by Toho Chemical Industry Co., Ltd.) | |
| Carplex #80 | 2 parts |
| (trade name for a synthetic hydrous silicate manufactured by Shionogi Pharmaceutical Co., Ltd.) | |

The above ingredients are homogenously pulverized and mixed to form a wettable powder.

**[Formulation Example 2] Emulsifiable concentrate**

| | |
|---|---|
| Compound I-1 of the present invention | 5 parts |
| Xylene | 75 parts |
| N-Methylpyrrolidone | 15 parts |
| Sorpol 2680 | 5 parts |
| (trade name for a mixture of a nonionic surfactant and an anionic surfactant manufactured by Toho Chemical Industry Co., Ltd.) | |

The above ingredients are mixed to form an emulsifiable concentrate.

**[Formulation Example 3] Flowable**

| | |
|---|---|
| Compound I-1 of the present invention | 25 parts |
| Agrizole S-710 | 10 parts |
| (trade name for a nonionic surfactant manufactured Kao Corporation) | |
| Lunox 1000C | 0.5 part |
| (trade name for an anionic surfactant manufactured by Toho Chemical Industry Co., Ltd.) | |
| Xanthan gum | 0.02 part |
| Water | 64.48 parts |

The above ingredients are homogeneously mixed and wet-pulverized to form a flowable.

**[Formulation Example 4] Dry flowable**

| | |
|---|---|
| Compound I-1 of the present invention | 75 parts |
| HITENOL NE-15 | 5 parts |
| (trade name for an anionic surfactant manufactured by Dai-Ichi Kogyo Seiyaku Co., Ltd.) | |
| Vanilex N | 10 parts |
| (trade name for an anionif surfactant manufactured by Nippon Paper Industries Co., Ltd.) | |
| Carplex #80 | 10 parts |
| (trade name for a synthetic hydrous silicate manufactured by Shionogi Parmaceutical Co., Ltd.) | |

The above ingredients are homogeneously mixed and pulverized, then kneaded with a small amount of water by stirring and granulated through an extrusion granulator and dried to form a dry flowable.

**[Formulation Example 5] Granule**

| | |
|---|---|
| Compound I-1 of the present invention | 1 part |
| Bentonite | 55 parts |
| Talc | 44 parts |

The above ingredients are homogenously mixed and pulverized, then kneaded with a small amount of water by stirring and granulated through an extrusion granulator and dried to form a granule.

**[Formulation Example 6] Granule**

| | |
|---|---|
| Compound I-1 of the present invention | 1 part |
| Compound (A) | 0.07 part |
| DBSN | 3 parts |
| Epoxylated soybean oil | 1 part |
| Bentonite | 30 parts |
| Talc | 64.93 parts |

| | |
|---|---|
| DBSN means sodium dodecylbenzenesulfonate. | |

Now, the syntheses of the compounds of the present invention will be described with reference to Examples. However, it should be understood that the present invention is by no means restricted to such specific Examples.

### EXAMPLES

### EXAMPLE 1

### Synthesis of 4-[2-(N-ethoxycarbonyl-N-trifluoromethanesulfonyl)aminobenzyl]-1,4-oxazinan-3-one (Compound I-2 of the present invention)

### Step 1:

### Synthesis of 2-(2-nitrobenzylamino)ethanol

2-Nitrobenzylbromide (12.4 g, 57.3 mmol) was added to 2-aminoethanol (17.5 g, 280 mmol), followed by stirring at room temperature for 1 hour. After the reaction, chloroform and water were added, and the organic layer was separated, washed with water, then dried with saturated aqueous sodium chloride and over anhydrous sodium sulfate and concentrated under reduced pressure to obtain 11.2 g of the desired product.

### Step 2:

### Synthesis of 2-chloro-N-(2-hydroxyethyl)-N-(2-nitrobenzyl)acetamide

2-(2-Nitrobenzylamino)ethanol (3.0 g, 15.3 mmol) was dissolved in 50 ml of dichloromethane, and triethylamine (2.3 g, 23.0 mmol) was added. 2-Chloroacetyl chloride (1.9 g, 16.7 mmol) was added with stirring under cooling with ice, followed by stirring for 2 hours. After the reaction, chloroform and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate=1/1 to 5/95) (volume ratio, the same applies hereinafter) to obtain 1.50 g of the desired product.

### Step 3:

### Synthesis of 4-(2-nitrobenzyl)-1,4-oxazinan-3-one

2-Chloro-N-(2-hydroxyethyl)-N-(2-nitrobenzyl)acetamide (0.3 g, 1.10 mmol) was dissolved in 9 ml of tetrahydrofuran, and 63 wt% sodium hydride (dispersed in mineral oil, 50 mg, 1.31 mmol) was added, followed by stirring at room temperature overnight. After the reaction, ethyl acetate and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate=9/1 to 1/1) to obtain 0.1 g of the desired product.

### Step 4:

### Synthesis of 4-(2-aminobenzyl)-1,4-oxazinan-3-one

4-(2-Nitrobenzyl)-1,4-oxazinan-3-one (2.0 g, 8.47 mmol) was dissolved in a mixed solvent comprising 50 ml of ethanol and 5 ml of water, and reduced iron (7.6 g, 136 mmol) and ammonium chloride (0.96 g, 16.9 mmol) were added, followed by reflux under heating for 6 hours with stirring. After the reaction, the reaction mixture was filtrated through Celite, the filtrate was concentrated under reduced pressure, and the resulting residue was purified by silica gel column chromatography (n-hexane/ethyl acetate=4/6) to obtain 0.96 g of the desired product.

### Step 5:

### Synthesis of 4-(2-trifluoromethanesulfonylaminobenzyl)-1,4-oxazinan-3-one (Compound I-1 of the present invention)

4-(2-Aminobenzyl)-1,4-oxazinan-3-one (0.96 g, 4.66 mmol) was dissolved in 40 ml of dichloromethane, and triethylamine (1.9 g, 18.6 mmol) was added. Then, trifluoromethanesulfonic anhydride (2.6 g, 9.32 mmol) was added dropwise with stirring at -78°C, followed by stirring for 1 hour. After the reaction, a saturated ammonium chloride aqueous solution and chloroform were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate=9/1 to 1/9) to obtain 1.34 g of the desired product (Compound I-1 of the present invention).

### Step 6:

### Synthesis of 4-[2-(N-ethoxycarbonyl-N-trifluoromethanesulfonyl)aminobenzyl]-1,4-oxazinan-3-one (Compound I-2 of the present invention)

4-(2-Trifluoromethanesulfonylaminobenzyl)-1,4-oxazinan-3-one (0.30 g, 0.89 mmol) was dissolved in 4 ml of acetonitrile, ethyl chloroformate (0.48 g, 4.44 mmol) and sodium hydrogencarbonate (0.37 g, 4.44 mmol) were added, followed by reflux under heating for 3 hours with stirring. After the reaction, ethyl acetate and water were added, and the organic layer was separated, dried with saturated aqueous sodium chloride and over anhydrous sodium sulfate and concentrated under reduced pressure, and the resulting residue was purified by silica gel column chromatography (n-hexane/ethyl acetate=9/1 to 1/9) to obtain 215 mg of the desired product (Compound I-2 of the present invention).

### EXAMPLE 2

### Synthesis of 2-methyl-4-[2-(N-ethoxycarbonyl-N-trifluoromethanesulfonyl)aminobenzyl]-1,4-oxazinan-3-one (Compound I-5 of the present invention)

### Step 1:

### Synthesis of 2-methyl-1,4-oxazinan-3-one

63 wt% sodium hydride (dispersed in mineral oil, 6.1 g, 160 mmol) was washed with hexane, and a mixed solution comprising 2-aminoethanol (8.9 g, 150 mmol) and 80 ml of 1,4-dioxane was added dropwise with stirring under cooling with ice, followed by reflux under heating for 10 minutes with stirring. Then, a mixed solution comprising ethyl 2-chloropropionate (17.9 g, 150 mmol) and 30 ml of 1,4-dioxane was added dropwise with stirring under cooling with ice, followed by reflux under heating for 1 hour with stirring. After the reaction, the reaction mixture was filtrated, the filtrate was concentrated under reduced pressure, and the resulting residue was purified by silica gel column chromatography (n-hexane/ethyl acetate=5/95) to obtain 9.39 g of the desired product.

### Step 2:

### Synthesis of 2-methyl-4-(2-trifluoromethanesulfonylaminobenzyl)-1,4-oxazinan-3-one (Compound I-4 of the present invention)

2-Methyl-1,4-oxazinan-3-one (4.15 g, 36.0 mmol) and p-toluenesulfonic acid monohydrate (3.4 g, 17.9 mmol) were added to a solution of 1,1,1-trifluoro-N-[2-(hydroxymethyl)phenyl]methanesulfonamide (4.6 g, 18.0 mmol) in 100 ml of m-xylene, followed by reflux under heating with stirring for 3 hours with azeotropic water separation with a dean-stark apparatus. After the reaction, the reaction mixture was concentrated under reduced pressure, and the resulting residue was purified by silica gel column chromatography (n-hexane/ethyl acetate=9/1 to 1/1) to obtain 0.64 g of the desired product (Compound I-4 of the present invention).

### Step 3:

### Synthesis of 2-methyl-4-[2-(N-ethoxycarbonyl-N-trifluoromethanesulfonyl)aminobenzyl]-1,4-oxazinan-3-one (Compound I-5 of the present invention)

2-Methyl-4-(2-trifluoromethanesulfonylaminobenzyl)-1,4-oxazinan-3-one (0.21 g, 0.60 mmol) was dissolved in 3 ml of acetonitrile, and ethyl chloroformate (0.26 g, 2.38 mmol) and sodium hydrogencarbonate (0.20 g, 2.38 mmol) were added, followed by reflux under heating for 3 hours with stirring. After the reaction, ethyl acetate and water were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/1) to obtain 198 mg of the desired product (Compound I-5 of the present invention).

### EXAMPLE 3

### Synthesis of 6,6-dimethyl-3-[2-(N-ethoxycarbonyl-N-trifluoromethanesulfonyl)aminobenzyl]-1,3-oxazinan-4-one Compound II-23 of the present invention)

### Step 1:

### Synthesis of 3-methyl-N-(2-nitrobenzyl)-2-butenamide

Triethylamine (10.6 g, 110 mmol) was added to a solution of 2-nitrobenzylamine hydrochloride (7.95 g, 42.2 mmol) in 200 ml of dichloromethane, 3-methylcrotonoyl chloride (5.0 g, 42.2 mmol) was added dropwise with stirring under cooling with ice, and the reaction mixture was stirred for 2 hours while it was gradually warmed to room temperature. After the reaction, chloroform and water were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/9) to obtain 8.54 g of the desired product as a yellow solid.

### Step 2:

### Synthesis of 3,3-dimethyl-N-(2-nitrobenzyl)-2-oxiranecarboxamide

3-Chloroperbenzoic acid (9.90 g, 40.2 mmol) was gradually added with stirring under cooling with ice to a solution of 3-methyl-N-(2-nitrobenzyl)-2-butenamide (8.54 g, 36.5 mmol) in 100 ml of dichloromethane, and the reaction mixture was stirred overnight while it was gradually warmed to room temperature. After the reaction, chloroform and a saturated sodium hydrogencarbonate aqueous solution were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/9) to obtain 6.18 g of the desired product.

### Step 3:

### Synthesis of 3-hydroxy-3-methyl-N-(2-nitrobenzyl)butanamide

A solution of 3,3-dimethyl-N-(2-nitrobenzyl)-2-oxiranecarboxamide (6.18 g, 24.7 mmol) in 20 ml of tetrahydrofuran was gradually added dropwise with stirring under cooling with ice to a suspension of lithium aluminum hydride (1.0 g, 24.7 mmol) in 80 ml of tetrahydrofuran, followed by stirring for 4 hours. After the reaction, saturated aqueous sodium chloride and 1 N hydrochloric acid in this order were gradually added dropwise with stirring under cooling with ice to acidify the reaction solution, ethyl acetate was added, and the organic layer was separated, dried over anhydrous sodium sulfate and concentrated under reduced pressure. The obtained residue was purified by silica gel column chromatography (n-hexane/ethyl acetate=9/1 to 1/9) to obtain 1.89 g of the desired product.

### Step 4:

### Synthesis of 6,6-dimethyl-3-(2-nitrobenzyl)-1,3-oxazinan-4-one (Preparation intermediate VII-7)

15 ml of trifluoroacetic acid was added dropwise with stirring to a suspension of 3-hydroxy-3-methyl-N-(2-nitrobenzyl)butanamide (7.5 g, 29.7 mmol) and paraformaldehyde (4.9 g, 149 mmol) in 75 ml of 1,2-dichloroethane, followed by stirring for 4 hours and further for 1 and a half hours at 50°C. After the reaction, chloroform and water were added, and the organic layer was separated, washed with water, then dried over anhydrous sodium sulfate and concentrated under reduced pressure to obtain 7.38 g of the crude desired product (Preparation intermediate VII-7).

### Step 5:

### Synthesis of 6,6-dimethyl-3-(2-aminobenzyl)-1,3-oxazinan-4-one (Preparation intermediate VIII-8)

6,6-Dimethyl-3-(2-nitrobenzyl)-1,3-oxazinan-4-one (7.38 g, 27.9 mmol) was dissolved in a mixed solvent comprising 100 ml of ethanol and 50 ml of water, and reduced iron (7.8 g, 140 mmol) and ammonium chloride (0.75 g, 14.0 mmol) were added, followed by reflux under heating for 1 hour with stirring. After the reaction, the reaction mixture was filtrated through Celite, the filtrate was concentrated under reduced pressure, ethyl acetate and water were added to the resulting residue, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/9) to obtain 5.14 g of the desired product (Preparation intermediate VIII-8).

### Step 6:

### Synthesis of 6,6-dimethyl-3-(2-trifluoromethanesulfonylaminobenzyl)-1,3-oxazinan-4-one (Compound II-17 of the present invention)

6,6-Dimethyl-3-(2-aminobenzyl)-1,3-oxazinan-4-one (4.75 g, 20.3 mmol) was dissolved in 85 ml of dichloromethane, and triethylamine (2.5 g, 24.3 mmol) was added. Then, a solution of trifluoromethanesulfonic anhydride (6.9 g, 24.3 mmol) in 5 ml of dichloromethane was added dropwise with stirring under cooling with ice, followed by stirring for 10 minutes. After the reaction, 1 N hydrochloric acid and chloroform were added, and the organic layer was separated, washed with water, then dried with saturated aqueous sodium chloride and over anhydrous sodium sulfate and concentrated under reduced pressure. The resulting solid was washed with hexane-isopropyl ether to obtain 6.14 g of the desired product (Compound II-17 of the present invention) as a white solid.

### Step 7:

### Synthesis of 6,6-dimethyl-3-[2-(N-ethoxycarbonyl-N-trifluoromethanesulfonyl)aminobenzyl]-1,3-oxazinan-4-one (Compound II-23 of the present invention)

6,6-Dimethyl-3-(2-trifluoromethanesulfonylaminobenzyl)-1,3-oxazinan-4-one (0.30 g, 0.82 mmol) was dissolved in 4 ml of toluene, and pyridine (0.10 g, 1.23 mmol) and ethyl chloroformate (0.14 g, 1.23 mmol) were added, followed by stirring at room temperature for 1 hour. After the reaction, ethyl acetate and 1 N hydrochloric acid were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/1) to obtain 275 mg of the desired product (Compound II-23 of the present invention) as a white solid.

### EXAMPLE 4

### Synthesis of 6,6-dimethyl-3-[2-(N-ethylcarbonyl-N-trifluoromethanesulfonyl)aminobenzyl]-1,3-oxazinan-4-one (Compound II-19 of the present invention)

63 wt% sodium hydride (dispersed in mineral oil, 80 mg, 2.05 mmol) was suspended in 10 ml of tetrahydrofuran, and a solution of 3-(2-trifluoromethanesulfonylaminobenzyl)-1,3-oxazinan-4-one (0.25 g, 0.68 mmol) in 5 ml of tetrahydrofuran was added dropwise with stirring under cooling with ice. The reaction mixture was stirred for 10 minutes as it was, propionyl chloride (0.19 g, 2.05 mmol) was added, and the reaction mixture was stirred further for 2 hours while it was gradually warmed to room temperature. After the reaction, water was added carefully, and ethyl acetate was added, and the organic layer was separated, dried with saturated aqueous sodium chloride and with anhydrous sodium sulfate and concentrated under reduced pressure. The resulting residue was purified by silica gel column chromatography (n-hexane/ethyl acetate=9/1 to 1/1) to obtain 251 mg of the desired product (Compound II-19 of the present invention).

### EXAMPLE 5

### Synthesis of 4-[2-(N-ethoxycarbonyl-N-trifluoromethanesulfonyl)aminobenzyl]-1,4-thiazinan-1,1-dioxide-3-one (Compound I-19 of the present invention)

### Step 1:

### Synthesis of 1,4-thiazinan-3-one

2-Aminoethanethiol hydrochloride (12.0 g, 106 mmol) was dissolved in 200 ml of ethanol, ethyl 2-bromoacetate (17.6 g, 106 mmol) and potassium carbonate (14.6 g, 106 mmol) were added, followed by reflux under heating overnight with stirring. After the reaction mixture was left to cool, potassium carbonate (14.6 g, 106 mmol) was added, followed by reflux under heating overnight with stirring. After the reaction, the reaction mixture was filtrated, the filtrate was concentrated under reduced pressure, and chloroform and 1 N hydrochloric acid were added. The organic layer was separated, dried with saturated aqueous sodium chloride and over anhydrous sodium sulfate and concentrated under reduced pressure to obtain 9.2 g of the desired product.

### Step 2:

### Synthesis of 4-(2-trifluoromethanesulfonylaminobenzyl)-1,4-thiazinan-3-one (Compound I-17 of the present invention)

1,4-Thiazinan-3-one (8.0 g, 68.2 mmol) and p-toluenesulfonic acid monohydrate (6.5 g, 34.1 mmol) were added to a solution of 1,1,1-trifluoro-N-[2-(hydroxymethyl)phenyl]methanesulfonamide (8.7 g, 34.1 mmol) in 100 ml of m-xylene, followed by reflux under heating with stirring for 3 hours with azeotropic water separation with a dean-stark apparatus. After the reaction, ethyl acetate and water were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/9) to obtain 3.74 g of the desired product (Compound I-17 of the present invention).

### Step 3:

### Synthesis of 4-[2-(N-ethoxycarbonyl-N-trifluoromethanesulfonyl)aminobenzyl]-1,4-thiazinan-3-one (Compound I-18 of the present invention)

4-(2-Trifluoromethanesulfonylaminobenzyl)-1,4-thiazinan-3-one (1.5 g, 4.23 mmol) was dissolved in 20 ml of acetonitrile, and ethyl chloroformate (1.8 g, 16.9 mmol) and sodium hydrogencarbonate (1.4 g, 16.9 mmol) were added, followed by reflux under heating with stirring for 2 hours. After the reaction, ethyl acetate and water were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/1) to obtain 1.54 g of the desired product (Compound I-18 of the present invention).

### Step 4:

### Synthesis of 4-[2-(N-ethoxycarbonyl-N-trifluoromethanesulfonyl)aminobenzyl]-1,4-thiazinan-1,1-dioxide-3-one (Compound I-19 of the present invention)

m-Chloroperbenzoic acid (0.87 g, 3.52 mmol) was added to a solution of 4-[2-(N-ethoxycarbonyl-N-trifluoromethanesulfonyl)aminobenzyl]-1,4-thiazinan-3-one (0.50 g, 1.17 mmol) in 10 ml of dichloromethane, followed by stirring at room temperature for 2 hours. After the reaction, chloroform and a 10% sodium hydrogen sulfite aqueous solution were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/1) to obtain 0.51 g of the desired product (Compound I-19 of the present invention).

### EXAMPLE 6

### Synthesis of 5-bromo-6,6-dimethyl-3-[2-(N-ethoxylcarbonyl-N-trifluoromethanesulfonyl)aminobenzyl]-1,3-oxazinan-4-one (Compound II-39 of the present invention)

Triethylamine (0.32 g, 3.10 mmol) was added to a solution of 6,6-dimethyl-3-[2-(N-ethoxycarbonyl-N-trifluoromethanesulfonyl)aminobenzyl]-1,3-oxazinan-4-one (0.68 g, 1.55 mmol) in 12 ml of dichloromethane, and trimethylsilyl trifluoromethanesulfonate (0.52 g, 2.33 mol) was gradually added dropwise with stirring under cooling with ice. The reaction mixture was stirred for 2 hours as it was, and phenyltrimethylammonium bromide (0.88 g, 2.33 mmol) was added little by little with stirring under cooling with ice, and the reaction mixture was further stirred overnight while it was gradually warmed to room temperature. After the reaction, ethyl acetate and 1 N hydrochloric acid were added, and the organic layer was separated, washed with a saturated sodium thiosulfate aqueous solution, then dried with saturated aqueous sodium chloride and over anhydrous sodium sulfate and concentrated under reduced pressure. The resulting residue was purified by silica gel column chromatography (n-hexane/ethyl acetate=9/1 to 1/1) to obtain 387 mg of the desired product (Compound II-39 of the present invention).

### EXAMPLE 7

### Synthesis of 6,6-dimethyl-3-(2-trifluoromethanesulfonylaminobenzyl)-1,3-oxazinan-4-thione (Compound II-80 of the present invention)

Lawesson's reagent (0.33 g, 0.82 mmol) was added to a solution of 6,6-dimethyl-3-(2-trifluoromethanesulfonylaminobenzyl)-1,3-oxazinan-4-one (0.30 g, 0.82 mmol) in 4 ml of toluene, followed by reflux under heating for 2 hours with stirring. After the reaction, the reaction solution was concentrated under reduced pressure, and the resulting residue was purified by silica gel column chromatography (n-hexane/ethyl acetate=9/1 to 1/2) to obtain 142 mg of the desired product (Compound II-80 of the present invention).

### EXAMPLE 8

### Synthesis of 3-(2-nitrobenzyl)-1,3-oxazinan-4-one (Preparation intermediate VIII-1) Step 1:

### Synthesis of 3-hydroxy-N-(2-nitrobenzyl)propanamide

3-(Acryloyloxy)propionic acid (5.7 g, 40.0 mmol), 1-ethyl-3-(3-dimethylaminopropyl)carbodiimide hydrochloride (7.6 g, 40.0 mmol) and triethylamine (5.4 g, 53.0 mmol) were added to a solution of 2-nitrobenzylamine hydrochloride (5.0 g, 26.5 mmol) in 20 ml of dichloromethane, followed by stirring at room temperature for 3 hours. After the reaction, chloroform and water were added, and the organic layer was separated, dried with saturated aqueous sodium chloride and over anhydrous sodium sulfate and concentrated under reduced pressure. The resulting residue was dissolved in 100 ml of ethanol, and sodium hydroxide (2.1 g, 53.0 mmol) and 50 ml of water were added, followed by stirring at room temperature for 2 hours. After the reaction, the reaction mixture was concentrated under reduced pressure and neutralized with concentrated hydrochloric acid, ethyl acetate was added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 0/1) to obtain 1.53 g of the desired product.

### Step 2:

### Synthesis of 3-(2-nitrobenzyl)-1,3-oxazinan-4-one (Preparation intermediate VIII-1)

Paraformaldehyde (1.0 g, 34.3 mmol) and p-toluenesulfonic acid monohydrate (0.65 g, 3.43 mmol) were added to a solution of 3-hydroxy-N-(2-nitrobenzyl)propanamide (1.54 g, 6.86 mmol) in 100 ml of m-xylene, followed by reflux under heating with stirring for 3 hours with azeotropic water separation with a dean-stark apparatus. After the reaction, the reaction mixture was concentrated under reduced pressure, and the resulting residue was purified by silica gel column chromatography (n-hexane/ethyl acetate=9/1 to 1/9) to obtain 0.92 g of the desired product (Preparation intermediate VIII-1).

### EXAMPLE 9

### Synthesis of 6-methyl-3-(2-nitrobenzyl)-1,3-oxazinan-4-one (Preparation intermediate VIII-3)

### Step 1:

### Synthesis of N-(2-nitrobenzyl)-3-oxobutanamide

Sodium hydroxide (1.06 g, 26. 5 mmol) was added to a solution of 2-nitrobenzylamine hydrochloride (5.0 g, 26.5 mmol) in a mixed solvent comprising 20 ml of methanol and 2 ml of water, followed by stirring at room temperature for 30 minutes. Then, the reaction solution was concentrated under reduced pressure, toluene was added to the resulting residue, followed by azeotropic water separation under reduced pressure to distill the solvent off. To the resulting residue, 100 ml of m-xylene and 2,2,6-trimethyl-1 ,3-dioxin-4-one (3.77 g, 26.5 mmol) were added, followed by reflux under heating with stirring for 1 hour with azeotropic water separation with a dean-stark apparatus. After the reaction, ethyl acetate and water were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/1) to obtain 2.27 g of the desired product.

### Step 2:

### Synthesis of 3-hydroxy-N-(2-nitrobenzyl)butanamide

Sodium borohydride (0.40 g, 9.61 mmol) was added to a solution of N-(2-nitrobenzyl)-3-oxobutanamide (2.27 g, 9.61 mmol) in 30 ml of methanol with stirring under cooling with ice, followed by stirring for 30 minutes. After the reaction, ethyl acetate and 1 N hydrochloric acid were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/1) to obtain 1.34 g of the desired product.

### Step 3:

### Synthesis of 6-methyl-3-(2-nitrobenzyl)-1,3-oxazinan-4-one (Preparation intermediate VIII-3)

Paraformaldehyde (0.82 g, 28.1 mmol) and p-toluenesulfonic acid monohydrate (0.53 g, 2.81 mmol) were added to a solution of 3-hydroxy-N-(2-nitrobenzyl)butanamide (1.34 g, 5.62 mmol) in 80 ml of m-xylene, followed by reflux under heating with stirring for 1 hour with azeotropic water separation with a dean-stark apparatus. After the reaction, ethyl acetate and water were added, and the organic layer was separated, dried with saturated aqueous sodium chloride and over anhydrous sodium sulfate and concentrated under reduced pressure. The resulting residue was purified by silica gel column chromatography (n-hexane/ethyl acetate=9/1 to 1/4) to obtain 1.24 g of the desired product (Preparation intermediate VIII-3) as a yellow solid.

### EXAMPLE 10

### Synthesis of 3-methyl-5-(2-nitrobenzyl)-1,3,5-oxadiazinan-4-one (Preparation intermediate IX-1)

### Step 1:

### Synthesis of 1-methyl-3-(2-nitrobenzyl)urea

A suspension of 2-nitrobenzylamine hydrochloride (1.00 g, 5.30 mmol) and triethylamine (560 mg, 5.53 mmol) in 10 ml of tetrahydrofuran was added in a suspension of 1,1'-carbonyl diimidazole (1.29 g, 7.96 mmol) in 30 ml of tetrahydrofuran, with stirring under cooling with ice over a period of about 1 hour. The reaction mixture was stirred for 30 minutes as it was, and a 40 wt% methylamine aqueous solution (1.28 g, 16.5 mmol) was added dropwise over a period of about 30 minutes. Then, the reaction mixture was stirred overnight while it was gradually warmed to room temperature. After the reaction, the reaction mixture was adjusted to pH 5 with 1 N hydrochloric acid, ethyl acetate was added, and the organic layer was separated and washed with 1 N hydrochloric acid, then dried over anhydrous sodium sulfate and concentrated under reduced pressure to obtain 980 mg of the crude desired product as a yellow solid.

### Step 2:

### Synthesis of 3-methyl-5-(2-nitrobenzyl)-1,3,5-oxadiazinan-4-one

1 ml of trifluoroacetic acid was added to a suspension of 1-methyl-3-(2-nitrobenzyl)urea (500 mg, 2.39 mmol) and paraformaldehyde (160 mg, 4.90 mmol) in 10 ml of 1,2-dichloroethane, with stirring, and the reaction mixture was stirred overnight as it was. After the reaction, chloroform and water were added, and the organic layer was separated, washed with water, then dried over anhydrous sodium sulfate and concentrated under reduced pressure to obtain 600 mg of the crude desired product (Preparation intermediate IX-1) as a yellow solid.

### REFERENCE EXAMPLE 1

### Synthesis of 1,4,2-dioxazinan-3-one

1,1'-Carbonyldiimidazole (4.23 g, 26.1 mmol) was added to a solution of 2-(aminooxy)ethanol (3.20 g, 26.1 mmol) in 30 ml of tetrahydrofuran, followed by reflux under heating with stirring for 2 hours. After the reaction, dichloromethane and a saturated sodium hydrogencarbonate aqueous solution were added, and the aqueous layer was separated and adjusted to pH 1 with 1 N hydrochloric acid. Dichloromethane was added, and the organic layer was separated, dried with saturated aqueous sodium chloride and over anhydrous sodium sulfate and concentrated under reduced pressure to obtain 70 mg of the crude desired product.

### REFERENCE EXAMPLE 2

### Synthesis of 1-ethyl-3-(2-nitrobenzyl)urea

Triethylamine (600 mg, 5.93 mmol) was added to a suspension of 2-nitrobenzylamine hydrochloride (1.00 g, 5.30 mmol) in 30 ml of tetrahydrofuran, and ethyl isocyanate (420 mg, 5.91 mmol) was added dropwise with stirring under cooling with ice over a period of about 30 minutes. Then, the reaction mixture was stirred overnight while it was gradually warmed to room temperature. After the reaction, the reaction mixture was adjusted to pH 5 with 1 N hydrochloric acid, ethyl acetate was added, and the organic layer was separated, washed with 1 N hydrochloric acid, then dried over anhydrous sodium sulfate and concentrated under reduced pressure to obtain 1.05 g of the crude desired product as a yellow solid.

### REFERENCE EXAMPLE 3

### Synthesis of 5,5-dimethyl-1,2-oxazepan-3-one

### Step 1:

### Synthesis of methyl 5-aminooxy-3,3-dimethylpentanoate

Methyl 5-bromo-3,3-dimethylpentanoate (16.4 g, 73.6 mmol) and triethylamine (9.30 g, 92.0 mmol) were added to a solution of N-hydroxyphthalimide (10.0 g, 61.3 mmol) in 100 ml of dimethylformamide, followed by stirring at 60°C for 4 hours. After the reaction, toluene and water were added, and the organic layer was separated, dried with saturated aqueous sodium chloride and over anhydrous sodium sulfate and concentrated under reduced pressure. Then, the resulting residue was dissolved in 100 ml of ethanol, hydrazine monohydrate (1.96 g, 61.3 mmol) was added dropwise with stirring under cooling with ice, and the reaction mixture was stirred for 16 hours while it was gradually warmed to room temperature. After the reaction, the reaction mixture was concentrated under reduced pressure, 2N hydrochloric acid was added to the resulting residue, and the precipitated insolubles were removed by filtration. The filtrate was neutralized with a saturated sodium hydrogencarbonate aqueous solution, ethyl acetate was added, and the organic layer was separated, washed with water, then dried with saturated aqueous sodium chloride and over anhydrous sodium sulfate and concentrated under reduced pressure to obtain 5.85 g of the crude desired product.

### Step 2:

### Synthesis of 5,5-dimethyl-1,2-oxazepan-3-one

Trimethylaluminum (2.0 M tetrahydrofuran solution) (17.1 ml, 34.2 mmol) was added to a solution of methyl 5-aminooxy-3,3-dimethylpentanoate (3.0 g, 17.1 mmol) in 50 ml of tetrahydrofuran with stirring under cooling with ice, and the reaction mixture was stirred for 6 hours while it was gradually warmed to room temperature. Then, 20 ml of acetone was added, followed by stirring for 30 minutes, and 50 ml of water was added. The reaction solution was concentrated under reduced pressure, dichloromethane and tetrahydrofuran were added, and the reaction solution was filtrated through Celite, and the filtrate was concentrated under reduced pressure. The resulting crude product was washed with diethyl ether to obtain 490 mg of the desired product as white crystals.

### REFERENCE EXAMPLE 4

### Synthesis of 1,1,1-trifluoro-N-[2-(hydroxymethyl)phenyl]methanesulfonamide

2-Aminobenzyl alcohol (4.0 g, 32.5 mmol) was dissolved in 60 ml of chloroform and cooled to 0°C, and triethylamine (3.94 g, 39.0 mmol) was added, and trifluoromethanesulfonic anhydride (11.0 g, 39.0 mmol) was added dropwise with stirring. The reaction solution was stirred further for 4 hours while it was gradually warmed to room temperature. After the reaction, chloroform and water were added, and the organic layer was separated, washed with water, then dried with saturated aqueous sodium chloride and over anhydrous sodium sulfate and concentrated under reduced pressure. The resulting residue was purified by silica gel column chromatography (n-hexane/ethyl acetate=9/1 to 1/1) (volume ratio, the same applies hereinafter) to obtain 5.13 g of the desired product as a yellow solid (melting point: 60 to 61°C).

The compounds of the present invention can be synthesized in the same manners as in the above Examples. The structural formulae and physical properties of synthesized compounds, including those synthesized in the preceding Examples, are shown in Tables 4 to 13, but the present invention is by no means restricted to those.

The symbols in Tables 4 to 12 have the following meanings.
Me: methyl group, Et: ethyl group, Pr: propyl group, Bu: butyl group, Pen: pentyl group, Hex: hexyl group, Ph: phenyl group.

Further, in Tables, *1 represents "resinoid".

Further, the symbols in Table 13 have the following meanings.
s: singlet, d: doublet, t: triplet, m: multiplet.

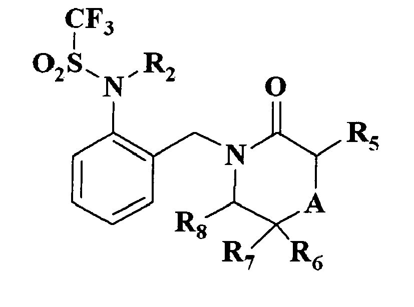

**TABLE 4**

| No. | R₂ | R₅ | R₆ | R₇ | R₈ | A | [m.p. (°C)] |
|---|---|---|---|---|---|---|---|
| I-1 | H | H | H | H | H | O | *1 |
| I-2 | CO₂ Et | H | H | H | H | O | *1 |
| I-3 | CO₂ Bu-i | H | H | H | H | O | *1 |
| I-4 | H | Me | H | H | H | O | *1 |
| I-5 | CO₂ Et | Me | H | H | H | O | *1 |
| I-6 | H | H | Me | H | H | O | *1 |
| I-7 | CO₂ Et | H | Me | H | H | O | *1 |
| I-8 | CO₂ CH₂ CH₂ Cl | H | Me | H | H | O | *1 |
| I-9 | CO₂ Bu-i | H | Me | H | H | O | *1 |
| I-10 | H | H | Et | H | H | O | *1 |
| I-11 | CO₂ Et | H | Et | H | H | O | *1 |
| I-12 | CO₂ Bu-i | H | Et | H | H | O | *1 |
| I-13 | H | H | H | H | Me | O | 102-103 |
| I-14 | CO₂ Et | H | H | H | Me | O | 124-125 |
| I-15 | H | H | Me | Me | H | O | *1 |
| I-16 | CO₂ Et | H | Me | Me | H | O | *1 |
| I-17 | H | H | H | H | H | S | *1 |
| I-18 | CO₂ Et | H | H | H | H | S | 78-79 |
| I-19 | CO₂ Et | H | H | H | H | SO₂ | *1 |

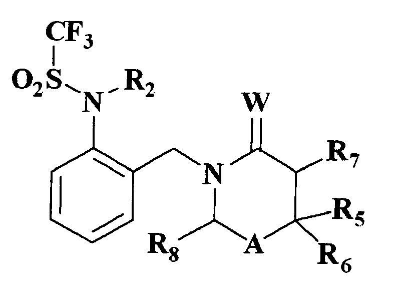

**TABLE 5**

| No | R₂ | R₅ | R₆ | R₇ | R₉ | A | W | m.p.(°C) |
|---|---|---|---|---|---|---|---|---|
| II-1 | H | H | H | H | H | O | O | *1 |
| II-2 | CO₂ Et | H | H | H | H | O | O | *1 |
| II-3 | CO₂ Bu-i | H | H | H | H | O | O | *1 |
| II-4 | H | Me | H | H | H | O | O | 107-109 |
| II-5 | COEt | Me | H | H | H | O | O | *1 |
| II-6 | COPh | Me | H | H | H | O | O | *1 |
| II-7 | CO₂ Et | Me | H | H | H | O | O | *1 |
| II-8 | CO₂ Bu-i | Me | H | H | H | O | O | *1 |
| II-9 | H | Ph | H | H | H | O | O | 115-118 |
| II-10 | CO₂ E t | Ph | H | H | H | O | O | *1 |
| II-11 | CO₂ Pr-n | Ph | H | H | H | O | O | *1 |
| II-12 | CO₂ CH₂ CH₂Cl | Ph | H | H | H | O | O | *1 |
| II-13 | CO₂ Bu-i | Ph | H | H | H | O | O | 105-107 |
| II-14 | H | CF₃ | H | H | H | O | O | *1 |
| II-15 | CO₂ Et | CF₃ | H | H | H | O | O | 126-127 |
| II-16 | CO₂ Bu-i | CF₃ | H | H | H | O | O | 161-162 |
| II-17 | H | Me | Me | H | H | O | O | *1 |
| II-18 | Me | Me | Me | H | H | O | O | *1 |
| II-19 | COEt | Me | Me | H | H | O | O | *1 |
| II-20 | COPr-c | Me | Me | H | H | O | O | *1 |
| II-21 | COPh | Me | Me | H | H | O | O | *1 |
| II-22 | CO₂ Me | Me | Me | H | H | O | O | *1 |
| II-23 | CO₂ Et | Me | Me | H | H | O | O | *1 |
| II-24 | CO₂ Pr-n | Me | Me | H | H | O | O | *1 |
| II-25 | CO₂ Bu-n | Me | Me | H | H | O | O | *1 |
| II-26 | CO₂ Bu-i | Me | Me | H | H | O | O | *1 |
| II-27 | E-1 | Me | Me | H | H | O | O | *1 |
| II-28 | CO₂ CH₂ CH₂ Cl | Me | Me | H | H | O | O | *1 |
| II-29 | H | Me | Me | Me | H | O | O | 83-84 |
| II-30 | CO₂ Et | Me | Me | Me | H | O | O | *1 |
| II-31 | CO₂ Bu- i | Me | Me | Me | H | O | O | *1 |
| II-32 | H | Me | Me | F | H | O | O | 117-118 |
| II-33 | CO₂ Me | Me | Me | F | H | O | O | *1 |
| II-34 | CO₂ Et | Me | Me | F | H | O | O | *1 |
| II-35 | CO₂ Pr-n | Me | Me | F | H | O | O | *1 |
| II-36 | CO₂ Bu-i | Me | Me | F | H | O | O | *1 |
| II-37 | SO₂ CF₃ | Me | Me | F | H | O | O | 108-109 |
| II-38 | H | Me | Me | Br | H | O | O | 107-108 |
| II-39 | CO₂ Et | Me | Me | Br | H | O | O | 89-90 |
| II-40 | H | Me | Et t | H | H | O | O | 113-114 |
| II-41 | COEt | Me | E t | H | H | O | O | *1 |
| II-42 | CO₂ Et | Me | Et t | H | H | O | O | *1 |
| II-43 | CO₂ Bu-i | Me | Et t | H | H | O | O | *1 |
| II-44 | H | Me | Pr-c | H | H | O | O | 98-99 |
| II-45 | CO₂ | Me | Pr-c | H | H | O | O | *1 |
| II-46 | H | Me | CF₃ | H | H | O | O | 84-85 |
| II-47 | COEt | Me | CF₃ | H | H | O | O | *1 |
| II-48 | COPr-c | Me | CF₃ | H | H | O | O | *1 |
| II-49 | CO₂Et | Me | CF₃ | H | H | O | O | *1 |
| II-50 | CO₂Bu-i | Me | CF₃ | H | H | O | O | *1 |
| II-51 | H | Me | CH₂CF₃ | H | H | O | O | 89-90 |
| II-52 | COEt | Me | CH₂CF₃ | H | H | O | O | *1 |
| II-53 | CO₂Et | Me | CH₂CF₃ | H | H | O | O | 117-119 |
| II-54 | H | Me | CH₂OMe | H | H | O | O | 75-77 |
| II-55 | CO₂Et | Me | CH₂OMe | H | H | O | O | *1 |
| II-56 | CO₂Bu-i | Me | CH₂OMe | H | H | O | O | *1 |
| II-57 | H | Me | Ph | H | H | O | O | 113-114 |
| II-58 | CO₂Et | Me | Ph | H | H | O | O | *1 |
| II-59 | H | Me | E-8 | H | H | O | O | *1 |
| II-60 | CO₂Et | Me | E-8 | H | H | O | O | *1 |
| II-61 | H | - (CH₂)₃- | | H | H | O | O | 132-134 |
| II-62 | COEt | - (CH₂)₃- | | H | H | O | O | 82-83 |
| II-63 | CO₂Et | - (CH₂)₃- | | H | H | O | O | *1 |
| II-64 | H | - (CH₂)₄- | | H | H | O | O | 137-139 |
| II-65 | COEt | - (CH₂)₄- | | H | H | O | O | *1 |
| II-66 | CO₂Et | - (CH₂)₄- | | H | H | O | O | *1 |
| II-67 | H | - (CH₂)₅₋ | | H | H | O | O | 109-111 |
| II-68 | CO₂Et | - (CH₂)₅- | | H | H | O | O | *1 |
| II-69 | H | E-9 | | H | H | O | O | *1 |
| II-70 | CO₂Et | E-9 | | H | H | O | O | *1 |
| II-71 | CO₂Et | Me | H | Me | H | O | O | *1 |
| II-72 | H | Me | Me | H | Me | O | O | 83-84 |
| II-73 | COEt | Me | Me | H | Me | O | O | *1 |
| II-74 | CO₂Et | Me | Me | H | Me | O | O | 130-131 |
| II-75 | CO₂Et | Me | CF₃ | Br | H | O | O | *1 |
| II-76 | CO₂Et | Me | Me | Br | Me | O | O | 137-138 |
| II-77 | CO₂Et | -(CH₂)₄- | | Br | H | O | O | 121-122 |
| II-78 | H | H | H | H | H | S | O | 89-90 |
| II-79 | CO₂Et | H | H | H | H | S | O | *1 |
| II-80 | H | H | H | H | H | O | S | *1 |
| II-81 | CO₂Et | Me | Me | H | H | O | S | *1 |

In Table, E-1 and E-8 are the same as defined above, and E-9 is -(CH₂)₂O(CH₂)₂-.

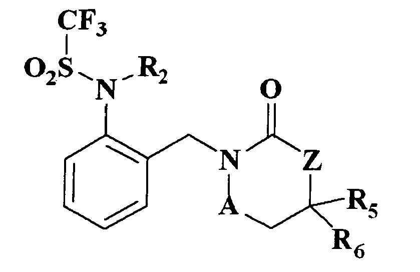

**TABLE 6**

| No. | R₂ | R₅ | R₆ | A | Z | m.p.(°C) |
|---|---|---|---|---|---|---|
| III-1 | H | H | H | O | O | *1 |
| III-2 | CO₂Et | H | H | O | O | *1 |
| III-3 | H | Me | H | O | O | *1 |
| III-4 | CO₂Et | Me | H | O | O | *1 |
| III-5 | H | Et | H | O | O | *1 |
| III-6 | CO₂Et | Et | H | O | O | *1 |
| III-7 | CO₂CH₂CH₂Cl | Et | H | O | O | *1 |
| III-8 | H | CF₃ | H | O | O | *1 |
| III-9 | CO₂Et | CF₃ | H | O | O | *1 |
| III-10 | H | Me | Me | O | O | *1 |
| III-11 | CO₂Et | Me | Me | O | O | *1 |
| III-12 | H | H | H | O | CH₂ | *1 |
| III-13 | CO₂Et | H | H | O | CH₂ | *1 |
| III-14 | CO₂CH₂CH₂Cl | H | H | O | CH2 | *1 |
| III-15 | CO₂Bu-i | H | H | O | CH₂ | *1 |

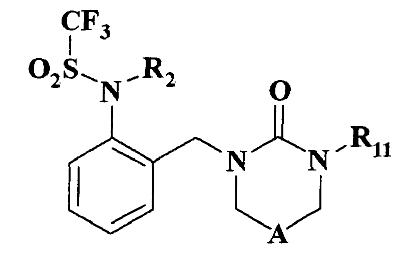

**TABLE 7**

| No. | R₂ | R₁₁ | A | W | m.p.(°C) |
|---|---|---|---|---|---|
| IV-1 | H | Me | O | O | *1 |
| IV-2 | CO₂Et | Me | O | O | *1 |
| IV-3 | CO₂CH₂CH₂Cl | Me | O | O | *1 |
| IV-4 | CO₂Bu-i | Me | O | O | *1 |
| IV-5 | H | Et | O | O | *1 |
| IV-6 | CO₂Et | Et | O | O | *1 |
| IV-7 | CO₂CH₂CH₂Cl | Et | O | O | *1 |
| IV-8 | CO₂Bu-i | Et | O | O | *1 |
| IV-9 | H | Me | O | S | *1 |
| IV-10 | CO₂Et | Me | O | S | *1 |

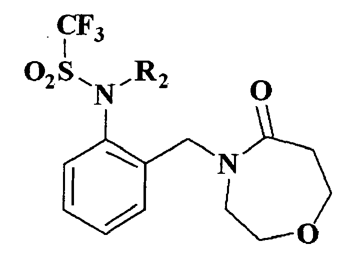

**TABLE 8**

| No. | R₂ | m.p.(°C) |
|---|---|---|
| V-1 | H | *1 |
| V-2 | CO₂Et | *1 |

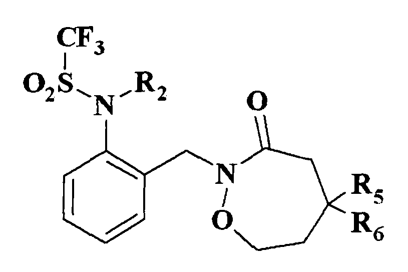

**TABLE 9**

| No. | R₂ | R₂ | R₂ | m.p.(°C) |
|---|---|---|---|---|
| VI-1 | H | Me | Me | *1 |
| VI-2 | CO₂ Et | Me | Me | *1 |

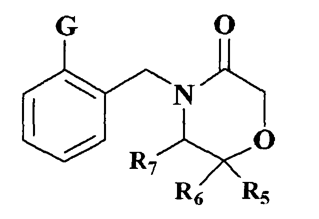

**TABLE 10**

| No. | G | R₅ | R₆ | R₇ | m.p.(°C) |
|---|---|---|---|---|---|
| VII-1 | NO₂ | H | H | H | *1 |
| VII-2 | NH₂ | H | H | H | *1 |
| VII-3 | NO₂ | Et | H | H | 68-69 |
| VII-4 | NH₂ | Et | H | H | *1 |
| VII-5 | NO₂ | H | H | Me | 82-83 |
| VII-6 | NH₂ | H | H | Me | 101-102 |
| VII-7 | NO₂ | Me | Me | H | *1 |
| VII-8 | NH₂ | Me | Me | H | *1 |

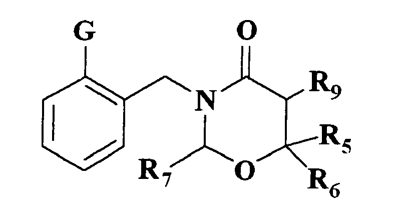

**TABLE 11**

| No. | G | R₅ | R₆ | R₇ | R₉ | m.p.(°C) |
|---|---|---|---|---|---|---|
| VIII-1 | NO₂ | H | H | H | H | *1 |
| VIII-2 | NH₂ | H | H | H | H | *1 |
| VIII-3 | NO₂ | Me | H | H | H | *1 |
| VIII-4 | NH₂ | Me | H | H | H | *1 |
| VIII-5 | NO₂ | CF₃ | H | H | H | 80-81 |
| VIII-6 | NH₂ | CF₃ | H | H | H | *1 |
| VIII-7 | NO₂ | Me | Me | H | H | *1 |
| VIII-8 | NH₂ | Me | Me | H | H | *1 |
| VIII-9 | NO₂ | Me | Et | H | H | 79-80 |
| VIII-10 | NH₂ | Me | Et | H | H | *1 |
| VIII-11 | NO₂ | Me | Ph | H | H | 103-104 |
| VIII-12 | NH₂ | Me | Ph | H | H | *1 |
| VIII-13 | NO₂ | Me | CF₃ | H | H | 91-92 |
| VIII-14 | NH₂ | Me | CF₃ | H | H | *1 |
| VIII-15 | NO₂ | Me | CH₂CF₃ | H | H | 93-94 |
| VIII-16 | NH₂ | Me | CH₂CF₃ | H | H | *1 |
| VIII-17 | NO₂ | Me | E-8 | H | H | 121-123 |
| VIII-18 | NH₂ | Me | E-8 | H | H | *1 |
| VIII-19 | NO₂ | Me | CH₂OMe | H | H | 69-70 |
| VIII-20 | NH₂ | Me | CH₂OMe | H | H | *1 |
| VIII-21 | NO₂ | | - (CH₂)₃- | H | H | 94-95 |
| VIII-22 | NH₂ | | - (CH₂)₃- | H | H | *1 |
| VIII-23 | NO₂ | | - (CH₂)₄- | H | H | 103-104 |
| VIII-24 | NH₂ | | - (CH₂)₄- | H | H | *1 |
| VIII-25 | NO₂ | | - (CH₂)₅- | H | H | 68-70 |
| VIII-26 | NH₂ | | - (CH₂)₅- | H | H | *1 |
| VIII-27 | NO₂ | Me | E-9 | H | H | 79-80 |
| VIII-28 | NH₂ | Me | E-9 | H | H | *1 |
| VIII-29 | NO₂ | Me | Me | Me | H | 75-76 |
| VIII-30 | NH₂ | Me | Me | Me | H | *1 |
| VIII-31 | NO₂ | Me | Me | H | Me | *1 |
| VIII-32 | NH₂ | Me | Me | H | Me | *1 |

In TABLE, E-8 and E-9 are the same as defined above.

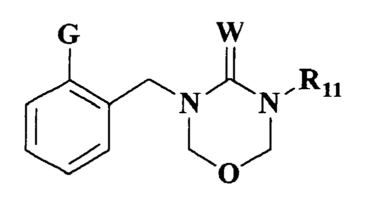

**TABLE 12**

| No. | G | R₁₁ | W | m.p.(°C) |
|---|---|---|---|---|
| IX-1 | NO₂ | Me | O | *1 |
| IX-2 | NH₂ | Me | O | *1 |
| IX-3 | NO₂ | Et | O | *1 |
| IX-4 | NH₂ | Et | O | *1 |
| IX-5 | NO₂ | Me | S | *1 |
| IX-6 | NH₂ | Me | S | *1 |

**TABLE 13**

| No. Proton nuclear magnetic chemical shift (in CDCl₃):δ (ppm) |
|---|
| I-1:11.33(brs,1H),7.60-7.65(m,1H),7.35-7.45(m,1H),7.20-7.30(m,2H),4.52(s,2H),4.21 (s,2H),3.87(t,J=5.1Hz,2H),3.54(t,J=5.1Hz,2H) |
| I-2:7.35-7.55(m,3H),7.20-7.25(m,1H),4.81 (d,J=15.0Hz, 1H),4.66(d,J=15.0Hz,1H),4.25-4.45(m,4H),3.87(t,J=5.1Hz,2H),3.15-3.35(m,2H),1.33(t,J=7.2Hz,3H) |
| I-3:7.35-7.55(m,3H),7.20-7.25(m,1H),4.79(d,J=15.3Hz,1H),4.69(d,J=15.3Hz, 1H),4.28(s,2H),4.00-4.15(m,2H),3.85-3.90(m,2H),3.20-3.30(m,2H), 1.90-2.05(m, 1H),0.89(d,J=6.9Hz,6H) |
| I-4:11.41 (brs,1H),7.60-7.65(m,1H),7.35-7.45(m,1H),7.20-7.30(m,2H),4.72(d,J=14.7Hz,1H),4.27(d,J=14.7Hz,1H),4.15-4.25(m,1H),3.95-4.05(m,1H),3.60-3.75(m,2H),3.35-3.45(m,1H),1.47(d,J=6.6Hz,3H) |
| I-5:7.20-7.50(m,4H),4.90-5.05(m,1H),4.30-4.55(m,4H),3.90-4.00(m,1H),3.70-3.85(m,1H),3.40-3.50(m,1H),3.05-3.15(m,1H),1.50-1.55(m,3H),1.25-1.35(m,3H) |
| I-6:11.34(brs,1H),7.60-7.65(m,1H),7.35-7.45(m,2H),7.20-7.30(m,1H),4.69(d,J=14.6Hz,1H),4.25-4.45(m,2H),4.05-4.20(m,1H),3.75-3.85(m,1H),3.35(d,J=6.6Hz,2H),1.28(d,J=6.3Hz,3H) |
| I-7:7.30-7.55(m,3H),7.20-7.25(m,1H),4.95(d,J=14.9Hz,1H),4.05-4.50(m,5H),3.80-4.00(m, 1H),3.00-3.20(m,2H),1.20-1.35(m,6H) |
| I-8:7.30-7.55(m,3H),7.20-7.30(m,1H),4.02(dd,J=15.3,63.3Hz,1H),4.20-4.60(m,5H),3.80-4.00(m,1H),3.60-3.80(m,2H),3.00-3.25(m,2H),1.15-1.30(m,3H) |
| I-9:7.30-7.55(m,2H),7.15-7.25(m,2H),4.95(dd,J=3.3,15.6Hz,1H),4.20-4.55(m,3H),3.80-4.20(m,3H),2.90-3.20(m,2H),1.85-2.05(m,1H),1.15-1.30(m,3H),0.89(d,J=6.9Hz,6H) |
| I-10:11.37(brs,1H),7.63(dd,J=0.6,8.1Hz,1H),7.40(dt,J=2.1,7.8Hz,1H),7.20-7.30(m,2H),4.70(d,J=14.7Hz,1H),4.25-4.35(m,2H),4.13(d,J=14.7Hz, 1H),3.50-3.60(m,1H),3.30-3.40(m,2H),1.50-1.70(m,2H),0.98(t,J=7.2Hz,3H) |
| I-11:7.45-7.55(m,1H),7.35-7.45(m,2H),7.23(d,J=7.8Hz,1H),4.93(dd,J=3.6,15.3Hz,1H),4.20-4.55(m,5H),3.55-3.70(m,1H),3.00-3.20(m,2H),1.45-1.60(m,2H),1.33(dt,t=2.7,7.2Hz,3H),0.93(dt,t=1.5,7.5Hz,3H) |
| I-12:7.45-7.55(m,1H),7.35-7.45(m,2H),7.23(d,J=8.4Hz,1H),4.92(dd,J=8.1,15.3Hz,1H),4.20-4.60(m,3H),4.05-4.15(m,2H),3.55-3.70(m,1H),3.00-3.15(m,2H),1.90-2.00(m,1H),1.45-1.60(m,2H),0.93(dt,t=1.5,7.5Hz,3H),0.85-0.90(m,6H) |
| I-15:11.34(brs,1H),7.60-7.70(m,1H),7.35-7.45(m,1H),7.20-7.30(m,2H),4.51 (s,2H),4.19(s,2H),3.35(s,2H),1.25(s,6H) |
| I-16:7.35-7.55(m,3H),7.20-7.30(m,1H),4.87(d,J=15.9Hz,1H),4.55(d,J=15.9Hz,1H),4.30-4.40(m,2H),4.29(s,2H),3.09(s,2H),1.32(d,t=6.9Hz,3H),1.25(s,6H) |
| I-17:11.26(brs,1H),7.60-7.65(m,1H),7.15-7.45(m,3H),4.55(s,2H),3.78(t,J=5.71Hz,2H),3.39(s,2H),2.85(t,J=5.7Hz,2H) |
| I-19:7.25-7.55(m,4H),5.32(d,J=15.3Hz,1H),3.95-4.50(m,5H),3.55-3.65(m,2H),3.15-3.40(m,2H),1.35(t,J=7.2Hz,3H) |
| II-1:11.59(brs,1H),7.55-7.65(m,1H),7.35-7.45(m,1H),7.15-7.25(m,2H),4.90(s,2H),4.43(s,2H),3.99(t,J=6.3Hz,2H),2.58(t,J=6.3Hz,2H) |
| II-2:7.20-7.50(m,4H),4.60-4.70(m,4H),4.30-4.45(m,2H),4.00-4.10(m,2H),2.66(t,J=6.3Hz,2H),1.32(t,J=7.2Hz,3H) |
| II-3:7.35-7.50(m,3H),7.15-7.25(m,1H),4.65-4.70(m,4H),4.00-4.15(m,4H),2.67(t,J=6.0Hz,2H),1.85-2.05(m,1H),0.85-0.95(m,6H) |
| II-5:7.20-7.55(m,4H),3.95-5.10(m,5H),2.20-2.60(m,4H),1.30-1.40(m,3H),1.05-1.15(m,3H) |
| II-6:7.05-7.60(m,9H),4.25-5.30(m,4H),3.95-4.15(m,1H),2.40-2.65(m,2H),1.35-1.40(m,3H) |
| II-7:7.35-7.50(m,3H),7.15-7.25(m,1H),4.30-4.95(m,6H),3.95-4.05(m,1H),2.35-2.65(m,2H),1.25-1.35(m,6H) |
| II-8:7.35-7.50(m,3H),7.20-7.25(m,1H),4.40-4.95(m,4H),3.95-4.15(m,3H),2.35-2.65(m,2H),1.90-2.00(m,1H),1.32(d,J=6.0Hz,3H),0.85-0.90(m,6H) |
| II-10:7.30-7.55(m,8H),7.23(d,J=8.1Hz,1H),4.75-5.00(m,4H),4.50-4.70(m,1H),4.30-4.50(m,2H),2.80-2.90(m,2H),1.34(t,J=7.2Hz,3H) |
| II-11:7.30-7.55(m,8H),7.23(d,J=7.9Hz,1H),4.75-5.00(m,4H),4.50-4.70(m,1H),4.28(t,J=6.9Hz,2H),2.80-2.95(m,2H),1.65-1.80(m,2H),0.85-0.95(m,3H) |
| II-12:7.30-7.55(m,8H),7.24(d,J=8.0Hz,1H),4.65-5.05(m,5H),4.45-4.60(m,2H),3.65-3.75(m,2H),2.70-2.95(m,2H) |
| II-14:10.84(brs,1H),7.61(d,J=8.1Hz,1H),7.35-7.45(m,1H),7.15-7.25(m,2H),5.09(d,J=8.7Hz,1H),4.98(d,J=8.7Hz,1H),4.55(d,J=15.0Hz,1H),4.44(d,J=15. 0Hz,1H),4.20-4.35(m,1H),2.65-2.80(m,2H) |
| II-17:11.62(brs,1H),7.55-7.65(m,1H),7.35-7.45(m,1H),7.20-7.25(m,2H),4.91 (s,2H),4.43(s,2H),2.42(s,2H),1.27(s,6H) |
| II-18:7.30-7.45(m,4H),5.07(d,J=16.5Hz,1H),4.83(d,J=9.3Hz,1H),4.79(d,J=9.3Hz,1H),4.30(d,J=16. 5Hz,1H),3.44(s,3H),2.53(d,J=16.8Hz,1H),2.46(d,J=16.8Hz,1H),1.35(s,3H),1.34(s,3H) |
| II-19:7.35-7.55(m,3H),7.15-7.25(m,1H),5.04(d,J=16.5Hz,1H),4.75-4.90(m,2H),4.15(d,J=16.5Hz,1H),2.25-2.60(m,4H),1.35-1.40(m,6H),1.12(t,J=7.2Hz,3H) |
| II-20:7.35-7.55(m,3H),7.25-7.30(m,1H),5.08(d,J=16.5Hz,1H),4.75-4.85(m,2H),4.31 (d,J=16.5Hz,1H),2.50-2.55(m,2H),1.50-1.55(m,1H),1.35-1.40(m,6H), 1.25-1.35(m,1H), 1.10-1.20(m,1H), 1.00-1.10(m, 1H),0.85-0.95(m,1H) |
| II-21:7.55-7.60(m,2H),7.10-7.45(m,7H),5.17(d,J=16.5Hz,1H),4.75-4.85(m,2H),4.43(d,J=16.5Hz,1H),2.45-2.65(m,2H),1.37(s,6H) |
| II-22:7.30-7.50(m,3H),7.20(d,J=8.1Hz,1H),4.50-4.80(m,4H),3.90(s,3H),2.52(s,2H),1.33(s,6H) |
| II-23:7.30-7.50(m,3H),7.15-7.25(m,1H),4.55-4.75(m,4H),4.30-4.45(m,2H),2.52(s,2H),1.30-1.35(m,9H) |
| II-24:7.30-7.50(m,3H),7.19(d,J=7.5Hz,1H),4.55-4.80(m,4H),4.26(t,J=6.3Hz,2H),2.52(s,2H),1.65-1.75(m,2H),1.33(s,6H),0.90(t,J=7.5Hz,3H) |
| II-25:7.30-7.50(m,3H),7.19(d,J=8.4Hz,1H),4.55-4.80(m,4H),4.30(t,J=6.9Hz,2H),2.52(s,2H),1.60-1.70(m,2H),1.33(s,6H),1.20-1.35(m,2H),0.90(t,J=7.2Hz,3H) |
| II-26:7.30-7.50(m,3H),7.15-7.25(m,1H),4.55-4.80(m,4H),4.00-4.15(m,2H),2.52(s,2H),1.85-2.05(m,1H),1.33(s,6H),0.85-0.90(m,6H) |
| II-27:7.35-7.50(m,3H),7.21(d,J=7.5Hz,1H),4.40-4.95(m,4H),4.83(d,J=10.5Hz,1H),3.87(d,J=10.5Hz,1H),2.52(s,2H),1.33(s,6H),0.85(s,9 H) |
| II-28:7.30-7.50(m,3H),7.23(d,J=6.5Hz,1H),4.74(d,J=12.0Hz,1H),4.69(s,2H),4.65(d,J=12.0Hz,1H), 4.53(t,J=5.2Hz,2H),3.69(t,J=5.2Hz,2H),2.52(s,2H),1.33(s,6H) |
| II-30:7.30-7.50(m,3H),7.19(d,J=7.8Hz,1H),4.50-4.95(m,6H),2.55(q,J=7.2Hz,1H),1.30-1.35(m,6H),1.24(d,J=7.2Hz,3H),1.21 (d,J=4.8Hz,3H) |
| II-31:7.30-7.50(m,3H),7.19(d,J=8.1Hz,1H),4.35-4.95(m,4H),4.00-4.15(m,2H),2.56(q,J=7.2Hz,1H),1.90-2.00(m,1H),1.30-1.35(m,3H),1.24(d,J=7.2Hz,3H),1.2(d,J=5.4Hz,3H),0.85-0.90(m,6H) |
| II-33:7.40-7.55(m,3H),7.21(d,J=9.0Hz,1H),4.30-4.95(m,5H),3.91 (d,J=1.8Hz,3H),1.41 (d,J=3.0Hz,3H),1.35(q,J=3.0Hz,3H) |
| II-34:7.35-7.50(m,3H),7.21(d,J=8.7Hz,1H),4.30-4.95(m,7H),1.41(d,J=3.0z,3H),1.30-1.40(m,6H) |
| II-35:7.35-7.50(m,3H),7.21(d,J=8.1Hz,1H),4.35-4.95(m,5H),4.27(d,J=2.4,6.3Hz,2H),1.65-1.75(m,2H), 1.41 (d,J=3.0Hz,3H),1.35(t,J=3.0Hz,3H),0.91 (dt,J=2.4,7.8Hz,3H) |
| II-36:7.35-7.50(m,3H),7.21(d,J=8.1Hz,1H),4.35-4.95(m,5H),4.00-4.15(m,2H),1.90-2.00(m,1H),1.41(d,J=3.0Hz,3H),1.35(t,J=3.0Hz,3H),0.85-0.90(m,6H) |
| II-41:7.45-7.55(m,1H),7.35-7.45(m,2H),7.21(d,J=7.8Hz,1H),5.04(dd,J=10.2,15.9Hz,1H),4.75-4.90(m,2H),4.17(dd,J=15.9,19.2Hz,1H),2.30-2.50(m,4H),1.55-1.75(m,2H),1.29(s,3H),1.12(t,J=7.2Hz,3H),0.96(dt,J=2.1,7.2Hz,3H) |
| II-42:7.30-7.50(m,3H),7.19(d,J=7.8Hz,1H),4.55-4.80(m,4H),4.30-4.45(m,2H),2.56(dd,J=3.0,16.8Hz,1H),2.43(dd,J=1.5,16.8Hz,1H),1.55-1.70(m,2H),1.32(t,J=7.2Hz,3H),1.26(s,3H),0.93(t,J=7.5Hz,3H) |
| II-43:7.30-7.50(m,3H),7.19(d,J=8.1Hz,1H),4.50-4.85(m,4H),4.00-4.15(m,2H),2.56(dd,J=2.1,16.5Hz,1H),2.44(dd,J=1.2,16.5Hz,1H),1.85-2.05(m,1H),1.55-1.70(m,2H),1.26(s,3H),0.93(dt,J=0.9,7.5Hz,3H),0.85-0.90(m,6H) |
| II-45:7.30-7.50(m,3H),7.19(d,J=7.8Hz,1H),4.50-4.85(m,4H),4.30-4.40(m,2H),2.46(s,2H),1.32(d,J=7.2Hz,3H),1.26(s,3H),0.95-1.05(m,1H),0.40-0.55(m,3H),0.30-0.40(m,1H) |
| II-47:7.35-7.60(m,3H),7.15-7.25(m,1H),4.75-5.10(m,3H),4.30-4.65(m,1H),2.80-3.00(m,1H),2.50-2.65(m,1H),2.35-2.50(m,2H),1.43(d,J=28.8Hz,3H),1.13(dt,J=3.0,7.2Hz,3H) |
| II-48:7.40-7.55(m,3H),7.25-7.30(m,1H),4.70-5.15(m,3H),4.52(dd,J=16.5,39.6Hz,1H),2.97(dd,J=4.5,15.9Hz,1H),2.60(d,J=15.9Hz,1H ),1.49(s,3H),1.20-1.40(m,2H),1.10-1.20(m,1H),0.90-1.20(m,2H) |
| II-49:7.45-7.50(m,1H),7.35-7.45(m,2H),7.20(d,J=8.1Hz,1H),4.55-4.95(m,4H),4.30-4.45(m,2H),2.97(dd,J=12.0,16.2Hz,1H),2.61(dd,J=3.0,16.2Hz,1H),1.46(s,3H),1.34(dt,J =2.4,7.2Hz,3H) |
| II-50:7.45-7.50(m,1H),7.35-7.45(m,2H),7.21(d,J=7.8Hz,1H),4.55-4.95(m,4H),4.00-4.15(m,2H),2.97(dd,J=11.1,16.2Hz,1H),2.61(dd,J=2.4,16.2Hz,1H),1.90-2.00(m,1H),1.46(s,3H),0.85-0.95(m,6H) |
| II-52:7.50-7.55(m,1H),7.40-7.45(m,2H),7.21 (d,J=7.8Hz,1H),5.02(dd,J=16.2,51.0Hz,1H),4.75-4.90(m,2H),4.25(dd,J=16.2,69.3Hz,1H),2.74(d,J=16.5Hz,1H),2.35-2.60(m,5H),1.48(s,3H),1.13(dt,J=3.0,6.9Hz,3H) |
| II-55:7.30-7.50(m,3H),7.19(d,J=8.0Hz,1H),4.50-4.85(m,4H),4.25-4.45(m,2H),3.25-3.45(m,5H),2.79(dd,J=6.0,15.2Hz,1H),2.40(dd,J=6.0,15.2Hz,1H),1.20-1.35(m,6H) |
| II-56:7.30-7.50(m,3H),7.20(d,J=7.9Hz,1H),4.55-4.90(m,4H),3.95-4.20(m,2H),3.30-3.45(m,5H),2.80(dd,J=5.0,14.9Hz,1H),2.40(dd,J=3.6,14.9Hz,1H),1.85-2.05(m,1H),1.29(s,3H),0.80-0.95(m,6H) |
| II-58:7.10-7.45(m,9H),4.55-4.70(m,2H),4.30-4.55(m,4H),3.17(dd,J=3.0,16.8Hz,1H),2.88(dd,J=1.8,16.8Hz,1H),1.60(s,3H),1.30(dt,J= 3.6,7.2Hz,3H) |
| 11-59:11.12(brs,1H),7.61(d,J=8.0Hz,1H),7.39(t,J=8.0Hz,1H),7.18(t,J=8.0Hz,1H),7.07(d,J= 8.0Hz,1H),5.95(s,1H),4.91(d,J=8.2Hz,1H),4.57(d,J=15.0Hz,1H),4.42(d,J=8.2Hz,1H),4. 10(d,J=15.0Hz,1H),3.93(s,3H),2.96(d,J=17.5Hz,1H),2.70(d,J=17.5Hz,1H),1.64(s,3H) |
| II-60:7.30-7.50(m,2H),7.05-7.25(m,2H),6.16(d,J=6.8Hz,1H),4.50-4.70(m,3H),4.20-4.45(m,3H),3.89(s,3H),3.02(dd,J=6.9,15.5Hz,1H),2.80(dd,J=6.9,15.5Hz,1H),1.67(s,3H) ,1.20-1.40(m,3H) |
| II-63:7.30-7.50(m,3H),7.19(d,J=7.8Hz,1H),4.50-4.75(m,4H),4.25-4.45(m,2H),2.73(s,2H),2.15-2.30(m,2H),1.95-2.10(m,2H),1.60-1.90(m,2H),1.20-1.40(m,3H) |
| II-65:7.51 (d,J=8.0Hz,1H),7.35-7.45(m,2H),7.20(d,J=8.0Hz,1H),5.04(d,J=16.5Hz,1H),4.80(dd,J=3.5,10.2Hz,2H),4.13(d ,J=16.5Hz,1H),2.25-2.65(m,4H),1.90-2.15(m,2H),1.50-1.85(m,6H),1.12(t,J=7.2Hz,3H) |
| II-66:7.30-7.50(m,3H),7.19(d,J=7.9Hz,1H),4.55-4.80(m,4H),4.25-4.45(m,2H),2.63(s,2H),1.85-2.00(m,2H),1.50-1.80(m,6H),1.32(t,J=7.2Hz,3H) |
| II-68:7.30-7.50(m,3H),7.18(d,J=8.0Hz,1H),4.55-4.80(m,4H),4.25-4.45(m,2H),2.48(s,2H),1.65-1.85(m,2H),1.40-1.60(m,8H),1.32(t,J=7.1Hz,3H) |
| II-69:11.36(brs,1H),7.60(d,J=7.8Hz,1H),7.30-7.45(m,1H),7.15-7.25(m,2H),4.94(s,2H),4.45(s,2H),3.60-3.75(m,4H),2.45(s,2H),1.60-1.75(m,4H) |
| II-70:7.30-7.50(m,3H),7.20(d,J=8.0Hz,1H),4.55-4.80(m,4H),4.25-4.45(m,2H),3.65-3.80(m,4H),2.54(s,2H),1.65-1.85(m,4H),1.33(t,J=7.2Hz,3H) |
| II-71:7.30-7.50(m,3H),7.19(d,J=7.8Hz,1H),4.60-5.05(m,3H),4.25-4.45(m,3H),3.55-3.70(m,1H),2.35-2.45(m,1H),1.20-1.35(m,9H) |
| II-73:7.45-7.55(m,1H),7.35-7.45(m,2H),7.20(d,J=7.8Hz,1H),5.14(dq,J=5.4,37.2Hz,1H),4.93(dd,J=17.1,26.7Hz,1H), 4.06(d,J=17.1Hz,1H),2.35-2.60(m,3H),2.15-2.25(m,1H),1.35-1.45(m,9H),1.10(t,J=7.5Hz,3H) |
| II-75:7.40-7.55(m,3H),7.22(d,J=7.5Hz,1H),5.07(t,J=10.5Hz,1H),4.60-4.85(m,4H),4.35-4.45(m,2H),1.64(s,3H),1.35(t,J=6.9Hz,3H) |
| II-79:7.45-7.50(m,1H),7.35-7.45(m,2H),7.21(d,J=7.5Hz,1H),4.84(d,J=15.6Hz,1H),4.74(d,J=15.6Hz,1H),4.25-4.45(m,3H),4.15(dd,J=0.9,13.5Hz,1H),2.95-3.10(m,2H),2.54(t,J=6.3Hz,2H),1.33(t,J=7.2Hz,3H) |
| II-80:10.18(brs,1H),7.59(d,J=8.7Hz,1H),7.40(dt,J=1.8,7.2Hz,1H),7.25-7.35(m,2H),5.13(s,2H),4.99(s,2H),2.98(s,2H),1.26(s,6H) |
| II-81:7.35-7.50(m,3H),7.22(d,J=7.2Hz,1H),5.47(d,J=15.6Hz,1H),5.18(d,J=15.6Hz,1H),4.78(d,J=1 0.2Hz,1H),4.73(d,J=10.2Hz,1H),4.30-4.45(m,2H),3.12(d,J=16.8Hz,1H),3.05(d,J=16.8Hz,1H),1.30-1.35(m,9H) |
| III-1:10.00(brs,1H),7.55-7.60(m,1H),7.40-7.45(m,2H),7.20-7.30(m,1H),4.75(s,2H),4.40-4.45(m,2H),4.15(t,J=7.7Hz,2H) |
| III-2:7.40-7.60(m,3H),7.20-7.25(m,1H),4.85(dd,J=13.0,16.0Hz,2H),4.30-4.45(m,4H),4.00-4.15(m,2H),1.32(t,J=7.0Hz,3H) |
| III-3:10.12(brs,1H),7.55-7.60(m,1H),7.35-7.40(m,2H),7.20-7.30(m,1H),4.75(s,2H),4.60-4.65(m,1H),4.10-4.15(m,1H),3.70-3.75(m,1H),1.33(d,J=6.3Hz,3H) |
| III-4:7.40-7.60(m,3H),7.20-7.25(m,1H),4.65-5.00(m,3H),4.30-4.40(m,2H),4.00-4.10(m,1H),3.65-3.85(m,1H),1.25-1.40(m,6H) |
| III-5:10.11(brs,1H),7.55-7.60(m,1H),7.35-7.45(m,2H),7.20-7.30(m,1H),4.73(dd,J=3.3,15.8Hz,2H),4.40-4.45(m,1H),4.05-4.20(m,1H),3.75-3.85(m,1H),1.50-1.80(m,2H),1.00(t,J=7.4Hz,3H) |
| III-6:7.40-7.60(m,3H),7.20-7.25(m,1H),4.70-5.00(m,2H),4.30-4.50(m,3H),4.05-4.20(m,1H),3.65-3.90(m,1H),1.45-1.85(m,2H),1.32(t,J=6.8Hz,3H),0.95-1.05(m,3H) |
| III-7:7.40-7.60(m,3H),7.20-7.25(m,1H),4.70-5.00(m,2H),4.40-4.55(m,3H),4.05-4.20(m,1H),3.70-3.90(m,1H),1.45-1.85(m,2H),0.95-1.05(m,3H) |
| III-8:9.29(brs,1H),7.50-7.60(m,1H),7.40-7.45(m,2H),7.25-7.35(m,1H),4.70-4.85(m,2H),4.25-4.45(m,2H),4.05-4.15(m,1H) |
| III-9:7.40-7.60(m,3H),7.20-7.25(m,1H),4.70-4.95(m,3H),4.05-4.50(m,4H),1.30-1.40(m,3H) |
| III-10:10.11(brs,1H),7.55-7.60(m,1H),7.35-7.45(m,2H),7.20-7.25(m,1H),4.74(s,2H),3.82(s,2H),1.38(s,6H) |
| III-11:7.40-7.60(m,3H),7.20-7.25(m,1H),4.85(dd,J=6.8,16.1Hz,2H),4.30-4.45(m,2H),3.78(dd,J=11.9,14.6Hz,2H),1.41(s,6H),1.32(t,J=6.8Hz,3H) |
| III-12:7.56(d,J=8.2Hz,1H),7.30-7.45(m,2H),7.10-7.30(m,1H),4.74(s,2H),4.01(t,J=6.3Hz,2H),2.52(t,J=6.9Hz,2H),1.95-2.15(m,2H) |
| III-13:7.35-7.60(m,3H),7.15-7.25(m,1H),4.87(d,J=16.0Hz,1H),4.82(d,J=16.0Hz,1H),4.25-4.45(m,2H),3.80-4.05(m,2H),2.45-2.60(m,2H),2.00-2.15(m,2H),1.32(t,J=6.9Hz,3H) |
| III-14:7.35-7.60(m,3H),7.15-7.25(m,1H),4.89(d,J=16.2Hz,1H),4.81(d,J=16.2Hz,1H),4.52(t,J=5.9Hz,2H),3.80-4.10(m,2H),3.71 (t,J=5.9Hz,2H),2.45-2.65(m,2H),2.00-2.20(m,2H) |
| III-15:7.30-7.60(m,3H),7.15-7.25(m,1H),4.89(d,J=15.8Hz,1H),4.82(d,J=15.8Hz,1H),3.80-4.20(m,4H),2.45-2.60(m,2H),2.00-2.20(m,2H),1.80-2.00(m,1H),0.87(d,J=6.6Hz,6H) |
| IV-1:11.97(brs,1H),7.62(d,J=8.9Hz,1H),7.30-7.40(m,1H),7.10-7.20(m,2H),4.86(s,2H),4.73(s,2H),4.42(s,2H),2.91 (s,3H) |
| IV-2:7.45-7.55(m,2H),7.25-7.40(m,1H),7.19(d,J=8.1Hz,1H),4.55-4.85(m,6H),4.30-4.45(m,2H),2.94(s,3H), 1.31 (t,J=6.9Hz,3H) |
| IV-3:7.45-7.55(m,2H),7.35-7.40(m,1H),7.20(d,J=7.7Hz,1H),4.65-4.85(m,6H),4.52(t,J=5.4Hz,2H),3.69(t,J=5.4Hz,2H),2.93(s,3H) |
| IV-4:7.45-7.55(m,2H),7.36(t,J=7.5Hz,1H),7.19(d,J=7.8Hz,1H),4.55-4.85(m,6H),4.00-4.15(m,2H),2.94(s,3H),1.90-2.00(m,1H),0.87(d,J=6.6Hz,6H) |
| IV-5:12.09(brs,1H),7.64(d,J=8.1Hz,1H),7.10-7.40(m,3H),4.85(s,2H),4.76(s,2H),4.42(s,2H)),3.39(q,J=7.2Hz,2H),1.13(t,J=7.2Hz,3H) |
| IV-6:7.45-7.50(m,2H),7.30-7.40(m,1H),7.18(d,J=7.7Hz,1H),4.55-4.85(m,6H),4.30-4.40(m,2H),3.42(q,J=7.1Hz,2H),1.31(t,J=7.1Hz,3H),1.18(t,J=7.1Hz,3H) |
| IV-7:7.48(d,J=4.2Hz,2H),7.35-7.40(m,1H),7.20(d,J=7.7Hz,1H),4.65-4.85(m,6H),4.52(t,J=6.0Hz,2H),3.69(t,J=6.0Hz,2H),3.41(q,J=7.1Hz,2H),1.18(t,J=7.1Hz, 3H) |
| IV-8:7.40-7.55(m,2H),7.30-7.40(m,1H),7.18(d,J=7.7Hz,1H),4.55-4.90(m,6H),3.95-4.15(m,2H),3.42(q,J=7.1Hz,2H),1.90-2.05(m,1H),1.18(t,J=7.1Hz,3H),0.87(d,J=6.6Hz,6H) |
| IV-9:10.67(brs,1H),7.64(d,J=8.4Hz,1H),7.30-7.40(m,1H),7.15-7.25(m,2H),5.17(s,2H),4.84(s,2H),4.82(s,2H),3.27(s,3H) |
| IV-10:7.55(d,J=7.5Hz,1H),7.48(t,J=7.5Hz,1H),7.36(t,J=7.8Hz,1H),7.19(d,J=7.8Hz,1H),5.4 8(d,J=16.5Hz,1H),5.06(d,J=16.5Hz,1H),4.80-4.95(m,2H),4.70-4.80(m,2H),4.25-4.45(m,2H),3.35(s,3H),1.32(t,J=6.9Hz,3H) |
| V-1:11.26(brs,1H),7.63(d,J=8.0Hz,1H),7.39(t,J=8.0Hz,1H),7.15-7.30(m,2H),4.52(s,2H),3.70-3.80(m,2H),3.60-3.70(m,4H),2.75-2.85(m,2H) |
| V-2:7.48(d,J=7.8Hz,1H),7.30-7.45(m,2H),7.18(d,J=7.8Hz,1H),4.76(d,J=16.2Hz,1H),4.64(d,J=16.2Hz,1H),4.25-4.45(m,2H),3.75-3.85(m,2H),3.60-3.70(m,2H),3.35-3.45(m,2H),2.80-2.90(m,2H),1.33(t,J=7.2Hz,3H) |
| VI-1:10.86(brs,1H),7.55-7.65(m,1H),7.35-7.45(m,2H),7.20-7.30(m,1H),4.72(s,2H),3.95-4.05(m,2H),2.54(brs,2H),1.60-1.75(m,2H),1.00(s,6H) |
| VI-2:7.30-7.60(m,3H),7.20-7.25(m,1H),4.97(d,J=15.6Hz,1H),4.69(d,J=15.6Hz,1H),4.25-4.45(m,2H),3.85-4.00(m,2H),2.60(brs,2H),1.55-1.75(m,2H),1.30(t,J=7.2Hz,3H),1.09(s,6H) |
| VII-1:8.00-8.10(m,1H),7.60-7.70(m,1H),7.40-7.50(m,2H),5.00(s,2H),4.30(s,2H),3.90-4.00(m,2H),3.35-3.45(m,2H) |
| VII-2:7.10-7.15(m,1H),7.00-7.05(m,1H),6.60-6.70(m,2H),4.53(brs,4H),4.20(s,2H),3.82(t,J=5.4Hz,2H),3.30(t,J=5.4Hz,2H) |
| VII-4:7.13(dt,J=1.5,7.8Hz,1H),7.01(dd,J=1.5,7.8Hz,1H),6.60-6.70(m,2H),4.77(d,J=14.7Hz,1H),4.54(brs,2H),4.31(d,J=16.2Hz,1H),4.23(d,J=14.7Hz,1 H),4.14(d,J=16.2Hz,1H),3.50-3.60(m,1H),3.05-3.20(m,2H),1.40-1.60(m,2H),0.94(t,J=7.5Hz,3H) |
| VII-7:8.00-8.10(m,1H),7.60-7.70(m,1H),7.40-7.55(m,2H),4.98(s,2H),4.29(s,2H),3.21 (s,2H),1.33(s,6H) |
| VII-8:7.05-7.20(m,1H),6.95-7.05(m,1H),6.60-6.70(m,2H),4.48(brs,2H),4.52(s,2H),4.20(s,2H),3.08(s,2H),1.20(s,6H) |
| VIII-1:8.05-8.10(m,1H),7.60-7.70(m,1H),7.40-7.50(m,2H),4.91(s,2H),4.78(s,2H),4.11(t,J=6.0Hz,2H),2.69(t,J=6.0Hz,2H) |
| VIII-2:7.05-7.15(m,1H),6.90-7.00(m,1H),6.60-6.65(m,2H),4.70(s,2H),4.57(brs,4H),4.48(s,2H),3.96(t,J=6.3Hz,2H),2.56(t,J=6.3Hz,2H) |
| VIII-3:8.05-8.10(m,1H),7.60-7.65(m,1H),7.40-7.50(m,2H),5.22(d,J=17.7Hz,1H),4.70-4.90(m,2H),4.59(d,J=17.7Hz,1H),4.00-4.10(m,1H),2.40-2.65(m,2H),1.36(d,J=6.0Hz,3H) |
| VIII-4:7.05-7.15(m,1H),6.90-7.00(m,1H),6.60-6.65(m,2H),4.35-4.80(m,6H),3.85-4.00(m,1H),2.25-2.55(m,2H),1.28(d,J=6.3Hz,3H) |
| VIII-6:7.13(dt,J=1.2,7.5Hz,1H),6.98(dd,J=1.5,7.5Hz,1H),6.60-6.70(m,2H),4.90(d,J=9.3Hz,1H),4.74(d,J=9.3Hz,1H),4.54(s,2H),4.40(brs,2H),4.20-4.35(m,1H),2.65-2.80(m,2H) |
| VIII-7:8.00-8.10(m,1H),7.60-7.65(m,1H),7.40-7.50(m,2H),4.93(s,2H),4.79(s,2H),2.53(s,2H),1.37(s,6H) |
| VIII-8:7.05-7.15(m,1H),6.95-7.00(m,1H),6.60-6.70(m,2H),4.70(s,2H),4.51 (brs,4H),2.42(s,2H),1.27(s,6H) |
| VIII-10:7.10(dt,J=1.5,7.8Hz,1H),6.98(dd,J=1.5,7.8Hz,1H),6.64(d,J=7.8Hz,2H),4.68(s,2H),4. 58(brs,2H),4.50(s,2H),2.45(d,J=16.8Hz,1H),2.33(d,J=16.8Hz,1H),1.45-1.70(m,2H),1.20(s,3H),0.89(t,J=7.5Hz,3H) |
| VIII-12:7.25-7.40(m,5H),7.07(dt,J=1.2,7.8Hz,1H),6.87(dd,J=1.2,7.8Hz,1H),6.55-6.65(m,2H),4.71 (d,J=8.7Hz,1H),4.68(d,J=15.0Hz,1H),4.47(brs,2H),4.43(d,J=8.7Hz,1H) ,4.08(d,J=15.0Hz,1H),3.02(d,J=17.1Hz,1H),2.78(d,J=17.1Hz,1H),1.56(s,3H) |
| VIII-14:7.13(dt,J=1.5,7.5Hz,1H),6.99(dd,J=1.5,7.5Hz,1H),6.60-6.70(m,2H),4.85(d,J=9.9Hz,1H),4.71 (d,J=9.9Hz,1H),4.61(d,J=14.7Hz,1H),4.50(d,J=14. 7Hz,1H),4.35(brs,2H),2.89(d,J=16.2Hz,1H),2.54(d,J=16.2Hz,1H),1.41(s,3H) |
| VIII-16:7.11(dt,J=1.8,7.8Hz,1H),6.97(dd,J=1.8,7.8Hz,1H),6.60-6.70(m,2H),4.71 (s,2H),4.56(d,J=14.7Hz,1H),4.48(d,J=14.7Hz,1H),4.44(brs,2H),2.65(d, J=16.8Hz,1H),2.48(d,J=16.8Hz,1H),2.30-2.50(m,2H),1.39(s,3H) |
| VIII-18:7.10(t,J=8.0Hz,1H),6.89(d,J=8.0Hz,1H),6.55-6.70(m,2H),6.07(s,1H),4.65-4.80(m,2H),4.40(brs,2H),4.27(d,J=8.5Hz, 1H),4.04(d,J=15.7Hz,1H),3.89(s,3H),2.93(d,J =17.0Hz,1H),2.71(d,J=17.0Hz,1H),1.62(s,3H) |
| VIII-20:7.09(t,J=7.7Hz,1H),6.97(d,J=7.7Hz,1H),6.55-6.70(m,2H),4.76(d,J=7.7Hz,1H),4.68(d,J=7.7Hz,1H),4.52(brs,2H),4.50(s,2H),3.34(s,3H ),3.29(d,J=2.7Hz,2H),2.69(d,J=16.5Hz,1H),2.30(d,J=16.5Hz,1H),1.23(s,3H) |
| VIII-22:7.11(t,J=7.8Hz,1H),6.96(d,J=7.8Hz,1H),6.55-6.70(m,2H),4.64(s,2H),4.49(brs,2H),4.47(s,2H),2.63(s,2H),2.10-2.25(m,2H),1.90-2.05(m,2H),1.55-1.90(m,2H) |
| VIII-24:7.09(t,J=8.0Hz,1H),6.97(d,J=8.0Hz,1H),6.55-6.70(m,2H),4.68(s,2H),4.52(brs,2H),4.49(s,2H),2.52(s,2H),1.80-1.95(m,2H),1.45-1.80(m,6H) |
| VIII-26:7.23(t,J=7.8Hz,1H),6.90(d,J=7.8Hz,1H),6.85(d,J=7.8Hz,1H),6.59(t,J=7.8Hz,1H),4.6 9(brs,2H),4.59(s,2H),4.42(s,2H),2.28(s,2H),1.15-1.70(m,10H) |
| VIII-28:7.11(t,J=7.9Hz,1H),6.98(d,J=7.9Hz,1H),6.55-6.70(m,2H),4.71(s,2H),4.52(s,2H),4.47(brs,2H),3.60-3.70(m,4H),2.44(s,2H),1.50-1.80(m,4H) |
| VIII-30:7.00-7.15(m,2H),6.60-6.70(m,2H),5.44(d,J=15.0Hz,1H),4.73(q,J=5.4Hz,1H),4.32(brs,2H),3.94(d,J=15.0Hz,1 H),2.51(d,J=16.5Hz,1H),2.35(d,J=16.5Hz,1H),1.49(d,J=5.4Hz,3H),1.26(s,3H),1.16(s,3 H) |
| VIII-31:8.04(dd,J=0.9,8.1Hz,1H),7.55-7.65(m,1H),7.40-7.50(m,2H),5.00(d,J=16.8Hz,1H),4.83(d,J=9.0Hz,1H),4.77(d,J=16.8Hz,1H),4.76(d,J=9. 0Hz,1H),2.57(q,J=7.2Hz,1H),1.36(s,3H),1.24(d,J=7.2Hz,3H),1.24(s,3H) |
| VIII-32:7.10(dt,J=1.2,7.5Hz,1H),6.96(d,J=7.5Hz,1H),6.65-6.90(m,2H),4.72(d,J=8.4Hz,1H),4.68(d,J=8.4Hz,1H),4.58(d,J=15.0Hz,1H),4.51(brs,2H) ,4.40(d,J=15.0Hz,1H),2.44(q,J=7.2Hz,1H),1.28(s,3H),1.19(d,J=7.2Hz,3H),1.15(s,3H) |
| IX-1:8.04(d,J=8.2Hz,1H),7.55-7.65(m,2H),7.35-7.50(m,1H),4.88(s,2H),4.84(s,4H),2.94(s,3H) |
| IX-2:7.10(t,J=7.8Hz,11H),6.93(d,J=7.8Hz1H),6.55-6.70(m,2H),4.80(s,4H),4.67(brs,2H),4.46(s,2H),2.89(s,3H) |
| IX-3:8.03(d,J=7.8Hz,1H),7.55-7.65(m,2H),7.35-7.50(m,1H),4.88(s,2H),4.87(s,2H),4.82(s,2H),3.41(q,J=7.4Hz,2H),1.18(t,J=7.4Hz,3H) |
| IX-4:7.09(t,J=7.9Hz,1H),6.92(d,J=7.9Hz,1H),6.55-6.70(m,2H),4.76(s,2H),4.71 (s,2H),4.45(s,2H),3.36(q,J=7.3Hz,2H),1.14(t,J=7.3Hz,3H) |
| IX-5:8.09(d,J=8.0Hz,1H),7.55-7.70(m,2H),7.35-7.50(m,1H),5.48(s,2H),4.94(s,2H),4.88(s,2H),3.36(s,3H) |
| IX-6:7.12(t,J=7.8Hz,1H),7.00(d,J=7.8Hz,1H),6.55-6.70(m,2H),5.17(s,2H),4.80(s,2H),4.68(s,2H),4.56(brs,2H),3.30(s,3H) |
| |

The proton nuclear magnetic resonance chemical shifts in Table 11 were measured by using Me₄Si (tetramethylsilane) as the standard at 300 MHz.

Now, the usefulness of the compounds of the present invention as herbicides will be described in detail with reference to the following Test Examples.

### [TEST EXAMPLE 1] Test on the herbicidal effects in preemergence treatment on weeds under submerged conditions

Alluvial soil was put in 1/30000-are styrol cups and admixed with water so that it was submerged at a depth of 4 cm. In each cup, seeds of barnyardgrass, bulrush and ducksalad were sown together, and rice seedlings at the 2.5-leaf stage were transplanted. On the same day as the seeding, emulsifiable concentrates containing compounds of the present invention formulated in accordance with Formulation Example 2 were applied to the water surfaces at predetermined doses after diluted with water. The cups were placed in a greenhouse at 25 to 30°C to grow the plants. Three weeks after the application, the herbicidal effects against various plants were determined on the following evaluation scale. The results are shown in Table 14.

### Evaluation scale

| | |
|---|---|
| 5 | mortality of 90% or above (nearly complete death) |
| 4 | mortality of at least 70% and less than 90% |
| 3 | mortality of at least 40% and less than 70% |
| 2 | mortality of at least 20% and less than 40% |
| 1 | mortality of at least 5% and less than 20% |
| 0 | mortality of less than 5% (almost ineffective) |

### [TEST EXAMPLE 2] Test on the herbicidal effects on weeds during the vegetation period under submerged conditions

Alluvial soil was put in 1/30000-are styrol cups and admixed with water so that it was submerged at a depth of 4 cm. In each cup, seeds of barnyardgrass, bulrush and ducksalad were sown together, and the cups were placed in a greenhouse at 25 to 30°C to grow the plants. When the barnyardgrass, bulrush and ducksalad reached the 1- or 2-leaf stage, emulsifiable concentrates containing compounds of the present invention formulated in accordance with Formulation Example 2 were applied to the water surfaces at predetermined doses after diluted with water. Three weeks after the application, the herbicidal effects against various plants were determined on the same evaluation scale as in Test Example 1. The results are shown in Table 15.

### [TEST EXAMPLE 3] Test on the herbicidal effects in soil treatment

Sterilized diluvial soil was put in plastic boxes with a length of 21 cm, a width of 13 cm and a depth of 7 cm, and seeds of barnyardgrass, large crabgrass, giant foxtail, wild oat, blackgrass, velvetleaf, common ragweed, redroot pigweed, common lambsquater, posumbu knotweed, common chickweed corn, soybean, rice, wheat and sugar beet were sown in spots and covered with an about 1.5 cm-thick soil layer. Emulsifiable concentrates containing compounds of the present invention formulated in accordance with Formulation Example 2 were uniformly applied to the soil surfaces at predetermined doses through a small sprayer after dilute with water. Three weeks after the application, the herbicidal effects against various plants were determined on the same evaluation scale as in Test Example 1. The results are shown in Table 16.

### [TEST EXAMPLE 4] Test on the herbicidal effect in foliage treatment

Sterilized diluvial soil was put in plastic boxes with a length of 21 cm, a width of 13 cm and a depth of 7 cm, and seeds of barnyardgrass, large crabgrass, giant foxtail, wild oat, blackgrass, velvetleaf, common ragweed, redroot pigweed, common lambsquater, posumbu knotweed, common chickweed, corn, soybean, rice, wheat and sugar beet were sown in spots and covered with an about 1.5 cm-thick soil layer. The boxes were placed in a greenhouse at 25 to 30°C to grow the plants. After 14 days, emulsifiable concentrates containing compounds of the present invention formulated in accordance with Formulation Example 2 were uniformly applied to the foliage at predetermined doses through a small sprayer after diluted with water. Three weeks after the application, the herbicidal effects against various plants were determined on the same evaluation scale as in Test Example 1. The results are shown in Table 17.

The symbols in Tables 14 to 17 have the following meanings.
A: barnyardgrass, B: bulrush, C: ducksalad, D: large crabgrass, E: giant foxtail, F: wild oat, G: water foxtail, H: Italian ryegrass, I: velvetleaf, J: common ragweed, K: redroot pigweed, L: common lambsquater, M: posumbu knotweed, N: common chickweed, O: beadstraw, a: transplanted rice, b: corn, c: soybean, d: direct-seeded rice, e: wheat, f: sugar beet.

The doses (g/a) represents the amounts in g per 1 are (a) applied after adjustment of concentration adjustment.

**TABLE 14**

| Compound No. | Dose (g/a) | A | B | C | a |
|---|---|---|---|---|---|
| I-1 | 2.52 | 5 | 5 | 5 | 1 |
| I-2 | 2.52 | 5 | 5 | 5 | 1 |
| I-3 | 2.52 | 5 | 5 | 5 | 0 |
| I-4 | 2.52 | 5 | 5 | 5 | 0 |
| I-5 | 2.52 | 5 | 5 | 5 | 0 |
| I-7 | 2.52 | 5 | 5 | 5 | 2 |
| I-8 | 2.52 | 5 | 5 | 5 | 0 |
| I-9 | 2.52 | 5 | 5 | 5 | 0 |
| I-13 | 2.52 | 5 | 5 | 5 | 1 |
| I-14 | 2.52 | 5 | 5 | 5 | 0 |
| I-15 | 2.52 | 5 | 5 | 5 | 1 |
| I-16 | 2.52 | 5 | 5 | 5 | 2 |
| I-17 | 2.52 | 4 | 5 | 5 | 0 |
| I-18 | 2.52 | 5 | 5 | 5 | 0 |
| I-19 | 2.52 | 5 | 5 | 5 | 0 |
| II-1 | 2.52 | 5 | 5 | 5 | 3 |
| II-2 | 2.52 | 5 | 5 | 5 | 1 |
| II-3 | 2.52 | 5 | 5 | 5 | 0 |
| II-4 | 2.52 | 5 | 5 | 5 | 0 |
| II-5 | 2.52 | 5 | 5 | 5 | |
| II-6 | 2.52 | 5 | 5 | 5 | 0 |
| II-7 | 2.52 | 5 | 5 | 5 | 0 |
| II-8 | 2.52 | 5 | 5 | 5 | 0 |
| II-9 | 2.52 | 5 | 5 | 5 | 0 |
| II-10 | 2.52 | 5 | 5 | 5 | 0 |
| II-11 | 2.52 | 5 | 5 | 5 | 0 |
| II-12 | 2.52 | 5 | 5 | 5 | 0 |
| II-13 | 2. 52 | 5 | 5 | 5 | 0 |
| II-14 | 2.52 | 5 | 5 | 5 | 0 |
| II-15 | 2. 52 | 5 | 5 | 5 | 0 |
| II-16 | 2.52 | 5 | 5 | 5 | 0 |
| II-17 | 2.52 | 5 | 5 | 5 | 0 |
| II-18 | 2.52 | 2 | 5 | 0 | 0 |
| II-19 | 2.52 | 5 | 5 | 5 | 0 |
| II-20 | 2. 52 | 5 | 5 | 5 | |
| II-21 | 2.52 | 5 | 5 | 5 | 0 |
| II-22 | 2.52 | 5 | 5 | 5 | 0 |
| II-23 | 2.52 | 5 | 5 | 5 | 0 |
| II-24 | 2.52 | 5 | 5 | 5 | 0 |
| II-25 | 2.52 | 5 | 5 | 5 | 0 |
| II-26 | 2.52 | 5 | 5 | 5 | 0 |
| II-27 | 2.52 | 5 | 5 | 5 | 0 |
| II-28 | 2.52 | 5 | 5 | 5 | 4 |
| II-32 | 2.52 | 5 | 5 | 5 | 0 |
| II-33 | 2.52 | 5 | 5 | 5 | 0 |
| II-34 | 2.52 | 5 | 5 | 5 | 0 |
| IT-35 | 2.52 | 5 | 5 | 5 | 0 |
| II-36 | 2.52 | 5 | 5 | 5 | 0 |
| II-37 | 2.52 | 5 | 5 | 5 | 2 |
| II-39 | 2.52 | 5 | 5 | 5 | 0 |
| II-40 | 2. 52 | 5 | 5 | 5 | 0 |
| II-41 | 2.52 | 5 | 5 | 5 | 0 |
| II-42 | 2.52 | 5 | 5 | 5 | 0 |
| II-43 | 2.52 | 5 | 5 | 5 | 0 |
| II-44 | 2.52 | 5 | 5 | 5 | 3 |
| II-45 | 2.52 | 5 | 5 | 5 | 0 |
| II-46 | 2.52 | 4 | 5 | 5 | 0 |
| II-47 | 2.52 | 5 | 5 | 5 | 0 |
| II-48 | 2.52 | 5 | 5 | 5 | 0 |
| II-49 | 2.52 | 5 | 5 | 5 | 0 |
| II-50 | 2.52 | 5 | 5 | 5 | 0 |
| II-51 | 2.52 | 5 | 5 | 5 | 0 |
| II-52 | 2.52 | 5 | 5 | 5 | 0 |
| II-53 | 2.52 | 5 | 5 | 5 | 0 |
| II-54 | 2.52 | 5 | 5 | 5 | 0 |
| II-55 | 2.52 | 5 | 5 | 5 | 0 |
| II-56 | 2.52 | 5 | 5 | 5 | 0 |
| II-57 | 2.52 | 5 | 5 | 5 | 0 |
| II-58 | 2. 52 | 5 | 5 | 5 | 0 |
| II-59 | 2. 52 | 5 | 5 | 5 | 0 |
| II-60 | 2.52 | 5 | 5 | 5 | 0 |
| II-61 | 2.52 | 5 | 5 | 5 | 2 |
| II-62 | 2. 52 | 5 | 5 | 5 | 0 |
| II-63 | 2.52 | 5 | 5 | 5 | 1 |
| II-64 | 2. 52 | 5 | 5 | 5 | 0 |
| II-65 | 2. 52 | 5 | 5 | 5 | 0 |
| II-66 | 2. 52 | 5 | 5 | 5 | 0 |
| II-67 | 2.52 | 5 | 5 | 5 | 0 |
| II-68 | 2.52 | 5 | 5 | 5 | 3 |
| II-70 | 2. 52 | 5 | 5 | 5 | 3 |
| II-71 | 2.52 | 5 | 5 | 5 | 0 |
| II-72 | 2.52 | 5 | 5 | 5 | 3 |
| II-73 | 2.52 | 5 | 5 | 5 | 3 |
| II-74 | 2. 52 | 5 | 5 | 5 | 3 |
| II-75 | 2.52 | 5 | 5 | 5 | 0 |
| II-77 | 2.52 | 5 | 5 | 5 | 0 |
| II-78 | 2.52 | 5 | 5 | 5 | 0 |
| II-79 | 2.52 | 5 | 5 | 5 | 0 |
| III-2 | 2.52 | 4 | 5 | 5 | 0 |
| III-3 | 2.52 | 4 | 5 | 5 | 0 |
| III-4 | 2.52 | 5 | 5 | 5 | 0 |
| III-5 | 2.52 | 5 | 5 | 5 | 0 |
| III-6 | 2.52 | 5 | 5 | 5 | 0 |
| III-7 | 2.52 | 5 | 5 | 5 | 0 |
| III-9 | 2.52 | 4 | 5 | 0 | 0 |
| III-10 | 2.52 | 5 | 5 | 5 | 0 |
| III-11 | 2.52 | 5 | 5 | 5 | 0 |
| III-12 | 2.52 | 5 | 5 | 5 | 2 |
| III-13 | 2.52 | 5 | 5 | 5 | 1 |
| III-14 | 2.52 | 5 | 5 | 5 | 3 |
| III-15 | 2.52 | 5 | 5 | 5 | 2 |
| IV-1 | 2.52 | 5 | 5 | 4 | 0 |
| IV-2 | 2.52 | 5 | 5 | 3 | 0 |
| IV-3 | 2.52 | 5 | 5 | 5 | 0 |
| IV-4 | 2.52 | 5 | 5 | 4 | 0 |
| IV-5 | 2.52 | 5 | 5 | 4 | 0 |
| IV-6 | 2.52 | 5 | 5 | 5 | 0 |
| IV-7 | 2.52 | 5 | 5 | 4 | 0 |
| IV-8 | 2.52 | 5 | 5 | 4 | 0 |
| V-1 | 2.52 | 5 | 5 | 5 | 0 |
| V-2 | 2.52 | 5 | 5 | 5 | 0 |

**TABLE 15**

| Compound No. | Dose (g/a) | A | B | C |
|---|---|---|---|---|
| I-1 | 2. 52 | 5 | 5 | 5 |
| I-2 | 2. 52 | 5 | 5 | 5 |
| I-3 | 2. 52 | 5 | 5 | 5 |
| I-4 | 2. 52 | 5 | 5 | 5 |
| I-5 | 2. 52 | 5 | 5 | 5 |
| I-7 | 2. 52 | 5 | 5 | 5 |
| I-8 | 2. 52 | 5 | 5 | 5 |
| I-9 | 2. 52 | 5 | 5 | 5 |
| I-13 | 2. 52 | 5 | 5 | 5 |
| I-14 | 2. 52 | 5 | 5 | 5 |
| I-15 | 2. 52 | 5 | 5 | 5 |
| I-16 | 2. 52 | 5 | 5 | 5 |
| I-17 | 2. 52 | | 5 | 4 |
| I-18 | 2.52 | | 5 | 4 |
| I-19 | 2. 52 | 3 | 5 | 4 |
| II-1 | 2. 52 | 5 | 5 | 5 |
| II-2 | 2. 52 | 5 | 5 | 4 |
| II-3 | 2. 52 | 5 | 5 | 4 |
| II-4 | 2. 52 | 5 | 5 | 5 |
| II-5 | 2. 52 | 5 | 5 | 5 |
| II-6 | 2. 52 | 5 | 5 | 4 |
| II-7 | 2. 52 | 5 | 5 | 4 |
| II-8 | 2. 52 | 5 | 5 | 4 |
| II-9 | 2. 52 | 5 | 5 | 5 |
| II-10 | 2. 52 | 4 | 5 | 5 |
| II-11 | 2. 52 | 5 | 5 | 5 |
| II-12 | 2. 52 | 4 | 5 | 5 |
| II-13 | 2. 52 | 5 | 5 | 5 |
| II-14 | 2. 52 | 5 | 5 | 5 |
| II-15 | 2. 52 | 5 | 5 | 5 |
| II-16 | 2. 52 | 1 | 5 | 4 |
| II-17 | 2. 52 | 5 | 5 | 5 |
| II-18 | 2. 52 | 5 | 5 | 2 |
| II-19 | 2. 52 | 5 | 5 | 5 |
| II-20 | 2. 52 | 5 | 5 | 5 |
| II-21 | 2. 52 | 5 | 5 | 5 |
| II-22 | 2. 52 | 5 | 5 | 5 |
| II-23 | 2. 52 | 5 | 5 | 5 |
| II-24 | 2. 52 | 5 | 5 | 5 |
| II-25 | 2. 52 | 5 | 5 | 5 |
| II-26 | 2. 52 | 5 | 5 | 5 |
| II-27 | 2. 52 | 5 | 5 | 5 |
| II-28 | 2. 52 | 5 | 5 | 5 |
| II-32 | 2.52 | 5 | 5 | 5 |
| II-33 | 2.52 | 5 | 5 | 5 |
| I I-34 | 2.52 | 5 | 5 | 5 |
| II-35 | 2.52 | 5 | 5 | 5 |
| II-36 | 2.52 | 5 | 5 | 5 |
| II-37 | 2.52 | 5 | 5 | 5 |
| II-39 | 2.52 | 5 | 5 | 5 |
| II-40 | 2.52 | 4 | 5 | 5 |
| II-41 | 2.52 | 5 | 5 | 5 |
| II-42 | 2.52 | 5 | 5 | 5 |
| II-43 | 2.52 | 5 | 5 | 5 |
| II-44 | 2.52 | 5 | 5 | 5 |
| II-45 | 2.52 | 5 | 5 | 5 |
| II-46 | 2.52 | 4 | 5 | 5 |
| II-47 | 2.52 | 4 | 5 | 5 |
| II-48 | 2.52 | 5 | 5 | 5 |
| II-49 | 2.52 | 5 | 5 | 5 |
| II-50 | 2.52 | 5 | 5 | 5 |
| II-51 | 2.52 | 5 | 4 | 5 |
| II-52 | 2.52 | 5 | 5 | 5 |
| II-53 | 2.52 | 5 | 5 | 5 |
| II-54 | 2.52 | 5 | 5 | 5 |
| II-55 | 2.52 | 5 | 5 | 5 |
| II-56 | 2.52 | 5 | 5 | 5 |
| II-57 | 2.52 | 2 | 5 | 5 |
| II-58 | 2.52 | 4 | 5 | 5 |
| II-59 | 2.52 | 5 | 5 | 5 |
| II-60 | 2.52 | 5 | 5 | 5 |
| II-61 | 2.52 | 5 | 5 | 5 |
| II-62 | 2.52 | 5 | 5 | 5 |
| II-63 | 2.52 | 5 | 5 | 5 |
| II-64 | 2.52 | 5 | 5 | 5 |
| II-65 | 2.52 | 5 | 5 | 5 |
| II-66 | 2.52 | 5 | 5 | 5 |
| II-67 | 2.52 | 5 | 5 | 5 |
| II-68 | 2.52 | 5 | 5 | 5 |
| II-70 | 2.52 | 5 | 5 | 5 |
| II-71 | 2.52 | 5 | 5 | 5 |
| II-72 | 2.52 | 5 | 5 | 5 |
| II-73 | 2.52 | 5 | 5 | 5 |
| II-74 | 2.52 | 5 | 5 | 5 |
| II-75 | 2.52 | 5 | 5 | 5 |
| II-77 | 2.52 | 0 | 5 | 4 |
| II-78 | 2.52 | 5 | 5 | 5 |
| II-79 | 2.52 | 5 | 5 | 5 |
| III-2 | 2.52 | 3 | 5 | 4 |
| III-3 | 2.52 | 5 | 5 | 5 |
| III-4 | 2.52 | 5 | 5 | 5 |
| III-5 | 2.52 | 5 | 5 | 5 |
| III-6 | 2.52 | 5 | 5 | 5 |
| III-7 | 2.52 | 5 | 5 | 5 |
| III-9 | 2.52 | 4 | 5 | 4 |
| III-10 | 2.52 | 5 | 5 | 5 |
| III-11 | 2.52 | 5 | 5 | 5 |
| III-12 | 2.52 | 5 | 5 | 5 |
| III-13 | 2.52 | 5 | 5 | 5 |
| III-14 | 2.52 | 5 | 5 | 5 |
| III-15 | 2.52 | 5 | 5 | 5 |
| IV-1 | 2.52 | 4 | 5 | 4 |
| IV-2 | 2.52 | 5 | 5 | 4 |
| IV-3 | 2.52 | 5 | 5 | 4 |
| IV-4 | 2.52 | 5 | 5 | 5 |
| IV-5 | 2.52 | 5 | 5 | 5 |
| IV-6 | 2.52 | 5 | 5 | 5 |
| IV-7 | 2.52 | 5 | 5 | 5 |
| IV-8 | 2.52 | 5 | 5 | 4 |
| V-1 | 2.52 | 5 | 5 | 5 |
| V-2 | 2.52 | 4 | 5 | 5 |

**TABLE 16**

| Compound No. | dose (g/a) | A | D | E | F | G | H | I | J | K | L | M | N | O | b | c | d | e | f |
|---|---|---|---|---|---|---|---|---|---|---|---|---|---|---|---|---|---|---|---|
| I-1 | 6.3 | 5 | 5 | 5 | 4 | 5 | 5 | 5 | 4 | 5 | 5 | 5 | 5 | | 5 | 1 | 5 | 5 | 5 |
| I-2 | 6.3 | 5 | 5 | 5 | 3 | 5 | 5 | 5 | 5 | 5 | 5 | 5 | 5 | | 3 | 4 | 4 | 5 | 4 |
| I-3 | 6.3 | 5 | 5 | 5 | 0 | 5 | 5 | 5 | 4 | 3 | 5 | 3 | 5 | | 3 | 2 | 4 | 4 | 3 |
| I-4 | 6.3 | 4 | 5 | 3 | 0 | 5 | 3 | 4 | 5 | 4 | 5 | 4 | 5 | | 4 | 3 | 4 | 5 | 4 |
| I-5 | 6.3 | 5 | 5 | 5 | 3 | 5 | 1 | 4 | 4 | 4 | 5 | 4 | 5 | | 5 | 2 | 3 | 5 | 4 |
| I-7 | 3.2 | 5 | 5 | 5 | | 5 | 5 | 5 | 5 | 5 | 5 | 5 | 5 | | 5 | 0 | 5 | 4 | 5 |
| I-8 | 3.2 | 5 | 5 | 4 | 1 | 5 | 5 | 5 | 5 | 5 | 3 | | 5 | | 1 | 0 | 5 | 4 | 4 |
| I-9 | 3.2 | 5 | 5 | 4 | 3 | 5 | 3 | 5 | 5 | 4 | 0 | 0 | 5 | 4 | 0 | 0 | 4 | 3 | 4 |
| I-13 | 3.2 | 5 | 5 | 5 | 3 | 5 | 5 | 5 | 5 | 5 | 5 | 5 | 5 | | 3 | 3 | 5 | 3 | 4 |
| I-14 | 3.2 | 5 | 5 | 5 | 4 | 5 | 5 | 5 | 5 | 5 | 5 | 5 | 5 | | 0 | 0 | 5 | 4 | 4 |
| I-15 | 3.2 | 5 | 5 | 3 | 3 | 5 | 5 | 5 | 5 | 5 | 4 | | 4 | | 0 | 0 | 5 | 4 | 4 |
| I-16 | 3.2 | 5 | 5 | 5 | 3 | 5 | 4 | 5 | 5 | 5 | 3 | | 5 | 0 | 1 | 0 | 5 | 0 | 3 |
| I-17 | 6.3 | 5 | 5 | 3 | 0 | 5 | 3 | 1 | 4 | 3 | 5 | 2 | 5 | | 0 | 1 | 1 | 4 | 3 |
| I-18 | 6.3 | 5 | 4 | 5 | 0 | 5 | 4 | 1 | 4 | 4 | 5 | 3 | 4 | 3 | 0 | 1 | 1 | 4 | 0 |
| I-19 | 6.3 | 4 | 4 | 4 | 0 | 5 | 2 | 2 | 4 | 4 | 5 | 4 | 5 | | 0 | 2 | 3 | 0 | 4 |
| II-1 | 6.3 | 5 | 5 | 5 | 0 | 5 | 3 | 5 | 5 | 1 | 5 | 1 | 5 | | 3 | 2 | 5 | 4 | 4 |
| II-2 | 6.3 | 5 | 5 | 5 | 0 | 5 | 4 | 5 | 5 | 0 | 5 | 1 | 5 | | 3 | 1 | 5 | 1 | 4 |
| II-3 | 6.3 | 5 | 5 | 3 | 0 | 5 | 4 | 4 | 3 | | 1 | 1 | 3 | | 5 | 0 | 4 | 5 | 4 |
| II-4 | 6.3 | 5 | 5 | 5 | 0 | 5 | 3 | | 4 | 0 | 4 | | 5 | | 3 | 1 | 4 | 5 | 3 |
| II-5 | 6.3 | 5 | 5 | 3 | 0 | 5 | 4 | | 4 | 1 | 4 | | 5 | | 0 | 0 | 3 | 0 | 3 |
| II-6 | 6.3 | 5 | 4 | 4 | 0 | 5 | 3 | | 4 | 0 | 3 | | | | 0 | 1 | 3 | 0 | 1 |
| II-7 | 6.3 | 5 | 5 | 5 | 2 | 5 | 2 | | 4 | 0 | 5 | | 4 | | 1 | 1 | 3 | 1 | 0 |
| II-8 | 6.3 | 5 | 4 | 4 | 0 | 5 | 0 | | 5 | 0 | 4 | | 3 | | 0 | 0 | 2 | 1 | 1 |
| II-9 | 3.2 | 5 | 5 | 4 | 3 | 5 | 4 | 5 | 0 | 0 | 0 | 5 | 3 | | 1 | 0 | 0 | 4 | 0 |
| II-10 | 3.2 | 4 | 4 | 4 | | 5 | 2 | | 0 | 0 | 3 | 0 | 5 | | 0 | 0 | 0 | 0 | 0 |
| II-11 | 3.2 | 3 | 3 | 4 | 0 | 4 | 3 | 5 | 2 | 0 | 0 | 0 | 3 | | 0 | 0 | 0 | 0 | 0 |
| II-12 | 3.2 | 4 | 4 | 5 | 0 | 5 | 4 | | 0 | 0 | 0 | 0 | 3 | | 0 | 0 | 1 | 0 | 0 |
| II-13 | 3.2 | 3 | 2 | 4 | 0 | 0 | 0 | 0 | 0 | 0 | 0 | | 0 | | 0 | 0 | 0 | 0 | 0 |
| II-14 | 3.2 | 5 | 5 | 4 | 0 | 5 | 3 | 5 | 4 | 4 | 4 | 5 | 5 | 5 | 0 | 0 | 0 | 0 | 3 |
| II-15 | 3.2 | 4 | 5 | 0 | 0 | 5 | 4 | | 4 | 4 | 4 | 0 | 4 | 0 | 0 | 0 | 0 | 3 | 2 |
| II-16 | 3.2 | 0 | 0 | 0 | 0 | 5 | 0 | 0 | 3 | 0 | 1 | 0 | | 0 | 0 | 0 | 0 | 0 | 0 |
| II-17 | 6.3 | 5 | 5 | 5 | 0 | 5 | 4 | | 5 | 1 | 2 | | 4 | | 5 | 1 | 4 | 0 | 0 |
| II-18 | 3.2 | 4 | 0 | 0 | 0 | 0 | 0 | 0 | 4 | 0 | 0 | 0 | 4 | 4 | 0 | 0 | 0 | 0 | 0 |
| II-19 | 6.3 | 5 | 5 | 2 | 0 | 5 | 5 | | | 2 | 0 | | 4 | 4 | 3 | 0 | 1 | 2 | 0 |
| II-20 | 6.3 | 5 | 4 | 4 | 0 | 5 | 5 | | 4 | 0 | 0 | | 4 | | 0 | 2 | 2 | 3 | 0 |
| II-21 | 6.3 | 5 | 5 | 3 | 0 | 5 | 5 | | 5 | | 0 | | 4 | | 5 | 0 | 4 | 3 | 4 |
| II-22 | 3.2 | 5 | 5 | 3 | 0 | 5 | 5 | 5 | 3 | 0 | 4 | | 4 | 5 | 0 | 0 | 0 | 0 | 3 |
| II-23 | 6.3 | 5 | 5 | 5 | 0 | 5 | 4 | | 5 | | 2 | | 4 | | 3 | 0 | 1 | 0 | 0 |
| II-24 | 3.2 | 4 | 4 | 3 | 0 | 5 | 5 | 5 | 4 | 0 | | 4 | 3 | | 0 | 0 | 0 | 0 | 0 |
| II-25 | 3.2 | 4 | 4 | 3 | 0 | 5 | 1 | 5 | 4 | 0 | 0 | | 4 | 5 | 0 | 0 | 0 | 0 | 0 |
| II-26 | 6.3 | 5 | 5 | 4 | 0 | 5 | 3 | | 5 | 0 | 0 | | 2 | | 1 | 0 | 2 | 0 | 0 |
| II-27 | 3.2 | 5 | 5 | 4 | 0 | 5 | 3 | | 4 | 0 | 0 | 3 | 4 | | 0 | 0 | 0 | 0 | 4 |
| II-28 | 3.2 | 5 | 5 | 5 | | 5 | 2 | 4 | 4 | 0 | 0 | 5 | 4 | 5 | 0 | 0 | 0 | 0 | 3 |
| II-32 | 3.2 | 5 | 5 | 4 | 0 | 5 | 5 | 3 | 5 | 3 | 0 | 4 | 3 | 3 | 2 | 0 | 0 | 0 | 0 |
| II-33 | 3.2 | 5 | 5 | 5 | 0 | 5 | 4 | 4 | 5 | 3 | 0 | | 3 | 4 | 4 | 0 | 1 | 0 | 0 |
| II-34 | 3.2 | 5 | 5 | 4 | 0 | 5 | 2 | 3 | 4 | 3 | 3 | 5 | 3 | 4 | 0 | 0 | 0 | 0 | 0 |
| II-35 | 3.2 | 5 | 5 | 3 | 3 | 5 | 4 | 3 | 4 | 2 | 0 | | 1 | 4 | 1 | 0 | 0 | 0 | 0 |
| II-36 | 3.2 | 5 | 4 | 3 | 0 | 5 | 0 | 3 | 4 | 0 | 0 | 4 | 4 | 4 | 0 | 0 | 0 | 0 | 0 |
| II-37 | 3.2 | 5 | 5 | 3 | 4 | 5 | 0 | 0 | 5 | 3 | 0 | 0 | 2 | 4 | 0 | 1 | 0 | 0 | 0 |
| II-39 | 3.2 | 5 | 5 | 5 | | 5 | 5 | 5 | 4 | 1 | 5 | 5 | 5 | | 0 | 0 | 0 | 3 | 5 |
| II-40 | 3.2 | 5 | 5 | 5 | | 5 | 5 | 5 | 5 | 4 | 0 | 4 | 5 | | 2 | 0 | 5 | 5 | 0 |
| II-41 | 3.2 | 5 | 4 | 4 | | 5 | 5 | 5 | 4 | 2 | 3 | 4 | 5 | | 0 | 0 | 4 | 3 | 0 |
| II-42 | 3.2 | 5 | 5 | 1 | | 5 | 5 | 5 | 4 | 3 | 0 | 4 | 5 | | 0 | 0 | 4 | 3 | 0 |
| II-43 | 3.2 | 4 | 3 | 2 | | 5 | 5 | 5 | 3 | 2 | 5 | 4 | 5 | | 0 | 0 | 0 | 1 | 0 |
| II-44 | 3.2 | 5 | 4 | 4 | | 5 | 5 | 3 | 3 | 0 | 0 | 3 | 2 | 0 | 3 | 0 | 1 | 0 | 0 |
| II-45 | 3.2 | 5 | 4 | 4 | | 5 | 4 | 0 | 4 | 0 | 0 | 4 | 3 | | 0 | 0 | 0 | 0 | 3 |
| II-46 | 3.2 | 5 | 5 | 5 | | 5 | 3 | 3 | 4 | 0 | 4 | 3 | 4 | | 0 | 0 | 0 | 2 | 3 |
| II-47 | 3.2 | 5 | 5 | 4 | | 5 | 3 | 4 | 4 | 0 | 3 | 3 | | | 0 | 0 | 0 | 2 | 0 |
| II-48 | 3.2 | 5 | 5 | 4 | | 5 | 0 | | 4 | 0 | 0 | 3 | 4 | | 0 | 0 | 0 | 0 | 0 |
| II-49 | 3.2 | 3 | 3 | 3 | | 5 | 3 | 0 | 4 | 0 | 2 | 4 | 4 | | 0 | 0 | 0 | 0 | 0 |
| II-50 | 3.2 | 4 | 3 | 2 | | 4 | 0 | | 3 | 0 | 3 | 0 | 3 | | 0 | 0 | 0 | 0 | 0 |
| II-51 | 3.2 | 5 | 5 | 4 | | 5 | 3 | 5 | 4 | 0 | 0 | 5 | 5 | | 3 | 0 | 4 | 0 | 4 |
| II-52 | 3.2 | 5 | 5 | 5 | | 5 | 5 | 5 | 5 | 1 | 4 | 5 | 5 | 0 | 5 | 0 | 5 | 0 | 3 |
| II-53 | 3.2 | 5 | 5 | 5 | | 5 | 3 | 5 | 4 | 0 | 0 | 5 | 5 | | 0 | 0 | 2 | 0 | 1 |
| II-54 | 3.2 | 5 | 5 | 5 | | 5 | 5 | 5 | 4 | 3 | 3 | 5 | 5 | | 4 | 0 | 3 | 3 | 3 |
| II-55 | 3.2 | 5 | 5 | 5 | | 5 | 5 | 5 | 5 | 3 | 4 | 5 | 5 | | 4 | 0 | 4 | 3 | 5 |
| II-56 | 3.2 | 5 | 4 | 5 | | 5 | 5 | 5 | 4 | 2 | 3 | 3 | 5 | 0 | 0 | 0 | 0 | 0 | 3 |
| II-57 | 3.2 | 2 | 3 | 5 | | 5 | 5 | 5 | 0 | 0 | 0 | | 4 | 0 | 0 | 0 | 0 | 0 | 0 |
| II-58 | 3.2 | 0 | 0 | 0 | | 5 | 4 | 0 | 0 | 0 | 0 | 4 | 0 | | 0 | 0 | 0 | 0 | 5 |
| II-59 | 3.2 | 4 | 1 | 4 | | 0 | 5 | 1 | 3 | 3 | 0 | 1 | 4 | 1 | 0 | 0 | 0 | 0 | 0 |
| II-60 | 3.2 | 5 | 4 | 5 | | 5 | 3 | 4 | 1 | 4 | 1 | 3 | 4 | 0 | 0 | 0 | 0 | 0 | 4 |
| II-61 | 3.2 | 5 | 5 | 5 | | 5 | 5 | 5 | 4 | 5 | 5 | | 5 | 5 | 4 | 0 | 5 | 5 | 3 |
| II-62 | 3.2 | 5 | 5 | 5 | | 5 | 5 | 5 | 4 | 4 | 4 | 5 | 5 | 4 | 2 | 0 | 4 | 4 | 1 |
| II-63 | 32 | 5 | 5 | 5 | | 5 | 4 | 5 | 4 | 4 | 3 | 4 | 5 | 4 | 4 | 0 | 5 | 5 | 0 |
| II-64 | 3. 2 | 5 | 5 | 5 | | 5 | 5 | 3 | 4 | 4 | 5 | 5 | 4 | 4 | 3 | 0 | 5 | 5 | 0 |
| II-65 | 3.2 | 5 | 5 | 5 | 0 | 5 | 5 | 3 | 4 | 2 | 0 | 4 | 5 | 0 | 0 | 0 | 1 | 0 | 3 |
| II-66 | 3.2 | 5 | 5 | 5 | | 5 | 5 | 4 | 3 | 2 | 0 | | 5 | | 3 | 0 | 3 | 0 | 0 |
| II-67 | 3.2 | 5 | 5 | 5 | | 5 | 5 | 5 | 2 | 3 | 5 | 5 | 5 | 5 | 1 | 0 | 4 | 5 | 4 |
| II-68 | 3.2 | 5 | 5 | 5 | | 5 | 5 | 5 | 2 | 3 | 4 | 5 | 4 | | 2 | 0 | 5 | 4 | 4 |
| II-70 | 3.2 | 5 | 5 | 5 | | 5 | 5 | 5 | 3 | 3 | 5 | 5 | 4 | | 1 | 0 | 5 | 3 | 4 |
| II-71 | 3.2 | 5 | 5 | 4 | | 5 | 2 | | 5 | 5 | 0 | 3 | 3 | | 1 | 0 | 3 | 0 | 0 |
| II-72 | 3.2 | 5 | 5 | 5 | | 5 | 5 | | 4 | 3 | 0 | | 4 | 4 | 0 | 0 | 4 | 0 | 0 |
| II-73 | 3.2 | 5 | 5 | 5 | | 5 | 5 | 5 | 3 | 0 | 0 | | 0 | 4 | 0 | 0 | 5 | 4 | 0 |
| II-74 | 3.2 | 5 | 5 | 5 | | 5 | 4 | 5 | 2 | 0 | 1 | 5 | 3 | 4 | 0 | 0 | 4 | 0 | 0 |
| II-76 | 3.2 | 3 | 3 | 0 | 0 | 5 | 0 | 3 | 0 | 3 | 3 | | 3 | 5 | 0 | 0 | 0 | 0 | 0 |
| II-77 | 3.2 | 4 | 5 | 1 | | 3 | 1 | 5 | 0 | 4 | 5 | | 4 | 0 | 0 | 0 | 0 | or | 0 |
| II-78 | 3.2 | 5 | 5 | 5 | 0 | 5 | 5 | 5 | 4 | 3 | 4 | 4 | 5 | 0 | 4 | 2 | 3 | 0 | 0 |
| II-79 | 3.2 | 5 | 5 | 5 | 0 | 5 | 3 | 5 | 4 | 2 | 3 | | 4 | 0 | 1 | 0 | 0 | 0 | 0 |
| III-2 | 3.2 | 4 | 0 | 0 | | 5 | 3 | 5 | 0 | 0 | 5 | 4 | 3 | | 0 | 0 | 0 | 3 | 0 |
| III-3 | 3.2 | 5 | 5 | 5 | | 5 | 0 | 1 | 5 | 0 | 0 | 5 | 4 | 0 | 0 | 0 | 3 | 0 | 0 |
| III-4 | 3.2 | 5 | 5 | 5 | | 5 | 5 | 5 | 3 | 3 | 5 | 5 | 4 | | 1 | 0 | 5 | 3 | 4 |
| III-5 | 3.2 | 5 | 5 | 5 | | 5 | 5 | 5 | 4 | 0 | 5 | 4 | 5 | 4 | 0 | 0 | 1 | 0 | 0 |
| III-6 | 3.2 | 5 | 5 | 4 | | 5 | 5 | 5 | 4 | 3 | 4 | 5 | 5 | 4 | 0 | 0 | 0 | 3 | 5 |
| III-7 | 3.2 | 5 | 5 | 5 | | 5 | 4 | 0 | 4 | 1 | 4 | 4 | 5 | | 0 | 0 | 0 | 2 | 3 |
| III-9 | 3.2 | 2 | 0 | 2 | | 5 | 0 | | 0 | 0 | 3 | 1 | 1 | | 0 | 0 | 0 | 0 | 0 |
| III-10 | 3.2 | 5 | 5 | 4 | | 5 | 4 | | 5 | 2 | 4 | 5 | 4 | | 4 | 0 | 3 | 1 | 3 |
| III-11 | 3.2 | 5 | 5 | 5 | | 5 | 4 | 4 | 5 | 3 | 5 | 5 | 5 | | 4 | 0 | 0 | 2 | 4 |
| III-12 | 3.2 | 5 | 5 | 5 | 2 | 5 | 3 | 5 | 5 | 0 | 0 | | | | 0 | 0 | 0 | 0 | |
| III-13 | 3.2 | 5 | 5 | 5 | 3 | 5 | 5 | 5 | 5 | 3 | 0 | 0 | 4 | 0 | 1 | 1 | 4 | 1 | 3 |
| III-14 | 3.2 | 5 | 5 | 5 | 0 | 5 | 3 | 5 | 5 | 1 | 0 | 3 | 5 | | 1 | 1 | 4 | 0 | 0 |
| III-15 | 3.2 | 5 | 5 | 5 | 0 | 5 | 3 | 4 | 3 | 2 | 0 | 0 | 4 | 0 | 0 | 0 | 0 | 0 | 0 |
| IV-1 | 3.2 | 5 | 4 | 3 | 0 | 5 | 0 | 3 | 2 | 0 | 0 | 0 | 0 | 0 | 0 | 0 | 3 | 0 | 0 |
| IV-2 | 3.2 | 5 | 5 | 4 | 0 | 5 | 2 | 2 | 0 | 0 | 0 | 0 | 0 | 0 | 1 | 0 | 0 | 0 | 0 |
| IV-3 | 3.2 | 5 | 4 | 3 | 0 | 5 | 0 | 5 | 0 | 0 | 0 | 0 | 0 | 0 | 0 | 0 | 0 | 0 | 0 |
| IV-4 | 3.2 | 4 | 4 | 3 | 0 | 4 | 0 | 0 | 0 | 0 | 0 | 0 | 0 | 0 | 0 | 0 | 0 | 0 | 0 |
| IV-5 | 3.2 | 5 | 5 | 5 | 2 | 5 | 4 | 5 | 0 | 3 | 3 | 4 | 5 | | 0 | 0 | 0 | 3 | 4 |
| IV-6 | 3.2 | 5 | 5 | 5 | 0 | 5 | 0 | 5 | 0 | 1 | 0 | 0 | 4 | 4 | 0 | 0 | 0 | 0 | 0 |
| IV-7 | 3.2 | 5 | 5 | 3 | 1 | 5 | 4 | 5 | 0 | 1 | 4 | 0 | 4 | | 1 | 0 | 0 | 0 | 0 |
| IV-8 | 3.2 | 4 | 1 | 1 | 0 | 5 | 0 | 0 | 0 | 0 | 0 | 0 | 0 | | 0 | 0 | 0 | 0 | 0 |
| V-1 | 6.3 | 5 | 0 | 4 | 3 | 5 | 3 | | 5 | 4 | 5 | | 5 | | 0 | 0 | 0 | 3 | 4 |
| V-2 | 6.3 | 4 | 1 | 3 | 0 | 5 | 0 | | 5 | | 5 | | 5 | | 0 | 0 | 0 | 0 | 4 |

**TABLE 17**

| Compound No. | dose (g/a) | A | D | E | F | G | H | I | J | K | L | M | N | O | b | c | d | e | f |
|---|---|---|---|---|---|---|---|---|---|---|---|---|---|---|---|---|---|---|---|
| I-1 | 6.3 | 5 | 5 | 5 | 3 | 5 | 5 | 5 | 4 | 3 | 5 | 5 | 4 | 5 | 4 | 4 | 5 | 4 | 5 |
| I-2 | 6.3 | 5 | 5 | 5 | 3 | 5 | 4 | 5 | 3 | 3 | 4 | 4 | 4 | 5 | 3 | 3 | 5 | 2 | 5 |
| I-3 | 6.3 | 5 | 5 | 4 | 0 | 5 | 3 | 5 | 4 | 2 | 4 | 0 | 4 | 4 | 4 | 4 | 5 | 0 | 5 |
| I-4 | 6.3 | 5 | 5 | 4 | 0 | 5 | 2 | 5 | 4 | 3 | 4 | 3 | 4 | 4 | 5 | 4 | 4 | 1 | 4 |
| I-5 | 6.3 | 5 | 5 | 4 | 2 | 5 | 4 | 4 | 4 | 3 | 3 | 4 | 4 | 3 | 5 | 3 | 4 | 0 | 4 |
| I-7 | 6.3 | 5 | 5 | 5 | | 5 | 5 | 5 | 4 | 1 | 4 | 4 | 5 | 5 | 1 | 3 | 4 | 0 | 5 |
| I-8 | 3.2 | 5 | 4 | 4 | 1 | 5 | 4 | 5 | 5 | 4 | 2 | 5 | 3 | 4 | 4 | 4 | 5 | 3 | 5 |
| I-9 | 3.2 | 5 | 5 | 3 | 0 | 5 | 3 | 5 | 4 | 2 | 0 | 0 | 3 | 4 | 4 | 3 | 5 | 2 | 3 |
| I-13 | 3.2 | 5 | 5 | 4 | 0 | 5 | 5 | 5 | 4 | 3 | 3 | 3 | 4 | 4 | 0 | 3 | 4 | 0 | 4 |
| I-14 | 3.2 | 5 | 4 | 4 | 0 | 5 | 5 | 5 | 4 | 4 | 5 | 4 | 4 | 4 | 0 | 3 | 4 | 0 | 4 |
| I-15 | 3.2 | 5 | 4 | 4 | 1 | 5 | 4 | 5 | 4 | 3 | 3 | 4 | 4 | 4 | 3 | 3 | 4 | 2 | 2 |
| I-16 | 3.2 | 5 | 4 | 4 | 3 | 5 | 4 | 5 | 5 | 2 | 2 | 3 | 5 | 3 | 0 | 2 | 4 | 0 | 0 |
| I-17 | 6.3 | 4 | 5 | 3 | 1 | 5 | 3 | 4 | 4 | 4 | 5 | 3 | 4 | 5 | 1 | 4 | 3 | 0 | 5 |
| I-18 | 6.3 | 5 | 4 | 4 | 3 | 5 | 4 | 4 | 3 | 4 | 4 | 3 | 4 | 4 | 1 | 4 | 3 | 2 | 4 |
| I-19 | 6.3 | 4 | 4 | 4 | 0 | 5 | 3 | 3 | 4 | 4 | 4 | 3 | 4 | 4 | 0 | 3 | 3 | 0 | 4 |
| II-1 | 6.3 | 5 | 5 | 5 | 1 | 5 | 4 | 5 | 4 | 0 | 4 | 0 | 4 | 4 | 5 | 3 | 3 | 3 | 2 |
| II-2 | 6.3 | 5 | 5 | 5 | 0 | 5 | 4 | 5 | 4 | 0 | 4 | 3 | 3 | 4 | 5 | 3 | 3 | 0 | 2 |
| II-3 | 6.3 | 5 | 5 | 5 | 0 | 5 | 3 | 5 | 2 | 0 | 4 | 0 | 3 | 3 | 5 | 3 | 3 | 0 | 1 |
| II-4 | 6.3 | 5 | 5 | 5 | 0 | 5 | 3 | | 5 | 0 | 3 | | 3 | 4 | 5 | 4 | 4 | 3 | 3 |
| II-5 | 6.3 | 5 | 5 | 5 | 0 | 5 | 3 | | 5 | 0 | 3 | | 3 | 0 | 5 | 4 | 5 | 0 | 0 |
| II-6 | 6.3 | 5 | 4 | 4 | 0 | 5 | 3 | | 5 | 0 | 1 | | 1 | 2 | 4 | 3 | 4 | 0 | 1 |
| II-7 | 6.3 | 5 | 5 | 5 | 0 | 5 | 3 | | 5 | 0 | 5 | | 2 | 3 | 5 | 3 | 4 | 0 | 0 |
| II-8 | 6.3 | 5 | 5 | 4 | 0 | 5 | 0 | | 4 | 0 | 5 | | 1 | 1 | 5 | 4 | 3 | 0 | 1 |
| II-9 | 3.2 | 5 | 0 | 4 | 0 | 4 | 1 | 1 | 3 | 0 | 0 | 5 | 3 | 4 | 0 | 2 | 0 | 0 | 0 |
| II-10 | 3.2 | 4 | 0 | 3 | 0 | 4 | 1 | 4 | 3 | 0 | 3 | 4 | 4 | 3 | 0 | 2 | 0 | 0 | 3 |
| II-11 | 3.2 | 4 | 2 | 2 | 0 | 3 | 1 | 4 | 4 | 0 | 2 | 3 | 4 | 4 | 1 | 3 | 0 | 0 | 3 |
| II-12 | 3.2 | 5 | 2 | 3 | 0 | 4 | 0 | 3 | 4 | 0 | 0 | 1 | 3 | 4 | 0 | 2 | 0 | 0 | 3 |
| II-13 | 3.2 | 2 | 2 | 0 | 0 | 0 | 0 | 0 | 4 | 0 | 0 | 1 | 3 | 4 | 0 | 2 | 0 | 0 | 0 |
| II-14 | 3.2 | 5 | 4 | 4 | 0 | 5 | 3 | 4 | 3 | 0 | 4 | | 3 | 3 | 4 | 1 | 2 | 0 | 4 |
| II-15 | 3.2 | 4 | 4 | 3 | 0 | 5 | 1 | 5 | 3 | 0 | 4 | 3 | 4 | 4 | 0 | 0 | 0 | 0 | 4 |
| II-16 | 3.2 | 0 | 0 | 0 | 0 | 5 | 0 | 0 | 2 | 0 | 3 | 0 | 2 | 0 | 0 | 0 | 0 | 0 | 0 |
| II-17 | 6.3 | 5 | 5 | 5 | 0 | 5 | 4 | | 5 | 1 | 4 | | 4 | 5 | 5 | 4 | 5 | 1 | 3 |
| II-18 | 3.2 | 4 | 0 | 0 | 0 | 0 | 0 | 0 | 3 | 0 | 0 | 3 | 4 | 5 | 0 | 2 | 0 | 0 | 1 |
| II-19 | 6.3 | 5 | 4 | 4 | 0 | 5 | 4 | | 5 | | 0 | | 4 | 5 | 5 | 4 | 5 | 0 | 2 |
| II-20 | 6.3 | 5 | 5 | 5 | 0 | 5 | 4 | | 5 | 0 | 0 | | 3 | 4 | 5 | 4 | 4 | 0 | 3 |
| II-21 | 6.3 | 5 | 4 | 4 | 0 | 5 | 4 | | 5 | 2 | 4 | | 4 | 4 | 4 | 4 | 5 | 0 | 3 |
| II-22 | 3.2 | 5 | 5 | 5 | 0 | 5 | 4 | 5 | 4 | 0 | 3 | 3 | 3 | 5 | 3 | 3 | 1 | 0 | 4 |
| II-23 | 6.3 | 5 | 5 | 4 | 0 | 5 | 4 | | 5 | | 4 | | 4 | 5 | 4 | 3 | 5 | 0 | 0 |
| II-24 | 3.2 | 5 | 4 | 4 | 0 | 5 | 4 | 4 | 3 | 0 | 0 | 3 | 4 | 5 | 4 | 3 | 4 | 1 | 3 |
| II-25 | 3.2 | 5 | 5 | 5 | 0 | 5 | 0 | 4 | 3 | 0 | 1 | 1 | 4 | 5 | 4 | 3 | 3 | 0 | 4 |
| II-26 | 6.3 | 4 | 4 | 4 | 0 | 5 | 3 | | 5 | 0 | 0 | | 3 | 4 | 5 | 3 | 3 | 0 | 2 |
| II-27 | 3.2 | 5 | 4 | 3 | 0 | 3 | 3 | 5 | 4 | 0 | 3 | 3 | 3 | 4 | 4 | 3 | 0 | 0 | 3 |
| II-28 | 3.2 | 5 | 4 | 4 | | 5 | 3 | 4 | 3 | 0 | 1 | 4 | 4 | 5 | 4 | 2 | 0 | 0 | 3 |
| II-32 | 3.2 | 5 | 4 | 4 | 2 | 5 | 4 | 4 | 3 | 1 | 2 | 2 | 3 | 4 | 5 | 4 | 3 | 0 | 2 |
| II-33 | 3.2 | 4 | 4 | 4 | 0 | 5 | 4 | 3 | 3 | 1 | 0 | 3 | 3 | 4 | 4 | 3 | 3 | 0 | 0 |
| II-34 | 3.2 | 5 | 4 | 3 | 0 | 5 | 4 | 3 | 4 | 2 | 3 | 4 | 4 | 4 | 4 | 3 | 1 | 0 | 1 |
| II-35 | 3.2 | 5 | 5 | 3 | 0 | 5 | 4 | 0 | 3 | 1 | 1 | 3 | 3 | 4 | 4 | 3 | 1 | 0 | 1 |
| II-36 | 3.2 | 5 | 4 | 3 | 0 | 4 | 2 | 2 | 3 | 0 | 1 | 2 | 3 | 3 | 4 | 3 | 3 | 0 | 2 |
| II-37 | 3.2 | 5 | 4 | 3 | 1 | 5 | 3 | 3 | 4 | 2 | 2 | 3 | 4 | 4 | 3 | 3 | 3 | 0 | 3 |
| II-39 | 3.2 | 5 | 4 | 5 | | 5 | 3 | 5 | 5 | 4 | 3 | 3 | 4 | 4 | 4 | 4 | 3 | 0 | 3 |
| II-40 | 3.2 | 5 | 3 | 4 | | 5 | 5 | 5 | 4 | 0 | 4 | 0 | 4 | 3 | 4 | 4 | 4 | 2 | 0 |
| II-41 | 3.2 | 5 | 1 | 4 | | 5 | 4 | 5 | 3 | 0 | 2 | 0 | 3 | 4 | 3 | 4 | 4 | 0 | 0 |
| II-42 | 3.2 | 5 | 3 | 5 | | 5 | 3 | 5 | 3 | 0 | 3 | 0 | 4 | 3 | 3 | 3 | 2 | 0 | 0 |
| II-43 | 3.2 | 5 | 1 | 4 | | 5 | 4 | 5 | 3 | 0 | 2 | 0 | 3 | 4 | 3 | 4 | 4 | 0 | 0 |
| II-44 | 3.2 | 4 | 2 | 3 | | 4 | 4 | 3 | 2 | 0 | 1 | 1 | 4 | | 4 | 3 | 3 | 0 | 0 |
| II-45 | 3.2 | 4 | 3 | 2 | | 4 | 4 | 2 | 3 | 0 | 3 | 3 | 3 | | 4 | 4 | 4 | 0 | 2 |
| II-46 | 3.2 | 4 | 4 | 4 | | 2 | 0 | 1 | 3 | 0 | 2 | 2 | 4 | 4 | 4 | 2 | 0 | 0 | 0 |
| II-47 | 3.2 | 4 | 1 | 2 | | 2 | 0 | 4 | 2 | 0 | 0 | 2 | 3 | 4 | 3 | 1 | 0 | 0 | 0 |
| II-48 | 3.2 | 4 | 1 | 2 | | 2 | 0 | 4 | 2 | 0 | 0 | 2 | 3 | 4 | 3 | 1 | 0 | 0 | 0 |
| II-49 | 3.2 | 4 | 0 | 3 | | 3 | 0 | 0 | 3 | 0 | 3 | 3 | 3 | 4 | 2 | 3 | 0 | 0 | 0 |
| II-50 | 3.2 | 3 | 0 | 0 | | 1 | 0 | 0 | 3 | 0 | 2 | 0 | 3 | 4 | 3 | 1 | 0 | 0 | 0 |
| II-51 | 3.2 | 5 | 4 | 4 | | 5 | 3 | 4 | 3 | 0 | 3 | 5 | 5 | 4 | 5 | 4 | 2 | 3 | 3 |
| II-52 | 3.2 | 5 | 4 | 5 | | 5 | 4 | 3 | 3 | 0 | 4 | 1 | 4 | 5 | 5 | 4 | 2 | 3 | 3 |
| II-53 | 3.2 | 4 | 3 | 3 | | 5 | 4 | 4 | 3 | 0 | 2 | 4 | 5 | 5 | 2 | 4 | 0 | 0 | 3 |
| II-54 | 3.2 | 5 | 4 | 5 | | 5 | 5 | 5 | 4 | 1 | 2 | 2 | 4 | 4 | 3 | 1 | 5 | 0 | 3 |
| II-55 | 3.2 | 5 | 4 | 5 | | 5 | 5 | 5 | 4 | 0 | 4 | 3 | 4 | 4 | 0 | 0 | 2 | 0 | 4 |
| II-56 | 3.2 | 5 | 0 | 4 | | 5 | 3 | 5 | 4 | 2 | 3 | 0 | 4 | 3 | 0 | 1 | 0 | 0 | 3 |
| II-57 | 3.2 | 4 | 2 | 2 | | 3 | 5 | 3 | 3 | 0 | 3 | 3 | 4 | | 3 | 4 | 3 | 0 | 0 |
| II-58 | 3.2 | 3 | 0 | 0 | | 3 | 4 | 0 | 3 | 0 | 1 | 3 | 4 | | 0 | 4 | 3 | 1 | 3 |
| II-59 | 3.2 | 5 | 3 | 1 | | 5 | 1 | 2 | 4 | 0 | 1 | 1 | 4 | 5 | 3 | 1 | 1 | 0 | 2 |
| II-60 | 3.2 | 4 | 3 | 3 | | 5 | 3 | 3 | 3 | 0 | 2 | 2 | 4 | 4 | 0 | 1 | 0 | 0 | 4 |
| II-61 | 3.2 | 5 | 4 | 5 | | 5 | 4 | 5 | 3 | 0 | 3 | 4 | 4 | 3 | 4 | 3 | 3 | 3 | 1 |
| II-62 | 3.2 | 5 | 5 | 5 | | 5 | 4 | 5 | 4 | 3 | 4 | 4 | 4 | 4 | 4 | 4 | 3 | 2 | 3 |
| II-63 | 3.2 | 5 | 4 | 4 | | 5 | 4 | 5 | 4 | 2 | 3 | 4 | 4 | 3 | 4 | 4 | 3 | 2 | 3 |
| II-64 | 3.2 | 4 | 4 | 4 | | 5 | 3 | 1 | 3 | 0 | 2 | 3 | 4 | | 3 | 3 | 1 | 0 | 0 |
| II-65 | 3.2 | 3 | 3 | 4 | | 5 | 3 | 3 | 3 | 0 | 2 | 3 | 5 | 0 | 3 | 3 | 1 | 0 | 3 |
| II-66 | 3.2 | 5 | 3 | 4 | | 5 | 3 | 3 | 3 | 0 | 3 | 4 | 4 | 0 | 0 | 3 | 1 | 0 | 1 |
| II-67 | 3.2 | 5 | 5 | 5 | | 5 | 2 | 5 | 3 | 0 | 4 | 3 | 4 | 3 | 3 | 4 | 4 | 3 | 4 |
| II-68 | 3.2 | 5 | 4 | 5 | | 3 | 1 | 4 | 3 | 2 | 4 | 2 | 4 | 3 | 0 | 4 | 3 | 3 | 3 |
| II-70 | 3.2 | 5 | 5 | 5 | | 5 | 4 | 5 | 5 | 4 | 5 | 3 | 4 | 4 | 0 | 4 | 4 | 4 | 4 |
| II-71 | 3.2 | 5 | 4 | 4 | | 5 | 1 | 5 | 3 | 0 | 0 | 0 | 4 | | 4 | 2 | 4 | 0 | 1 |
| II-72 | 3.2 | 4 | 4 | 4 | 1 | 5 | 5 | 5 | 3 | 2 | 1 | 3 | 3 | | 4 | 3 | 4 | 2 | 3 |
| II-73 | 3.2 | 5 | 4 | 4 | | 5 | 5 | 5 | 2 | 0 | 0 | 4 | 2 | | 4 | 2 | 3 | 0 | 4 |
| II-74 | 3.2 | 5 | 5 | 3 | | 5 | 5 | 5 | 3 | 0 | 3 | 4 | 4 | | 4 | 3 | 3 | 2 | 4 |
| II-75 | 3.2 | 4 | 1 | 1 | 0 | 2 | 3 | 3 | 0 | 3 | 4 | 1 | 3 | 5 | 0 | 1 | 0 | 0 | 3 |
| II-77 | 3.2 | 4 | 1 | 1 | | 5 | 0 | 3 | 3 | 3 | 4 | 3 | 3 | | 1 | 3 | 0 | 0 | 4 |
| II-78 | 3.2 | 4 | 4 | 4 | 0 | 5 | 5 | 5 | 4 | 4 | 4 | 4 | 4 | 5 | 3 | 3 | 3 | 0 | 4 |
| II-79 | 3.2 | 5 | 4 | 4 | 0 | 5 | 3 | 4 | 4 | 1 | 3 | 4 | 4 | 4 | 1 | 2 | 3 | 0 | 3 |
| III-2 | 3.2 | 4 | 0 | 0 | | 5 | 3 | 4 | 4 | 3 | 3 | 4 | 3 | 3 | 0 | 0 | 0 | 0 | 3 |
| III-3 | 3.2 | 5 | 4 | 4 | | 5 | 0 | 2 | 4 | 0 | 0 | 2 | 4 | 4 | 1 | 0 | 3 | 0 | 2 |
| III-4 | 3.2 | 5 | 3 | 4 | | 5 | 1 | 3 | 4 | 0 | 1 | 3 | 5 | 3 | 0 | 0 | 3 | 0 | 1 |
| III-5 | 3.2 | 5 | 0 | 3 | | 5 | 1 | 3 | 4 | 0 | 3 | 0 | 3 | 3 | 0 | 0 | 0 | 0 | 3 |
| III-6 | 3.2 | 5 | 3 | 5 | | 5 | 2 | 3 | 4 | 4 | 3 | 5 | 4 | 3 | 0 | 0 | 0 | 1 | 3 |
| III-7 | 3.2 | 5 | 3 | 3 | | 5 | 0 | 0 | 4 | 3 | 4 | 0 | 3 | 2 | 0 | 0 | 0 | 0 | 0 |
| III-9 | 3.2 | 0 | 0 | 0 | | 5 | 0 | 3 | 0 | 0 | 2 | 2 | 3 | 3 | 0 | 0 | 0 | 0 | 0 |
| III-10 | 3.2 | 5 | 4 | 1 | | 5 | 4 | 3 | 5 | 4 | 4 | 5 | 1 | 5 | 0 | 1 | 0 | 0 | 3 |
| III-11 | 3.2 | 5 | 0 | 4 | | 5 | 3 | 4 | 4 | 2 | 4 | 4 | 5 | 5 | 0 | 3 | 0 | 0 | 3 |
| III-12 | 3.2 | 5 | 5 | 3 | 0 | 5 | 3 | 5 | 4 | | 0 | 0 | | 4 | 0 | | 4 | 0 | |
| III-13 | 3.2 | 5 | 5 | 5 | 0 | 5 | 4 | 5 | 4 | 0 | 0 | 0 | 4 | 4 | 4 | 4 | 4 | 0 | 0 |
| III-14 | 3.2 | 5 | 5 | 5 | 0 | 5 | 4 | 5 | 4 | 3 | 0 | 0 | 4 | 5 | 3 | 4 | 4 | 0 | 1 |
| III-15 | 3.2 | 5 | 5 | 4 | 0 | 5 | 3 | 4 | 4 | 2 | 0 | 0 | 3 | 3 | 1 | 4 | 4 | 0 | 0 |
| IV-1 | 3.2 | 4 | 4 | 3 | 0 | 5 | 3 | 5 | 1 | 0 | 0 | 0 | 1 | 3 | 0 | 1 | 2 | 0 | 1 |
| IV-2 | 3.2 | 4 | 5 | 3 | 0 | 5 | 0 | 3 | 1 | 0 | 0 | 0 | 0 | 0 | 0 | 2 | 3 | 0 | 1 |
| IV-3 | 3.2 | 4 | 4 | 3 | 0 | 5 | 1 | 3 | 1 | 0 | 0 | 0 | 5 | 0 | 0 | 1 | 2 | 0 | 4 |
| IV-4 | 3.2 | 4 | 3 | 3 | 0 | 4 | 1 | 3 | 0 | 0 | 0 | 0 | 3 | 0 | 0 | 0 | 0 | 0 | 3 |
| IV-5 | 3.2 | 5 | 4 | 4 | 0 | 5 | 3 | 5 | 3 | 0 | 3 | 0 | 4 | 3 | 0 | 2 | 1 | 0 | 4 |
| IV-6 | 3.2 | 4 | 4 | 4 | 0 | 5 | 2 | 5 | 3 | 0 | 3 | 0 | 4 | 4 | 1 | 2 | 3 | 0 | 4 |
| IV-7 | 3.2 | 4 | 4 | 4 | 0 | 5 | 3 | 4 | 3 | 0 | 4 | 0 | 4 | 3 | 0 | 1 | 3 | 2 | 3 |
| IV-8 | 3.2 | 5 | 4 | 4 | 0 | 5 | 0 | 3 | 3 | 0 | 0 | 0 | 3 | 3 | 0 | 2 | 0 | 0 | 4 |
| V-1 | 6.3 | 5 | 4 | 5 | 0 | 5 | 3 | | 5 | 1 | 4 | | 4 | 4 | 1 | 3 | 3 | 0 | 4 |
| V-2 | 6.3 | 5 | 2 | 4 | 0 | 5 | 0 | | 5 | | 5 | | 4 | 3 | 1 | 3 | 3 | 0 | 3 |

Although the compounds of the present invention are novel, the following comparative compound a is known to have an analogous structure.

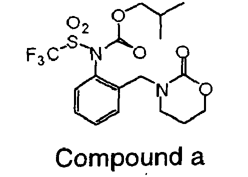

Although Comparative Compound a is disclosed in Patent Document 6, the present inventors discovered that the compounds of the present invention are clearly superior to the comparative compound in herbicidal effect. To demonstrate this, in the following Comparative Examples, their herbicidal effects against barnyardgrass, a major paddy field weed, are compared.

### [COMPARATIVE EXAMPLE 1] Test on the herbicidal effects in preemergence treatment on barnyardgrass under submerged conditions

Alluvial soil was put in 1/30000-are styrol cups and admixed with water so that it was submerged at a depth of 4 cm. In each cup, seeds of barnyardgrass were sown, and on the same day as the seeding, emulsifiable concentrates containing compounds of the present invention formulated in accordance with Formulation Example 2 were applied to the water surfaces at predetermined doses after diluted with water. The cups were placed in a greenhouse at 25 to 30°C to grow the plants. Three weeks after the application, the herbicidal effects against barnyardgrass were determined on the following evaluation scale. The results are shown in Table 18.

The symbol A in the table means barnyardgrass.

**TABLE 18**

| Compound No. | Dose (g/a) | A |
|---|---|---|
| a | 0.16 | 0 |
| a | 0.64 | 5 |
| a | 2.52 | 5 |
| II-3 | 0.16 | 5 |
| II-3 | 0.64 | 5 |
| II-3 | 2.52 | 5 |

### [COMPARATIVE EXAMPLE 2] Test on the herbicidal effects on barnyardgrass during the vegetation period under submerged conditions

Alluvial soil was put in 1/30000-are styrol cups and admixed with water so that it was submerged at a depth of 4 cm. In each cup, seeds of barnyardgrass were sown, and the cups were placed in a greenhouse at 25 to 30°C to grow the plants. When the barnyardgrass reached the 2- or 2.5-leaf stage, emulsifiable concentrates containing compounds of the present invention and the comparative compounds formulated in accordance with Formulation Example 2 were applied to the water surfaces at predetermined doses after diluted with water. Three weeks after the application, the herbicidal effects were determined on the same evaluation scale as in Test Example 1. The results are shown in Table 19.

The symbol A in the table is the same as defined above.

**TABLE 19**

| Compound No. | Dose (g/a) | A |
|---|---|---|
| a | 0.16 | 0 |
| a | 0.64 | 0 |
| a | 2.52 | 5 |
| II-3 | 0.16 | 0 |
| II-3 | 0.64 | 3 |
| II-3 | 2.52 | 5 |

### INDUSTRIAL APPLICABILITY

The haloalkylsulfonanilide compounds of the present invention are useful as selective herbicides for rice, corn, wheat, sugar beet and soybean.

The entire disclosures of Japanese Patent Application No. 2009-098429 filed on April 14, 2009, Japanese Patent Application No. 2010-046451 filed on March 3, 2010 and Japanese Patent Application No. 2010-086422 filed on April 2, 2010, including specifications, claims and summaries are incorporated herein by reference in their entireties.

## Claims

1. A haloalkylsulfonanilide derivative represented by the formula (1) or an agrochemically acceptable salt thereof:
the formula (1): 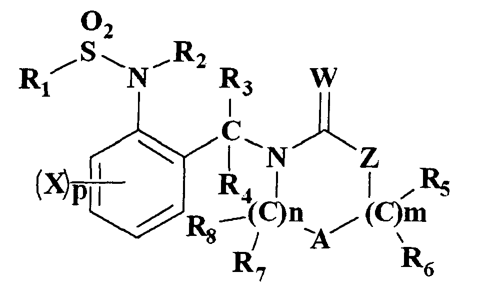
wherein Z is an oxygen atom, a sulfur atom, -C(R₉)(R₁₀)- or -N(R₁₁)-,
A is an oxygen atom, a sulfur atom or -N(R₁₂)-,
W is an oxygen atom or a sulfur atom,
m is an integer of from 0 to 3,
n is an integer of from 0 to 3,
m+n is from 1 to 3,
p is an integer of from 0 to 4,
R₁ is halo C₁-Cₛ alkyl,
R₂ is a hydrogen atom, C₁-C₆ alkyl, halo C₁-C₆ alkyl, C₂-C₆ alkenyl, halo C₂-C₆ alkenyl, C₂-C₆ alkynyl, halo C₂-C₆ alkynyl, C₁-C₆ alkoxy C₁-C₆ alkyl, halo C₁-C₆ alkoxy C₁-C₆ alkyl, C₁-C₆ alkoxy C₁-C₆ alkoxy C₁-C₆ alkyl, tri C₁-C₆ alkylsilyl C₁-C₆ alkoxy C₁-C₆ alkyl, phenyl C₁-C₆ alkyl, phenyl C₁-C₆ alkyl substituted with at least one Y, C₁-C₁₂ alkylcarbonyl, halo C₁-C₁₂ alkylcarbonyl, cyclo C₃-C₆ alkylcarbonyl, cyclo C₃-C₆ alkyl C₁-C₆ alkylcarbonyl, C₂-C₆ alkenylcarbonyl, phenylcarbonyl, phenylcarbonyl substituted with at least one Y, heterocyclic carbonyl, heterocyclic carbonyl substituted with at least one Y, C₁-C₁₂ alkoxycarbonyl, halo C₁-C₁₂ alkoxycarbonyl, C₁-C₆ alkoxy C₁-C₆ alkoxycarbonyl, C₁-C₆ alkylthio C₁-C₆ alkoxycarbonyl, C₁-C₆ alkylsulfinyl C₁-C₆ alkoxycarbonyl, C₁-C₆ alkylsulfonyl C₁-C₆ alkoxycarbonyl, C₂-C₆ alkenyloxycarbonyl, halo C₂-C₆ alkenyloxycarbonyl, C₂-C₆ alkynyloxycarbonyl, phenoxycarbonyl, phenoxycarbonyl substituted with at least one Y, phenyl C₁-C₆ alkoxycarbonyl, phenyl C₁-C₆ alkoxycarbonyl substituted with at least one Y, phenoxy C₁-C₆ alkylcarbonyl, phenoxy C₁-C₆ alkylcarbonyl substituted with at least one Y, mono(C₁-C₆ alkyl)aminocarbonyl, mono(halo C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, C₁-C₆ alkylthiocarbonyl, halo C₁-C₆ alkylthiocarbonyl, C₁-C₆ alkylsulfonyl, halo C₁-C₆ alkylsulfonyl, phenylsulfonyl, phenylsulfonyl substituted with at least one Y, C₁-C₆ alkylthio C₁-C₆ alkyl, halo C₁-C₆ alkylthio C₁-C₆ alkyl, phenyl C₁-C₆ alkoxy C₁-C₆ alkyl, phenyl C₁-C₆ alkoxy C₁-C₆ alkyl substituted with at least one Y, phenylthio C₁-C₆ alkyl, phenylthio C₁-C₆ alkyl substituted with at least one Y, phenyl C₁-C₆ alkylthio C₁-C₆ alkyl, phenyl C₁-C₆ alkylthio C₁-C₆ alkyl substituted with at least one Y, phenylsulfonyl C₁-C₆ alkyl, phenylsulfonyl C₁-C₆ alkyl substituted with at least one Y, phenyl C₁-C₆ alkylsulfonyl C₁-C₆ alkyl, phenyl C₁-C₆ alkylsulfonyl C₁-C₆ alkyl substituted with at least one Y, C₁-C₆ alkylcarbonyloxy C₁-C₆ alkyl, phenylcarbonyloxy C₁-C₆ alkyl, phenylcarbonyloxy C₁-C₆ alkyl substituted with at least one Y, phenylcarbonyl C₁-C₆ alkyl, phenylcarbonyl C₁-C₆ alkyl substituted with at least one Y, C₁-C₆
alkoxycarbonyloxy C₁-C₆ alkyl, phenylcarbonyloxy C₁-C₆ alkoxy C₁-C₆ alkyl, phenylcarbonyloxy C₁-C₆ alkoxy C₁-C₆ alkyl substituted with at least one Y, mono C₁-C₆ alkylaminocarbonyloxy C₁-C₆ alkyl, symmetric or asymmetric di(C₁-C₆ alkyl)aminocarbonyloxy C₁-C₆ alkyl, phenylaminocarbonyloxy C₁-C₆ alkyl, phenylaminocarbonyloxy C₁-C₆ alkyl substituted with at least one Y, C₁-C₆ alkyl(phenyl)aminocarbonyloxy C₁-C₆ alkyl, C₁-C₆ alkyl(phenyl)aminocarbonyloxy C₁-C₆ alkyl substituted with at least one Y, C₁-C₆ alkylcarbonyl C₁-C₆ alkyl, C₁-C₆ alkoxycarbonyl C₁-C₆ alkyl, halo C₁-C₆ alkylthio, symmetric or asymmetric di(C₁-C₆ alkyl)aminothio or cyano,
each of R₃ and R₄ is independently a hydrogen atom, C₁-C₆ alkyl, halo C₁-C₆ alkyl, cyclo C₃-C₆ alkyl, halocyclo C₃-C₆ alkyl, C₁-C₆ alkoxy, halo C₁-C₆ alkoxy, halogen or cyano, or R₃ and R₄ may form a 3- to 7-membered ring together with each other,
each of R₅, R₆, R₇, R₈, R₉ and R₁₀ is independently a hydrogen atom, a halogen, C₁-C₆ alkyl, halo C₁-C₆ alkyl, C₂-C₆ alkenyl, C₂-C₆ alkynyl, cyclo C₃-C₆ alkyl, halocyclo C₃-C₆ alkyl, C₁-C₆ alkoxy C₁-C₆ alkyl, halo C₁-C₆ alkoxy C₁-C₆ alkyl, hydroxy C₁-C₆ alkyl, C₁-C₆ alkylthio C₁-C₆ alkyl, halo C₁-C₆ alkylthio C₁-C₆ alkyl, mono(C₁-C₆ alkyl)amino C₁-C₆ alkyl, symmetric or asymmetric di(C₁-C₆ alkyl)amino C₁-C₆ alkyl, phenyl, phenyl substituted with at least one Y, heterocyclyl, heterocyclyl substituted with at least one Y, phenyl C₁-C₆ alkyl, phenyl C₁-C₆ alkyl substituted with at least one Y, phenoxy C₁-C₆ alkyl, phenoxy C₁-C₆ alkyl substituted with at least one Y, C₁-C₆ alkylcarbonyl, halo C₁-C₆ alkylcarbonyl, phenylcarbonyl, phenylcarbonyl substituted with at least one Y, C₁-C₆ alkoxycarbonyl, halo C₁-C₆ alkoxycarbonyl, carboxyl, mono(C₁-C₆ alkyl)aminocarbonyl, symmetric or asymmetric di(C₁-C₆ alkyl)aminocarbonyl, phenylaminocarbonyl, phenylaminocarbonyl substituted with at least one Y, phenyl C₁-C₆ alkylaminocarbonyl, phenyl C₁-C₆ alkylaminocarbonyl substituted with at least one Y, C₁-C₆ alkoxy, halo C₁-C₆ alkoxy, phenoxy, phenoxy substituted with at least one Y, C₁-C₆ alkylthio, halo C₁-C₆ alkylthio, phenylthio, phenylthio substituted with at least one Y, C₁-C₆ alkylsulfonyl, halo C₁-C₆ alkylsulfonyl, mono(C₁-C₆ alkyl)amino, di(C₁-C₆ alkyl)amino, C₁-C₆ alkylcarbonyloxy, hydroxy, amino, cyano or nitro,
each of R₁₁ and R₁₂ is independently a hydrogen atom, C₁-C₆ alkyl, halo C₁-C₆ alkyl, cyclo C₃-C₆ alkyl, halocyclo C₃-C₆ alkyl, C₂-C₆ alkenyl, halo C₂-C₆ alkenyl, C₂-C₆ alkynyl, C₁-C₆ alkoxy C₁-C₆ alkyl, halo C₁-C₆ alkoxy C₁-C₆ alkyl, hydroxy C₁-C₆ alkyl, cyclo C₃-C₆ alkyl C₁-C₆ alkyl, phenyl C₁-C₆ alkyl, phenyl C₁-C₆ alkyl substituted with at least one Y, phenyl, phenyl substituted with at least one Y, phenoxy C₁-C₆ alkyl, phenoxy C₁-C₆ alkyl substituted with at least one Y, C₁-C₆ alkylcarbonyl C₁-C₆ alkyl, halo C₁-C₆alkylcarbonyl C₁-C₆ alkyl, C₁-C₆ alkoxycarbonyl C₁-C₆ alkyl, halo C₁-C₆ alkoxycarbonyl C₁-C₆ alkyl, C₁-C₆ alkylcarbonyl, halo C₁-C₆ alkylcarbonyl, cyclo C₃-C₆ alkylcarbonyl, cyclo C₃-C₆ alkyl C₁-C₆ alkylcarbonyl, C₂-C₆ alkenylcarbonyl, phenylcarbonyl, phenylcarbonyl substituted with at least one Y, heterocyclic carbonyl, heterocyclic carbonyl substituted with at least one Y, C₁-C₆ alkoxycarbonyl, halo C₁-C₆ alkoxycarbonyl, phenoxycarbonyl, phenoxycarbonyl substituted with at least one Y, aminocarbonyl, mono(C₁-C₆ alkyl)aminocarbonyl, monohalo(C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, C₁-C₆ alkylthiocarbonyl, halo C₁-C₆ alkylthiocarbonyl, C₁-C₆ alkylsulfonyl, halo C₁-C₆ alkylsulfonyl, phenylsulfonyl, phenylsulfonyl substituted with at least one Y, C₁-C₆ alkylthio C₁-C₆ alkyl, halo C₁-C₆ alkylthio C₁-C₆ alkyl, phenylthio C₁-C₆ alkyl, phenylthio C₁-C₆ alkyl substituted with at least one Y, C₁-C₆ alkylsulfinyl C₁-C₆ alkyl, halo C₁-C₆ alkylsulfinyl C₁-C₆ alkyl, phenylsulfinyl C₁-C₆ alkyl, phenylsulfinyl C₁-C₆ alkyl substituted with at least one Y, C₁-C₆ alkylsulfonyl C₁-C₆ alkyl, halo C₁-C₆ alkylsulfonyl C₁-C₆ alkyl, phenylsulfonyl C₁-C₆ alkyl, phenylsulfonyl C₁-C₆ alkyl substituted with at least one Y, cyano, amino or hydroxy, or
R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), an olefin or carbonyl, or
R₅, R₆, R₇, R₈, R₉, R₁₀, R₁₁ or R₁₂ may form, together with R₅, R₆, R₇, R₈, R₉, R₁₀, R₁₁ or R₁₂ on an adjacent carbon or nitrogen, an optionally substituted 3- to 8-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), or
R₅, R₆, R₇, or R₈ may form a bond together with R₅, R₆, R₇, or R₈ on an adjacent carbon,
when m is 3 or 4, R₅ or R₆ may be the same as or different from adjacent R₅ or R₆, when n is 3 or 4, R₇ or R₈ may be the same as or different from adjacent R₇ or R₈,
X is independently a halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl, phenyl substituted with at least one Y, heterocyclyl, heterocyclyl substituted with at least one Y, phenoxy, phenoxy substituted with at least one Y, phenylthio, phenylthio substituted with at least one Y, phenylsulfinyl, phenylsulfinyl substituted with at least one Y, phenylsulfonyl, phenylsulfonyl substituted with at least one Y, C₁-C₆ alkylcarbonyl, halo C₁-C₆ alkylcarbonyl, phenylcarbonyl, phenylcarbonyl substituted with at least one Y, C₁-C₆ alkoxycarbonyl, halo C₁-C₆ alkoxycarbonyl, carboxyl, mono(C₁-C₆ alkyl)aminocarbonyl, monohalo(C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, phenylaminocarbonyl, phenylaminocarbonyl substituted with at least one Y, phenyl C₁-C₆ alkylaminocarbonyl, phenyl C₁-C₆ alkylaminocarbonyl substituted with at least one Y, hydroxy, amino, cyano or nitro,
p is an integer of from 0 to 4,
Y is a halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, phenyl, phenyl having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro}, heterocyclyl, heterocyclyl having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro}, phenoxy, phenoxy having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro}, phenylthio, phenylthio having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro}, phenylsulfinyl, phenylsulfinyl having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro}, phenylsulfonyl, phenylsulfonyl having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro}, C₁-C₆ alkylcarbonyl, halo C₁-C₆ alkylcarbonyl, phenylcarbonyl, phenylcarbonyl having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro}, C₁-C₆ alkoxycarbonyl, carboxyl, mono(C₁-C₆ alkyl)aminocarbonyl, symmetric or asymmetric di(C₁-C₆ alkyl)aminocarbonyl, phenylaminocarbonyl, phenylaminocarbonyl having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro}, phenyl C₁-C₆ alkylaminocarbonyl, phenyl C₁-C₆ alkylaminocarbonyl having at least one substituent selected from the group consisting of {halogen, C₁-C₆ alkyl, C₂-C₆ alkenyl, C₂-C₆ alkynyl, cyclo C₃-C₆ alkyl, halo C₁-C₆ alkyl, halocyclo C₃-C₆ alkyl, C₁-C₆ alkoxy, halo C₁-C₆ alkoxy, C₁-C₆ alkylthio, halo C₁-C₆ alkylthio, C₁-C₆ alkylsulfinyl, halo C₁-C₆ alkylsulfinyl, C₁-C₆ alkylsulfonyl, halo C₁-C₆ alkylsulfonyl, C₁-C₆ alkylcarbonyl, halo C₁-C₆ alkylcarbonyl, C₁-C₆ alkoxycarbonyl, carboxyl, mono(C₁-C₆ alkyl)aminocarbonyl, symmetric or asymmetric di(C₁-C₆ alkyl)aminocarbonyl, hydroxy, amino, cyano and nitro, hydroxy, amino, cyano or nitro}, or
Y may be C₁-C₄ alkylene, halo C₁-C₄ alkylene, C₂-C₄ alkenylene or halo C₂-C₄ alkenylene which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl) and form a 5- or 6-membered ring together with an adjacent carbon or nitrogen atom on a benzene ring or a heterocyclyl, and
the heterocyclyl is thienyl, furyl, pyrrolyl, oxazolyl, isoxazolyl, isoxazolinyl, thiazolyl, isothiazolyl, pyrazolyl, imidazolyl, 1,3,4-oxadiazolyl, 1,2,4-oxadiazolyl, 1,3,4-thiadiazolyl, 1,2,4-thiadiazolyl, 1,2,4-triazolyl, 1,2,3-thiadiazolyl, 1,2,3-triazolyl, 1,2,3,4-tetrazolyl, pyridyl, pyrimidinyl, pyrazinyl, pyridazinyl, 1,3,5-triazinyl, 1,2,4-triazinyl, benzothienyl, benzofuryl, indolyl, benzothiazolyl, benzoimidazolyl, benzoisoxazolyl, benzoisothiazolyl, indazolyl, benzoxazolyl, quinolyl, isoquinolyl, quinoxalinyl, phthalazinyl, cinnolinyl or quinazolinyl,
provided that in a group having two or more substituents, the substituents may be the same or different.

2. The haloalkylsulfonanilide derivative according to Claim 1 or an agrochemically acceptable salt thereof, wherein Z is an oxygen atom or -C(R₉)(R₁₀)-,
R₁ is halo C₁-C₂ alkyl,
R₂ is a hydrogen atom, C₁-C₁₂ alkylcarbonyl, halo C₁-C₁₂ alkylcarbonyl, cyclo C₃-C₆ alkylcarbonyl, cyclo C₃-C₆ alkyl C₁-C₆ alkylcarbonyl, C₂-C₆ alkenylcarbonyl, phenylcarbonyl, phenylcarbonyl substituted with at least one Y, heterocyclic carbonyl, heterocyclic carbonyl substituted with at least one Y, C₁-C₁₂ alkoxycarbonyl, halo C₁-C₁₂ alkoxycarbonyl, C₁-C₆ alkoxy C₁-C₆ alkoxycarbonyl, C₂-C₆ alkenyloxycarbonyl, halo C₂-C₆ alkenyloxycarbonyl, C₂-C₆ alkynyloxycarbonyl, phenoxycarbonyl, phenoxycarbonyl substituted with at least one Y, phenyl C₁-C₆ alkoxycarbonyl, phenyl C₁-C₆ alkoxycarbonyl substituted with at least one Y, phenoxy C₁-C₆ alkylcarbonyl, phenoxy C₁-C₆ alkylcarbonyl substituted with at least one Y, mono(C₁-C₆ alkyl)aminocarbonyl, mono(halo C₁-C₆ alkyl)aminocarbonyl, symmetric or asymmetric di(C₁-C₆ alkyl)aminocarbonyl, symmetric or asymmetric halo di(C₁-C₆ alkyl)aminocarbonyl, C₁-C₆ alkylsulfonyl or halo C₁-C₆ alkylsulfonyl,
R₃ and R₄ are hydrogen atoms,
each of R₅, R₆, R₇, R₈, R₉ and R₁₀ is independently a hydrogen atom, a halogen, C₁-C₆ alkyl, halo C₁-C₆ alkyl, cyclo C₃-C₆ alkyl, halocyclo C₃-C₆ alkyl, C₁-C₆ alkoxy C₁-C₆ alkyl, halo C₁-C₆ alkoxy C₁-C₆ alkyl, hydroxy C₁-C₆ alkyl, C₁-C₆ alkylthio C₁-C₆ alkyl, halo C₁-C₆ alkylthio C₁-C₆ alkyl, phenyl, phenyl substituted with at least one Y, heterocyclyl, heterocyclyl substituted with at least one Y, phenyl C₁-C₆ alkyl, phenyl C₁-C₆ alkyl substituted with at least one Y, C₁-C₆ alkylcarbonyl, halo C₁-C₆ alkylcabonyl, phenylcarbonyl, phenylcarbonyl substituted with at least one Y, C₁-C₆ alkoxycarbonyl, halo C₁-C₆ alkoxycarbonyl, carboxyl, mono(C₁-C₆ alkyl)aminocarbonyl, symmetric or asymmetric di(C₁-C₆ alkyl)aminocarbonyl, C₁-C₆ alkoxy, halo C₁-C₆ alkoxy, phenoxy, phenoxy substituted with at least one Y, C₁-C₆ alkylthio, halo C₁-C₆ alkylthio, phenylthio, phenylthio substituted with at least one Y, C₁-C₆ alkylsulfonyl, halo C₁-C₆ alkylsulfonyl or hydroxy, or
R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), an olefin or carbonyl, or
R₅, R₆, R₇, R₈, R₉, R₁₀, R₁₁ or R₁₂ may form, together with R₅, R₆, R₇, R₈, R₉, R₁₀, R₁₁ or R₁₂ on an adjacent carbon or nitrogen, an optionally substituted 3- to 8-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), or
R₅, R₆, R₇ or R₈ may form a bond together with R₅, R₆, R₇ or R₈ on an adjacent carbon,
X is a halogen, and
p is 0 or 1.

3. The haloalkylsulfonanilide derivative according to Claim 1 or an agrochemically acceptable salt thereof, wherein Z is an oxygen atom or -C(R₉)(R₁₀)-,
A is an oxygen atom or a sulfur atom,
R₁ is trifluoromethyl,
R₂ is a hydrogen atom, C₁-C₅ alkylcarbonyl, halo C₁-C₅ alkylcarbonyl, cyclo C₃-C₆ alkylcarbonyl, C₁-C₅ alkoxycarbonyl, halo C₁-C₅ alkoxycarbonyl or phenylcarbonyl,
R₃ and R₄ are hydrogen atoms,
each of R₅, R₆, R₇, R₈, R₉ and R₁₀ is independently a hydrogen atom, a halogen, C₁-C₃ alkyl, halo C₁-C₃ alkyl, C₁-C₃ alkoxy C₁-C₃ alkyl, phenyl, phenyl substituted with at least one Y, heterocyclyl, or heterocyclyl substituted with at least one Y, or
R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), an olefin or carbonyl, or
R₅, R₆, R₇, or R₈ may form a bond together with R₅, R₆, R₇, or R₈ on an adjacent carbon, and
p is 0.

4. The haloalkylsulfonanilide derivative according to Claim 3 or an agrochemically acceptable salt thereof, wherein Z is -C(R₉)(R₁₀)-, and m and n are 1.

5. The haloalkylsulfonanilide derivative according to Claim 3 or an agrochemically acceptable salt thereof, wherein Z is -C(R₉)(R₁₀)-, m is 0, and n is 2.

6. A compound represented by the formula (2):
the formula (2) 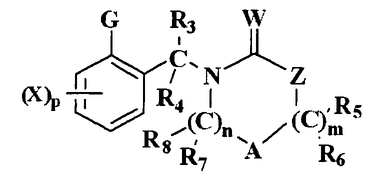
wherein Z, W, R₃, R₄, R₅, R₆, R₇, R₈, m, n, p and X are the same as defined in Claim 1,
and G is amino or nitro.

7. The compound according to Claim 6, wherein Z is an oxygen atom or -C(R₉)(R₁₀)-,
R₃ and R₄ are hydrogen atoms,
each of R₅, R₆, R₇, R₈, R₉ and R₁₀ is independently a hydrogen atom, a halogen, C₁-C₃ alkyl, halo C₁-C₃ alkyl, C₁-C₃ alkoxy C₁-C₃ alkyl, phenyl, phenyl substituted with at least one Y, heterocyclyl or heterocyclyl substituted with at least one Y, or
R₅ and R₆, R₇ and R₈, or R₉ and R₁₀ on the same carbon may form, together with each other, an optionally substituted 3- to 7-membered ring which may contain one or two hetero atoms selected from oxygen atoms, sulfur atoms and nitrogen atoms (hydrogen atoms on the nitrogen atoms may be substituted with C₁-C₆ alkyl, C₂-C₆ alkenyl, C₂-C₆ alkynyl or cyclo C₃-C₆ alkyl), an olefin or carbonyl, or
R₅, R₆, R₇ or R₈ may form a bond together with R₅, R₆, R₇ or R₈ on an adjacent carbon, and
p is 0.

8. A method of use of the compound of the formula (2) as defined in Claim 6 as an intermediated for production of the haloalkylsulfonanilide derivative as defined in Claim 1.

9. An agrochemical containing the haloalkylsulfonanilide derivative as defined in any one of Claims 1 to 5 or an agrochemically acceptable salt thereof, as an active ingredient.

10. A herbicide containing the haloalkylsulfonanilide derivative as defined in any one of Claims 1 to 5 or an agrochemically acceptable salt thereof, as an active ingredient.
